(19) Europäisches Patentamt
European Patent Office
Office européen des brevets

(11) EP 1 377 671 B1

(12) **EUROPEAN PATENT SPECIFICATION**

(45) Date of publication and mention of the grant of the patent:
**06.08.2008 Bulletin 2008/32**

(21) Application number: **02714960.8**

(22) Date of filing: **22.02.2002**

(51) Int Cl.:
***C12N 15/861*** *(2006.01)*

(86) International application number:
**PCT/US2002/005300**

(87) International publication number:
**WO 2002/067861 (06.09.2002 Gazette 2034/03)**

(54) **ONCOLYTIC ADENOVIRAL VECTORS**

ONKOLYTISCHE ADENOVIRALE VEKTOREN

VECTEURS ADENOVIRAUX ONCOLYTIQUES

(84) Designated Contracting States:
**AT BE CH CY DE DK ES FI FR GB GR IE IT LI LU MC NL PT SE TR**

(30) Priority: **23.02.2001 US 270922 P**
**01.06.2001 US 295037 P**
**14.01.2002 US 348670 P**

(43) Date of publication of application:
**07.01.2004 Bulletin 2004/02**

(73) Proprietor: **Cell Genesys Inc.**
**South San Francisco, CA 94080 (US)**

(72) Inventors:
- **ENNIST, David, Leonard**
  **Bethesda, MD 20817 (US)**
- **FORRY-SCHAUDIES, Suzanne**
  **Rockville, MD 20853 (US)**
- **GORZIGLIA, Mario**
  **Doylestown, PA 18091 (US)**
- **HALLEN-BECK, Paul, L.**
  **Gaithersburg, MD 20882 (US)**
- **HAY, Carl, M.**
  **Damascus, MD 20872 (US)**
- **JAKUBCZAK, John, Leonard**
  **Germantown, MD 20874 (US)**
- **KALEKO, Michael**
  **Rockville, MD 20854 (US)**
- **RYAN, Patricia, Clara**
  **Montgomery Village, MD 20886 (US)**
- **STEWART, David, A.**
  **Eldersburg, MD 21784 (US)**
- **XIE, Yuefeng**
  **Germantown, MD 20874 (US)**
- **CONNELLY, Sheila**
  **Ijamsville, MD 21754 (US)**
- **POLICE, Seshidhar Reddy**
  **Gaithersburg, MD 20878 (US)**
- **CLARKE, Lori**
  **Olney, MD 20832 (US)**
- **PHIPPS, Sandrina**
  **Reston, VA 20191 (US)**
- **CHENG, Cheng**
  **Rockville, MD 20852 (US)**

(74) Representative: **Hallybone, Huw George et al**
**Carpmaels & Ransford**
**43-45 Bloomsbury Square**
**London WC1A 2RA (GB)**

(56) References cited:
**WO-A-00/31286** **US-A- 5 998 205**

- **PARR M J ET AL: "TUMOR-SELECTIVE TRANSGENE EXPRESSION IN VIVO MEDIATED BY AN E2F-RESPONSIVE ADENOVIRAL VECTOR" NATURE MEDICINE, NATURE PUBLISHING, CO, US, vol. 3, no. 10, 1 October 1997 (1997-10-01), pages 1145-1149, XP002053131 ISSN: 1078-8956**
- **VASSAUX G ET AL: "INSULATION OF A CONDITIONALLY EXPRESSED TRANSGENE IN AN ADENOVIRAL VECTOR" GENE THERAPY, MACMILLAN PRESS LTD., BASINGSTOKE, GB, vol. 6, no. 6, June 1994 (1994-06), pages 1192-1197, XP008000930 ISSN: 0969-7128**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- STEINWAERDER D S ET AL: "INSULATION FROM VIRAL TRANSCRIPTIONAL REGULATORY ELEMENTS IMPROVES INDUCIBLE TRANSGENE EXPRESSION FROM ADENOVIRUS VECTORS IN VITRO AND IN VIVO" GENE THERAPY, MACMILLAN PRESS LTD., BASINGSTOKE, GB, vol. 7, no. 7, April 2000 (2000-04), pages 556-567, XP008000870 ISSN: 0969-7128

## Description

This application claims the benefit of US Patent Application No. 60/270,922, filed February 23, 2001; US Patent Application 60/295,037, filed June 1, 2001; and US Patent Application No. 60/348,670, filed January 14, 2002, which are herein incorporated by reference.

## FIELD OF THE INVENTION

The present invention generally relates to substances and methods useful for the treatment of neoplastic disease. More specifically, it relates to oncolytic adenoviral vectors and their use in methods of gene therapy.

## BACKGROUND OF THE INVENTION

Adenoviruses that replicate selectively in tumor cells are being developed as anticancer agents ("oncolytic adenoviral vectors"). Such oncolytic vectors amplify the input virus dose due to viral replication in the tumor, leading to spread of the virus throughout the tumor mass. In situ replication of adenoviruses leads to cell lysis. This in situ replication may allow relatively low, nontoxic doses to be highly effective in the selective elimination of tumor cells.

One approach to achieving selectivity is to introduce loss-of-function mutations in viral genes that are essential for growth in non-target cells but not in tumor cells. This strategy is exemplified by the use of Add/1520, which has a deletion in the E1b-55KD gene. In normal cells, the adenoviral E1b-55KD protein is needed to bind to p53 to prevent apoptosis. In p53-deficient tumor cells, E1b-55K binding to p53 is unnecessary. Thus, deletion of E1b-55KD should theoretically restrict vector replication to p53-deficient tumor cells.

Another approach is to use tumor-selective promoters to control the expression of early viral genes required for replication (US patent 5,998,205 (Hallenbeck et al., 1999)). Thus, in this approach the adenoviral vectors will specifically replicate and lyse tumor cells if the gene that is essential for replication is exclusively under the control of a promoter or other transcriptional regulatory element which is tumor-specific.

It is an object of the present invention to provide novel oncolytic adenoviral vectors for the treatment of neoplastic disease, which exhibit a high degree of tumor selectivity, therapeutic efficacy, and safety when administered to a host organism.

## BRIEF DESCRIPTION OF THE DRAWINGS

Figure 1: Cleavage and polyadenylation process for the SV40 early poly(A) site (SEQ ID NO:1).

Figure 2: E1A transcription control region (SEQ ID NO:2).

Figure 3: Sequence of Ar6pAE2fF from left and right ends of viral DNA. Regions of Ar6pAE2fF confirmed by DNA sequencing. Panel A. Regions in first 1802 nucleotides are the inverted terminal repeat (ITR) (nucleotides 1-103), poly-adenylation signal (nucleotides 116-261), a human E2F-1 promoter (nucleotides 283-555), E1A gene (nucleotides 574-1647) and a portion of the E1b gene (nucleotides 1648-1802) are indicated (SEQ ID NO:3). Panel B. Regions in the last 531 nucleotides are the PacI restriction site (nucleotides 33967-33974) (underlined), the packaging signal (nucleotides 34020-34217 and the ITR (34310-34412).

Figure 4: Sequence of Ar6F from left end of viral DNA (SEQ ID NO:4). The first 660 nucleotides at the left end of Ar6F. The ITR (nucleotides 1-103), a multiple cloning site (MCS) (nucleotides 104-134) and a portion of the E1A gene (nucleotides 135-660) are shown.

Figure 5: Sequence of Ar6pAF from left end of viral DNA. The first 660 nucleotides at the left end of Ar6pAF. The ITR (nucleotides 1-103), the SV40 early polyA signal (nucleotides 104-134) and a portion of the E1A gene (nucleotides298-660) are shown.

Figure 6: Schematic diagram of Ar6pAF and Ar6pAE2fF vectors. The backbone adenoviral sequences are derived from the pAr6pAF and pAr6pAE2fF infectious plasmids. The intermediate vector backbone adenoviral sequences are derived from Ad*dl*327, an E3-deleted adenovirus type 5, in which the packaging signal is located immediately upstream of the right ITR. The Ar6pAF vector backbone is deleted in the E1A promoter and the SV-40 poly(A) signal is inserted after the left ITR. The Ar6pAE2fF vector backbone contains, after the SV-40 poly(A) signal sequences, a E2F-1 promoter (bp-212 to +51), a DNA segment of four intact E2F, one NF-kB and four Sp-1 consensus sequences.

Figure 7: Comparison of body weight change after administration of vectors Add/327, AvPAE1A09i, Ar6F, Ar6pAF, Add/312.

Figure 8: Backbones of vectors Add/327, AvE1A09i, AvPAE1A09i, Ar6F, Ar6pAF, Add/312

Figure 9: Mean H460 tumor volume after intratumoral injections of Ar6pAE2fF. Comparison of *in vivo* growth of H460 tumors after five consecutive daily (study day 1-5) intratumoral injections of Ar6pAE2fF at $5x10^8$(n=13), $5x10^9$ (n=13), or $5x10^{10}$ (n=12) particles/dose/day. Control animals were treated IT with HBSS (n=13) or Addl327 (n=12, $5x10^{10}$ particles/dose/day) also for 5 consecutive days. Data is expressed as mean tumor volume + SE. *$p < 0.05$ compared to HBSS treated controls using one-way RM ANOVA with Tukey's test for multiple comparison.

Figure 10: Survival of tumor-bearing animals after intratumoral injections of Ar6pAE2fF. Survival of tumor bearing animals after treatment with Ar6pAE2fF. Animals were observed until study day 32. Numbers of animals in each treatment group were as follows: HBSS, n = 13; Ar6pAE2fF at $5x10^8$, n = 13; $5x10^9$, n = 13; and $5x10^{10}$ particles/dose/day, n=12; and Addl327 at $5x10^{10}$ particles/dose/day, n =12. The survival of animals was analyzed by the Mantel-Haenszel logrank test.

Figure 11: Mean Hep3B tumor volume after intratumoral injections of Ar6pAE2fF. Comparison of in vivo growth of Hep3B tumors after five consecutive daily (study day 1-5) intratumoral injections of Ar6pAE2fF at $5x10^8$(n=11), $5x10^9$(n=11), or $5x10^{10}$ (n=10) particles/dose/day. Control animals were treated IT with HBSS (n=10) or Add1327 (n=11, $5x10^{10}$ particles/dose/day) also for 5 consecutive days. Data is expressed as mean tumor volume + SE. *$p < 0.05$ compared to HBSS treated controls using one-way RM ANOVA with Tukey's test for multiple comparison. **$p<0.05$ compared to Ar6pAE2fF at $5x10^8$ particles/dose/day by t-test.

Figure 12: Survival of tumor-bearing animals after intratumoral injections of Ar6pAE2fF. Survival of tumor bearing animals after treatment with Ar6pAE2fF. Animals were observed until study day 32. Numbers of animals in each treatment group were as follows: HBSS, n = 11; Ar6pAE2fF at $5x10^8$, n = 11; $5x10^9$, n = 11; and $5x10^{10}$ particles/dose/day, n=10; and Addl327 at $5x10^{10}$ particles/dose/day, n =11. The survival of animals was analyzed by the Mantel-Haenszel logrank test.

Figure 13: Schematic diagram of adenovirus right donor plasmid pDR2F.

Figure 14: Schematic diagram of adenovirus right donor plasmid pDR2mGmF. The adenovirus right donor plasmid pDR2mGmF is an 11526bp circular molecule. The mGm-cDNA is at position 8059 to 8520.

Figure 15: Schematic diagram of plasmid pG1mGmSvNa.

Figure 16: Schematic diagram of plasmid pG1NaSvBg.

Figure 17: Sequence of the murine GM-CSF cDNA (SEQ ID NO:7) and protein (SEQ ID NO:8). Sequence of the 7878 to 8826 region of the pDR2mGmF plasmid was confirmed by DNA sequencing. This region includes the murine GM-CSF cDNA insert.

Figure 18: Pathway used to generate pAr6pAE2fmGmF plasmid. The 37763bp Ar6pAE2fmGmF large plasmid was generated through the homologous recombination of the pDR2mGmF/Fspl + Spel fragment (9284bps) and the pAr6pAE2fF/Pacl + Srfl fragment (30695bps) in E coli BJ5183 cells. In this plasmid, the mGM-CSF cDNA was cloned into the Xbal site of the adenoviral E3 region.

Figure 19: MTS assay of oncolytic vectors on different tumor cell lines.

Figure 20: Sequence of Ar6pAE2fhGmF region from 28536 to 29273 of the viral genome including the human GM-CSF cDNA insert (SEQ ID NO:19) and the human GM-CSF protein sequence (SEQ ID NO:20).

Figure 21: Pathway used to generate pAr6pAE2fhGmF. The 37587bp pAr6pAE2fhGmF large plasmid was generated through the homologous recombination of the pDR2hGmF/(Fsp I + Spe I) fragment (9284bps) and the pAr6pAE2fF/(Pac I + Srf I) fragment (30695bps) in E coli BJ5183 cells. In this plasmid, the hGM-CSF cDNA was cloned into the Xba I site of the adenoviral E3 region.

Figure 22: MTS assay of oncolytic vectors on different tumor cell lines.

Figure 23: Efficacy of GM-CSF armed oncolytic vectors in H460 non-small cell lung carcinoma tumor model. Volumes of H460 human xenograft tumors were measured periodically following treatment of pre-established tumors with oncolytic adenoviral vectors. Comparison of in vivo growth of H460 tumors after five intratumoral injections of PBS, or $2x10^{10}$ particles/injection (panel A) or $1x10^{11}$ particles/injection (panel B) of Addl312, Addl327, Ar6pAE2fF or Ar6pAE2fmGmF. Each treatment group is identified by symbols as listed in the graph insets. Vector injections were on study days 10, 12, 14, 17, and 19, as indicated by the arrows along the x-axis. Data are represented by average tumor volume +SEM. Asterisks indicate significant differences compared to Addl312 negative control vector-treated tumors (p<0.05, RM-OW-ANOVA using Tukey's test). At the low dose (panel A), differences were significant for Addl327, Ar6pAE2fF and Ar6pAE2fmGmF compared to Addl312. At the high dose (panel B), differences compared to Addl312 were significant only for the mouse GM-CSF containing Ar6pAE2fmGmF vector.

Figure 24: Efficacy of GM-CSF armed oncolytic vectors in Hep3B hepatocellular carcinoma tumor model. The in vivo growth of Hep3B tumors following intratumoral injections of AddI312, Ar6pAE2fF, Ar6pAE2fhGmF, or Ar6pAE2fmGmF at $2x10^7$ (panel A), $2x10^8$ (panel B), or $2x10^9$ (panel C) particles/injection (n=10/group) was analyzed. Vectors were injected on study days 15, 18, 20, 22, and 25 as indicated by the arrows along the x-axes. Control animals were treated with PBS (n=10). Data represent group averages +SEM. Asterisks indicate p<0.05 compared to dose-matched Addl312-treated tumors. Crosses indicate p<0.05 compared to the Ar6pAE2fF vector and pound symbols indicate p<0.05 for Addl312-treated tumors compared to PBS-treated tumors. Tumors were measured for 47 days following Hep3B tumor cell inoculation.

Figure 25: Schematic diagram of PCR and overlap PCR for □gp19 donor plasmids The mGM-CSF or hGM-CSF cDNA was inserted into the E3 region replacing the E3-gp19 open reading frame (ORF) using two steps of PCR amplification. In the first step, 3 individual PCR amplifications were carried out using 3 pairs of primers and corresponding DNA templates. In the second step, the 3 DNA fragments generated in first step were mixed as the template DNA for the overlap PCR amplification using primer 1 and primer 6 as primers. The overlap PCR product was then digested with BsiWI/NotI and used to replace the BsiWI/NotI region of adenoviral E3 containing the E3-gp19 open reading frame.

Figure 26: Schematic Diagram of ∆gp19 Vectors

Figure 27a: Pathway Used to Generate the pAr6pAE2f(E3+,mGm,Dg19b)F Large Plasmid The 38977bp pAr6pAE2 (E3+,mGm,Dg19b)F large plasmid was generated through the homologous recombination of the pDR2(E3+,mGm, Dg19b)F/(Fsp I + Spe I) fragment (9284bps) and the pAr6pAE2fF/(Pac I + Srf I) fragment (30695bps) in E coli BJ5183 cells. In this plasmid, the mGM-CSF cDNA was swapped into the E3 gp19kD ORF of the adenoviral E3 region.

Figure 27b: Pathway Used to Generate the pAr6pAE2f(E3+,hGm,Dg19b)F Large Plasmid The 38950bp pAr6pAE2 (E3+,hGm,Dg19b)F large plasmid was generated through the homologous recombination of the pDR2(E3+,hGm, Dg19b)F/(Fsp I + Spe I) fragment (9284bps) and the pAr6pAE2fF/(Pac I + Srf I) fragment (30695bps) in E coli BJ5183 cells. In this plasmid, the hGM-CSF cDNA was swapped into the E3 gp19kD ORF of the adenoviral E3 region.

Figure 28: MTS Assay of ∆gp19 mGM-CSF Vectors on H460 and Hep3B Tumor Cell Lines. Two human tumor cell lines, H460 (non-small cell lung carcinoma) and Hep3B (hepatocellular carcinoma), were used. For each cell line, two MTS assays have been performed for all vectors. One representative MTS assay from each cell line is presented.

Figure 29: GM-CSF expression mediated by ∆gp19 GM-CSF vectors in infected H460 cells detected by ELISA. The ∆gp19kD vectors were assayed for their ability to mediate GM-CSF transgene expression in the culture media of H460 cells infected with viral vectors.

Figure 30: Anti-tumor activity of oncolytic adenoviruses in the Hep3B xenograft subcutaneous tumor model. Comparison of the in vivo growth of Hep3B tumors following five intratumoral injections of PBS, or $2x10^9$ particles/injection of Ad*dl*312, Ar6pAE2fF, Ar6pAE2fmGmF, or Ar6pAE2f(E3+,mGm,Dg19b)F (n=10 per group). Symbols representing the different treatment groups are shown in the graph inset. Vector injections were on days 11, 13, 15, 17, and 19 as indicated by the arrows along the x-axis. Tumor volumes when vector injections began were 175 $mm^3$ for all groups, except for tumors treated with the Ar6pAE2f(E3+,mGM,Dg19b)F vector, where the initial tumor volumes

were 290 $mm^3$. Asterisks indicate a p value <0.05 compared to Add/312 vector-treated tumors (by repeat-measures, one-way ANOVA).

Figure 31: Anti-tumor activity of oncolytic adenoviruses in the H460 xenograft subcutaneous tumor model. Comparison of the in vivo growth of H460 tumors following five intratumoral injections of controls or oncolytic vectors. Panel A, H460 tumors injected with $2x10^{10}$ particles/injection of Add/312, Ar6pAE2fF, or Ar6pAE2fmGmF, or $1x10^{10}$ particles/injection of Ar6pAE2f(E3+,mGm,Dg19b)F (n=10 per group). Panel B, H460 tumors injected with $1x10^{11}$ particles/injection of Add/312, Ar6pAE2fF, or Ar6pAE2fmGmF, or $5x10^{10}$ particles/injection of Ar6pAE2f(E3+,mGm, Dg19b)F (n=10 per group). Symbols representing the different treatment groups are shown in the graph inset. Vector injections were on days 12, 14, 16, 19, and 21 as indicated by the arrows along the x-axis. Tumor volumes when vector injections began were 160 $mm^3$ for all groups, except for tumors treated with the Ar6pAE2f(E3+mGM,Dg19b) F vector, where the initial tumor volumes were 120 $mm^3$. Asterisks indicate a p value <0.05 compared to Ad*dl*312 vector-treated tumors (by repeat-measures, one-way ANOVA).

Figure 32: Schematic diagram of adenovirus pDr5hGmF and pDr5mGmF right donor plasmids. The adenovirus right donor plasmid pDr5hGmF and pDr5mGmF are circular molecules 12674bp and 12701 bp in size, respectively. The loxP sites were removed from their parental plasmids pDR2(E3+,mGm,Dg19b)F and pDR2(E3+,hGm,Dg19b)F by replacing the NotI/SphI fragment with the NotI/SphI fragment from wild type Ad5.

Figure 33: Pathway used to generate the pAr15pAE2fhGmF plasmid. The 38910 bp pAr15pAE2fhGmF large plasmid was generated through homologous recombination of the pDr5hGmF/(FspI + SpeI) fragment (10432 bps) and the pAr6pAE2fF/(PacI + SrfI) fragment (30695bp) in E coli BJ5183 cells. In this plasmid, the hGM-CSF cDNA was swapped into the E3-gp19 ORF of the adenoviral E3 region and the loxP site was removed from E3 region.

Figure 34: Pathway used to generate the pAr15pAE2fmGmF plasmid. The 38938bp pAr15pAE2fmGmF large plasmid was generated through the homologous recombination of the pDr5mGmF/(FspI + SpeI) fragment (10459bp) and the pAr6pAE2fF/(PacI + SrfI) fragment (30695bp) in E. coli BJ5183 cells. In this plasmid, the mGM-CSF cDNA was swapped into the E3 gp19 ORF of the adenoviral E3 region and the loxP site was removed from E3 region.

Figure 35: MTS assay of Ar15pAE2fhGmF and Ar15pAE2fmGmF vectors on H460 and Hep3B tumor cell lines. MTS assays were performed to evaluate the oncolytic potential of the Ar15pAE2fhGmF and Ar15pAE2fmGmF vectors. Two human tumor cell lines, H460 (non-small cell lung carcinoma) and Hep3B (hepatocellular carcinoma), were used. For each cell line, two MTS assays were performed using all the indicated vectors and one representative MTS assay from each cell line is presented. Ar15pAE2fF was used for comparison of LD50s of "armed" vectors vs "unarmed" vectors. Ar6pAE2fmGmF and Ar6pAE2f(E3+, mGm, Dg19b) were also included. In addition, control viruses Addl327 (replication competent Ad5 positive control virus) and Addl312 (E1 a deficient negative control) were also included in the MTS assays. The Ar15pAE2fhGmF and Ar15pAE2fmGmF vectors retained the oncolytic capacity of the Ar6pAE2fF vector in both cell lines tested.

Figure 36: GM-CSF expression mediated by Ar15pAE2fhGmF and Ar15pAE2fmGmF vectors in infected H460 cells detected by ELISA. The Ar15pAE2fGmF vectors were assayed for their ability to mediate human or mouse GM-CSF transgene expression in the culture media of H460 cells and Hep3B cells infected with viral vectors. The Ar15pAE2fGmF vectors induce the in vitro production of GM-CSF at levels similar to the E3 deleted Ar6pAE2fGmF series.

Figure 37: Schematic diagram of PCR and overlap PCR for ΔE3-14.7 plasmids. The human GM-CSF cDNA was inserted into the E3 region replacing the E3-14.7 ORF using two steps of PCR amplification. In the first step, 3 individual PCR amplifications were carried out using 3 pairs of primers and corresponding DNA templates as summarized in Tables 2 and 3. In the second step, the 3 DNA fragments generated in first step were mixed as the template DNA for the overlap PCR amplification using primers 1 and 6. The overlap PCR product was then digested with XhoI/SphI and used to replace the XhoI/SphI region of plasmid pDR4F containing the E3-14.7 open reading frame.

Figure 38: Schematic Diagram of ΔE3-14.7 Vectors.

Figure 39: Schematic Diagram of Adenovirus Right Donor Plasmid pDr6hGmF. The adenovirus right donor plasmid pDR6hGmF is a circular molecule of 12774 bp. The human GM-CSF (hGm) cDNA was swapped into the position of the E3-14.7 ORF in the pDR4F plasmid using PCR amplification and overlap PCR amplification followed by restriction enzyme digestion (XhoI/SphI) and ligation.

Figure 40: Pathway Used to Generate the pAr16pAE2fhGmF Large Plasmid. The 39011 bp pAr16pAE2fhGmF large plasmid was generated through the homologous recombination of the pDR6hGmF/(Fsp I + Spe I) fragment (10532bps) and the pAr6pAE2fF/(Pac I + Srf I) fragment (30695bps) in E coli BJ5183 cells. In this plasmid, the hGM-CSF cDNA was swapped into the E3-14.7 ORF of the adenoviral E3 region.

Figure 41: MTS Assay of ΔE3-14.7 hGM-CSF Vector on H460 Tumor Cell Line. To evaluate the oncolytic potential of the Ar16pAE2fhGmF vector, MTS assays were performed. Human tumor cell line H460 (non-small cell lung carcinoma) was used. Two MTS assays have been done for all vectors and the assays gave similar LD50 values for each vector. The oncolytic capacity of Ar16pAE2fhGmF is compared to Ar6pAE2fF, Ar6pAE2fhGmF, and Ar6pAE2f(E3+,hGm,Dg19)F. In addition, control viruses Addl327 (replication competent Ad5 positive control virus) and Addl312 (E1A deficient negative control) were also included in the MTS assays. The Ar16pAE2fhGmF vectors retained the oncolytic capacity of the Ar6pAE2fF vector.

Figure 42: GM-CSF Expression Mediated by ΔE3-14.7 hGM-CSF Vector (Ar16pAE2fhGmF) Compared to Ar6pAE2fF, Ar6pAE2fhGmF and Ar6pAE2f(E+,hGm,Dg19)F in Infected H460 Cells 24 Hours Post-Infection. The Ar16pAE2fhGmF vector was assayed for its ability to mediate GM-CSF transgene expression in the culture media of H460 cells infected with viral vectors. H460 cells were plated in 6-well plates using 2 ml/well of culture media at a density of $2.5x10^5$ cells/well. The next day, the media were removed and the cultured cells were transduced in duplicate with viral vectors at 10, 100, and 1000 particles/cell in 5000□l serum-free medium. After two hours of incubation at 37°C in a 5% $CO_2$ incubator, virus was aspirated and 2 ml of fresh complete culture medium was added to each well. At 24 hours post infection, the supernatants were collected for hGM-CSF ELISA.

Figure 43: Spread of adenoviruses in H460 xenograft tumors detected by FACS. At day 1, 4, and 7 after the injection of viruses or PBS, tumors were analyzed for hexon staining using intracellular flow cytometry. The percentage of hexon positive cells from each mouse was displayed in the graph, with the bar as the mean (n=10). The PBS negative control group had fewer mice.
*: p<0.05 between Ar6pAE2fF or Ar6pAE2fE3F and Addl312, ANOVA
□: p<0.05 between Ar6pAE2fF and Ar6pAE2fE3F vectors, ANOVA

Figure 44: Spread of adenoviruses in Hep3B xenograft tumors detected by FACS. On days 1, 4 and 7 after the injection of viruses or PBS, tumors were analyzed for hexon staining using flow cytometry. The percentage of hexon positive cells from each mouse was displayed in the graph, with the bar as the mean (n=10). The PBS negative control group had fewer mice.
*: p<0.05 between Ar6pAE2fhGmF or Ar6pAE2f(E3+,hGm,Dg19)F and Add1312, ANOVA
□: p<0.05 between Ar6pAE2fhGmF and Ar6pAE2f(E3+,hGm,Dg19)F vectors, ANOVA

Figure 45: Flowchart for construction of pArpAE2fFTrtex. The plasmids that were used for the construction of the large plasmid for the oncolytic vector Ar17pAE2fFTrtex are depicted. The specific alterations are noted and described in the text in more detail.

Figure 46: The final right end shuttle plasmid (pDr17TrtexF) and the large plasmid (pAr17pAE2fFTrtex) used to make the oncolytic vector Ar17pAE2fFTrtex are shown here.

Figure 47: Sequence of the right end of Ar17pAE2fFTrtex (SEQ ID NO:17): The right end of the vector was sequenced and is shown here. The viral ITR and packaging signal are at 36305 to 36203. The Trtex promoter is located at 35843 to 35606. Additional regions were also sequenced including the E3 region and the left end of the virus.

Figure 48: Diagram of Ar17pAE2fFTrtex: The diagram of the final vector is depicted schematically in this figure. The known transcription factor binding sites are indicated above each added promoter. Briefly, a E2F-1 promoter is driving the E1a transcription unit and a telomerase reverse transcriptase (Tert) promoter is driving the E4 transcription unit. The E3 region is completely wild type.

Figure 49: Adenoviral E4 expression measured by semi-quantitative RT-PCR. The E4 region is encoded on the opposite strand in the viral genome. Total RNA was isolated from Hep3B cells 24 hours after infection with 10 ppc of Ar17pAE2fFTrtex. Depicted is a schematic diagram of the right end of the Ar17pAE2fFTrtex viral genome with relative positions of primers used in RT-PCR reactions along with the approximate size of the products. PCR 2.f paired with PCR 3.r or PCR 4.r were designed to detect all E4 transcripts. PCR 2.f paired with PCR 5.r was used to detect transcripts that initiated from any cryptic start sites upstream of the E4 region. +1, indicates the approximate position of transcriptional initiation site of the native hTERT promoter.

Figure 50: Sequence of a hTERT promoter and a portion of the E4 region of Ar17pAE2fFTrtex is shown (SEQ ID NO:21). In the Ad genome, the E4 genes are oriented in the reverse direction. A hTERT promoter sequence is indicated by the double underline. The boxed sequence labeled "ExtP1" indicates the antisense oligonucleotide primer used in the primer extension assay to map the transcriptional initiation sites for the E4 region. Nucleotides indicated by the gray boxes are the three transcription initiation sites we identified. The start sites previously identified by Horikawa I, Cable PL, Afshari C, Barrett JC. Cloning and characterization of the promoter region of human telomerase reverse transcriptase gene. Cancer Res. 1999 Feb 15;59(4):826-30 and Takakura M, Kyo S, Kanaya T, Hirano H, Takeda J Yutsudo M, Inoue M. Cloning of human telomerase catalytic subunit (hTERT) gene promoter and identification of proximal core promoter sequences essential for transcriptional activation in immortalized and cancer cells. Cancer Res. 1999 Feb 1;59(3):551-7 in the endogenous hTERT gene are indicated by bold solid underlines.

Figure 51: Tumors were established by injecting $1x10^7$ Hep3B cells subcutaneously into the right flank of 6-8 week old female nude mice (Harlan). Two weeks after implantation, mice with tumors ranging from 91.6 - 218.5 $mm^3$ were selected and randomly distributed into groups (n=17-18). Each mouse was weighed prior to intravenous injection. The control groups received HBSS or Addl312 at 4.5 x $10^{12}$ vp/kg (n=18). Ar17pAE2fFTrtex treatment groups received $1.5\times10^{12}$ (n=18), $3.0\times10^{12}$ (n=17), or $4.5\times10^{12}$ (n= 18) vp/kg. All dose volumes were 10 ml/kg. Groups means + SEM are represented. *, $p < 0.05$ vs. HBSS controls (Dunnett test).

Figure 52: Survival of tumor bearing animals after treatment with HBSS, Addl312 (4.5 x $10^{12}$ vp/kg), or Ar17pAE2fFTrtex (1.5 x $10^{12}$ vp/kg, 3.0 $x10^{12}$ vp/kg, or 4.5 $x10^{12}$ vp/kg). Animals were observed until study day 42, n= 17-18 per group. The survival curves were plotted using GraphPad Prism, and analyzed by the Mantel-Haenszel logrank test ($p<0.004$ for all treatment doses compared to HBSS).

Figure 53: Group mean body weights are shown following a single intravenous injection of the indicated test article. The number of animals evaluated at each scheduled data collection time point was 18-33, except for SD29 when n = 9-22. Vector doses were adjusted on the basis of individual animal body weight on the day of dosing. Lo Dose: 1.5 x $10^{12}$ vp/kg; Mid Dose: 3.0 x $10^{12}$ vp/kg; Hi Dose: 4.5 x $10^{12}$ vp/kg. Group means + SD are represented, with no statistically significant differences between groups.

Figure 54: Comparison of *in vivo* growth of Hep3B tumors after a single iv injection of Ar17pAE2fFTrtex at $3x10^{12}$ (n=16) or $4.5x10^{12}$ (n=16) particles/kg. Control groups were injected with HBSS (n=16) or Addl312 (n=16) at $4.5x10^{12}$ particles/kg. Data is expressed as mean tumor volume + SE. (*$p < 0.05$) For both Ar17pAE2fFTrtex treated groups compared to HBSS treated controls using one-way ANOVA with Student-Newman-Keuls test for multiple comparison.

Figure 55: Survival of tumor bearing animals after treatment with Ar17pAE2fFTrtex. Animals were observed until study day 58. Numbers of animals in each treatment group were as follows: HBSS, n = 16; Ar17pAE2fFTrtex at $3x10^{12}$, n = 16; and $4.5x10^{12}$, n = 16; and Addl312 at $4.5x10^{12}$ particles/kg, n =16. The survival of animals was analyzed by the Mantel-Haenszel logrank test.

Figure 56: Analysis of mean % body weight change from Hep3B tumor bearing animals treated with the oncolytic adenoviral vector Ar17pAE2fFTrtex, Addl312 or HBSS. Body weights were measured once per week. Data is expressed as mean tumor volume + SD.

Figure 57: Tumors were established by injecting $1x10^7$ Hep3B cells subcutaneously into the right flank of 6-8 week old female nude mice (Harlan). Two weeks after implantation, mice with tumors ranging from 90-215 $mm^3$ were selected and randomly distributed into groups (n=12/group). Each mouse was weighed prior to intravenous injection. The control mice received HBSS. Ar17pAE2fFTrtex treatment groups received $3\times10^{11}$ (n=12), $6x10^{11}$ (n=12), $1x10^{12}$ (n=12), or $3x10^{12}$ (n=12) vp/kg. All dose volumes were 10 ml/kg. Groups means (+SEM) are represented. *, $p < 0.05$ vs. HBSS controls (Dunnett's method).

Figure 58: Individual tumor volumes for study days 3 through 22 are presented. All dose volumes were 10 ml/kg. A) The control group treated with HBSS. Treatment groups received Ar17pAE2fFTrtex at B) $3x10^{11}$ vp/kg, C) $6x10^{11}$ vp/kg, D) $1\times10^{12}$ , or E) $3\times10^{12}$ vp/kg. (n=12 / group).

Figure 59: Survival of tumor bearing animals after treatment with HBSS or Ar17pAE2fFTrtex (3 x $10^{11}$ vp/kg, 6 x

$10^{11}$ vp/kg, 1 x $10^{12}$ vp/kg, or 3 x $10^{12}$ vp/kg). Animals were observed until study day 39, n= 12 per group. The survival curves were plotted using GraphPad Prism, and analyzed by the Mantel-Haenszel logrank test.

Figure 60: Percent body weight change from study day 1 are shown following a single intravenous injection of the indicated dose (n=12). Vector doses were adjusted on the basis of individual animal body weight on the day of dosing. A single intravenous injection of Ar17pAE2fFhTrtex at 3x$10^{11}$ (n=12), 6x$10^{11}$ (n=12), 1x$10^{12}$ (n=12), or 3x$10^{12}$ (n=12) viral particles/kg in a final volume of 10 ml/kg was administered on study day 1. *, $p < 0.05$ vs study day 1 percent body weight change, Kruskal-Wallis One-Way ANOVA on Ranks.

Figure 61: Vector DNA copies per cell in tumors and livers collected from mice prior to treatment (n=3) and at indicated times and after intravenous injection of Ar17pAE2fFTrtex at 3.0 x $10^{12}$ vp/kg (n=5). Molecular analysis was done by PCR using primers specific for adenoviral hexon DNA. Results are expressed as hexon copy number per cell.

Figure 62: The mean body weight change as a percent of the SD1 body weight +st dev was followed for a cohort of five mice in each treatment group. Animals were injected with a single intravenous dose of the indicated vectors on SD1. *, $p < 0.05$ vs. HBSS (one-way ANOVA).

Figure 63: Improved isobologram with additivity envelope for Ar17pAE2fFTrtex and Taxol against Hep3B and PC3M. 2AC6 cells. In the table, $EC_{50}$ of virus or chemotherapy single treatment was termed as 1. $EC_{50}$ of virus or chemotherapy agents in the combination were divided by the $EC_{50}$ of single treatments.

Figure 64: Improved isobologram with additivity envelope for Ar17pAE2fFTrtex and Doxorubicin against Hep3B and PC3M.2AC6 cells. In the table, $EC_{50}$ of virus or chemotherapy single treatment was termed as 1. $EC_{50}$ of virus or chemotherapy agents in the combination were divided by the $EC_{50}$ of single treatments.

Figure 65: Improved isobologram with additivity envelope for Ar17pAE2fFTrtex and Epothilone B against Hep 3B cells. In the table, $EC_{50}$ of virus or chemotherapy single treatment was termed as 1. $EC_{50}$ of virus or chemotherapy agents in the combination were divided by the $EC_{50}$ of single treatments.

Figure 66: Tumor growth curve in the doxorubicin combination study. Comparison of in vivo growth of Hep3B tumors after a single i.v. injection of Ar17pAE2fFTrtex at $3\times10^{12}$ particle/kg (n=10) alone or in combination with doxorubicin given i.p. at 10 mg/kg. A group was given doxorubicin alone (n=10) i.p. at 10 mg/kg. A control group was injected with HBSS (n=10). Other details are described in the text. * means $p < 0.001$ by t-test compared to all other groups at study day 20. One mouse was found dead at study day 27 in combination group, so the n=9 to end of study. Data is expressed as mean tumor volume + SEM.

Figure 67: Tumor growth curve in the Doxil® combination study. Comparison of in vivo growth of Hep3B tumors after a single i.v. injection of Ar17pAE2fFTrtex at 1x$10^{12}$ (n=10) vp/kg alone or at 1x$10^{12}$ or 6x$10^{11}$ vp/kg in combination with Doxil® given i.v. at 9 mg/kg. A group was given Doxil® alone (n=10) i.v. at 9 mg/kg. Other details are described in the text. A negative control group was injected with HBSS (n=9). *means $p < 0.01$ by t-test compared to all other groups at study day 21. Data is expressed as mean tumor volume $\pm$ SEM.

Figure 68: Toxicity of Ar17pAE2fFTrtex in primary human hepatocytes (PHH). PHH were transduced with indicated vectors at 1, 10 and 50 ppc. Panel A: Cytotoxicity as measured by LDH release was measured five days after transduction. Means $\pm$ sd from triplicate wells is shown. * $p<0.05$ Ar17pAE2fFTrtex versus Ar13pAE2fF by t-test. Panel B: Cytotoxicity measured seven days after transduction. Means $\pm$ range from 1-3 wells is shown. Statistical comparisons not possible for data in panel B due to low replicate number.

Figure 69: Ad35-based oncolytic vectors. Ar350scE1A and Ar35E2FE1A both contain the E1a region under the control of a tumor-specific promoter, a osteocalcin or a E2F promoter, respectively. Ar35E2F+E1A contains in addition, the E4 region under the control of a tumor-specific promoter.

Figure 70: Effect of Ar350scE1A on a subcutaneous PC3 tumor in nude mice. Groups of 10 animals each were treated with vehicle (HBSS), Ad35v0.5 (an E1a deficient Ad35 based vector), Ar6pAOscE3F (the Ad5-based oncolytic vector containing the osteocalcin promoter driving expression of E1a), Ad35 (wt virus), and Ar350scE1a (the Ad35-based oncolytic vector containing the osteocalcin promoter driving expression of E1a). All vectors were delivered intratumorally (IT), using a single dose of 2 x $10^{11}$ particles/mouse (1 x $10^{13}$ particles/kg).

## SUMMARY OF THE INVENTION

The present invention provides novel and improved oncolytic adenoviral vectors and their uses in methods of gene therapy. In a preferred embodiment, the oncolytic adenoviral vector has an E2F promoter operably linked to the E1a gene. In a particularly preferred embodiment, the oncolytic adenoviral vectors has an E2F promoter operably linked to the E1a gene and the human telomerase reverse transcriptase promoter operably linked to the E4 gene.

Accordingly, in one aspect, the present invention provides a recombinant viral vector comprising an adenoviral nucleic acid backbone, wherein said nucleic acid backbone comprises in sequential order: A left ITR, a termination signal sequence, an E2F responsive promoter which is operably linked to a first gene essential for replication of the recombinant viral vector, an adenoviral packaging signal and a right ITR.

In a second aspect, there may be provided a recombinant viral vector comprising an adenoviral nucleic acid backbone, wherein said nucleic acid backbone comprises in sequential order: A left ITR, a termination signal sequence, an E2F responsive promoter which is operably linked to a first gene essential replication of the recombinant viral vector, a telomerase promoter operably linked to a second gene essential for replication, an adenoviral packaging signal and a right ITR.

In another aspect, the invention provides adenoviral particles comprising these vectors. Preferably, the particles further comprise a targeting ligand included in a capsid protein of the particles.

In another aspect, the adenoviral particles carry at least one therapeutic transgene. Preferably, the particles further comprise a polynucleotide encoding a cytokine such as GM-CSF that can stimulate a systemic immune response against tumor cells.

In another aspect, there is provided a method of selectively killing a neoplastic cell in a cell population which comprises contacting a suitable amount of the recombinant viral vector of the invention with said cell population under conditions where the recombinant viral vector can transduce the cells of said cell population.

In a further aspect a pharmaceutical composition comprising the recombinant viral vector of the invention and a pharmaceutically acceptable carrier is provided.

In yet another aspect a method of treating a host organism having a neoplastic condition is provided, comprising administering a therapeutically effective amount of the composition of the invention to said host organism.

## DETAILED DESCRIPTION OF THE INVENTION

The present invention provides novel viral vectors based on the oncolytic adenoviral vector strategy as described in US patent 5,998,205, issued December 7, 1999 to Hallenbeck et al.. In particular, oncolytic adenoviral vectors are disclosed in which expression of an adenoviral gene, which is essential for replication, is controlled by E2F-responsive promoters which are selectively transactivated in cancer cells. Examples of E2F-responsive promoters are disclosed in PCT publication WO 98/13508, published April 2, 1998.

Without being bound by theory, the inventors believe that the mechanism of action is as follows. The selectivity of E2F-responsive promoters (hereinafter sometimes referred to as E2F promoters) is based on the derepression of the E2F promoter/transactivator in Rb-pathway defective tumor cells. In quiescent cells, E2F binds to the tumor suppressor protein pRB in ternary complexes. In its complexed form, E2F functions to repress transcriptional activity from promoters with E2F binding sites, including the E2F-1 promoter itself (Zwicker J, and Muller R. Cell cycle-regulated transcription in mammalian cells. Prog. Cell Cycle Res 1995; 1:91-99). Thus the E2F-1 promoter is transcriptionally inactive in resting cells. In normal cycling cells, pRB-E2F complexes are dissociated in a regulated fashion, allowing for controlled derepression of E2F and subsequent cell cycling (Dyson, N. The regulation of E2F by pRB-family proteins. Genes and Development 1998: 12:2245-2262).

In the majority of tumor types, the Rb cell cycle regulatory pathway is disrupted, suggesting that Rb-pathway deregulation is obligatory for tumorigenesis (Strauss M. Lukass J and Bartek J. Unrestricted cell cycling and cancer. Nat Med 1995; 12:1245-1246). These mutations can be in Rb itself or in other factors that have an effect on upstream regulators of pRB, such as the cyclin-dependent kinase, p16 (Weinberg, RA. The retinoblastoma protein and cell cycle control. Cell 1995: 81:323-330). One consequence of these mutations is the disruption of E2F-pRB binding and an increase in free E2F in tumor cells. The abundance of free E2F in turn results in high level expression of E2F responsive genes in tumor cells, driving them into S phase. The E2F-1 promoter used here has been shown to up-regulate the expression of marker genes in an adenovirus vector in a rodent tumor model but not normal proliferating cells in vivo (Parr MJ et al. Tumor-selective transgene expression in vivo mediated by an E2F-responsive adenoviral vector. Nature Med 1997; Oct;3(10):1145-1149).

In one aspect the present invention now provides recombinant viral vector comprising an adenoviral nucleic acid backbone, wherein said nucleic acid backbone comprises in sequential order: A left ITR, a termination signal sequence, an E2F responsive promoter which is operably linked to a gene essential for replication of the recombinant viral vector, an adenoviral packaging signal, and a right ITR.

In another aspect, may be provided a recombinant viral vector comprising an adenoviral nucleic acid backbone, wherein said nucleic acid backbone comprises in sequential order: A left ITR, a termination signal sequence, an E2F responsive promoter which is operably linked to a first gene essential for replication of the recombinant viral vector, a telomerase promoter operably linked to a second gene essential for replication, an adenoviral packaging signal, and a right ITR.

The recombinant viral vectors of this invention are useful as therapeutics for cancer therapy. In particular, the vectors of the invention preferentially kill Rb-pathway defective tumor cells as compared to cells which are non-defective in the Rb-pathway. Furthermore, such vectors exhibit a favorable toxicity profile, which is clinically acceptable for the condition to be treated. Without wishing to be limited by theoretical considerations, the specific regulation of viral replication by a E2F promoter, which is preferably shielded from readthrough transcription by the upstream termination signal sequence, avoids toxicity that would occur if it replicated in non-target tissues, allowing for the favorable efficacy / toxicity profile. Preferably, the specificity of the regulation of viral replication by a E2F promoter may be further enhanced in the vectors of the invention because of the positioning of the packaging signal downstream of the E2F-linked gene essential for replication. This positioning provides for the possibility to delete sequences of the adenoviral backbone which are located upstream of the E2F-linked gene and which would encompass the packaging signal in its wild-type position. Such deletions further improve the specificity of regulation of viral replication by a E2F promoter. Thus, the combination and the sequential positioning of the genetic elements employed in the vectors of this invention provide for the vector's therapeutic efficacy, while at the same time synergistically minimizing toxicity and side effects in the patient. The recombinant viral vectors of the invention may further comprise a telomerase promoter operably linked to the E4 gene.

The present invention contemplates the use of all adenoviral serotypes. The adenoviral nucleic acid backbone may be derived from adenovirus serotype 2(Ad2), 5 (Ad5) or 35 (Ad35). A vector may comprise an Ad5 nucleic acid backbone, wherein the backbone comprises in sequential order a left ITR, an SV40 early polyA site, a human E2F-1 promoter operably linked to the E1A gene, a telomerase promoter operably linked to the E4 gene, an adenoviral packaging signal, and a right ITR.

A preferred vector is Ar6pAE2fF. The vector Ar6pAE2fF is an adenovirus vector that uses a fragment of the human E2F-1 promoter to selectively regulate E1A expression and thus adenoviral replication in tumor cells. Characterization of the Ar6pAE2fF vector in vitro shows that it selectively kills Rb-pathway defective tumor cells over normal primary cells. Likewise, this vector is shown to be preferentially replicated in human tumor cell lines versus normal primary cells. Studies in vivo show that this vector has a superior early toxicity profile to the non-selective replication competent virus, Ad*dl*327, when administered intravenously in SCID mice. Further in vivo studies in subcutaneous xenograft models in nude mice show efficacy against different tumors, in particular against tumors of the liver and lung. Furthermore, intra-tumoral administration of Ar6pAE2fF in two independent human xenograft models elicited dose-dependent effects on tumor growth and progression. Ar6pAE2fF is shown to provide advantages in efficacy, selectivity, and safety as compared to the oncolytic adenoviral vector Ad*dl*1520.

A particularly preferred vector is Ar17pAE2fFTrtex. Ar17pAE2fFTrtex is a tumor-selective oncolytic adenovirus designed for the treatment of a broad range of cancer indications. Without being bound by theory, the inventors engineered Ar17pAE2fFTrtex to be dependent on the presence of the two most common alterations in human cancer, namely defects in the Rb-pathway (~85% of all cancers) and over expression of telomerase (~85% of all cancers). Like the intratumoral oncolytic adenovirus Ar6pAE2fF, Ar17pAE2fFTrtex utilizes a E2F-1 promoter to control expression of the adenoviral E1a gene. To increase tumor selectivity appropriate for systemic delivery, the adenoviral E4 gene in Ar17pAE2fFTrtex is controlled by a hTERT (human telomerase reverse transcriptase) promoter. Ar17pAE2fFTrtex is expected to replicate in the majority of cancer cells, lead to tumor selective-expression of toxic viral proteins, cytolysis, and enhancement of sensitivity to chemotherapy, cytokines and cytotoxic T lymphocytes.

As used herein, the term "viral vector" is used according to its art-recognized meaning. It refers to a nucleic acid vector construct which includes at least one element of viral origin and may be packaged into a viral vector particle. The viral vector particles may be utilized for the purpose of transferring DNA into cells either in vitro or in vivo.

A nucleic acid sequence is "operably linked" when it is placed into a functional relationship with another nucleic acid sequence. For instance, a promoter is operably linked to a gene if it affects the transcription of said gene. Operably linked DNA sequences are typically contiguous.

A termination signal sequence within the meaning of the invention may be any genetic element that causes RNA polymerase to terminate transcription, such as for example a polyadenylation signal sequence. A polyadenylation signal sequence is a recognition region necessary for endonuclease cleavage of an RNA transcript that is followed by the polyadenylation consensus sequence AATAAA. A polyadenylation signal sequence provides a "polyA site", i.e. a site on a RNA transcript to which adenine residues will be added by post-transcriptional polyadenylation. Polyadenylation signal sequences are useful insulating sequences for transcription units within eukaryotic cells and eukaryotic viruses. Generally, the polyadenylation signal sequence includes a core poly(A) signal which consists of two recognition elements flanking a cleavage-polyadenylation site (Figure 1). Typically, an almost invariant AAUAAA hexamer lies 20 to 50 nucleotides upstream of a more variable element rich in U or GU residues. Cleavage between these two elements is usually on the 3' side of an A residue and, in vitro, is mediated by a large, multicomponent protein complex. The choice of a suitable polyadenylation signal sequence will consider the strength of the polyadenylation signal sequence, as completion of polyadenylation process correlates with poly(A) site strength (Chao et al., Molecular and Cellular Biology, Aug. 1999, pp5588-5600). For example, the strong SV40 late poly(A) site is committed to cleavage more rapidly than the weaker SV40 early poly(A) site. The person skilled in the art will consider to choose a stronger polyadenylation signal sequence if a more substantive reduction of nonspecific transcription is required in a particular vector construct. In principle, any polyadenylation signal sequence may be useful for the purposes of the present invention. However, in preferred embodiments of this invention the termination signal sequence is either the SV40 late polyadenylation signal sequence or the SV40 early polyadenylation signal sequence. Preferably, the termination signal sequence is isolated from its genetic source and inserted into the viral vector at a suitable position upstream of a E2F responsive promoter.

The termination signal sequence increases the therapeutic effect because it will reduce replication and toxicity of the oncolytic adenoviral vectors in non-target cells. Oncolytic vectors of the present invention with a polyadenylation signal inserted upstream of the E1A coding region are superior to their non-modified counterparts as they demonstrated the lowest level of E1A expression in nontarget cells. Thus, insertion of a polyadenylation signal sequence to stop nonspecific transcription from the left ITR will improve the specificity of E1A expression from the respective promoter. Insertion of the polyadenylation signal sequences will reduce replication of the oncolytic adenoviral vector in nontarget cells and therefore toxicity. A termination signal sequence could also be placed before (5') any promoter in the vector. In one embodiment, the terminal signal sequence is placed before a heterologous promoter operably linked to the E4 gene.

A E2F-responsive promoter has at least one E2F binding site. Preferably, the E2F-responsive promoter is a mammalian E2F promoter, more preferred is a human E2F promoter. In a preferred embodiment of the invention, the E2F-responsive promoter is the human E2F-1 promoter, particularly preferred is the human E2F-1 promoter having the sequence as described in Figure 3.

The E2F-responsive promoter does not have to be the full length wild type promoter, but should have a tumor-selectivity of at least 3-fold, preferably at least 10-fold, at least 30-fold or even at least 300-fold. Tumor-selectivity can be determined by a number of assays using known techniques, such as the techniques employed in example 4, for example RT-PCR. Preferably the tumor-selectivity of the adenoviral vectors is quantified by E1A RNA levels, as further described in example 4, and preferably the E1A RNA levels obtained in H460 cells are compared to those in PrEC cells in order to determine tumor-selectivity for the purposes of this invention. The relevant conditions of the experiment should follow those described in example 4. For example, Ar6pAE2fF in example 4 displays a tumor-selectivity of 2665/8-fold, i.e. about 332-fold.

E2F responsive promoters typically share common features such as Sp I and/or ATT7 sites in proximity to their E2F site(s), which are frequently located near the transcription start site, and lack of a recognizable TATA box. E2F-responsive promoters include E2F promoters such as the E2F-1 promoter, dihydrofolate reductase (DHFR) promoter, DNA polymerase A (DPA) promoter, c-myc promoter and the B-myb promoter. The E2F-1 promoter contains four E2F sites that act as transcriptional repressor elements in serum-starved cells. Preferably, an E2F-responsive promoter has at least two E2F sites.

Without being bound by theory, the understanding of selective hTERT expression in cancer is based on the current knowledge of the molecular underpinnings involved in tumorigenesis. hTERT is the rate-limiting catalytic subunit of telomerase, a multicomponent ribonucleoprotein enzyme that has also been shown to be active in - 85 % of human cancers but not normal somatic cells (Kilian A et al. Hum Mol Genet. 1997 Nov;6(12):2011-9; Kim NW et al. Science. 1994 Dec 23;266(5193):2011-5; Shay JW et al. European Journal of Cancer 1997; 5, 787-791; Stewart SA et al., Semin Cancer Biol. 2000 Dec;10(6):399-406). Telomerase synthesizes telomeric DNA to enable cells to proliferate without senescence. In humans this activity is restricted to germ line cells, stem cells, and activated B and T cells, an attribute necessary for these cells to repopulate diminished cell populations or mediate an immune response (Kim NW et al. Science. 1994 Dec 23;266(5193):2011-5; Hiyama K et al. J Natl Cancer Inst. 1995 Jun 21;87(12):895-902). However, most other normal human cells have a limited lifespan due to lack of telomerase (Poole JC et al. Gene. 2001 May 16; 269(1-2):1-12; Shay JW et al. Hum Mol Genet. 2001 Apr;10(7):677-85). Cancer cells appear to require immortalization for tumorigenesis and telomerase activity is almost always necessary for immortalization (Kim NW et al. Science. 1994 Dec 23;266(5193):2011-5; Kiyono T et al. Nature 1998;396:84), although there is an alternative pathway not involving telomerase that maintains telomere length in a small percentage of tumors (Bryan TM et al. Nat Med. 1997 Nov;3(11): 1271-4). Interestingly, immortalization appears to require an Rb-pathway defect (Kiyono T et al. Nature 1998;396:84). Thus, the majority of tumors have both an Rb-pathway defect and disregulated telomerase, two pathways specifically targeted by Ar17pAE2fFTrtex.

The term TERT promoter refers to the native TERT promoter and functional fragments, mutations and derivatives thereof. The TERT promoter does not have to be the full-length wild type promoter. One skilled in the art knows how to derive fragments from a TERT promoter and test them for the desired specificity. Preferably, the TERT promoter of the invention is a mammalian TERT promoter, more preferred is a human TERT promoter (hTERT). In one embodiment of the invention, the TERT promoter consists essentially of SEQ ID NO:93 which is a 397 bp fragment of the hTERT promoter. In a preferred embodiment of the invention, the TERT promoter consists essentially of SEQ ID NO:94, which is a 245 bp fragment of the hTERT promoter. In a preferred embodiment, a TERT promoter is operably linked to the adenovirus E4 region.

SEQ ID NO:93

ccctcgctggcgtccctgcaccctgggagcgcgagcggcgcgcgggcggggaagcgcggcccagacccccgggtccgcccgg
agcagctgcgctgtcggggccaggccgggctcccagtggattcgcgggcacagacgcccaggaccgcgcttcccacgtggcgg
agggactggggacccgggcacccgtcctgccccttcaccttccagctccgcctcctccgcgcggaccccgccccgtcccgacccct
cccgggtccccggcccagccccctccgggccctcccagcccctccccttcctttccgcggccccgccctctcctcgcggcgcgagttt
caggcagcgctgcgtcctgctgcgcacgtgggaagccctggccccggccacccccgcg

SEQ ID NO:94

ccccacgtggcggagggactggggacccgggcacccgtcctgccccttcaccttccagctccgcctcctccgcgcggaccccgcc
ccgtcccgacccctcccgggtccccggcccagccccctccgggccctcccagcccctccccttcctttccgcggccccgccctctcct
cgcggcgcgagtttcaggcagcgctgcgtcctgctgcgcacgtgggaagccctggccccggccacccccgcg

The recombinant viral vector comprises a gene essential for replication. The term "gene essential for replication" refers to a nucleic acid sequence whose transcription is required for the vector to replicate in the target cell. For example, as the vector construct of the invention is an adenoviral vector, the gene essential for replication may be selected from the group consisting of E1A, E1b, E2 and E4 coding sequences. Most preferably, the gene essential for replication is selected from the group consisting of the E1A, E1b, and E4 coding sequences. Particularly preferred is the adenoviral E1A gene as the gene essential for replication.

In a preferred embodiment, the recombinant viral vector further comprises a deletion upstream of the termination signal sequence. Preferred deletions may be between nucleotides 103 and 551 of the adenoviral type 5 backbone or corresponding positions in other serotypes. Deletion may be between nucleotides 189 and 551 or corresponding positions in other serotypes.

A deletion in the packaging signal 5' to the termination signal sequence may be such that the packaging signal becomes non-functional. The deletion may comprise a deletion 5' to the termination signal sequence wherein the deletion may span at least the nucleotides 189 to 551. The deletion may comprise a deletion 5' to the termination signal sequence wherein the deletion may span at least nucleotides 103 to 551 (Figure 2). In these situations, the packaging signal may be located (i.e. re-inserted) at a position 3' to the termination signal sequence and downstream of the E2F-linked gene essential for replication.

In the context of adenoviral vectors, the term "5" is used interchangeably with "upstream" and means in the direction of the left ITR. In the context of adenoviral vectors, the term "3'" is used interchangeably with "downstream" and means in the direction of the right ITR.

In one embodiment, the invention further comprises a mutation or deletion in the E3 region. However all or a part of the E3 region may be preserved or re-inserted in the oncolytic adenoviral vector. Presence of all or a part of the E3 region may decrease the immunogenicity of the adenoviral vector. It may also increase cytopathic effect in tumor cells and decreases toxicity to normal cells. The vector may expresse more than half of the E3 proteins.

In an alternative embodiment, the invention comprises a mutation or deletion in the E1b gene. Preferably the mutation or deletion in the E1 b gene is such that the E1b-19kD protein becomes non-functional. This modification of the E1b region may be combined with vectors where all or a part of the E3 region is present.

In a preferred embodiment, the oncolytic adenoviral vector further comprises at least one therapeutic gene. The therapeutic gene, preferably in the form of cDNA, can be inserted in any position that does not adversely affect the infectivity or replication of the vector. Preferably, it is inserted in the E3 region in place of at least one of the polynucleotide sequences coding for the E3 proteins. Most preferably, the therapeutic gene is inserted in place of the 19kD or 14.7 kD E3 gene.

A therapeutic gene may be one that exerts its effect at the level of RNA or protein. Therapeutic genes that may be introduced into the adenovirus include a factor capable of initiating apoptosis, antisense or ribozymes, which among other capabilities may be directed to mRNAs encoding proteins essential for proliferation, such as structural proteins, transcription factors, polymerases, etc., genes encoding cytotoxic proteins, genes that encode an engineered cytoplasmic variant of a nuclease (e.g. RNase A) or protease (e.g. trypsin, papain, proteinase K, carboxypeptidase, etc.), or encode the Fas gene, and the like.

Other therapeutic genes of interest include, but are not limited to, immunostimulatory, anti-angiogenic, and suicide genes. Immunostimulatory genes include, but are not limited to, cytokines (GM-CSF, IL1, IL2, IL4, IL5, IFN$\alpha$, IFN$\gamma$, TNF$\alpha$, IL12, IL18, and flt3), proteins that stimulate interactions with immune cells (B7, CD28, MHC class I, MHC class II, TAPs), tumor-associated antigens (immunogenic sequences from MART-1, gp100(pmel-17), tyrosinase, tyrosinase-related protein 1, tyrosinase-related protein 2, melanocyte-stimulating hormone receptor, MAGE1, MAGE2, MAGE3, MAGE12, BAGE, GAGE, NY-ESO-1, $\beta$-catenin, MUM-1, CDK-4, caspase 8, KIA 0205, HLA-A2R1701, $\alpha$-fetoprotein, telomerase catalytic protein, G-250, MUC-1, carcinoembryonic protein, p53, Her2/neu, triosephosphate isomerase, CDC-27, LDLR-FUT, telomerase reverse transcriptase, and PSMA), cDNAs of antibodies that block inhibitory signals (CTLA4 blockade), chemokines (MIP1$\alpha$, MIP3$\alpha$, CCR7 ligand, and calreticulin), and other proteins. Anti-angiogenic genes include, but are not limited to, METH-1, METH -2, TrpRS fragments, proliferin-related protein, prolactin fragment, PEDF, vasostatin, various fragments of extracellular matrix proteins and growth factor/cytokine inhibitors. Various fragments of extracellular matrix proteins include, but are not limited to, angiostatin, endostatin, kininostatin, fibrinogen-E fragment, thrombospondin, tumstatin, canstatin, and restin. Growth factor/cytokine inhibitors include, but are not limited to, VEGFNEGFR antagonist, sFlt-1, sFlk, sNRP1, angiopoietin/tie antagonist, sTie-2, chemokines (IP-10, PF-4, Gro-beta, IFN-gamma (Mig), IFN$\alpha$, FGF/FGFR antagonist (sFGFR), Ephrin/Eph antagonist (sEphB4 and sephrinB2), PDGF, TGF$\beta$ and IGF-1.

A "suicide gene" encodes for a protein which itself can lead to cell death, as with expression of diphtheria toxin A, or the expression of the protein can render cells selectively sensitive to certain drugs, e.g., expression of the Herpes simplex thymidine kinase gene (HSV-TK) renders cells sensitive to antiviral compounds, such as acyclovir, gancyclovir and FIAU (1-(2-deoxy-2-fluoro-.beta.-D-arabinofuranosil)-5-iodouracil). Other suicide genes include, but are not limited to, genes that encode for carboxypeptidase G2 (CPG2), carboxylesterase (CA), cytosine deaminase (CD), cytochrome P450 (cyt-450), deoxycytidine kinase (dCK), nitroreductase (NR), purine nucleoside phosphorylase (PNP), thymidine phosphorylase (TP), varicella zoster virus thymidine kinase (VZV-TK), and xanthine-guanine phosphoribosyl transferase (XGPRT). Alternatively, the therapeutic gene can exert its effect at the level of RNA, for instance, by encoding an antisense message or ribozyme, a protein that affects splicing or 3' processing (e.g., polyadenylation), or a protein that affects the level of expression of another gene within the cell, e.g. by mediating an altered rate of mRNA accumulation, an alteration of mRNA transport, and/or a change in post-transcriptional regulation. The addition of a therapeuitc gene to the virus would result in a virus with an additional antitumor mechanism of action. Thus, a single entity (i.e., the virus carrying a therapeutic transgene) would be capable of inducing multiple antitumor mechanisms.

The DNA sequence encoding the therapeutic gene may be selected from either GM-CSF, thymidine kinase, Nos, FasL, or sFasR (soluble Fas receptor). In a particularly preferred embodiment, the therapeutic gene is GM-CSF.

Granulocyte macrophage colony stimulating factor (GM-CSF) is a multi-functional glycoprotein produced by T cells, macrophages, fibroblasts and endothelial cells. It stimulates the production of granulocytes (neutrophils, eosinophils & basophils) and cells of the monocytic lineage, including monocytes, macrophages and dendritic cells (reviewed in Armitage JO et al. Blood 1998 Dec 15:92(12):4491-508). In addition, it activates the effector functions of these cells and also appears to stimulate the differentiation of B cells. Since the early 1990's, a number of groups have investigated the clinical use of recombinant human GM-CSF for the treatment of cancer.

Of central importance in the oncology setting is the ability of GM-CSF to augment the antigen presentation capability of the subclass of dendritic cells (DC) capable of stimulating robust anti-tumor responses (Gasson et al. Blood 1991 Mar 15;77(6):1131-45; Mach et al. Cancer Res. 2000 Jun 15;60(12):3239-46; reviewed in Mach and Dranoff, Curr Opin Immunol. 2000 Oct;12(5):571-5). In the vaccine setting, DCs that are recruited by GM-CSF to the vaccine site are presumed to capture tumor proteins. Among the proteins captured by DCs will be tumor antigens (i.e., proteins expressed specifically by the tumor, Boon and Old, Curr Opin Immunol. 1997 Oct 1;9(5):681-3). Presentation of tumor antigen epitopes to T cells in the draining lymph nodes is then expected to result in systemic immune responses to tumor metastases. Also, irradiated tumor cells expressing GM-CSF function as potent vaccines against tumor challenge (Dranoff, et al. Proc National Acad Sciences 1993; 90:3539-3543; Jaffee, et al. J Clin Oncol 2001; 19:145-156; reviewed in Pardoll, Annu Rev Immunol 1995;13:399-415). Data such as these have stimulated a number of clinical trials, most notably in melanoma, and prostate, renal and pancreatic carcinoma (Simons JW et al. Cancer Res. 1999; 59:5160-5168; Simons JW et al. Cancer Res 1997; 57:1537-1546; Solffer R et al. Proc. Natl. Acad. Sci USA 1998; 95:13141-13146; Jaffee, et al. J Clin Oncol 2001; 19:145-156). In addition, GM-CSF expression has been shown preclinically to elicit a protease that cleaves plasminogen to produce angiostatin, a known anti-angiogenic protein (Dong Z et al, Cell. 1997 Mar 21;88(6):801-10; Dona Z et al. J Exp Med 1998: 188:755-763).

The DNA sequence encoding a therapeutic gene may be under the control of a suitable promoter. Suitable promoters which may be employed include, but are not limited to, adenoviral promoters, such as the adenoviral major late promoter and/or the E3 promoter, or hetorologous promoters, such as the cytomegalovirus (CMV) promoter; the Rous Sarcoma Virus (RSV) promoter; inducible promoters, such as the MMT promoter, the metallothionein promoter, heat shock promoters; the albumin promoter; and the ApoAI promoter. In a preferred embodiment, the promoter is a tissue-specific promoter as disclosed in U.S. Patent No. 5,998,205, issued December 7, 1999 to Hallenbeck, et al. An E2F-responsive promoter is particularly preferred, such as the human E2F-1 promoter.

Combinations of two or more transgenes with synergistic, complementary and/or nonoverlapping toxicities and methods of action may be utilised. The resulting oncolytic adenovirus would retain the viral oncolytic functions and would, for example, additionally be endowed with the ability to induce immune and anti-angiogenic responses, etc.

The invention further comprises adenoviral vector particles, which comprise the viral vectors of the invention. Preferably, the viral particles further comprise a targeting ligand included in a capsid protein of the particle. Preferably, the capsid protein is a fiber protein, and most preferably, the ligand is in the HI loop of the fiber protein.

The adenoviral vectors may be made by standard techniques known to those skilled in the art. The vectors may be transferred into packaging cells by techniques known to those skilled in the art. Packaging cells may provide complementing functions to the functions provided by the genes In the adenovirus genome that are to be packaged into the adenovirus particle. The production of such particles requires that the vector be replicated and that those proteins necessary for assembling an infectious virus be produced. The packaging may be cultured under conditions that permit the production of the desired viral vector particle. The particles may be recovered by standard techniques. The preferred packaging cells may be those that have been designed to limit homologous recombination that could lead to wild-type adenoviral particles. Such cells are disclosed in U.S. Patent Nos. 5,994,128, Issued November 30, 1999 to Fallaux, et al., and 6,033,908, issued March 7, 2000 to Bout, et al. The packaging cell known as PER.C6, which is disclosed in these patents, may be particularly preferred.

The recombinant viral vectors and particles may selectively replicate in and lyse Rb-pathway defective cells in the majority of tumor types, the Rb/cell cycle regulatory pathway is disrupted, suggesting that Rb-pathway disregulation may be obligatory for tumorgenesis (Strauss M, Lukass J and Bartek J. Unrestricted cell cycling and cancer. Nat Med 1995: 12:1245-1246). Rb itself is mutated in some tumor types, and in other tumor types factors upstream of Rb are deregulated (Weinberg, RA. The retinoblastoma protein and cell cycle control. Cell 1995; 81:323-330). One effect of these Rb-pathway changes in tumors is the loss of pRB binding to E2F, and an apparent increase in free E2F in tumor cells. The abundance of free E2F in turn results in high level expression of E2F responsive genes in tumor cells, including the E2F-1 gene. Accordingly, the term "Rb-pathway defective cells" may be functionally defined as cells which display an abundance of "free" E2F, as measured by gel mobility shift assay or by chromatin immunoprecipitation (Takahashi Y, Rayman JB, Dynlacht BD. Analysis of promoter binding by the E2F and pRB families in vivo: distinct E2F proteins mediate activation and repression. Genes Dev. 2000 Apr 1;14(7):804-16).

In particular, cells which have mutations in genes encoding factors that phosphorylate pRB may be Rb-pathway defective cells within the meaning of the invention. pRB is temporally regulated by phosphorylation during the cell cycle. Among the factors that phosphorylate pRB is the complex of cyclin-dependent-kinase 4 (CDK4) and its regulatory subunit, D-type cyclins (CycD). CDK4 is in turn regulated by the p16 small molecular weight CDK inhibitor. Phosphorylation by CDKs reversibly inactivates pRB, resulting in transcriptional activation by E2F-DP-1 dimers and entry into S phase of the cell cycle. Dephosphorylation of pRB after mitosis causes re-entry into $G_1$ phase. In tumor cells, any one or several of the cell cycle checkpoint proteins may be modified, leading to cell cycle deregulation and unrestricted cell cycling. Loss of the pRB-E2F-DP-1 interaction, or abundance of "free E2F," results in derepression/activation of promoters having E2F sites. Although the inventors do not wish to be limited by these theoretical considerations, we believe that derepression of the E2F-1 promoter in Ar6pAE2fF leads to transcription of E1A, viral replication, and oncolysis.

Accordingly, in another aspect there is provided a method of selectively killing a neoplastic cell in a cell population which comprises contacting an effective amount of the viral vectors or viral particles of the invention with said cell population under conditions where the viral vectors or particles can transduce the neoplastic cells in the cell population, replicate, and kill the neoplastic cells. Preferably, the neoplastic cell has a defect in the Rb-pathway.

The viral vectors of the invention are useful in studying methods of killing neoplastic cells in vitro or in animal models. Preferably, the cells are mammalian cells. More preferably, the mammalian cells are primate cells. Most preferably, the primate cells are human cells.

In a further aspect of the invention, a pharmaceutical composition comprising the recombinant viral vectors and particles of the invention and a pharmaceutically acceptable carrier is provided. Such compositions, which can comprise an effective amount of adenoviral vectors and particles of this invention in a pharmaceutically acceptable carrier, may be suitable for local or systemic administration to individuals in unit dosage forms, sterile parenteral solutions or suspensions, sterile non-parenteral solutions or oral solutions or suspensions, oil in water or water in oil emulsions and the like. Formulations for parenteral and non-parenteral drug delivery are known in the art. Compositions may also include lyophilized and/or reconstituted forms of the adenoviral vectors and particles of the invention. Acceptable pharmaceutical carriers may be, for example, saline solution, protamine sulfate (Elkins-Sinn, Inc., Cherry Hill, N.J.), water, aqueous buffers, such as phosphate buffers and Tris buffers, or Polybrene (Sigma Chemicel, St. Louis MO) and phosphate-buffered saline and sucrose. The selection of a suitable pharmaceutical carrier is deemed to be apparent to those skilled in the art from the teachings contained herein. These solutions are sterile and generally free of particulate matter other than the desired adenoviral virions. The compositions may contain pharmaceutically acceptable auxiliary substances as required to approximate physiological conditions such as pH adjusting and buffering agents, toxicity adjusting agents and the like, for example sodium acetate, sodium chloride, potassium chloride, calcium chloride, sodium lactate, etc. Excipients which enhance infection of cells by adenovirus may be included.

The viral vectors may be administered to a host in an amount which is effective to inhibit, prevent, or destroy the growth of the tumor cells through replication of the viral vectors in the tumor cells.
Such administration may be by systemic administration as hereinabove described, or by direct injection of the vectors in the tumor. In general, the vectors may be administered systemically in an amount of at least $5x10^9$ particles per kilogram body weight and in general, such an amount does not exceed $2.5 \times 10^{12}$ particles per kilogram body weight. The vectors may be administered intratumorally in an amount of at least $2x10^{10}$ particles and in general such an amount does not exceed $2x10^{13}$ particles. The exact dosage to be administered is dependent upon a variety of factors including the age, weight, and sex of the patient, and the size and severity of the tumor being treated. The viruses may be administered one or more times, depending upon the immune response potential of the host. Single or multiple administrations of the compositions can be carried out with dose levels and pattern being selected by the treating physician. If necessary, the immune response may be diminished by employing a variety of immunosuppressants, so as to permit repetitive administration, without a strong immune response. Antineoplastic adenoviral therapy of the present invention may be combined with other antineoplastic protocols.

Delivery may be achieved in a variety of ways, employing liposomes, direct injection, catheters, topical applications, etc.

In yet another aspect, a method of treating a host organism having a neoplastic condition is provided, comprising administering a therapeutically effective amount of the composition of the invention to said host organism.

The neoplastic tissue may be abnormally proliferating, and may be malignant tumor tissue. The viral vector may be distributed essentially throughout the tissue or tumor mass due to its capacity for selective replication in the tumor tissue.

All neoplastic conditions are potentially amenable to treatment with the methods of the invention. Tumor types may include, but are not limited to hematopoietic, pancreatic, neurologic, hepatic, gastrointestinal tract, endocrine, biliary tract, sinopulmonary, head and neck, soft tissue sarcoma and carcinoma, dermatologic, reproductive tract, and the like. Preferred tumors for treatment are those with a high mitotic index relative to normal tissue. Preferred tumors are solid tumors.

In a preferred embodiment of the method of treatment, the neoplastic condition is lung, colon, breast, or prostate cancer.

In a preferred embodiment the host organism is a human patient: For human patients, if a therapeutic gene is included in the vector, the therapeutic gene will generally be of human origin although genes of closely related species that exhibit high homology and biologically identical or equivalent function in humans may be used if the gene does not produce an adverse immune reaction in the recipient. A therapeutic active amount of a nucleic acid sequence or a therapeutic gene is an amount effective at dosages and for a period of time necessary to achieve the desired result. This amount may vary according to various factors including but not limited to sex, age, weight of a subject, and the like.

## EXAMPLES

It is to be understood by the following examples that the scope of the present invention is not intended to be limited.

### Example 1: Construction and molecular characterization of replication-selective adenoviruses Ar6F, Ar6pAF and Ar6pAE2fF

Two adenovirus backbones that were designed to minimize nonspecific activation of the E1A gene were developed. The Ar6F adenoviral vector contains the left side ITR directly linked to the E1A coding region (SEQ ID NO:5), with the intervening nucleotides deleted (nucleotides 104-551 in the Ad5 sequence, GenBank accession number M73260) and replaced with a multiple cloning site (Figure 4). The Ar6pAF adenoviral vector is identical to Ar6F except that it contains the 145 nucleotide SV-40 early poly(A) signal inserted between the left ITR and the E1A coding region (SEQ ID NO: 6, Figure 5). In both of these vectors, the packaging signal normally present near the left ITR was moved to the right ITR (Figure 3, panel B; Seq ID NO:4). This was performed by replacing the right ITR with the reverse complementary sequence of the first 392bp of Ad5, which contains the left ITR and the packaging signal. Finally, to generate the adenoviral vector Ar6pAE2fF, the tumor selective promoter E2F-1 was inserted between the SV-40 early poly(A) signal and the E1A coding region present in Ar6pAF (Figure 3, panel A; Seq ID NO:3).

The first 1802 nucleotides of the Ar6pAE2fF adenoviral vector, including the ITR, poly(A), E2F-1 promoter and the E1A gene was confirmed by DNA sequencing (SEQ ID NO:3). In addition, the last 531 nucleotides at the right end of the vector, containing the packaging signal and right ITR was confirmed by sequencing (SEQ ID NO: 4, Figure 3).

Adenoviral genomes containing these modifications were cloned by standard methods in bacterial plasmids. Homologous recombination in E. coli was performed between these bacterial shuttle plasmids containing fragments of the Ad genome to generate plasmids (pAr6F, pAr6pAF, and pAr6pAE2fF) containing full-length infectious viral genomes (He et al.. 1998. A simplified system for generating recombinant adenoviruses. PNAS 95, 2509-2514). These plasmids containing full length adenoviral genomes were linearized with a restriction enzyme to release the adenoviral genome DNA from the bacterial plasmid sequences. The adenoviral DNA was then transfected into a complementing cell line AE1-2a (Gorziglia et al., 1996. Elimination of both E1 and E2a from adenovirus vectors further improves prospects for in vivo human gene therapy. J. Virol. 6,4173-4178) using the LipofectaAMINE-PLUS reagent system (Life Technologies, Rockville, MD). The cells were incubated at 37°C for approximately 5-7 days. Adenovirus was amplified and purified by CsCl gradient as described (Jakubczak et al., 2001. Adenovirus type 5 viral particles pseudotyped with mutagenized fiber proteins show diminished infectivity of coxsackie B-Adenovirus receptor-bearing cells. J. Virol. 75:2972-2981). Virus particle concentrations were determined by spectrophotometric analysis (Mittereder et al., 1996. Evaluation of the concentration and bioactivity of adenovirus vectors for gene therapy. J Virol. 70, 7498-7509).

1.2 Viral DNA isolation and Southern analysis

DNA was isolated from CsCl-purified virus preparation as described (Puregene Kit, Gentra). Viral DNA was digested with the indicated restriction enzymes and analyzed on 1% agarose/TAE gels containing ethidium bromide. A total of 1 ug of each DNA sample was digested with ClaI, XbaI, HpaI, SalI and BamHI and subjected to Southern analysis according to standard procedures. The probe was prepared by random oligonucleotide priming and contained the E2F-1 promoter.

Figure 6 summarizes the cloning and structures of Ar6pAF and Ar6pAE2fF vectors. The DNA structure of a research lot of Ar6pAE2fF vector was confirmed by Southern analysis. The expected left DNA region fragments were obtained using five independent restriction endonucleases. Southern blot analysis with an E2F promoter DNA probe demonstrated the expected hybridization pattern for all restriction endonucleases. Thus, these results confirmed the presence of the E2F-1 promoter in the correct position and verified the integrity of the viral DNA.

1.3 Limiting Dilution Cloning of Ar6pAE2fF vector in PER.C6 cells

A seed lot of Ar6pAE2fF vector was produced for further evaluations. To obtain a pure seed lot of a virus it is necessary to isolate a clone derived from a single virus particle. The cloning of Ar6pAE2fF virus was accomplished through viral limiting dilution as described in below.

Ten 96 well plates of PER.C6 cells (Fallaux et al., 1998. New helper cells and matched early region 1-deleted adenovirus vectors prevent generation of replication-competent adenoviruses. Human. Gene Ther 9, 1909-1917) were plated at 5 x $10^3$ cells/well in 0.04 ml volume /well. PER.C6 cells were grown in DMEM with the addition of 10% FBS and 10 mM $MgCl_2$. 10 ul of Ar6pAE2fF containing 1 x $10^{-2}$ particles/ul was added to each well, giving a final infection of 0.1 particle/well. Infected cells were incubated at 37 °C and 5% $CO_2$ for 4 hours, after which 150 ul of media was added. The virus infected cells were incubated at 37 °C and 5% $CO_2$ for 12 days followed by scoring for CPE. The 0.1 particle /cells clones 7-9 from PER.C6 cells were harvested on day 13.Three clones, 7-9 showed CPE and were freeze thawed 5 times and amplified on PER.C6 cells plated in 6 well dishes. On day 3, CVL were prepared from clones 7-9 and clone 7 was further amplified in a T150 of PER.C6 cells. Ar6pAE2fF clone 7 T150 was harvested 2 days post-infection, a time at which the cells had reached complete CPE.

The CVL was freeze thawed 5 times and cellular debris was spun out. A T75 flask of PER.C6 cells was plated and infected with 0.5 ml of the above CVL.

Of the 960 wells infected with 0.1 particle/cell, three wells showed CPE. These 3 clones were in the range of the theoretical numbers of clones expected. Statistically, only 4 wells out of the 10 plates should give CPE. This gives odds of 1:2500 that there will be more than one infectious particle/well when assuming a particle:pfu ratio of 25. The three clones were amplified in PER.C6 cells and the genome of clone 7 showed the expected size DNA fragments when analyzed with HpaI, XhoI and XbaI restriction endonuclease.

1.4 Sequence analysis.

The 5'-end first 1802 nucleotides and the last 3'-end nucleotides from bp 33881-34412 of the plasmids pDL6pAE2f and Ar6pAE2fF clone 7 were directly sequenced (SEQ ID NO:3, Figure 3A).

Regions of Ar6pAE2fF were confirmed by DNA sequencing. Regions in first 1802 nucleotides are ITR (nucleotides 1-103), poly-adenylation signal (nucleotides 116-261), human E2F-1 promoter (nucleotides 283-555), E1A gene (nucleotides 574-1647) and a portion of the E1b gene (nucleotides 1648-1802) are indicated (SEQ ID NO:3, Figure 3A). Regions in the last 531 nucleotides are the Pacl restriction site (nucleotides 33967-33974) (underlined), the packaging signal (nucleotides 34020-34217 and the ITR (34310-34412) (SEQ ID NO:4, Figure 3B).

Example 2: Characterization of E1 A expression by FACS

To determine if deletions of enhancer elements and insertion of a polyA signal would be sufficient for efficient transcription termination, a quantitative E1A FACS assay was used to evaluate E1A expression in a non-complementing A549 cell background (p16- p53+ Rb+). We compared the E1A expression from cells infected with Ad*dl*327, Ad*dl*312, Ar6F, Ar6pAF or Ar6pAE2fF at doses of 10, 50, 250 and 1250 virus particles per cell (VPC) (Table 1). The highest level of E1A expression was observed with the Ad*dl*327 at all range of doses. In contrast, the E1A deleted mutant Ad*dl*312 showed no E1A expression. Under the conditions used in this experiment (10 to 1250 VPC) there was about 80% to 22% less E1A detected in cells transduced with Ar6F than in those transduced with Add1327. The E1A expression in cells transduced with Ar6pAF was significantly reduced about 100% to 96%, in all doses, as compared to the expression from cells infected with the Add/327. The expression of E1A from cells infected with the Ar6pAE2fF oncolytic vector was reduced 50% as compared with the Add/327 virus at a dose of 50VPC.

In conclusion, the insertion of a poly(A) signal in the Ar6pAF vector reduced the E1A expression in A549 cells. In contrast, insertion of the E2F-1 promoter reestablished the E1A expression, thus demonstrating that E1A expression was exclusively due to the inserted promoter.

**Table 1**. E1A expression in A549 noncomplementing cells

| | 10 vpc | 50 vpc | 250 vpc | 1250 vpc |
|---|---|---|---|---|
| Addl327 | 27.5±2.2 | 72.9±3.8 | 94.4±0.7 | 98.4±0.4 |
| Addl312 | 0.0±0.0 | 0.0±0.0 | 0.0±0.0 | 0.0±0.0 |
| Ar6F | 5.6±0.8 | 28.3±1.1 | 59.4±4.7 | 76.9±3.6 |
| Ar6pAF | 0.0±0.0 | 0.1 ±0.1 | 0.3±0.1 | 3.8±2.4 |
| Ar6pAE2fF | ND | 39.7±0.1 | ND | ND |

Noncomplementing A549 cells were infected with either vector at 10, 50, 250 and 1250 VPC. E1A expression was determined 24 hours postinfection by FACS.

Protocol for E1A FACS Assays

Cells were plated the day before infection in 12-well plates. The next day, media was aspirated from cells, virus dose formulations in particles per cell (ppc) were added to the wells and the plates were rocked at 37°C for 4 hours. Virus/media was aspirated, washed one time, then replaced with complete growth media and incubated 20 hours at 37°C. Cells were harvested by trypsin-EDTA digestion, and fixed in 70% ethanol for 20 minutes at room temperature. Then the cells were washed one time and resuspended in FACS buffer (PBS, 3% FBS, 0.1 % $NaN_3$). 10□l of a 1:10 dilution of unconjugated anti-E1A antibody (Calbiochem, Anti-Adenovirus 2E1A, Human (Ab-1)) or mouse $IgG_{2a}$ isotype control (Sigma M-5409) was added and incubated at room temperature for 30 minutes. The cells were washed one time with FACS buffer. Then 50□l of 1:40 dilution of GAM PE (Sigma P-9670) was added and incubated at room temperature for 30 minutes. Then the cells were washed, resuspended in 200□l FACS buffer, and 20,000 events on FACSCAN were acquired.

Example 3: Toxicity of adenoviral vectors

Acute hepatic toxicity in Balb/c SCID male mice was used to distinguish between adenoviral vectors with different levels of E1A activity. A profound difference in serum liver enzyme elevations was observed between vectors with wild-type E1A expression and those with minimal or silent E1A expression.

Studies were designed with ten animals per group. Control groups were HBSS vehicle alone, the negative control E1A-deleted Ad*dl*312 and the E1A-containing positive control Ad*dl*327. Viruses were injected at a dose of 6.25 x $10^{11}$ particles/kg intravenously into the tail vein in a volume of 10ml/kg; an equivalent dose volume of HBSS (10 mL/kg) was injected in the vehicle control group. Animals were injected on study day 1, with an interim sacrifice of half of each group on study day 4 and a terminal sacrifice of the remaining animals on study day 15. On study days 4 and 15, serum was collected from all mice, and the livers removed from the animals scheduled for sacrifice (5/group). In addition, body weights were measured on all surviving mice on study days -3, 1, 3, 4, 8 and 15.

**Table 2**. Acute toxicity of E1A containing adenoviral ectors

| | ALT | | AST | | DB | |
|---|---|---|---|---|---|---|
| Vector | mean | sd | Mean | sd | mean | Sd |
| Ar6F | 2213.40 | 1018.61 | 1500.40 | 922.53 | 0.19 | 0.33 |
| Ar6pAF | 57.6* | 24.59 | 130.7* | 40.33 | 0.01* | 0.03 |

*significant difference versus Ar6F (*p*<0.05) The acute toxicity of E1A-containing adenoviral vectors in the backbones Ar6F, Ar6PAF was compared. Viruses are prepared as described in Example 1. Based on body weight change (Figure 7, map of corresponding constructs see Figure 8) and serum ALT and AST levels (Table 2), the hepatotoxicity of Ar6F was higher than Ar6pAF.

Example 4: Selective regulation of E1 A gene expression in tumor cells by the oncolytic adenoviral vector Ar6pAE2fF - quantitative RT-PCR assay

In this study, E1A gene expression in Ar6pAE2fF in tumor cells versus non-tumor cells was compared using a quantitative RT-PCR assay. The E2F-1 promoter dependency of E1A expression was assessed by comparing the level of E1A RNA in Ar6pAE2fF with the level in Ar6pAF, an adenovirus identical to Ar6pAE2fF but lacking the E2F-1 promoter. Adenoviral transduction was determined by the viral DNA copy number. The impact of selective E1A gene expression on adenoviral replication was also examined in certain cell types. E1A gene expression was selective in tumor cells. This tumor selectivity was dependent on E2F-1 promoter transactivation. In association with E1A gene expression, selective adenoviral DNA replication of Ar6pAE2fF was also observed in Hep3B versus primary hepatocytes.

Viruses were prepared as described in Example 1.

4.1 Methods

4.1.1 Human tumor cell lines and normal cells

Selectivity of E1A gene expression by the adenoviral vectors was evaluated in pRB/p16 pathway defective human tumor cell lines as well as in human normal non-tumor cells. The tumor cells were all obtained from American Type Culture Collection (ATCC, Manasass VA), including hepatocellular carcinoma (Hep 3B2.1-7; ATCC #HB-8064), non-small cell lung cancer (NCI-H460; ATCC #HTB-177 and NIH-H1299; ATCC #CRL-5803) and epithelioid carcinoma of pancreas (Panc-1; ATCC #CRL-1469). Hep3B cells were grown in EMEM with 10% FBS, Panc-1 in DMEM with 10% FBS, H460 in RPMI1640 containing 10% FBS, 4.5g/L D-(+)-glucose, 0.75g/L sodium bicarbonate, 10 mM HEPES and 2 mM L-glutamine, and H1299 in RPMI1640 with 10% FBS. Non-tumor human cells included small airway epithelial cells (SAEC, Clonetics Cat. #CC2547) and prostate epithelial cells (PrEC, Clonetics Cat. #CC2555). These cells were isolated from a single donor and passaged through early culture upon receipt in cryopreserved form. Since they are capable of proliferating up to 15 population doublings when grown in Small Airway Epithelial Cell Growth Medium (SAGM) and Prostate Epithelial Cell Growth Medium (PrEGM) respectively (Biowhittaker/Clonetics, Walkersville, MD), the SAEC and PrEC are considered "normal" cell lines to differentiate them from primary hepatocytes that are not subjected to subculture (Freshney RI. (Ed.) Biology of the cultured cell in Culture of Animal Cells, pp.9-19, 3rd Edition, 1994, Wiley-Liss, New York). The primary human hepatocytes were purchased from In Vitro Technology (Baltimore, MD) and Dr. Steven Stom, University of Pittsburgh (Pittsburgh, PA) as primary cultures in HCGM (Biowhittaker/Clonetics, Waldersville, MD). Tested by the suppliers, these primary hepatocytes were negative in HIV, hepatitis B virus and hepatitis C virus. Experiments were performed on the hepatocytes after less than one day in culture.

4.1.2 Conditions for proliferating and quiescent culture and cell cycle analysis

E2F-1 promoter is highly active in tumor cells but relatively inactive in normal proliferating cells (Parr MJ et al., Nat Med 1997 Oct; 3(10):1145- 9). As preliminary study, we attempted to address E2F-1 promoter driven E1A expression in Ar6pAE2fF in association with cell cycle status. Tumor cells were expected to be persistently proliferating in culture. To obtain 50 to 60% confluent cells upon viral infection, tumor cells were seeded in 6-well plates at 4 x $10^5$/well one-day prior to viral infection. Non-tumor cells require relatively lower seeding density for growth (Airway Epithelial Cell System Instruction Manual, Biowhittaker/Clonetics), therefore, SAEC and PrEC were plated at 2 x $10^5$/well one-day prior to infection. The cells were 40 to 50% confluent upon viral infection. Same cell culture experiment was used for analyses of E1A RT-PCR, adenoviral DNA PCR and cell cycle status.

In order to test whether the non-tumor cell proliferation can be arrested, quiescent cultures were generated for SAEC and used for cell cycle analysis. Cells were seeded at 1 x $10^5$/well in 6-well plates and allowed to grow to 100% confluency in SAGM. Confluent SAECs were then starved by incubation in Small Airway Epithelial Cell Basal Medium (SABM, Biowhittaker/Clonetics) for 24 hours before infection with adenoviral vectors. SABM is SAGM but lacking the following growth nutrients: bovine pituitary extract, hydrocortisone, human epidermal growth factor, epinephrine, transferrin, insulin, retinoic acid, triiodothyronine, bovine serum albumin-fatty acid free, as well as gentamicin and amphotericin-B (Airway Epithelial Cell System Instruction Manual, Biowhittaker/Clonetics). Primary hepatocytes were a positive control for quiescent cultures in this study.

Cell cycling status in the above culture conditions was examined at the time of infection. The relative cell population undergoing DNA synthesis (S phase) in uninfected cells was analyzed by using a BrdU Flow Kit purchased from BD PharmMingen (San Diego, CA). Cells were first labeled with bromodeoxyuridine (BrdU), an analog of the DNA precursor thymidine, as follows: cells grown in 6-well plates were pulsed by 10 □l/ml 1 mM BrdU (BD PharMingen) for 4 hours at 37°C in a total of 3 ml culture media. Cells were then harvested with trypsin EDTA, fixed, permeablized and frozen at -70°C. Cells were then costained by immunofluorescent antiBrdU antibody and 7-amino-actinomycin D (7-AAD) for total DNA according to the manufacturer's manual (BD PharmMingen). The BrdU-labled cell population was quantitated by flow cytometric analysis using FACSCalibur Manufactured by Becton Dickinson with CellQuest software. The cell cycle position was determined by analyzing the correlated expression of total DNA and incorporated BrdU levels. FACS analysis of the cells costained with anti-BrdU and 7-AAD discriminates cell subsets that are in phases of G0/G1, S or G2 (BD PharmMingen). Cell cycle status was presented as % of cells in each cycle phase (Table 3). For each sample, 8,000 to 19,000 events were collected. The BrdU pulsing and FACS analysis were performed in two cell samples and the average value is reported. Unstained cells were used for background settings in the Flow Cytometry.

**Table 3**. Cell cycling in uninfected tumor and normal cells

| Cells | S phase (%) | G0/G1 phase (%) | G2 phase (%) | Rb/p16 & p53 status |
|---|---|---|---|---|
| Hep3B | 38.5 | 40.7 | 16.4 | $Rb^-p16^+p53^-$ |
| H460 | 46.4 | 47.5 | 3.1 | $Rb^+$ $p16^-$ $p53^+$ |
| H1299 | 58.6 | 37.8 | 1.7 | $Rb^+$ $p16^-$ p53- |
| Panc-1 | 43.3 | 36.3 | 18.8 | $Rb^+$ $p16^-$ $p53^-$ |
| Primary hepatocytes | 0.33 | 23.9 | 66.4 | wild type |
| SAEC (Q) | 0.78 | 90.6 | 7.8 | wild type |
| SAEC (P) | 22.2 | 68.6 | 7.8 | wild type |

Uninfected cells were stained by BrdU followed by co-staining of anti-BrdU and 7-AAD, and cell cycle position was determined by analyzing the correlated expression of total DNA and incorporated BrdU levels as described in Methods. Tumor cell lines: Hep3B, H460, H1299 and Panc-1; Normal non-tumor cells: SAEC and primary hepatocytes. Q, quiescent cell culture conditions; P, proliferating cell culture conditions. Data represent mean of two cell samples. Rb/p16 and p53 status in tumor cells was reported by Farshid et al. J Viral Hepat 1994; 1(1):45-53, Kaino M. J Gastroenterol 1997 Feb; 32(1):40-6, Kataoka M, et al. Oncogene 2000 Mar 16; 19(12):1589-95, Spillare EA, et al. Mol Carcinog 1996 May;16(1):53-60.

4.1.3 Cell infection for E1A transcription study

E1A gene transcription occurs in both early and late stage of adenoviral life cycle after infection of host cells. Since the cell population in culture may not be infected simultaneously by adenoviruses, the E1A gene transcription may occur in each cell at different time, therefore, the level of early E1A gene transcription may be obscured. To control the time for initiation of E1A gene transcription, the infection was synchronized for viral internalization into the cell. The infections were carried out in 0.5 ml/well infectious media (basic media with 2% FBS) at 4°C, rocking for one hour to allow attachment of the virus to the cytoplasmic membrane. Viral attachment is efficient at cold temperature but subsequent steps in the infectious process require energy. Thus, after viral attachment, the viral media were removed, the cells were washed with cold PBS, and incubated in growth media at 37°C for variable times to allow viral internalization and gene transcription. The time course for E1A gene transcription was tested in Hep3B cells infected with 1, 10, 100 particles/cell of Ad*dl*/327 for 4, 8 and 16 hours. Based on the results from the time course study, the infection for all the other experiments was carried out at a viral concentration of 10 and 100 ppc with one-hour incubation at 4°C for virus attachment and four hours at 37 °C for virus internalization. The reason for choosing 10 and 100 ppc is to assure the level of E1A expression can be detected in all cell types infected with different vectors.

4.1.4 Real time E1A RT-PCR analysis

RNA samples were collected from approximate $4 \times 10^5$ cells for E1A RT-PCR analysis. The cells were washed with PBS, lysed in 1 ml RNAzol B and stored in -70°C freezer until isolation of the RNA. Total RNA from the cell lysates was isolated using the RNAzol B method (Tel-TEST, Friendswood, TX). RNA concentration was determined spectrophotometrically ($A_{260}$ and $A_{280}$). First strand cDNA was generated from 100 ng of RNA using Taqman Reverse Transcription Reagents (Applied Biosystems). Primers specific for the adenovirus E1A sequences were:

E1A Forward primer: 5'-AGCTGTGACTCCGGTCCTTCT-3' (SEQ ID NO:22)

E1A Reverse primer: 3'-GCTCGTTAAGCAAGTCCTCGA-3' (SEQ ID NO:23)

E1A Probe: 5'-FAM-TGGTCCCGCTGTGCCCCATTAAA -TAMRA-3' (SEQ ID NO:24)

Amplification was performed in a reaction volume of 50 μl under the following conditions: 20 μl of sample cDNA, 1X Taqman Universal PCR Master Mix (Applied Biosystems), 300 nM forward primer, 900 nM reverse primer and 100 nM E1A probe. Thermal cycling conditions were: a 2 minute incubation at 50°C, a 10 minute 95°C activation step for the Amplitaq Gold, followed by 35 cycles of successive incubation at 95°C for 15 seconds and 60°C for 1 minute. Thermal cycling was carried out with 7700 Sequence Detection System (Applied Biosystems). To assess RNA input (endogenous control), 10 μl of a 1:1000 dilution of each cDNA was amplified using a Pre-Developed Taqman Assay Reagent 18S kit (Applied Biosystems) as per manufacturer's instruction. Data was collected and analyzed using the 7700 Sequence Detection System software v. 1.6.3 (Applied Biosystems). Relative levels of E1A were determined based on an E1 A -plasmid curve with dilutions from 1,500,000 - 15 copies.

4.1.5 Hexon DNA PCR analysis

For viral DNA copy number analysis, cells were infected with 10 ppc adenoviral vectors. The cells were washed with PBS and harvested with trypsin EDTA after the infection. Cell pellets were frozen on dry ice and stored in -70°C freezer until isolation of the DNA. DNA was isolated from 3 x $10^6$ - 5 x $10^6$ cells using Qiagen Qiamp Mini Columns (Qiagen Inc., Chatsworth, CA), according to the manufacturer's instructions. Elution of DNA was done in 275 μl of water and concentrations were determined spectrophotometrically ($A_{260}$ and $A_{280}$). PCR primers and a Taqman probe specific to adenovirus hexon sequences were designed using Primer Express software v. 1.0 (Applied Biosystems, Foster City, CA). Primer and probe sequences were:

Hexon Forward primer: 5'-CTTCGATGATGCCGCAGTG-3' (SEQ ID NO:25)

Hexon Reverse primer: 3'-GGGCTCAGGTACTCCGAGG-3' (SEQ ID NO:26)

Hexon Probe: 5'-FAM-TTACATGCACATCTCGGGCCAGGAC-TAMRA-3' (SEQ ID NO:27) Amplification was performed in a reaction volume of 50 μl under the following conditions: 10 ng of sample DNA, 1X Taqman Universal PCR Master Mix (Applied Biosystems), 600 nM forward primer, 900 nM reverse primer and 100 nM hexon probe. Thermal cycling conditions were: 2 minute incubation at 50°C, 10 minutes at 95°C, followed by 35 cycles of successive incubation at 95°C for 15 seconds and 60°C for 1 minute. Data was collected and analyzed using the 7700 Sequence Detection System software v. 1.6.3 (Applied Biosystems). Quantification of adenovirus copy number was performed using a standard curve consisting of dilutions of adenovirus DNA from 1,500,000 copies to 15 copies in 10ng human DNA. The average number of total copies was normalized to copies per cell based on the input DNA weight amount and a genome size of $6 \times 10^9$ bp.

4.2 Results

4.2.1 Time course of E1A gene expression

Our goal was to quantitate the level of E1A RNA for evaluation of the promoter transcriptional activity. E1A gene transcription occurs shortly after infection of host cells and lasts approximately 6-8 hours, after which viral DNA replication is first detected (Shenk T. Adenoviridae: The viruses and their replication. 1996; in Fields Virology , Fields BN, Knipe DM and Howley PM, eds. (Lippincott-Raven, Philadelphia) pp. 2111-2148; Russell WC. Update on adenovirus and its vectors. J. General Virol 2000; 81:2573-2604). In order to determine the earliest time point at which E1A RNA could be detected by real time RT-PCR assay after adenoviral infection, we performed a time course for E1A RNA levels in Hep3B cells infected with 1, 10 and 100 ppc Add*l*/327. The cellular E1A RNA was quantitated at 0, 4, 8, and 16 hours post infection as described in Methods. E1A RNA was detected at 4 hours post infection at all three viral doses in a dose-dependent manner (Table 4). The level of E1A RNA continued to increase until the last time point (16 hours) post infection used in this study. The E1A RNA detected at 4-hour post infection is very likely the result of gene transcription at early phase of viral infection cycle since E1A transcription in the late phase is coupled with viral DNA replication and occurs 6-8 hours after infection (Shenk T. Adenoviridae: The viruses and their replication. 1996; in Fields Virology, Fields BN, Knipe DM and Howley PM, eds. (Lippincott-Raven, Philadelphia) pp. 2111-2148). The rapidly rising E1A RNA level after 4-hour post infection indicates that E1A gene expression is not limited by the host cellular factors, indicating the viral infection cycle is progressing to replication stage.

**Table 4**. Time and dose-dependence of E1A gene expression in Addl327

| Time after infection (hrs) | E1A mRNA levels* | | |
|---|---|---|---|
| | 1 ppc | 10ppc | 100ppc |
| | E1A mRNA levels* | | |
| 0 | 1 | 0.2 | 0.1 |
| 4 | 1,200 | 8,800 | 110,000 |
| 8 | 6,600 | 55,000 | 570,000 |
| 16 | 46,000 | 390,000 | 2,000,000 |

Hep3B cells were infected and E1A RNA expression was measured by quantitative RT-PCR. *E1A RNA levels shown are units of RT-PCR product relative to an E1A plasmid standard curve in a total of 28.5 ng input RNA. ppc, particles of Add/327per cell.

4.2.2 Adenoviral DNA copy numbers

Adenoviral DNA copy number at 4-hours post infection

E1A RNA detected at 4-hour post infection is the result of gene transcription at an early phase of the viral infection cycle. Since viral DNA replication marks the late phase of viral the infection cycle, we examined the viral DNA copy number 4 hours post infection to confirm the early phase of the viral infection cycle. It has been reported that all adenoviral types exhibit similar kinetics for viral transduction (Kasamatsu H, Nakanishi A. Annu Rev Microbiol 1998;52: 627-86). Replication competent Ar6pAE2fF encodes identical capsid proteins as replication defective Add/312. Therefore, the Add/312 was used as a negative control for viral DNA replication. Hep3B cells were infected with 10 ppc of Add/312 or Ar6pAE2fF for 4 hour and adenoviral DNA copy number was analysed by PCR. We detected an average 0.5 Ad*dl*312 DNA copy/cell as compared with 0.4 Ar6pAE2fF DNA copy/cell in Hep3B cells (Table 5), indicating that no obvious adenoviral DNA replication was taking place at 4-hour post infection in Ar6pAE2fF-infected Hep3B cells. This observation is again consistent with adenoviral DNA replication after 6-8 hour post infection. This result coupled with the ability to detect E1A transcripts by the RT-PCR assay lead us to assess the transcriptional control of the E1A gene at 4-hour post infection.

**Table 5.** Adenoviral vector transduction of cells

| Cells | Vectors | Adenoviral DNA copies / cell | | |
|---|---|---|---|---|
| | | Exprmt.1 | Exprmt. 2 | Mean |
| H460 | Ar6pAE2fF | 0.03 | 0.03 | 0.03 |
| H1299 | Ar6pAE2fF | 0.1 | NA | 0.1 |
| Panc-1 | Ar6pAE2fF | 0.1 | NA | 0.1 |
| Hep3B | Ar6pAE2fF | 0.3 | 0.4 | 0.4 |
| Hep3B | Add/312 | 0.5 | 0.5 | 0.5 |
| SAEC | Ar6pAE2fF | 0.01 | 0.01 | 0.01 |

(continued)

| Cells | Vectors | Adenoviral DNA copies / cell Exprmt.1 | Exprmt. 2 | Mean |
|---|---|---|---|---|
| Primary hepatocytes | Ar6pAE2fF | 0.11 | 0.1 | 0.1 |

Cells were infected with 10 ppc of the appropriate adenoviral vectors and the adenoviral DNA copy number was measured by hexon DNA PCR assay as described in Methods. Tumor cell lines: H460, H1299, Hep3B and Panc-1; Non-tumor cells: SAEC and primary hepatocytes. NA, not available. Data for SAEC and primary hepatocytes were from two separate experiments with different donors. For all tumor cell lines, data were from two experiments except H1299 and Panc-1.

Adenovirus transduction of cell lines

Since virus attachment, internalization, and nuclear transfer depend on host cell factors, their efficiency was expected to vary between different cell types. In order to control for the effect of transduction on the level of E1A gene expression in different cell types, we determined the adenoviral DNA copy number in four tumor cell lines and two normal cell types following infection with the same dose. Table 5 shows the virus DNA copy numbers measured in cells infected with 10 ppc. The data indicate that Hep3B had the highest, H1299, Panc-1 and primary hepatocytes intermediate, and H460 and SAEC the lowest amount of adenovirus DNA, indicating up to 50-fold variation among different cell types in viral transduction. This finding is generally consistent with the reported 20-fold differences in the uptake efficiencies between different tumor cell lines (Steinwaerder, 2000). The high transduction variation among cell types in our result may relate to broad cell types including normal cells covered in the study. In addition, the data in Table 5 is relatively preliminary since only two experiments were performed (except H1299 and Panc-1). However, since the variation in viral DNA copy number between the two experiments within the same cell type was small (Table 5), the values were used for normalization of E1A RNA level in different cell types.

4.2.3 Selective E1A gene expression in tumor cells

Selective E1A gene expression is critical to restrict viral DNA replication in tumor cells. We tested the ability of Ar6pAE2fF to mediate selective E1A gene expression through the tumor-selective E2F-1 promoter. Tumor and non-tumor cells were infected with 10 and 100 ppc Ar6pAE2fF or Ar6pAF for 4 hours as described in Methods. To discern cell type variation in adenoviral genome uptake (Table 5), the E1A RNA levels were normalized to the adenoviral DNA copy number per cell and is presented as E1A RNA level per Ad genome (Table 6). As shown in Table 6, there is a clear trend of higher E1A RNA expressed from all four Ar6pAE2fF-infected tumor cells (381 - 2655 units/Ad genome) than the level expressed from the two Ar6pAE2fF-infected non-tumor cells (57-123 units/Ad genome), suggesting tumor selective E1A gene expression. Within each of the tumor cell types, Ar6pAE2fF tended to mediate higher levels of E1A RNA than that mediated by Ar6pAF (Table 6). The apparent difference in E1A levels between Ar6pAE2fF-infected cells versus Ar6pAF-infected cells at the high dose was approximately 30 to 100-fold, showing that the tumor selective E1A gene expression is E2F-1 promoter-dependent.

Table 6. E1A gene expression in tumor and primary cells

| Cell line | Ad vector | Dose (ppc) | E1A RNA / Ad genome |
|---|---|---|---|
| Hep3B | Ar6pAE2fF | 100 | 2538 |
| | | 10 | 194 |
| | Ar6pAF | 100 | 53 |
| | | 10 | 3.3 |
| | Addl327 | 100 | 26471 |
| | | 10 | 1929 |
| | Addl312 | 100 | 1.1 |
| H1299 | Ar6pAE2fF | 100 | 381 |
| | | 10 | 38 |
| | Ar6pAF | 100 | 8 |
| | | 10 | 2.9 |

(continued)

| Cell line | Ad vector | Dose (ppc) | E1A RNA / Ad genome |
|---|---|---|---|
| | Addl327 | 100 | 1464 |
| | | 10 | 165 |
| | Addl312 | 100 | 0 |
| H460 | Ar6pAE2fF | 100 | 2655 |
| | | 10 | 385 |
| | Ar6pAF | 100 | 0 |
| | | 10 | 37 |
| | Addl327 | 100 | 3145 |
| | | 10 | 162 |
| | Addl312 | 100 | 12 |
| Panc-1 | Ar6pAE2fF | 100 | 1103 |
| | | 10 | 85 |
| | Ar6pAF | 100 | 42 |
| | | 10 | 0 |
| | Addl327 | 100 | 4792 |
| | | 10 | 267 |
| | Addl312 | 100 | 2.2 |
| SAEC | Ar6pAE2fF | 100 | 123 |
| | | 10 | 60 |
| | Ar6pAF | 100 | 20 |
| | | 10 | 18 |
| | Addl327 | 100 | 17105 |
| | | 10 | 1165 |
| | Addl312 | 100 | 75 |
| Hepatocytes | Ar6pAE2fF | 100 | 57 |
| | | 10 | 6 |
| | Ar6pAF | 100 | 3 |
| | | 10 | 5 |
| | Addl327 | 100 | 16475 |
| | | 10 | 1080 |

E1A RNA levels at 4 hours post-infection relative to an E1A plasmid standard curve were quantitated by RT-PCR. The E1A RNA level was then normalized to the adenoviral DNA copy number per cell determined as in Table 8. Data represent mean of two experiments. ppc, particles per cell. SAEC, small airway epithelial cells.

Cell lines differ from one another in the ability to transactivate specific promoters, including the presence of cellular factors and efficiency of basal transcription machinery. In order to estimate the E2F-1 promoter activity, the E1A RNA level expressed from 100 ppc Ar6pAE2fF-infected cells was analyzed relative to E1A RNA level from Add/327-infected cells. Both Ar6pAE2fF and Add/327 mediated E1A gene expression in tumor cells (Table 7); thus, both the E2F-1 and the E1A promoters are active in tumor cells tested. The E2F-1 promoter was 10-84% as active as the wild-type E1A promoter in the tumor cells (Table 7). In contrast, the E2F-1 promoter was 0.2-0.7% as active as the wild-type promoter in the non-tumor cells (Table 7). The low E2F-1 promoter activity from non-tumor cells is selective because the wild-type promoter in these cells (E1A RNA 4491-16475 units/Ad genome) was as active as in tumor cells (E1A RNA 1464-26471 units/Ad genome, Table 7). These data showed that replacement of the wild-type promoter with the E2F-1 promoter introduces tumor selective E1A expression and that the E2F-1 promoter conveys substantial transcription activity in tumor cell but not in non-tumor cells.

E1A gene expression in viral vectors lacking the E2F-1 promoter is a measure of E1A gene expression independent of the E2F-1 promoter. We observed 2 to 60 units/Ad genome E1A RNA expressed from high dose Ar6pAF-infected cells (Table 6) while little E1A RNA was detected in the negative control of Ad*d*/312-infected cells (Table 6).

Compared to 381 to 2655 units/Ad genome E1A RNA expressed by the selective E2F-1 promoter, the level of E1A RNA expressed by Ar6pAF lacking the E2F-1 promoter was very low. In a study of E1A protein level by FACS analysis, 0.1% E1A positive cell population was detected in 50 ppc Ar6pAF-infected-A549 cells. The E1A RNA analysis is consistent with the E1A protein analysis and indicates trace amount of E1A gene transcribed possibly by nonselective promoters in the adenoviral backbone.

**Table 7**. Relative E1A gene expression in Ar6pAE2fF-infected cells

| | E1A RNA level / Ad | genome | E1A RNA level |
|---|---|---|---|
| Cells | Ar6pAE2fF | Add/327 | (% of Add1327) |
| H460 | 2655 | 3145 | 84 |
| H1299 | 381 | 1464 | 26 |
| Panc-1 | 1103 | 4792 | 23 |
| Hep3B | 2538 | 26471 | 10 |
| Primary hepatocytes | 57 | 16475 | 0.3 |
| SAEC (P) | 123 | 17105 | 0.7 |
| PrEC (P) | 8 | 4491 | 0.2 |

Cells were infected with 100 ppc Ar6pAE2fF to compare relative E1A expression in the tumor cells (Hep3B, H460, H1299 and Panc-1) versus normal non-tumor cells (primary hepatocyte, SAEC and PrEC). Data represent mean of two experiments.

4.2.4 Cell cycling in tumor and normal cells

Deregulation of cell cycle control upregulates free E2F protein which is required for E2F-1 promoter transactivation. In this study, cell cycle analysis showed that tumor cells in culture were highly proliferating (39-59% in S-phase), non-tumor SAECs were proliferating (22% in S-phase), and primary hepatocytes were quiescent (0.3% in S-phase, Table 3). The E2F-1 promoter activity in Ar6pAE2fF-infected tumor cells (10-84% relative E1A RNA) was higher than in proliferating Ar6pAE2fF-infected SAECs (0.7% relative E1A RNA, Table 7), suggesting that E2F-1 promoter transactivation is more effective in tumor cells than in proliferating non-tumor cells although statistic significance remains to be determined.

4.2.5 Adenoviral replication in Hep3B and primary hepatocytes

The question remained as to whether the level of E1A expression had an impact on late stages in the viral life cycle. We measured Ar6pAE2fF DNA replication in comparison with Add/1520, a prototype replication-based selective oncolytic adenoviral vector and currently in clinic trial (Heise CC, et al. Cancer Res 1999 Jun 1; 59(11):2623-8, Ganly I, et al. Clinical Cancer Res 2000 Mar; 6:798-806). Ad*dl*327 and Ad*dl*312 were included as positive and negative replication controls. As a preliminary study, a comparison was made in Hep3B and primary hepatocytes. Because of 5-fold difference in viral transduction between these two cell types (Table 5), the adenoviral DNA copy numbers in Table 8 cannot be directly compared to one another. Therefore, Ar6pAE2fF DNA copy number in the two cell types were compared to Add/327 DNA copy number. At 24 hours post-infection with Ar6pAE2fF, the relative replication capability of Ar6pAE2fF in Hep3B (44%) was approximately 10 fold higher than the relative replication capability in the primary hepatocytes (5%). This selective viral DNA replication was also observed with Add/1520 in Hep3B (29%) and primary hepatocytes (5%). These data show that selective Ar6pAE2fF DNA replication in Hep3B is consistent with the selective E1A gene expression Hep3B (Table 6). The similar level of Ar6pAE2fF selective replication compared to the level of Ad*dl*1520 selective replication shows that the E2F-1 promoter-dependent E1A expression is capable of mediating viral DNA replication that is potentially sufficient for tumor killing activity.

**Table 8**. Adenoviral DNA replication in Hep3B and primary hepatocytes

| Cell line | Ad vector | Ad copies/cell |
|---|---|---|
| Hep3B | Ar6pAE 2fF | 1290.0 |
| | Ad*dl*327 | 2943.0 |

(continued)

| Cell line | Ad vector | Ad copies/cell |
|---|---|---|
| | Add/312 | 3.5 |
| Hepatocytes | Ar6pAE2fF | 5.9 |
| | Add/327 | 125.0 |
| | Add/312 | 0.6 |

Cells were infected with 10 particles per cell with the indicated vectors for 2 hours, washed and incubated at 37°C for 24 hours. Adenoviral DNA copy was measured by a quantitative PCR assay for adenoviral hexon DNA. The numbers represent the mean of duplicate samples.

Example 5: Efficacy of intratumoral administration of the Ar6pAE2fF oncolytic vector in a H460 model - xenograft model in nude mice

In this study, we tested the ability of the oncolytic vector Ar6pAE2fF to inhibit the growth and progression of pre-established human non-small cell lung carcinoma tumors in a subcutaneous xenograft model in nude mice. Results showed that five daily intratumoral injections of Ar6pAE2fF to H460 xenograft tumors in nude mice at doses of $5\times10^8$, $5\times10^9$ or $5\times10^{10}$ particles/dose/day lead to a dose-dependent inhibition in tumor growth. In addition, Ar6pAE2fF significantly increased the median survival time of animals in all treatment groups when compared to the HBSS treated control group. There were no significant differences in mean body weight change among any of the treatment or control groups and no mortality as a result of oncolytic vector treatment. Assessment of *in vivo* oncolytic vector function revealed E1A to be expressed intratumorally in all Ar6pAE2fF dose groups as measured by E1A-RNA-specific RT-PCR. Preliminary vector tissue distribution in the liver and lung was determined by adenoviral hexon gene specific PCR. Results indicated that animals treated intratumorally with Ar6pAE2fF at $5x10^{10}$ particles/dose/day had vector DNA present in both the liver (1.73 $\pm$ 0.77 copies/cell) and lung (1.25 $\pm$ 0.79 copies/cell). Nevertheless, E1A expression was minimal in those same tissues. These results show that intratumoral injections of Ar6pAE2fF lead to significant dose-dependent tumor growth inhibition and tumor-specific E1A expression.

5.1 Methods

5.1.1 Viruses and tumor cell line

The oncolytic vector Ar6pAE2fF is an E3-deleted adenoviral vector in which the native E1A promoter is replaced with the E2F-1 promoter, the packaging signal has been moved from the left ITR to the right ITR, and an SV40 polyadenylation signal has been inserted between the left ITR and the E2F-1 promoter. Add/327 is a replication competent serotype 5 adenovirus deleted in the Xbal-D fragment of E3 (bp 28,592-30,470) resulting in the deletion of all E3 genes except the E3-12.5K gene (Tollefson AE, et al. J Virol. 1996 Apr;70(4):2296-306, Young CSH, et al. The genetic system. In: Ginsberg HS ed. The adenoviruses comprehensive virology, Vol. 4, New York: Plenum Press. 1984:125-172). This virus is used as a replication competent positive control. Ar6pAE2fF and Ad*d*/327 were prepared using standard CsCl gradient purification methods. Vector concentration was determined by optical particle titer (Mittereder N, Marck KL, and Trapnell BC. J. Virology 1996; 70:7498-7509).

The human, non-small cell lung carcinoma line H460 (large cell lung cancer, NCI-H460; ATCC #HTB-177 lot number 945778) was obtained from American Type Culture Collection (Manassas, VA) and found to be free of mycoplasma contamination. The H460 cells are cultured in RPMI 1640 media containing 10% FBS, 4.5 g/L glucose, 2 mM glutamine, 10 mM HEPES, 1 mM sodium pyruvate, and 1.5 g/L (w/v) sodium bicarbonate.

5.1.2 Mouse tumor model study

Female athymic outbred nu/nu mice (Harlan Sprague Dawley), 6-8 weeks of age, were implanted with $3x10^6$ H460 cells (resuspended in 0.1 ml of HBSS) subcutaneously in the right flank. Tumor measurements were recorded (in two dimensions) twice weekly using calipers. Tumor volume was calculated by the equation $[(W \times L)/4]^3\pi$. Body weights were recorded once per week for the duration of the study. When tumor volume reached 100 - 250mm$^3$ animals were randomly distributed into groups and injected intratumorally with Ar6pAE2fF at $5x10^8$ (n=13), $5x10^9$ (n=13), or $5x10^{10}$ (n=12) particles/dose/day in a volume of 50 $\mu$l for 5 consecutive days. A negative control group was injected with HBSS (n=13) and a positive control group was injected with Add/327 (n=12) at $5x10^{10}$ particles/dose/day also for 5 consecutive days. Four days after the last intratumoral injection (study day 9), 5 animals per group were euthanized and a gross necropsy performed. Separate samples of tumor, liver and lung were collected for E1A RT-PCR and hexon DNA PCR analysis. E1A RT-PCR samples were placed in RNAlater™ (Ambion Inc.) and kept at 4°C for 24 hours, then stored at -20°C until processed. Hexon DNA PCR samples were snap frozen and stored at -70°C until processed.

5.1.3 Molecular Analyses

Adenoviral DNA quantitation.

DNA from tissues was isolated using the Qiagen Blood and Cell Culture DNA Midi or Mini Kits (Qiagen Inc., Chatsworth, CA). Frozen tissues were partially thawed and minced using sterile disposable scalpels. Tissues were then lysed by incubation overnight at 55°C in Qiagen buffer G2 containing 0.2mg/ml RNaseA and 0.1 mg/ml protease. Lysates were vortexed briefly and then applied to Qiagen-tip 100 or Qiagen-tip 25 columns. Columns were washed and DNAs were eluted as described in the manufacturer's instructions. After precipitation, DNAs were dissolved in water and the concentrations were spectrophotometrically determined ($A_{260}$ and $A_{280}$). Quantitation of adenoviral DNA copy number was determined by quantitative PCR detection of the hexon gene, as described in example 4.

Adenoviral E1A RNA quantitation

Tissue samples were collected, directly placed into RNAlater™ (Ambion, Auston, TX) and stored at 4°C. For RNA isolation, tissues were cut into approximately 100-200 $mm^3$ pieces using sterile scalpels. Following disruption in RNAzol B, samples were extracted 0.1 volume chloroform. The RNA was precipitated with one volume isopropanol, washed with 75% ethanol and resuspended in nuclease-free water. RNA samples were then treated with 10 Units DNase I (Life Technologies, Rockville, MD) at room temperature and purified using the RNeasy Mini Kit (Qiagen Inc., Chatsworth, CA). RNA concentration was determined photometrically ($A_{260}$ and $A_{280}$). Reverse transcription and PCR analysis was performed as described in example 4.

5.1.4 Statistical Analyses

Statistical tests were done using the SigmaStat program. Mean tumor volume values were compared by one-way repeated measures analysis of variance with Tukey's test used for multiple comparison. Survival of animals was analyzed by the Mantel-Haenszel logrank test using the GraphPad Prism program.

5.2 Results

5.2.1 Tumor Volumes

A subcutaneous xenograft model of non-small cell lung carcinoma was used to assess the efficacy of the oncolytic adenovirus Ar6pAE2fF. H460 cells formed tumors of 100-250 $mm^3$ between one and two weeks after subcutaneous injection into nude mice. Intratumoral injection of Ar6pAE2fF for 5 consecutive days at doses of $5x10^8$ (n=13), $5x10^9$ (n=13), or $5x10^{10}$ (n=12) particles/dose/day all showed a significant inhibition in tumor growth by study day 16 ($p<0.05$ by RM ANOVA) when compared to HBSS (n=13) injected controls (Figure 9, Table 9). Tumor volumes at day 16 in the high dose group ($5 \times 10^{10}$) were significantly different than tumor volumes in the low dose group ($5 \times 10^8$) demonstrating a dose-dependent anti-tumor response (p=0.007 by t-test). Intratumoral injections of the positive control virus, Ad*dl*327, (n=12) also lead to significant tumor growth inhibition ($p<0.05$).

**Table 9.** T/C values

| Treatment Group | % T/C |
|---|---|
| HBSS | 100 |
| Ar6pAE2fF: $5 \times 10^8$ | 63.7 |
| Ar6pAE2fF: $5 \times 10^9$ | 55.9 |
| Ar6pAE2fF: $5 \times 10^{10}$ | 35.2 |

(continued)

| Treatment Group | % T/C |
|---|---|
| Add/327: 5 x $10^{10}$ | 10.2 |
| Percent T/C = percent mean tumor volume for treatment group over HBSS control group; determined at study day 16. | |

5.2.2 Survival

Comparison of long term survival (Figure 10) shows that treatment of tumors with Ar6pAE2fF at all doses significantly increased survival over negative control animals (p=0.0002, p=0.009 and p=0.0111 for 5x$10^{10}$, 5x$10^9$ and 5x$10^8$ particles/dose/day, respectively). Median survival times increased from 16 days for the HBSS treated animals to 22 days for Ar6pAE2fF treated at 5x$10^8$ and 5x$10^9$ particles/dose/day and to 29 days for animals treated with Ar6pAE2fF at 5x$10^{10}$ particles/dose/day. Long term survival of mice treated with the positive control virus, Add/327, was significantly increased over negative control animals (p<0.001), and 80% of Add/327-treated mice survived to the end of the study.

5.2.3 Body Weights

Body weights increased for all groups for the duration of the study. Comparison of mean body weights showed no significant differences between all treatment and control groups (Table 10).

**Table 10.** Mean body weights from H460 tumor bearing mice

| Treatment Group | Virus dose (particles/ injection) | Study Day | | | | | |
|---|---|---|---|---|---|---|---|
| | | -3 | 4 | 11 | 18 | 25 | 32 |
| HBSS | 0 | 21.7±0.4 | 22.4 ±0.4 | 22.6 ±0.4 | 23.5 ±0.1 | 26.1 | 25.8 |
| Ar6pAE2fF | 5x$10^8$ | 22.3 ±0.5 | 22.9±0.5 | 23.8±0.5 | 24.3 ±0.4 | 24.7 ±0.4 | 23.9±0.3 |
| Ar6pAE2fF | 5x$10^9$ | 22.1 ±0.5 | 22.7 ±0.5 | 23.5±0.4 | 24.2±0.5 | 24.7 ±0.3 | 23.7 ±0.3 |
| Ar6pAE2fF | 5×$10^{10}$ | 21.9±0.5 | 22.1 ±0.4 | 22.7±0.4 | 23.6±0.5 | 24.6±0.4 | 24.8 ±0.5 |
| Add/327 | 5x$10^{10}$ | 21.8 ±0.3 | 22.4 ±0.3 | 23.2±0.4 | 24.0±0.3 | 24.1 ±0.3 | 24.1 ±0.4 |
| Body weights were measured on indicated study days. Mice were treated on study day 1. Values represent mean ± SE. | | | | | | | |

5.2.4 E1A expression in tumors

Assessment of *in vivo* oncolytic vector function was determined by analyzing tumor samples isolated on study day 9 from 5 animals from each dose group for expression of E1A. Detection and relative quantitation of E1A expression was carried out by reverse transcription of E1A RNA followed by Taqman real-time PCR. Data were reported as mean E1A RNA units per ng total RNA ± standard error (Table 11). Tumor samples treated with the positive control virus, Add/327 (5x$10^{10}$ particles/dose/day) were found to have the highest relative E1A at 6351 ± 871 E1A RNA units/ng total RNA. All Ar6pAE2fF treated tumor samples expressed E1A message with relative E1A RNA values of 5064 ± 1969, 3291 ± 147 and 3993 ± 1951 E1A RNA units/ng total RNA for dose groups of 5×$10^{10}$, 5×$10^9$ and 5×$10^8$ particles/dose/day respectively. Tumor samples treated with HBSS were found to have barely detectable levels of E1A RNA present.

**Table 11**. E1A expression in tumors and distal tissues

| Treatment Group | Virus dose (particles/injection) | E1A RNA units/ng total RNA | | |
|---|---|---|---|---|
| | | Tumor | Liver | Lung |
| HBSS | 0 | 0.0413 ± 0.041 | 0 | 0 |
| Ar6pAE2fF | 5 x $10^8$ | 3993 ± 1951 | ND | ND |
| Ar6pAE2fF | 5 x $10^9$ | 3291 ± 147 | ND | ND |

(continued)

| Treatment Group | Virus dose (particles/injection) | E1A RNA units/ng total RNA | | |
|---|---|---|---|---|
| | | Tumor | Liver | Lung |
| Ar6pAE2fF | 5 x $10^{10}$ | 5064 ± 1969 | 0.029 ± 0.014 | 0.209 ± 0.077 |
| Add/327 | 5 x $10^{10}$ | 6351.6 ± 871 | 2.49 ± 1.57 | 0.32 ± 0.21 |

Average E1A RNA units/ng total RNA ± SE from tissue samples isolated on study day 9. RNA units are determined relative to an E1A-plasmid copy number control. ND, not determined.

5.2.5 Vector tissue distribution and E1A expression

Preliminary vector tissue distribution was assessed by analyzing the liver and lung from animals treated intratumorally with Ar6pAE2fF at 5x$10^{10}$ particles/dose/day for expression of E1A by E1A RNA RT-PCR assay (Table 11). In addition, vector genome copy number was assessed by hexon gene DNA PCR assay in the same liver and lungs to see if Ar6pAE2fF vector DNA was present even in the absense of E1A expression (Table 12). Hexon DNA copy number indicated that Ar6pAE2fF vector DNA was present in both the liver (1.73 ± 0.77 copies/cell) and lung (1.25 ± 0.79 copies/cell). Ad*d*/327 vector DNA was also detected in liver and lung (0.62 ± 0.51 and 0.06 ± 0.07, respectively).

For the Ar6pAE2fF-treated mice, E1A expression was barely detectable in the liver (0.029±0.014 E1A RNA units/ng total RNA) and lung (0.209±0.077 E1A RNA units/ng total RNA). E1A expression was higher in liver of Add/327 treated mice (2.49 ± 1.57) and similar in lung (0.32 ± 0.21). (Table 11.)

**Table 12.** Hexon DNA PCR Results

| Treatment Group | Vector genome (copies/cell) | | |
|---|---|---|---|
| | Tumor | Liver | Lung |
| HBSS | ND | 0.0 | 0.0 |
| Ar6pAE2fF | ND | 1.73±0.77 | 1.25±0.79 |
| Add/327 | ND | 0.62±0.51 | 0.06±0.07 |

Viral genome copies determined by hexon gene specific PCR assay from samples isolated on study day 9. Mean Hexon DNA copies/cell ± SE are listed. ND - not determined.

Example 6: Efficacy of intratumoral administration of the Ar6pAE2fF oncolytic vector in a Hep3B model - xenograft model in nude mice

In this study, we tested the ability of the oncolytic vector Ar6pAE2fF to inhibit the growth and progression of pre-established human hepatocellular carcinoma (Hep3B) tumors in a subcutaneous xenograft model in nude mice. Results showed that five daily intratumoral injections of Ar6pAE2fF to Hep3B xenograft tumors in nude mice at doses of 5x$10^8$, 5x$10^9$ or 5x$10^{10}$ particles/dose/day lead to a dose-dependent inhibition in tumor growth. In addition, Ar6pAE2fF significantly increased the median survival time of animals in all treatment groups when compared to the HBSS treated control group. There were no significant differences in mean body weight change among any of the treatment or control groups and no mortality as a result of oncolytic vector treatment. Assessment of *in vivo* oncolytic vector function revealed E1A to be expressed intratumorally in the Ar6pAE2fF (5x$10^{10}$ particles/dose/day) dose group as measured by E1A-RNA-specific RT-PCR. Preliminary vector tissue distribution in the liver and lung was determined by adenoviral hexon gene specific PCR. Results indicated that animals treated intratumorally with Ar6pAE2fF at 5x$10^{10}$ particles/dose/day had vector DNA present in both the liver (0.50 ± 0.62 copies/cell) and lung (0.80 ± 0.93 copies/cell). Nevertheless, E1A expression was minimal in those same tissues. These results show that intratumoral injections of Ar6pAE2fF lead to significant dose-dependent tumor growth inhibition and tumor-specific E1A expression.

The human, liver hepatocellular carcinoma line Hep3B (Hep 3B2.1-7; ATCC #HB-8064, batch number F-9462) was obtained from American Type Culture Collection (Manassas, VA) and found to be free of mycoplasma contamination. The Hep3B cells are cultured in Eagle minimal essential media (EMEM) containing 10% fetal bovine serum (FBS).

6.1 Methods

6.1.1 Mouse tumor model study

Female athymic outbred nu/nu mice (Harlan Sprague Dawley), 6-8 weeks of age, were implanted with 1 x $10^7$ Hep3B cells (resuspended in 0.1 ml of HBSS) subcutaneously in the right flank. Tumor measurements were recorded (in two dimensions) twice weekly using calipers. Tumor volume was calculated by the equation $[(W \times L)/4]^3\pi$. Body weights were recorded once per week for the duration of the study. When tumor volume reached 100 - 250mm$^3$ animals were randomly distributed into groups and injected intratumorally with Ar6pAE2fF at 5x$10^8$ (n=11), 5x$10^9$ (n=11), or 5x$10^{10}$ (n=10) particles/dose/day in a volume of 50 $\mu$l for 5 consecutive days. A negative control group was injected with HBSS (n=10) and a positive control group was injected with Ad*dl*/327 (n=11) at $5\times10^{10}$ particles/dose/day also for 5 consecutive days. Five additional animals per group were treated as above for molecular analysis of vector distribution and E1A expression. Four days after the last intratumoral injection (study day 9), 5 animals per group were euthanized and a gross necropsy performed. Separate samples of tumor, liver and lung were collected for E1A RT-PCR and hexon DNA PCR analysis. E1A RT-PCR samples were placed in RNAlater™ (Ambion Inc.) and kept at 4°C for 24 hours, then stored at -20°C until processed. Hexon DNA PCR samples were snap frozen and stored at -70°C until processed.

6.1.2 Molecular Analyses

Molecular Analyses; DNA isolation from tissue; Hexon Taqman real-time PCR assay; RNA isolation from tissue; cDNA synthesis; Detection and relative quantitation of E1A expression by Taqman real-time PCR and Statistical Analyses were performed as described in Example 5.

6.2 Results

6.2.1 Tumor Volumes

A subcutaneous xenograft model of hepatocellular carcinoma was used to assess the efficacy of the oncolytic adenovirus Ar6pAE2fF. H460 cells formed tumors of 100-250 mm$^3$ between one and two weeks after subcutaneous injection into nude mice. Intratumoral injection of Ar6pAE2fF for 5 consecutive days at doses of $5\times10^8$ (n=11), $5\times10^9$ (n=11), or $5\times10^{10}$ (n=10) particles/dose/day all showed a significant inhibition in tumor growth by study day 25 ($p<0.05$ by RM ANOVA) when compared to HBSS (n=10) injected controls (Figure 11). Tumor volumes at day 32 in the high and medium dose group (5 x $10^{10}$ and 5x$10^9$ particles/dose/day) were significantly different than tumor volumes in the low dose group (5 x $10^8$) demonstrating a dose-dependent anti-tumor response ($p<0.05$ by t-test). Intratumoral injections of the positive control virus, Add/327, (n=11) also lead to significant tumor growth inhibition compared to HBSS injected controls ($p<0.05$).

Tumor volume data on study day 25 expressed as percent treatment/control (T/C) is shown in Table 13. These results show a dose-dependent anti-tumor response with T/C equal to 29.5 for the low dose and 12.8 for the high dose groups.

**Table 13.** T/C values

| Treatment Group | % T/C |
|---|---|
| HBSS | 100.0 |
| Ar6pAE2fF: 5 x $10^8$ | 29.5 |
| Ar6pAE2fF: 5 x $10^9$ | 12.7 |
| Ar6pAE2fF: 5 x $10^{10}$ | 12.8 |
| Ad*dl*327: 5 x $10^{10}$ | 4.4 |

Percent T/C = percent mean tumor volume for treatment group over HBSS control group; determined at study day 25.

6.2.2 Survival

Comparison of survival (Figure 12) shows that treatment of tumors with Ar6pAE2fF or Ad*d*/327 at all doses significantly increased survival over HBSS treated control animals ($p<0.0001$ by Mantel-Haenszel logrank test, for all groups). Median survival time was 32 days for the HBSS treated animals and undefined for all the other treatment groups since 100% of the Ar6pAE2fF treated animals survived to the end of the study, day 32. Long term survival of mice treated with the positive control virus, Add/327, was also significantly increased over negative control animals ($p<0.0001$), and 100% of Add/327-treated mice survived to the end of the study.

6.2.3 Body Weights

Comparison of mean body weights showed no significant differences between all treatment and control groups (Table 14).

**Table 14.** Mean body weights from Hep3B tumor bearing mice

| Treatment Group | Virus dose (particles/ injection) | Week 0 | 1 | 2 | 3 | 4 |
|---|---|---|---|---|---|---|
| HBSS | 0 | 24.5 ±0.5 | 24.2 ±0.4 | 24.4 ±0.5 | 24.3 ±0.6 | 23.9 ±0.7 |
| Ar6pAE2fF | $5x10^8$ | 24.9 ±0.6 | 24.4 ±0.4 | 25.1 ±0.6 | 25.1 ±0.5 | 24.9 ±0.5 |
| Ar6pAE2fF | $5x10^9$ | 24.2 ±0.5 | 23.7 ±0.5 | 24.0±0.6 | 25.0 ±0.7 | 24.7 ±0.6 |
| Ar6pAE2fF | $5x10^{10}$ | 24.2 ±0.3 | 23.6 ±0.4 | 24.4 ±0.4 | 25.0 ±0.5 | 25.1 ±0.5 |
| Ad*d*/327 | $5x10^{10}$ | 23.2±0.5 | 23.0 ±0.5 | 24.2 ±0.7 | 24.8 ±0.6 | 25.0 ±0.6 |

Body weights were measured on indicated study weeks. Values represent mean ± SE.

6.2.4 Vector tissue distribution and E1A expression

Preliminary vector tissue distribution was assessed by analyzing the tumor, liver and lung from animals treated intratumorally with HBSS, Ar6pAE2fF ($5x10^{10}$ particles/dose/day) and Add/327 ($5x10^{10}$ particles/dose/day) for expression of E1A by E1A RNA RT-PCR assay (Table 15). Detection and relative quantitation of E1A expression was carried out by reverse transcription of E1A RNA followed by Taqman real-time PCR. Data were reported as mean E1A RNA units per ng total RNA ± standard error. Tumor samples treated with the positive control virus, Add/327 ($5x10^{10}$ particles/ dose/day) were found to have relative E1A values of 723 ± 353 (E1A RNA units/ng total RNA). The Ar6pAE2fF ($5x10^{10}$ particles/dose/day) treated tumor samples also expressed E1A message with relative E1A RNA value of 869 ± 235 (E1A RNA units/ng total RNA). Tumor samples treated with HBSS were found to have little or no E1A RNA present (0.01 ± 0.01 units/ng total RNA). For distal tissues E1A expression of Ar6pAE2fF-treated mice was barely detectable in the liver (0.18 ± 0.14 E1A RNA units/ng total RNA) and lung (0.45 ± 0.40 E1A RNA units/ng total RNA). E1A expression was similar in the liver (0.07 ± 0.04) and lung (0.14 ± 0.07) of Add1327 treated mice.

**Table 15.** E1A expression in tumors and distal tissues

| Treatment Group | Virus dose (particles/injection) | E1A RNA units/ng total RNA | | |
|---|---|---|---|---|
| | | Tumor | Liver | Lung |
| HBSS | 0 | 0.01 ± 0.01 | 0 | 0 |
| Ar6pAE2fF | $5 \times 10^{10}$ | 869.3 ± 234.8 | 0.18 ± 0.14 | 0.45 ± 0.40 |
| Ad*d*/327 | $5 \times 10^{10}$ | 723.5 ± 352.8 | 0.07 ± 0.04 | 0.14 ± 0.07 |

Average E1A RNA units/ng total RNA ± SE from samples isolated on study day 9. RNA units are determined relative to an E1A-plasmid copy number control.

Vector genome copy number was assessed by hexon gene DNA PCR assay in the same tumor, liver and lungs to see if Ar6pAE2fF vector DNA was present (Table 16). Hexon DNA copy number indicated that Ar6pAE2fF vector DNA was present in the tumor (311 ± 150), the liver (0.50 ± 0.62 copies/cell) and lung (0.80 ± 0.93 copies/cell). Add/ 327 vector DNA was also detected in the tumor (291.40 ± 89.36), liver (0.34 ± 0.15), and lung (0.17 ± 0.14).

**Table 16.** Hexon DNA PCR Results

| Treatment Group | Vector genome (copies/cell) Tumor | Liver | Lung |
|---|---|---|---|
| HBSS | 0.10±0.00 | 0.01 ±0.02 | 0.01 ±0.01 |
| Ar6pAE2fF | 311.5 ± 150.3 | 0.50± 0.62 | 0.80± 0.93 |
| Add/327 | 291.4 ± 89.4 | 0.34±0.15 | 0.17±0.14 |

Viral genome copies determined by hexon gene specific PCR assay from samples isolated on study day 9. Mean Hexon DNA copies/cell ± SE is shown.

Example 7: Reconstitution of the E3 region, deletion of the E1 B-19kD gene and combination of E3-reconstitution and E1B-19K deletion in the genome of the Ar6pAE2fF vector

7.1 Vector construction

Modified oncolytic vectors were generated in the Ar6pAE2fF backbone to reconstitute the wildtype E3 region, delete the E1B-19KD gene, or combine of the E3 and E1B-19kD modifications.

7.1.1 Construction of Ar6pAE2fE3F

Ar6pAE2fF is based on the Ad*dl*327 backbone which is deleted in the 1878 nucleotide XbaI fragment in the E3 region, leaving a unique XbaI site in E3 (nucleotide 28592 in Ad5, GenBank accession number M73260). To regenerate the entire E3 region in the Ar6pAE2fF vector, an 1878 nucleotide XbaI fragment from adenovirus type 5 (nucleotides 28593-30470) containing the missing portion of the E3 region was inserted into the single XbaI site in an Ar6pAE2fF right arm shuttle. An infectious adenoviral plasmid was generated in bacteria by homologous recombination as described (He et al., 1998. A simplified system for generating recombinant adenoviruses. PNAS 95, 2509-2514). The viruses were generated as described above in example 1. This vector was named Ar6pAE2fE3F.

7.1.2 Construction of Ar6pAE2fdl19K

To remove the E1b 19K protein from the Ar6pAE2fF backbone, nucleotides 1716 to 2010 (as numbered in Ad5, GenBank accession number M73260) were deleted from a left end Ar6pAE2fF shuttle plasmid. An infectious adenoviral plasmid was generated in bacteria by homologous recombination as described (He et al., 1998. A simplified system for generating recombinant adenoviruses. PNAS 95, 2509-2514). The viruses were generated as described above in example 1. This vector was named Ar6pAE2fFdl19K.

7.1.3 Construction of Ar6pAE2fE3dl19K

An infectious adenoviral plasmid containing both the E3 addition (described in section 7.1.1) and the E1b 19K deletion (described in section 7.1.2) was generated in bacteria by homologous recombination as described (He et al., 1998. A simplified system for generating recombinant adenoviruses. PNAS 95, 2509-2514). The viruses were generated as described above in example 1. This vector was named Ar6pAE2fdl19KE3F.

7.2 In vitro cytotoxicity

In order to compare the cytotoxicity of Ar6pAE2fF versus Ar6pAE2fE3F, Ar6pAE2fdl19k and Ar6pAE2fE3dl19K oncolytic vectors in tumor and primary non-tumor cells, a quantitative method for cell killing was utilized (CellTiter 96® $AQ_{ueous}$ Assay by Promega, Madison, WI, or "MTS assay").

The tumor cells tested were H460 (ATCC#HTB-177), H1299 (ATCC#CRL-5803), and PANC-I (ATCC#CRL#1469) available from American Type Culture Collection (ATCC, Manassas, VA) and primary non-tumor cell PREC (Clonetics #CC2555).

One day prior to viral infection, cells were plated in 96-well dishes. The number of cells per well was determined empirically for each cell type such that they were 60-80% confluent at the time of viral infection.

The next day, cells were infected with the adenoviral vectors. The adenoviruses to be tested were four-fold serially diluted in growth media over a dose-range that yielded a sigmoidal dose response curve. Nine serial dilutions of each virus are added in a 10 μl volume across the plate, starting with the highest dose. To the blank wells and the control wells, 10 μl of media without virus was added at the time of infection to bring the total volume of all wells to 100 μl. The plate was then incubated at 37°C in a humidified 5% $CO_2$ incubator for seven days.

The MTS assay was performed according to the manufacturer's instructions (CellTiter 96® AQueous Assay by Promega, Madison, WI). The CellTiter 96® $AQ_{ueous}$ Assay uses the novel tetrazolium compound (3-(4,5-dimethylthiazol-2-yl)-5-(3-carboxymethoxyphenyl)-2-(4-sulfophenyl)-2H-tetrazolium, inner salt; MTS) and the electron coupling reagent, phenazine methosulfate (PMS).

7.2.1. Results

In the tumor cell lines, all the modified Ar6pAE2fF oncolytic vectors tested (Ar6pAE2fFdl19K, Ar6pAE2fE3F and Ar6pAE2fFE3dl19K) showed better killing activity than the parental Ar6pAE2fF virus, as demonstrated by the mean $LD_{50}$ values (Table 17). In H460 cells, the mean $LD_{50}$ values calculated for the three modified Ar6pAE2fF oncolytic vectors ranged from 14.7 - 34.2. For Ar6pAE2fF, the mean $LD_{50}$ value was 159, showing a 10 to 4.6- fold increase in cell killing activity for the modified oncolytic vectors. In the PANC1 cells the mean $LD_{50}$ values for the three modified oncolytic vectors ranged from 9.6-20.7 , while the mean $LD_{50}$ value for Ar6pAE2fF was 87. This represents between 9-4.2-fold increase in cell killing activity for the modified oncolytic vectors. In H1299 cells, the mean $LD_{50}$ values for the modified vectors ranged from 23-53, while for Ar6pAE2fF the $LD_{50}$ was 79, an increase between 3.4-1.4-fold in cell killing activity for the modified oncolytic vectors.

In the normal PREC cell, in contrast, the mean $LD_{50}$ values calculated for the three modified oncolytic vectors ranged from 98.4 to 158, while for Ar6pAE2fF the mean $LD_{50}$ value was 39, representing a 2.5-4.1 fold-reduction in killing activity for the three modified oncolytic vectors.

**Table 17**. Cytotoxicity of oncolytic vectors on tumor cell lines and normal cells

| | Tumor cell lines | | | Normal |
|---|---|---|---|---|
| Vector | H460 (n) | H1299 (n) | Panc-1 (n) | PrEC (n) |
| Ar6pAE2fF | 159 ±94 (5) | 79 ±78 (8) | 87 ±80 (14) | 39 ±32 (8) |
| Ar6pAE2fE3F | 15± 11* (4) | 54±52(9) | 21±24* (11) | 98±46* (8) |
| Ar6pAE2fdl19K | 34±8*(4) | 23 ±20 (7) | 17±17* (10) | 106 ±52* (8) |
| Ar6pAE2fE3dl19kF | 28 ±29 (3) | 29 ±28 (9) | 10 ±14* (9) | 158 ±13* (3) |

Shown are $LD_{50}$ values calculated in an MTS assay. Absorbance units were converted to percent uninfected control values and plotted versus vector dose on a logarithmic scale. A sigmoidal dose-response curve was fit to the data and an $LD_{50}$ value determined for each vector. *p<0.05 versus Ar6pAE2fF by t-test. n=number of replicates.

7.2.2 Summary

The cell cytopathic effect of the three modified oncolytic vectors was more closely related to one another than to the Ar6pAE2fF vector. The insertion of the adenovirus E3 region or the deletion of the E1b-19k gene in the parental Ar6pAE2fF genome effectively increases the killing of tumor cell lines while decreases the killing of normal primary cell cultures. The combination of the E3 insertion and E1b-19k deletion in the Ar6pAE2fF genome appeared to provide no synergistic effects in cell killing activity.

7.3 In vitro vector replication

The selectivity of the oncolytic vector production in tumor versus primary cell cultures was determined. Briefly, the tumor cell line H-460 and primary non-tumor cell PREC were infected with identical doses of the adenovirus vectors. After three and six days, cells were harvested and crude lysate were titered by a $TCID_{50}$ assay on AE12a indicator cells (Gorziglia et al., 1996. Elimination of both E1 and E2a from adenovirus vectors further improves prospects for in vivo human gene therapy. J. Virol 6,4173-4178).

7.3.1 Results

The vector titers by Ar6pAE2fE3F, Ar6pAE2fdl19kE3F and Ar6pAE2fdl19kF were 10, 3 and 2.4-fold higher respectively than the level observed for Ar6pAE2fF at day six post-infection (Table 18). For the oncolytic vector containing the entire E3 region or E1 B-19k deletion or both modifications, the titers of day 6 increased >250-fold as compared with the titers of day three. For the Ar6pAE2fF vector the difference in titer between day three and six was only 25-fold. For the primary non-tumor cell, the three new oncolytic vectors produced slighty less virus than Ar6pAE2fF vector.

**Table 18.** Vector production on tumor cell lines and normal cells

| | H460 | | SAEC | |
|---|---|---|---|---|
| Vector | Day 3 | Day 6 | Day 3 | Day 6 |
| Ar6pAE2fF | $1.2x10^6$ $\pm 9.1x10^5$ | $2.9x10^7$ $\pm 1.2x10^7$ | $5.2x10^6$ $\pm 4.0x10^6$ | $4.0x10^6$ $\pm 1.9x10^6$ |
| Ar6pAE2fE3F | $9.6x10^6$ $\pm 2.8x10^5$ | $2.6x10^8$ $\pm 1.1x10^8$ | $1.1x10^6$ $\pm 1.6x10^5$ | $2.5x10^6$ $\pm 1.7x10^4$ |
| Ar6pAE2fdl19K | $1.6x10^5$ $\pm 3.65x10^4$ | $7x10^7$ $\pm 8.8x10^7$ | $4.1x10^5$ $\pm 6.7x10^4$ | $6.3x10^5$ $\pm 1.9x10^5$ |
| Ar6pAE2fE3dl19kF | $2x10^5$ $\pm 1.3\times 10^5$ | $8.7x10^7$ $\pm 1.1\times 10^7$ | $7.3x10^5$ $\pm 2.7\times 10^4$ | $1.2x10^6$ $\pm 1.0\times 10^5$ |

Indicated cells were infected at a dose of 1 particle/cell. Data shown are plaque-forming units (pfu)/ml, as determined by $TCID_{50}$ assay at the indicated days post-infection (O'Reilly DR, et al., 1994 Appendix 6 In: Baculovirus Expression Vectors: A Laboratory Manual. Oxford: Oxford University press, pp132-134).

7.3.2 Summary

In the tumor cell lines examined, the reconstitution of the E3 region or the deletion of the E1 B-19k gene or the combination of both resulted in production of higher amounts of virus than the parental Ar6pAE2fF vector. In primary non-tumor cell the Ar6pAE2fF-derivative vectors replicated less efficiently than the parental vector.

Example 8: A tumor selective adenoviral vector (Ar6pAE2fmGmF) expressing murine granulocyte-macrophage colony stimulating factor (mGM-CSF)

8.1.1 Construction of pAr6pAE2fmGmF

The mGM-CSF cDNA has been cloned into an Xbal site located at the site of a major deletion in the E3 region, the result of which is that the only remaining E3 product is the 12.5kDa protein.

Since it has been demonstrated that GM-CSF exhibits species specificity, the mouse GM-CSF vector was prepared in order to facilitate in vivo mouse modeling studies.

The pDRF2 plasmid (Figure 13) was used to construct an adenovirus right donor plasmid, pDR2mGmF (Figure 14) containing an insertion of the mGM-CSF cDNA in the Xbal site of the pDR2F plasmid. Mouse GM-CSF cDNA was obtained from Gerald McMahon in 1992 (Sandoz, East Hanover, NJ) in plasmid pXMT2-muCSF. The plasmid pXMT2-muCSF is described in PCT publication WO 96/33746, published October 31, 1996. The mGM-CSF cDNA was excised from pXMT2-muCSF and used to create the pG1mGmSvNa plasmid (Figure 15). Plasmid pG1mGm was constructed from the directed ligation of Notl/Sall fragments of pG1 (4640bp, additionally treated with CIP) and pG1mGmSvNa (634bp carrying mGM-CSF cDNA). The pG1 plasmid is described in U.S. Patent No. 5,672,510. The pG1mGm plasmid was used to generate plasmid pKSmGm as follows:

I. Vector pBCKS+ (Stratagene Inc., La Jolla, CA) was digested with EcoRV, treated with CIP and gel purified.
II. Mouse GM-CSF (634bp fragment) was liberated from plasmid pG1mGm by digestion with Notl and Sall, filled in with Klenow Fragment DNA Polymerase I in the presence of dNTP and gel purified.
III. The insert and vector DNA were blunt ligated and transformed into E. coli HB101 competent cells to generate pKSmGm.

The pDR2F plasmid contains the adenoviral packaging signal at the right ITR followed by a Swal site. The packaging signal was moved from the left ITR to the right ITR using PCR primers (ITRF-2/PkgR3) designed to amplify the left ITR and the packaging signal through base pair 393 of the native Ad5 sequence. The PCR product was flanked with ClaI-SwaI restriction sites at the 5'-end and with a PacI restriction site at the 3'-end. This PCR product was digested with PacI/ClaI and cloned into p5FloxHRL, which is a derivative prePac (Gorziglia et al. "Generation of an adenovirus vector lacking E1, E2a, E3, and all of E4 except open reading frame 3," J Virol, 73 (7):6048-6055 (1999). pDR2F is the right end donor plasmid utilized to generate a plasmid that contains the entire adenovirus genome. pDR2F has a lox P site within the XbaI site that partially alters the 3' E3 14.7 kDa protein amino acid sequence.

Plasmid pDR2mGmF was constructed as follows:

I. Plasmid pDR2F was prepared by digestion with XbaI, filled with Klenow Fragment DNA Polymerase I in the presence of dNTP and treated with CIP to remove 5' phosphate groups. The digest was electrophoresed in a 0.8% agarose gel and the 10877bp fragment was purified using GeneClean II (BIO101, Inc., CA).
II. Mouse GM-CSF insert cDNA was isolated from pKSmGm by digestion with AvaI and filled with Klenow Fragment DNA Polymerase I in the presence of dNTP. The 641bp fragment was recovered from an agarose gel and purified with GeneClean II.
III. The insert and vector DNA were ligated and transformed into E. coli HB101 competent cells to generate pDR2mGmF.

Plasmid clones were screened using restriction enzyme digestion. The resulting sequence (Figure 17) for bp 7878 through 8826, containing the mGM-CSF insert in pDR2mGmF was as predicted.

The pAr6pAE2fmGmF plasmid (Figure 18) was generated as follows. Plasmid pDR2mGmF was digested with FspI and SpeI; the 9284bp fragment containing the mGM-CSF insertion was recovered from an agarose gel and purified using a GeneClean II kit. Fifty to 100ng of the DNA fragment was co-transformed into E. coli BJ5183 competent cells with 100ng of SrfI/PacI digested pAr6pAE2fF plasmid DNA (Figure 18). Transformed BJ5183 cells were plated onto LB agar plates containing 100□g/ml ampicillin and allowed to grow at 37°C overnight. Colonies were inoculated into 2ml LB medium containing 100μg/ml ampicillin and incubated at 30°C for 4-5 hours at 250 rpm. The plasmid DNA was then isolated from the BJ5183 culture using the alkali-lysis method described by Sambrook, et al. (Molecular cloning: A laboratory manual, Second Editions, pp 9.31-9.52, 1989) with minor modifications. Briefly, 1.5ml of BJ5183 culture was transferred into a microfuge tube, after centrifugation at 14,000 rpm for 1 minute at room temperature, the medium was aspirated and the bacterial pellet was resuspended in 100μl of ice-cold Solution I provided in the Plasmid DNA Mini-prep Kit from Qiagen. One hundred μl of Solution II (Qiagen) was added into each tube and mixed by inverting 5 times. After incubation at room temperature for 3 minutes, 100μl of N3 buffer (Qiagen) was added into each tube. Sample was mixed by inverting 5 times and centrifuged immediately at 14,000 rpm for 10 minutes at room temperature. The supernatant was transferred to a fresh tube and 1μl of glycogen (1μg/μl, Boehringer Mannheim) and 600μl of ethanol were added to precipitate DNA. Sample was kept at -20°C for at least 1 hour, and plasmid DNA was recovered by centrifugation at 14,000 rpm for 10 minutes at room temperature. Plasmid DNA was resuspended in 15μl of dH20 and 10μl was applied to a 0.8% agarose gel containing ethidium bromide. One microliter of mini-preps that contained large plasmids (i.e., >30kbp) were used to transform 100μl of E. coli HB101 competent cells (Life Technologies Inc.). The efficiency of homologous recombination was observed to be higher when the transformation was carried out immediately after isolation of the mini-prep. The plasmid DNA obtained from the second transformation was analyzed by restriction enzyme digestion and plasmids containing the correct restriction fragments were selected for production of the viral vector.

8.1.2 Viral Vector Generation of Ar6pAE2fmGmF

The AE1-2a clone S8 cells (S8 cells) (Gorziglia et al., 1996. Elimination of both E1 and E2a from adenovirus vectors further improves prospects for in vivo human gene therapy. J. Virol. 6,4173-4178) were cultured in IMEM containing 10% heat inactivated FBS. Two □g of SwaI-digested plasmid pAr6pAE2fmGmF (Figure 18) was transfected into S8 cells and cultured in a 6-well plate using the LipofectAMINE-PLUS reagent system (Life Technologies, Rockville, MD). After 7 days incubation at 37°C, 5% $CO_2$, humidified, the viral vector was further amplified and purified by CsCl gradient. Viral vector concentrations were determined by spectrophotometric analysis (Mittereder, et al., "Evaluation of the Concentration and Bioactivity of Adenovirus Vectors for Gene Therapy," J. Virol., 70:7498-7509 (1996)).

The viral genome DNA of Ar6pAE2fmGmF was isolated by incubation of 100□l viral vector solution ($4x10^{12}$ viral particles/ml) with 5□l of 10% SDS and 20□l of Proteinase K (10mg/ml) at 37°C overnight, followed by phenol/chloroform extraction and ethanol precipitation. The viral genome DNA was digested with restriction enzymes EcoRV, SalI, XbaI plus BamHI or BspeI and loaded to a 0.8% agarose gel. Southern blot analysis of the viral genome was then carried out by transferring the DNA fragments from the restriction endonuclease digestions to a nitrocellulose membrane (VWR, #28151-113) and hybridizing with a 32P-labeled mGM-CSF probe using standard procedures (Sambrook, et al., Molecular cloning: A laboratory manual Second Editions, pp 9.31-9.52, 1989). To generate the DNA probe, a 509 bp DNA fragment was PCR amplified using a pair of primers, 5'-CACCCTTGCGTCAGCCCACGGTACCATGGCCCACGAGAGAAAGGC-3' (SEQ ID NO:25) and 5'-CCTTAAAATCCACCTTTTGGGTTCATTTTTGGACTGGTTTTTTGC-3' (SEQ ID NO:26), using the pDR2mGmF plasmid DNA as the template. The resulting 509bp DNA fragment contains the 461 bp coding sequence for mGM-CSF, 26bp of adenoviral E3 sequence at the 5' end and 22bp of adenoviral E2 sequence at its 3' terminus. The 32P-labeled DNA probe was synthesized using the random primer extension method and 32P-dCTP. The specific activity of the probe was about 100□Ci/50 ng template DNA.

8.2 In vitro cytotoxicity (MTS Assay)

To evaluate the ability of the Ar6pAE2fmGmF vector to kill cells, MTS assays were performed.

8.2.1 Day -1 Plate set-up

One day prior to viral infection, cells are plated in 96-well dishes in a 90 µl volume of growth media using a multichannel pipettor. The number of cells per well is determined empirically for each cell type such that they are 60-80% confluent at the time of viral infection. This is typically 5,000 to 10,000 cells per well. For H460 cells, 10,000 cells per well were seeded. The outer perimeter wells are filled with 200 µl of dPBS to prevent media evaporation leading to edge effects; only the inner 60 wells are used in the assay. Three wells are filled with 90 µl of media only (blank) and three wells are used as uninfected controls.

8.2.2. Day 0 Virus infection

The next day, cells are infected with adenoviral vectors. The adenoviruses to be tested are four-fold serially diluted in growth media over a dose-range that yields a sigmoidal dose response curve. This dose-range is cell- and vector-specific and is usually between 100 PPC to 10,000 PPC. Nine serial dilutions of each virus are added in a 10 µl volume across the plate, starting with the highest dose in row B, column 3. One virus is added per row, in some cases in duplicate rows, such that three to six viruses are tested on one plate. The positive control virus Ad*dl*327 is tested in every experiment. To the blank wells and the control wells, 10 µl of media without virus is added at the time of infection to bring the total volume of all wells to 100 µl. The plate is then incubated at 37°C in a humidified 5% $CO_2$ incubator for seven days.

8.2.3. Day 7 MTS assay

The MTS assay is performed according to the manufacturer's instructions. The CellTiter 96® Aqueous Assay uses the tetrazolium compound (3-(4,5-dimethylthiazol-2-yl)-5-(3-carboxymethoxyphenyl)-2-(4-sulfophenyl)-2H-tetrazolium, inner salt; MTS) and the electron coupling reagent, phenazine methosulfate (PMS). These reagents are provided by the supplier in 100 ml and 5 ml solutions respectively (Catalog #G5430). The MTS Solution and the PMS Solution are thawed at room temperature (about 90 minutes) or in a 37°C waterbath (about 10 minutes). For each 96-well dish to be tested, 2 ml of MTS Solution is transferred to a disposable test tube. To this tube, 100 µl of PMS Solution per 2 ml of MTS Solution is added immediately before addition to the culture plate containing cells. The tube is gently swirled to ensure complete mixing of the combined MTS/PMS solution. A 20 µl aliquot of the combined MTS/PMS solution is pipetted into the medium in each well of the 96-well assay plate using a repeating pipettor. The plate(s) are incubated for 1-4 hours at 37°C in a humidified 5% $CO_2$ incubator. The total time required is determined by the absorbance levels measured and differs depending on cell type and seeding density. Absorbance units between approximately 0.5 and 1.5 are desirable with background levels of approximately 0.1 to 0.2 absorbance units. The absorbance is recorded at 490nm using a microtiter plate reader (Spectra Max Plus Model from Molecular Devices Corp., Sunnyvale, CA). Since color continues to develop throughout the time period, all plates should be read in rapid succession and should not be incubated longer than 4 hours.

8.2.4 Data analysis

The raw absorbance unit (AU) data from the plate reader is exported as a tab-delimited text file and imported into Microsoft Excel as a 6 row by 10 column spreadsheet for further manipulation. The Background is determined by calculating the average of the absorbance units (AU) from the three blank wells (media only). The average AU of the three "cells only" wells is determined and the Background subtracted from this to generate the Control value. The Background value is subtracted from each sample AU. The net sample AU value is then divided by the Control value and multiplied by 100 to generate Percent Control values for each sample well. If duplicates are done, mean Percent Control values are calculated.

$$\text{Background} = \text{Average (blank AU)}$$

$$\text{Control} = \text{Average (cells only AU)} - \text{Background}$$

$$\text{Percent Control} = [(\text{Sample AU} - \text{Background})/\text{Control}] \times 100$$

The dose of virus in particles per cell (X value) is plotted versus the corresponding Percent Control (Y value) in a semi-log scale. If the data forms a sigmoidal dose-response curve, the data can be used to calculate LD50. The LD50 was calculated using the GraphPad Prism 3.0 program.

LD50 is defined as the dose of vector in particles per cell which corresponds to one half the difference between the maximal (LD100 plateau) and minimal (LD0 plateau) response. Vector dose versus Percent Control values are imported into a template in the GraphPad Prism 3.0 program. In this template, a sigmoidal dose-response (variable slope) equation is used to fit a curve to the data points.

$$Y = \text{Bottom} + (\text{Top} - \text{Bottom})/(1 + 10^{\text{logEC50}-X\cdot\text{HillSlope}})$$

In this equation, the variable Bottom is the Y value at the bottom plateau and the variable Top is the Y value at the top plateau. The LogEC50 (effective concentration, 50%) is the X value when the response is halfway between Bottom and Top (LogLD50). The variable HillSlope describes the steepness of the curve. This variable, called the Hill slope, the slope factor, or the Hill coefficient, is negative when the curve decreases as X increases. A standard sigmoidal dose-response curve has a Hill Slope of 1.0. When the HillSlope is less than 1.0, the curve is more shallow. When HillSlope is greater than 1.0, the curve is steeper. The Hill slope has no units. Once a curve has been fit to the input data, the LD50 value for each dose-response curve is provided as output data from the Prism program. MTS assay results are reported as mean LD50 values ± standard deviation of three or more replicates. Each replicate represents a separate 96-well dish.

8.2.5 Cell lines and viruses tested

Several human and rodent tumor cell lines were tested, including human tumor cell lines H460 (non-small cell lung carcinoma, NCI-H460, ATCC #HTB-177) and Hep3B (hepatocellular carcinoma, Hep3B2.1-7, ATCC #HB-8064), a mouse tumor cell line RM-1 (prostate carcinoma, Nasu et al., 1999) and a rat tumor cell line McA-RH-7777 (hepatoma, ATCC #CRL-1601). The mGM-CSF armed vector Ar6pAE2fmGmF ($5.2\text{x}10^{12}$ viral particles/ml), an E1A-positive control vector Add/327 ($4.7\text{x}10^{12}$ viral particles/ml), an E1A-negative control vector Addl312 ($5.6\text{x}10^{12}$ viral particles/ml lot), and an unarmed oncolytic adenoviral vector (OAV) Ar6pAE2fF ($5.0\text{x}10^{12}$ viral particles/ml, in which E1A expression is driven by the E2F-1 promoter as in the mGM-CSF armed vector) were included in the MTS assays.

8.3 Results

A tumor replication selective adenoviral vector, Ar6pAE2fmGmF, expressing murine granulocyte-macrophage colony stimulating factor (mGM-CSF) was constructed for preclinical efficacy and toxicity modeling and its cytotoxic properties were characterized in vitro. The vector harbors a major E3 deletion such that the only E3 gene remaining codes for the E3 12.5kDa protein. The Ar6pAE2fmGmF oncolytic vector, "armed" with the ability to produce GM-CSF in tumors, was generated by cloning the mGM-CSF cDNA into the region of E3 deleted in Ar6pAE2fF from example #1. In Ar6pAE2fmGmF, the expression of mGM-CSF is driven by the E3 promoter that is in turn controlled by the adenoviral E1A protein. E1A expression in both Ar6pAE2fmGmF and Ar6pAE2fF is driven by the tumor selective E2F-1 promoter active in Rb-pathway disregulated cells. MTS cytotoxicity assay data demonstrated that the mGM-CSF armed Ar6pAE2fmGmF viral vector retains the in vitro oncolytic properties of the parental Ar6pAE2fF vector. The Ar6pAE2fmGmF viral vector was shown to kill human and murine cell lines at LD50 values very close to those of the parental Ar6pAE2fF vector (Figure 19). The mouse and rat tumor cells are less sensitive to killing by the human Ad5 derived oncolytic vectors than human tumor cell lines.

8.4 Summary

An oncolytic adenovirus, Ar6pAE2fmGmF that expresses mouse GM-CSF has been constructed. The mGM-CSF cDNA was inserted into an XbaI sequence located at the site of a major deletion of the adenoviral E3 region. The resulting vector lacks the E3-6.7kDa, gp19kDa, 11.6kDa (ADP), 10.4kDa (RIDα), 14.5kDa (RIDβ) and 14.7kDa proteins (E3 region reviewed in Wold, et al., E3 transcription unit of adenovirus. in "The Molecular Repertoire of Adenoviruses I," ed. by W Doerfler and P Bohm, pp 237-274, Springer-Verlag, Berlin. 1995) The pDR2mGmF plasmid also has a lox P site within the XbaI site that partially alters the 3' E3 14.7 κDa protein amino acid sequence. Only the 12.5kDa open reading frame is retained in the vector. Restriction digests and sequencing confirmed the integrity and orientation of the mGM-CSF cDNA insert contained in the adenoviral right end pDR2mGmF plasmid. This plasmid was co-transformed into the recombinase positive E. coli strain BJ5183 to generate the pAr6pAE2fmGmF 37.8kb plasmid that was then transfected into AE1-2a clone S8 cells to generate the Ar6pAE2fmGmF oncolytic adenoviral vector. Restriction digestion of this vector gave the predicted pattern. The Ar6pAE2fmGmF vector retained the in vitro oncolytic properties of the parental Ar6pAE2fF vector. Further studies have confirmed the production of substantial quantities of biologically active mGM-CSF following infection of tumor cells (Table 19).

**Table 19**. Comparison of mouse GM-CSF biological activity (proliferation) to mouse GM-CSF total protein (ELISA).

| Ar6pAE2fmGmF, Particles/cell | mGM-CSF activity, ng/$10^6$ cells/24 hours, detected by proliferation of MC/9 cells | mGM-CSF total protein, ng/$10^6$ cells/ 24 hours, detected by ELISA |
|---|---|---|
| 1000 | 803 | 420 |
| 250 | 219 | 160 |
| 50 | 98 | 40 |
| 10 | 20 | 15 |
| 2 | 22 | <7.8 |
| Serum-free supernatants from H460 cells infected with Ar6pAE2fmGmF at the indicated particles per cell were assayed for their ability to induce the proliferation of mouse MC/9 cells, or in an ELISA to detect mouse GM-CSF. In each assay, standard curves were generated using recombinant mouse GM-CSF and values of unknowns were calculated from the standard curves. Data are reported with identical units to facilitate a direct comparison. | | |

Example 9: A tumor selective adenoviral vector (Ar6pAE2fhGmF) expressing human granulocyte-macrophage colony stimulating factor (hGM-CSF)

9.1.1 Construction of pAr6pAE2fhGmF

An adenovirus right donor plasmid, pDR2hGmF containing the human GM-CSF cDNA in the XbaI site of the pDR2F plasmid was constructed.

Plasmid pDR2hGmF was constructed as follows:

I. Plasmid pG1NaSvGm was constructed as follows: The 466 base pair human GM-CSF cDNA was derived by Bgl II/Hind III digestion of the PCR amplified DNA fragment using plasmid pGMCSF as template. pGMCSF is a pBR322 based plasmid, which contains human GM-CSF. Plasmid pG1NaSvGm was isolated following the directed ligation of Bgl II/Hind III fragments of hGM-CSF cDNA (466bp) and pG1 NaSvBg (Figure 16, CIP treated, 5844 bp). The plasmid pG1NaSvBg is described in U.S. Patent No. 5,672,510.

II. Human GM-CSF (466 bp fragment) (Figure 20) was liberated from plasmid pG1NaSvGm by digestion with Bgl II and Hind III, filled in with Klenow Large Fragment of DNA Polymerase I in the presence of dNTP. The 466bp fragment was recovered from an agarose gel and purified with a GeneClean II kit (BIO101, Inc., CA), and was used as the insert DNA in the ligation reaction in step IV.

III. Plasmid pDR2F (as described in example #8 (Section 8.1.1)) was prepared by digestion with XbaI, filled with Klenow Large Fragment of DNA Polymerase I in the presence of dNTP and treated with CIP to remove 5' phosphate groups. The digest was electrophoresed in a 0.8% agarose gel and the 10877bp fragment was purified using a GeneClean II kit. The purified DNA fragment was used as the vector DNA in ligation reaction in step IV.

IV. The insert and vector DNA were ligated and transformed into E. coli HB101 competent cells to generate pDR2hGmF. (Figure 21)

The pAr6pAE2fhGmF plasmid was generated as follows. Plasmid pDR2hGmF was digested with Fspl and Spel; the 9109 bp fragment containing the hGM-CSF cDNA was recovered from an agarose gel and purified using a GeneClean II kit. Fifty to 100ng of the DNA fragment was co-transformed into E. coli BJ5183 competent cells with 100ng of Srfl/Pacl digested pAr6pAE2fF plasmid DNA (Figure 21). Transformed BJ5183 cells were plated onto LB agar plates containing 100μg/ml ampicillin and allowed to grow at 37°C overnight. Colonies were screened and the correct plasmid was isolated as described in example #8 (Section 8.1.1).

9.1.2 Viral Vector Generation of Ar6pAE2fhGmF

Viral vector was produced by transfecting 2μg of Swa I-digested plasmid pAr6pAE2fhGmF using the lipofectamine-plus reagent system (Life Technologies, Rockville, MD) into S8 cells and cultured in a 6-well plate. Virus was propagated and viral genomic DNA of Ar6pAE2fhGmF was analyzed as described in example #8 (Section 8.1.2). The viral genome DNA was digested with restriction enzymes EcoRV, Sall, Xbal plus BamHI or Bspel and loaded to a 0.8% agarose gel. Southern blot analysis of the viral genome was performed by transferring the DNA fragments from the restriction endonuclease digestions to a nitrocellulose membrane (VWR, #28151-113) and hybridizing with a 32P-labeled hGM-CSF probe using standard procedures (Sambrook, et al., Molecular cloning: A laboratory manual Second Editions, pp 9.31-9.52, 1989). To generate the DNA probe, a 482 bp DNA fragment was PCR amplified with a pair of primers,
5'-CACCCTTGCGTCAGCCCACGGTACCATGTGGCTGCAGAGCCTGCTGC-3'(SEQ ID NO:27)
5'-CCTTAAAATCCACCTTTTGGGTTCACTCCTGGACTGGCTCCCAGC-3' (SEQ ID NO:28),
using the pDR2hGmF plasmid DNA as the template. The resulting 482bp DNA fragment contains the 434bp coding sequence for human GM-CSF, 26bp of adenoviral E3 sequence at the 5' end and 22bp of adenoviral E2 sequence at its 3' terminus. The 32P-labeled DNA probe was synthesized using the random primer extension method and 32P-dCTP. The specific activity of the probe was approximately 100μCi/50ng template DNA.

9.1.3 Results

Following cloning, orientation of pDR2hGmF plasmids was screened with Sal I digestion. The structure was confirmed by further restriction enzyme analysis which showed the expected patterns. The cloning scheme and the structure of the pAr6pAE2fhGmF large plasmid are summarized in Figure 21. The integrity of pAr6pAE2fhGmF was confirmed by Sal I digestion.
After transfection of S8 cells with Swa I digested pAr6pAE2fhGmF plasmid DNA, the Ar6pAE2fhGmF viral vector was isolated and amplified. Viral genomic DNA of Ar6pAE2fmGmF was isolated and digested with restriction enzymes. The restriction enzyme digests showed the expected pattern. The integrity of the human GM-CSF cDNA insert was confirmed by sequencing bp 28536 to 29273, as shown in Figure 20 (SEQ ID NO:19).

9.2 In Vitro Cytotoxicity

To evaluate the ability of the Ar6pAE2fhGmF vector to kill cells, MTS assays were performed as described in example 8. Several human and rodent tumor cell lines were tested, including human tumor cell lines H460 (non-small cell lung carcinoma, NCI-H460, ATCC #HTB-177) and Hep3B (hepatocellular carcinoma, Hep3B2.1-7, ATCC #HB-8064), a mouse tumor cell line CMT-93 (rectal carcinoma, ATCC #CCL-223) and KLN 205 (mouse squamous cell carcinoma, ATCC #CRL-1453). Ar6pAE2fhGmF (the human GM-CSF armed vector, $2.4x10^{12}$ viral particles/ml), Ad*dl*/327 (an E1A-positive control vector, $4.7x10^{12}$ viral particles/ml,), Add/312 (an E1A-negative control vector, $5.6x10^{12}$ viral particles/ml), Ar6pAE2fF (an unarmed OAV, $5.0x10^{12}$ viral particles/ml), and Ar6pAE2fmGmF (the mouse GM-CSF armed vector, 5.2 $x10^{12}$ viral particles/ml) were included in the MTS assays. The results obtained from the MTS assays were subjected to sigmoidal dose-response curve fit analysis using the Prism GraphPad software. The program was used to calculate LD50; the effective concentration at which 50% of the cells were dead compared to untreated control cells 7 days after infection, as described in Example 8.

9.2.1 Results

The Ar6pAE2fhGmF viral vector was shown to kill human and murine cell lines at LD50 values very close to those of the parental Ar6pAE2fF vector (Figure 22). The mouse and rat tumor cells are less sensitive to killing by the human Ad5 derived oncolytic vectors than human tumor cell lines. Further studies have confirmed the production of substantial quantities of biologically active hGM-CSF following infection of tumor cells (Table 20).

**Table 20**. Comparison of human GM-CSF biological activity (proliferation) to human GM-CSF total protein (ELISA).

| Ar6pAE2fhGmF, Particles/cell | hGM-CSF activity, ng/$10^6$ cells/24 hours, detected by proliferation of TF-1 cells | HGM-CSF total protein, Ng/$10^6$ cells/ 24 hours, detected by ELISA |
|---|---|---|
| 500 | 2536 | 1500 |
| 250 | 846 | 500 |
| 100 | 472 | 344 |
| 50 | 149 | 70 |
| 10 | 34 | 20 |
| Serum-free supernatants from H460 cells infected with Ar6pAE2fhGmF at the indicated particles per cell were assayed for their ability to induce the proliferation of human TF-1 cells, or in an ELISA to detect human GM-CSF. In each assay, standard curves were generated using recombinant human GM-CSF and values of unknowns were calculated from the standard curves. Data are reported with identical units to facilitate a direct comparison. | | |

9.2.2 Summary

An oncolytic adenovirus, Ar6pAE2fhGmF that expresses human GM-CSF has been constructed. The hGM-CSF cDNA was inserted into an Xba I site located at the site of a major deletion of the adenoviral E3 region. The resulting vector lacks the E3-6.7kDa, gp19kDa, 11.6kDa (ADP), 10.4kDa (RIDα), 14.5kDa (RIDβ) and 14.7kDa proteins (E3 region reviewed in Wold, et al., 1995). Ar6pA2fhGmF also has a lox P site within the Xbal site that partially alters the 3' E3 14.7 kDa protein amino acid sequence. Only the 12.5kDa open reading frame is retained in the vector. Restriction enzyme digestions confirmed the integrity and orientation of the hGM-CSF cDNA insert contained in the adenoviral right end pDR2hGmF plasmid. This plasmid was co-transformed into the recombinase positive E. coli strain BJ5183 to generate the pAr6pAE2fhGmF 37.6kb plasmid that was then transfected into AE1-2a clone S8 cells to generate the Ar6pAE2fhGmF oncolytic adenoviral vector. Restriction digestion and partial sequencing of this vector gave the predicted pattern. The Ar6pAE2fhGmF vector retained the in vitro oncolytic properties of the parental Ar6pAE2fF vector and induced the production of substantial quantities of biologically active GM-CSF.

9.3 In Vivo Anti-tumor Efficay of Ar6pAE2fhGmF and Ar6pAE2fmGmF

9.3.1 Studies using H460 non-small cell lung carcinoma xenograft model

The H460 ($Rb^+$, $p16^-$, $p53^+$, Kataoka et al., "Downregulation of bcl-2 is associated with p16INK4 mediated apoptosis in non-small cell lung cancer cells," Oncogene 19(12):1589-1595 (2000)) human non-small cell lung carcinoma cell line provides a rigorous *in vivo* test of oncolytic vector function. These tumor cells have one of the highest LD50 values (370$\pm$108ppc) in the MTS assay of any human tumor cell line tested. This cell line was used to examine whether the addition of mouse GM-CSF in Ar6pAE2fmGmF (mouse version of NVP-OGM527) confers an added benefit to the treatment of pre-existing tumors. These studies were performed in T cell deficient nude mice. Thus, any added benefit would be due to the stimulation of non-immune anti-tumor mechanisms (e.g., anti-angiogenesis and/or inflammation). Pre-established H460 tumors (average 92$\pm$50$mm^3$) growing on the flanks of nude mice were injected with $2x10^{10}$ or $1x10^{11}$ of the indicated viral particles or PBS (n = 10/group) five times on days 10, 12, 14, 17 and 19. Tumors were measured twice weekly for 49 days and group tumor volumes were compared by repeated-measures one way analysis of variance (RM-OW-ANOVA) using Tukey's test.

9.3.1.1 Results

Both doses of the Ar6pAE2fF and Ar6pAE2fmGmF vectors significantly inhibited the growth of H460 tumors compared to saline-treated tumors, and the vectors significantly inhibited tumor growth compared to the E1A-negative control vector Add/312 at the lower $2x10^{10}$ particles dose. At the higher dose ($1x10^{11}$ particles/injection), only the Ar6pAE2fmGmF vector significantly inhibited tumor growth, as compared to the Add1312 vector, evidence of added benefit due to species-relevant GM-CSF expression by this vector (Figure 23).

The anti-tumor efficacy of vectors was evaluated by calculating T/C (treated/control) ratios as shown in Table 21. The data show that all adenoviral vectors at both doses elicited significant anti-tumor activity compared to saline-treated tumors. In addition, the Ar6pAE2fmGmF vector elicited significant anti-tumor activity at both low and high doses using the Ad*dl*312 control vector as the denominator value, whereas the Ar6pAE2fF vector treatment was significant only at the low dose. Unexpectedly in the nude immunodeficient mice, the data demonstrate that the Ar6pAE2fmGmF vector that expresses mouse GM-CSF induced more efficacious anti-tumor activity in vivo compared to the Ar6pAE2fF vector. In addition, tumor free survival was also enhanced such that 50% of Ar6pAE2fmGmF treated animals were free of tumor at day 49.

**Table 21**. T/C values and % tumor free survival of mice bearing H460 tumors

| Treatment Group (particles) | T/C Fraction vs PBS | T/C Fraction vs Ad*dl*312 | % Tumor free at day 49 | |
|---|---|---|---|---|
| Ad*dl*312: $2x10^{10}$ | 0.39* | na | 0 | |
| Ad*dl*312: $1x10^{11}$ | 0.13* | na | 0 | |
| Ad*dl*327: $2x10^{10}$ | 0.12* | 0.23* | 10 | ns |
| Ad*dl*327: $1x10^{11}$ | 0.08* | 0.39 | 0 | |
| Ar6pAE2fF: $2x10^{10}$ | 0.14* | 0.3* | 20 | ns |
| Ar6pAE2fF: $1x10^{11}$ | 0.11* | 0.67 | 10 | |
| Ar6pAE2fmGmF: $2x10^{10}$ | 0.29* | 0.25* | 40 | p<.001 |
| Ar6pAE2fmGmF: $1x10^{11}$ | 0.04* | 0.15* | 60 | |
| PBS | Na | na | 0 | |

T/C = mean of treatment tumor volume divided by tumor volume of control group at study day 21 for PBS, or over the Ad*dl*312-treated group on study day 31 (columns 2 and 3, respectively). Asterisks indicate significant differences (p<0.05) compared to the respective control by RM-OW-ANOVA. na; not applicable. Percent tumor free animals at day 49 were compared in the last column. Significantly increased tumor free survival was observed in mice treated with Ar6pAE2fmGmF (armed with mouse GM-CSF) as compared to the Ad*dl*312 treated group (p<.001, Fisher's Exact Test). In contrast, tumor free survival in the Ar6pAE2fF or Ad*dl*327 groups was not statistically greater than the Ad*dl*312 group. ns; not significant.

9.3.2 Studies using Hep3B hepatocellular carcinoma xenograft model

The Hep3B ($Rb^-$, $p16^+$, $p53^-$, Spillare et al., "Suppression of growth in vitro and tumorigenesis in vivo of human carcinoma cell lines by transfected p16INK4," Mol Carcinogenesis 16(1):53-60 (1996); Farshid et al., "Alterations of the RB tumor suppressor gene in hepatocellular carcinoma and hepatoblastoma cell lines in association with abnormal p53 expression," J Viral Hepat 1(1):45-53 (2000)) human hepatocellular carcinoma cell line was also used to test oncolytic vector function in vivo. Hep3B tumor cells are much more sensitive than H460 to the in vitro cytolytic effects of oncolytic adenoviruses (LD50 value 0.0134±0.0199ppc). As with H460 tumors, pre-existing xenografted Hep3B tumors were analyzed to determine whether the addition of mouse GM-CSF in Ar6pAE2fmGmF confers an added benefit in vivo. The slower growth of the Hep3B tumor afforded the opportunity to perform a dose response study beginning with a very low dose of virus. Pre-established Hep3B tumors (average 135±51 $mm^3$) growing on the flanks of nude mice were injected with $2x10^7$, $2x10^8$ or $2x10^9$ particles of the indicated viral vectors or PBS (n = 10/group) five times on days 15, 18, 20, 22 and 25. Tumors were measured twice weekly for 47 days and group volumes were compared by repeated-measures one way analysis of variance (RM-OW-ANOVA) using Tukey's test.

9.3.2.1 Results

The unarmed Ar6pAE2fF vector and the human GM-CSF-armed Ar6pAE2fhGmF vector displayed similar anti-tumor effects at each vector dose tested, indicating that the human GM-CSF is not bioactive in the mouse, as expected, but the vector retains the selective oncolytic activity of the parental vector. No significant differences between these two vectors were observed at any dose or timepoint.

Unexpectedly, there was significant enhancement of anti-tumor activity with the mouse GM-CSF secreting Ar6pAE2fmGmF vector compared to the unarmed Ar6pAE2fF vector at the low $2x10^7$ vector particle dose beginning on day 34, indicating that intratumoral expression of mouse GM-CSF in Hep3B tumor-bearing mice induced a more potent anti-tumor effect than the unarmed oncolytic vector, thus presenting the possibility that a GM-CSF armed OAV can be administered to patients at a lower dose than an unarmed vector. In addition, because the nude mice are immunodeficient, an oncolytic adenovirus expressing human GM-CSF may have activity due to the GM-CSF in cancer patients whose immune systems are compromised by chemotherapy or radiotherapy. These data show that Ar6pAE2fhGmF retains the oncolytic capacity of the parental Ar6pAE2fF vector and that, when measured in the appropriate species, GM-CSF confers a significant benefit to the oncolytic vector, even in the immunodeficient nude mouse (Figure 24).

As was done in the H460 model, anti-tumor efficacy of vectors in the Hep3B model was evaluated by calculating T/C (treated/control) ratios (Table 22). Treatment of Hep3B tumors with each dose of the Ar6pAE2fF, Ar6pAE2fhGmF, or Ar6pAE2fmGmF vectors resulted in T/C ratios less than 0.5 using either PBS or the Ad*dl*312 control adenovirus as the comparator, except for mice treated with $2\times10^7$ particles/injection of Ar6pAE2fF (T/C=0.53). The data demonstrate a therapeutic benefit following treatment with oncolytic vectors. When the unarmed vector Ar6pAE2fF is used as the comparator, the data showed T/C values >0.5 for all doses of the Ar6pAE2fhGmF vector, whereas all doses of the Ar6pAE2fmGmF vector yielded T/C values <0.5. This evaluation demonstrates a superior therapeutic benefit from treatment with the GM-CSF-armed oncolytic vector compared to the unarmed oncolytic vector.

**Table 22**. T/C values and % tumor free survival of mice bearing Hep3B tumors

| Treatment Group (particles) | T/C Fraction vs PBS | T/C Fraction vs Ad*dl*312 | T/C Fraction vs Ar6pAE2fF | % Tumor free at day 47 | |
|---|---|---|---|---|---|
| Ad*dl*312, 2x10$^7$ | 0.79 | Na | Na | 0 | |
| Ad*dl* 312, 2x10$^8$ | 0.83 | Na | Na | 0 | |
| Ad*dl* 312, 2x10$^9$ | 0.48* | Na | Na | 0 | |
| Ar6pAE2fF, 2x10$^7$ | 0.42* | 0.53* | Na | 0 | ns |
| Ar6pAE2fF, 2x10$^8$ | 0.25* | 0.30* | Na | 20 | |
| Ar6pAE2fF, 2x10$^9$ | 0.19* | 0.40* | Na | 0 | |
| Ar6pAE2fhGmF, 2x10$^7$ | 0.27* | 0.34* | 0.74 | 0 | |
| Ar6pAE2fhGmF, 2x10$^8$ | 0.17* | 0.20* | 0.61 | 0 | ns |
| Ar6pAE2fhGmF, 2x10$^9$ | 0.18* | 0.36* | 0.95 | 0 | |
| Ar6pAE2fmGmF, 2x10$^7$ | 0.16* | 0.20* | 0.32* | 10 | p<.0 |
| Ar6pAE2fmGmF, 2x10$^8$ | 0.10* | 0.11* | 0.34 | 10 | |
| Ar6pAE2fmGmF, 2x10$^9$ | 0.10* | 0.21* | 0.46 | 40 | |

T/C fraction, mean tumor volume for treatment group compared to the PBS control group at study day 27; or the Ad*dl*312 vector control group determined at study day 31; or the Ar6pAE2fF vector-treated group determined at study day 34 (columns 2, 3 and 4, respectively). Asterisks indicate p<0.05 compared to the respective control group by RM-OW-ANOVA. na; not applicable.

Percent tumor free animals at day 47 were compared in column 5. Significantly increased tumor free survival was observed in mice treated with Ar6pAE2fmGmF (armed with mouse GM-CSF) as compared to the Ad*dl*312 treated group (p<.05, Fisher's Exact Test). In contrast, tumor free survival in the Ar6pAE2fF or Ar6pAE2fhGmF groups was not statistically greater than in the Ad*dl*312 group. ns; not significant.

Example 10: Construction and Characterization of E3 Containing Oncolytic Adenoviruses with GM-CSF cDNA Inserted in the Position of the Adenoviral E3-qp19 Gene

10.1.1 Construction of pAr6pAE2f(E3+,mGm,Dgi19b)F and pAr6pAE2f(E3+,hGm,Dql9b)F

PCR amplifications were performed in order to precisely replace the adenoviral E3-gp19 gene with the GM-CSF cDNA from the start codon to the stop codon. Three DNA fragments were generated for each vector and 3-fragment overlap PCR was employed to SOE (Site Overlap Extension) these together, thus generating a single DNA fragment. The DNA fragments were then digested with BsiWI and NotI and used to replace the BsiWI/NotI region containing the E3-gp19 gene of the Ar6pAE2fE3F vector (Figure 25).

In the native adenovirus, the reading-frame of the E3-6.7 gene overlaps the E3-gp19 gene (Figure 26a). To optimize GM-CSF expression from the position of the E3-gp19 gene, viral vectors with alternative junctions (Figure 26b) between the E3-6.7 gene and the GM-CSF cDNA were generated and tested for GM-CSF expression. These alternatives include:

I. Δgp19a: The GM-CSF cDNA (ATG to TGA) was directly swapped for the E3-gp19 coding sequence (ATG to TGA) without regard to the effect on the E3-6.7 stop codon. As a result of the cloning, fusion proteins between E3-6.7 and GM-CSF can be predicted. For the Δgp19a human GM-CSF vector, the fusion protein is likely to contain the full-length E3-6.7kDa protein with an additional 33 aa at the carboxyl-terminal end. For the Δgp19a mouse GM-CSF vector, the fusion protein would include an additional nine aa at its carboxyl-terminal end.

II. Δgp19b: A stop codon (TAA) for the E3-6.7 gene was inserted 5' to the start codon (ATG) of the GM-CSF cDNA to ensure the correct termination of the E3-6.7kDa protein. A Kozak sequence was also included between the end of the E3-6.7 gene and the start of the GM-CSF cDNA in order to enhance the expression of GM-CSF.

III. Δgp19c: The E3-6.7 stop codon (TGA) was restored by mutating the 4$^{th}$ nucleotide of GM-CSF to an A, thus resembling the native overlapping ATG and TGA codons. This single nucleotide mutation results in a single aa mutation in the signal peptides of both hGM-CSF and mGM-CSF. For hGM-CSF, the 2$^{nd}$ aa is mutated from tryptophan to arginine. For mGM-CSF, the 2$^{nd}$ aa is mutated from alanine to threonine. If GM-CSF can be secreted, it is unlikely that the mutation will affect the biological activity of either hGM-CSF or mGM-CSF.

IV. Δgp19d: The E3-6.7 stop codon (TGA) was restored by overlapping with an ATGACC sequence added to the 5' end of GM-CSF. This construct will likely add two amino acids (a methionine and a threonine) to the amino terminus of the signal sequence of both mouse and human GM-CSF.

V. Δgp19IRES: The GM-CSF cDNA was attached to the ATG of the 573bp EMC IRES (Ghattas, et al. The encephalomyocarditis virus ribosomal entry site allows efficient coexpression of two genes from a recombinant provirus in cultured cells and embryos. Mol Cell Biol 11:5848-5859. 1991) using overlap PCR and the IRES/GM-CSF was inserted 3' to the native stop codon (TGA) of E3-6.7.

An adenovirus right donor plasmid was constructed for each Δgp19 vector (Tables 23 & 24). These donor plasmids contain hGM-CSF, mGM-CSF cDNA or IRES/mGM-CSF in the position of the E3-gp19 gene of the pDR2FE3 plasmid. Table 23 also shows the primer pairs used in generating each PCR product. The exact sequences of the primers are shown in Table 25.

**Table 23**. Primers for construction of □gp19 viral vectors

| Viral Vector (description) | Primer 1 / Primer 2 Template DNA | Primer 3 / Primer 4 Template DNA | Primer 5 / Primer 6 Template DNA |
|---|---|---|---|
| Ar6pAE2f(E3+,Dg19a)F (□gp19a control) | E3.1/E3.7 pDR2FE3 | | E3.8/E3a.4 PDR2FE3 |
| Ar6pAE2f(E3+,Dg19b) (□gp19b control) | E3.1/E3.7b pDR2(E3+, Dg19a)F | | E3.8b/E3a.4 PDR2(E3+, Dg19a)F |
| Ar6pAE2f(E3+,mGm, Dg19a)F (□gp19a, mGM-CSF) | E3.1/E3.5 pDR2FE3 | E3m.1/E3m.2 pDR2mGmF | E3.6/E3a.4 PDR2FE3 |
| Ar6pAE2f(E3+,mGm, Dg19b)F (□gp19b, mGM-CSF) | E3.1/E3.5b pDR2(E3+, mGm,Dg19a)F | E3m.b/E3m.2 pDR2(E3+, mGm,Dg19a)F | E3.6/E3a.4 PDR2(E3+, mGm,Dg19a)F |
| Ar6pAE2f(E3+,mGm, Dg19c)F (□gp19c, mGM-CSF) | E3.1/E3.5c pDR2(E3+, mGm,Dg19a)F | E3m.c/E3m.2 pDR2 (E3+.mGm,Dg19a)F | E3.6/E3a.4 PDR2(E3+, mGm.Dg19a)F |
| Ar6pAE2f(E3+,mGm, Dg19d)F (□gp19d, mGM-CSF) | E3.1/E3.5d pDR2(E3+, mGm,Dg19a)F | E3m.d/E3m.2 pDR2(E3+, mGm,Dg19a)F | E3.6/E3a.4 PDR2(E3+, mGm,Dg19a)F |
| Ar6pAE2f(E3,ImGm, Dg19b)F (□gp19 IRES, mGM-CSF*) | E3.1/E3IM-2 pDR2(E3+, mGm,Dg19a)F | E3IM-1/E3m.2 IRESmGm fragment | E3.6/E3a.4 PDR2(E3+, mGm,Dg19a)F |

(continued)

| Viral Vector (description) | Primer 1 / Primer 2 Template DNA | Primer 3 / Primer 4 Template DNA | Primer 5 / Primer 6 Template DNA |
|---|---|---|---|
| Ar6pAE2f(E3+,hGm, Dg19a)F (□gp19a, hGM-CSF) | E3.1/E3.2 pDR2FE3 | E3h.1/E3h.2 pDR2hGmTK3 | E3a.3/E3a.4 PDR2FE3 |
| Ar6pAE2f(E3+,hGm, Dg19b)F (□gp19b, hGM-CSF) | E3.1/E3.2b pDR2(E3+, hGm,Dg19a)F | E3h.b/E3h.2 pDR2(E3+, hGm,Dg19a)F | E3a.3/E3a.4 PDR2(E3+, hGm,Dg19a)F |
| Ar6pAE2f(E3+,hGm, Dg19c)F (□gp19c, hGM-CSF) | E3.1/E3.2c pDR2(E3+, hGm,Dg19a)F | E3h.c/E3h.2 pDR2(E3+, hGm,Dg19a)F | E3a.3/E3a.4 PDR2(E3+, hGm,Dg19a)F |
| (IRESmGm fragment) | IRES1/IRES3 pDR2mGmiresTK3 | mGm1/mGm2 pDR2mGmF | |
| * The IRES and mGM-CSF were first attached by PCR to generate an IRESmGm DNA fragment (as shown in the final row above) which was then used as the template for PCR amplification using primers E3IM-1 and E3m.2 | | | |

**Table 24.** Viral vectors, donor plasmids and large plasmids for □gp19 constructs

| Viral Vector (Description) | Donor Plasmid | Large Plasmid |
|---|---|---|
| Ar6pAE2f(E3+,Dg19a)F (□gp19a control) | pDR2(E3+,Dg19a)F | PAr6pAE2f(E3+,Dg19a)F |
| Ar6pAE2f(E3+,Dg19b) (□gp19b control) | pDR2(E3+,Dg19b)F | PAr6pAE2f(E3+,Dg19b)F |
| Ar6pAE2f(E3+,mGm,bg19a)F (□gp19a, mGM-CSF) | pDR2(E3+,mGm,Dg19a)F | PAr6pAE2f(E3+,mGm,Dg19a)F |
| Ar6pAE2f(E3+,mGm,Dg19b)F (□gp19b, mGM-CSF) | pDR2(E3+,mGm,Dg19b)F | PAr6pAE2f(E3+,mGm,Dg19b)F |
| Ar6pAE2f(E3+,mGm,Dg19c)F (□gp19c, mGM-CSF) | pDR2(E3+,mGm,Dg19c)F | PAr6pAE2f(E3+,mGm,Dg19c)F |
| Ar6pAE2f(E3+,mGm,Dg19d)F (□gp19d, mGM-CSF) | pDR2(E3+,mGm,Dg19d)F | PAr6pAE2f(E3+,mGm,Dg19d)F |
| Ar6pAE2f(E3,ImGm,Dg19b)F (□gp19 IRES, mGM-CSF) | pDR2(E3+,ImGm,Dg19b)F | PAr6pAE2f(E3,ImGm,Dg19b)F |
| Ar6pAE2f(E3+,hGm,Dg19a)F (□gp19a, hGM-CSF) | pDR2(E3+,hGm,Dg19a)F | PAr6pAE2f(E3+,hGm,Dg19a)F |
| Ar6pAE2f(E3+,hGm,Dg19b)F (□gp19b, hGM-CSF) | pDR2(E3+,hGm,Dg19b)F | PAr6pAE2f(E3+,hGm,Dg19b)F |
| Ar6pAE2f(E3+,hGm,Dg19c)F (□gp19c, hGM-CSF) | pDR2(E3+,hGm,Dg19c)F | PAr6pAE2f(E3+,hGm,Dg19c)F |

**Table 25**. Sequences of PCR primers

| Primer | Sequence | SEQ ID NO: |
|---|---|---|
| E3.1 | 5'-CCTGCCGGGAACGTACGAGTGC-3' | 29 |
| E3.2 | 5'-CTGCAGCCACATCTTGGGTGGCGACCCCAGC-3' | 30 |
| E3.2b | 5'-CTGCAGCCACATGGTTATCTTGGGTGGCGACCCCAGC-3' | 31 |
| E3.2c | 5'-CTGCAGCCTCATCTTGGGTGGCGACCCAGC-3' | 32 |

(continued)

| Primer | Sequence | SEQ ID NO: |
|---|---|---|
| E3.5 | 5'-CTCTCGTGGGCCATCTTGGGTGGCGACCCCAG-3' | 33 |
| E3.5b | 5'-CTCTCGTGGGCCATGGTTATCTTGGGTGGCGACCCCAG-3' | 34 |
| E3.5c | 5'-CTCTCGTGGGTCATCTTGGGTGGCGACCCCAG-3' | 35 |
| E3.5d | 5'-CTCTCGTGGGCCATGGTCATCTTGGGTGGCGACCCCAG-3' | 36 |
| E3.6 | 5'-CCAAAAATAATTTACTAAGTTACAAAGCTAAT-3' | 37 |
| E3.7 | 5'-GTAACTTAGTAAATTACTTGGGTGGCGACCCCAGCG-3' | 38 |
| E3.7b | 5'-GTAACTTAGTAAATTATCTTGGGTGGCGACCCCAGCG-3' | 39 |
| E3.8 | 5'-CGCCACCCAAGTAATTTACTAAGTTACAAAGC-3' | 40 |
| E3.8b | 5'-CGCCACCCAAGATAATTTACTAAGTTACAAAGC-3' | 41 |
| E3a.3 | 5'-CCAGGAGTAATTTACTAAGTTACAAAGC-3' | 42 |
| E3a.4 | 5'-GTCCGGTAGCGGCGGCCGCG-3' | 43 |
| E3m.1 | 5'-CGCCACCCAAGATGGCCCACGAGAGAAAGGC-3' | 44 |
| E3m.b | 5'-CGCCACCCAAGATAACCATGGCCCACGAGAGAAAGGC-3' | 45 |
| E3m.c | 5'-CGCCACCCAAGATGACCCACGAGAGAAAGGC-3' | 46 |
| E3m.d | 5'-CGCCACCCAAGATGACCATGGCCCACGAGAGAAAGGC-3' | 47 |
| E3h.1 | 5'-CGCCACCCAAGATGTGGCTGCAGAGCCTGCTG-3' | 48 |
| E3h.b | 5'-CGCCACCCAAGATAACCATGTGGCTGCAGAGCCTGCTGC-3' | 49 |
| E3h.c | 5'-CGCCACCCAAGATGAGGCTGCAGAGCCTGCTGC-3' | 50 |
| E3IM-1 | 5'-GGGGTCGCCACCCAAGATGACAATTCCGCCCCCCCCCTAACG-3' | 51 |
| E3IM-2 | 5'-GTCATCTTGGGTGGCGACCCCAGC-3' | 52 |
| IRES1 | 5'-TCCCCCCGGGCAATTCCGCCCCCCCCCTAA-3' | 53 |
| IRES3 | 5'-CTCTCGTGGGCCATGGTATTATCGTGTTTTTC-3' | 54 |
| MGm1 | 5'-GAAAAACACGATAATACCATGGCCCACGAGAGAAAGG-3' | 55 |
| MGm2 | 5'-GCATGTTAACTTCCTCATTTTTGGACTGG-3' | 56 |

The plasmid pDr2FE3 was cloned by ligating the 1.9kb XbaI fragment, containing the majority of the E3 genes, from the genomic DNA of wildtype Ad5 virus into the XbaI site of pDr2F. The plasmid pDR2F was previously described in example #8 (Section 8.1.1). pDr2F has a loxP site within the XbaI site that partially alters the 3' E3 14.7 kDa protein amino acid sequence.

The donor plasmids were constructed as follows:

I. PCR amplification of DNA fragments: As shown in Figure 25, three DNA fragments were PCR amplified for each donor plasmid using the primer pairs shown in Table 23 (only two fragments were generated for the control donor plasmids □gp19a and □gp19b that lack the GM-CSF transgene). The IRES containing pDR2(E3+,ImGm,Dg19b) F plasmid required two PCR amplifications followed by a two-fragment overlap PCR amplification in order to generate the IRESmGm fragment. All PCR amplifications were carried out using Platinum Taq DNA Polymerase High Fidelity (Invitrogen Inc., Carlsbad, CA, CAT# 11304-011) and conditions were as suggested by the manufacturer.

II. Overlap PCR: The PCR products from step I were purified using a StrataPrep PCR Purification Kit (Stratagene Inc., La Jolla, CA, CAT# 400771). An overlap PCR amplification was then performed for each vector using the mixture of the 3 purified DNA fragments (2 for control plasmids) as template and Primer 1 (primer E3.1) and Primer 6 (primer E3a.4) as primers. The overlap PCR generates a single DNA fragment in which the hGM-CSF, mGM-CSF or IRES/mGM-CSF cDNA is embedded within the E3 region. For the control plasmid, the fragment contains the E3 region with a deletion of the E3-gp19 gene. As in step I, all PCR amplifications were carried out using Platinum Taq DNA Polymerase High Fidelity and conditions as suggested by the manufacturer.

III. Preparation of Insert: The PCR products from step II were purified using a StrataPrep PCR Purification Kit and subjected to BsiWI/NotI double digestion. The digest was recovered from an agarose gel, purified with a GeneClean II kit (BIO101, Inc., CA), and used as the insert DNA in the ligation reaction of step V.

IV. Plasmid pDR2FE3 was digested with BsiWI/NotI and treated with CIP to remove 5' phosphate groups. The digest was electrophoresed in a 0.8% agarose gel and the 11635 bp fragment was purified using a GeneClean II kit. The purified DNA fragment was used as the vector DNA in ligation reaction of step V.

V. The insert and vector DNAs were ligated and transformed into E. coli HB101 competent cells to generate donor plasmids. Plasmid clones were screened using restriction enzyme digestion.

The donor plasmids were digested with FspI and SpeI; the large fragment containing the GM-CSF cDNA was recovered from an agarose gel and purified using a GeneClean II kit. Fifty to 100ng of the DNA fragment was co-transformed into E. coli BJ5183 competent cells with 100ng of SrfI/PacI digested pAr6pAE2fF plasmid DNA (generation of pAr6pAE2f (E3+,mGm,Dg19b)F and pAr6pAE2f(E3+,hGm,Dg19b)F shown in Figures 27a & b, respectively). Transformed BJ5183 cells were plated onto LB agar plates containing 100μg/ml ampicillin and allowed to grow at 37°C overnight. Colonies were screened and the correct plasmid was isolated as described in example #8 (Section 8.1.1). Table 24 shows the plasmids (donor and large) used in generating each viral vector..

10.1.2 Generation of Ar6pAE2f(E3+,mGm,Dg19b)F & Ar6pAE2f(E3+,hGm,Dg19b)F Viral Vectors

Viral vector was produced by transfecting 2 μg of SwaI-digested large plasmid using the LipofectAMINE-PLUS reagent system (Life Technologies, Rockville, MD) into S8 cells and cultured in a 6-well plate. Virus was propagated and viral genomic DNA was isolated as described in example #8 (Section 8.1.2). The viral genomic DNA was digested with restriction enzymes EcoRV, SalI, XbaI plus BamHI or BspeI and loaded to a 0.8% agarose gel. The restriction patterns were as expected and the sequences of the GM-CSF insert and the cloning junctions were confirmed.

10.2 In vitro cytotoxicity

To evaluate the ability of Δgp19 viral vectors to kill cells, MTS assays were performed as described in example #8 (Section 8.2). Human tumor cell lines H460 (non-small cell lung carcinoma, NCI-H460, ATCC #HTB-177) and Hep3B (hepatocellular carcinoma, Hep3B2.1-7, ATCC #HB-8064) were included in the MTS assay. All Agp19 viral vector backbones were included in the MTS assay. Experimental Δgp19 viruses used in the assay included Ar6pAE2f(E3+, mGm,Dg19a)F, Ar6pAE2f(E3+,mGm,Dg19b)F, Ar6pAE2f(E3+,mGm,Dg19c)F, Ar6pAE2f(E3+,mGm,Dg19d)F, Ar6pAE2f(E3+,lmGm,Dg19b)F, Ar6pAE2f(E3+,hGm,Dg19a)F, Ar6pAE2f(E3+,hGm,Dg19b)F and Ar6pAE2f(E3+,hGm, Dg19c)F. Control viruses included Ad*dl*312 (E1A negative control), Ad*dl*327 (E1A positive control), Ar6pAE2fF (E2F-1 promoted or E3 deleted backbone vector) and Ar6pAE2fmGmF (E2F-1 promoted, E3 deleted backbone vector with mGM-CSF).

The results (Figure 28) obtained from the MTS assays were subjected to sigmoidal dose-response curve fit analysis using Prism GraphPad software. The program was used to calculate LD50; the effective concentration at which 50% of the cells were dead compared to untreated control cells 7 days after infection. The Δgpl9 vectors retained the oncolytic capacity of the Ar6pAE2fF vector in all cell lines tested.

10.3 GM-CSF Transgene Expression

Mouse and human GM-CSF ELISAs were used as a screen to confirm expression of the mGM-CSF and hGM-CSF transgenes following infection of H460 cells with the □gp19 viral vectors using the same lots as in the MTS assays. H460 cells were plated in 6-well plates using 2 ml/well of serum-free culture media at a density of $2.5x10^5$ cells/well. The next day, the media was removed and the cultured cells were transduced in duplicate with the viral vector at 10, 100, and 1000 particles per cell in 500 μl serum-free medium. After two hours of incubation at 37°C in a 5% $CO_2$ incubator, virus was aspirated and 2 ml of fresh complete culture medium was added to each well. At 24 or 48 hours post infection, the supernatants were collected for ELISA. The results are shown if Figure 29.

10.4 H460 cell/nude mice xenograft tumor model

The H460 xenograft nude mouse tumor model was utilized to evaluate the in vivo anti-tumor efficacy of the Ar6pAE2f(E3+,mGm,Dg19b)F vector. In the H460 xenograft model, 2x$10^8$ H460 cells were injected subcutaneously into the right flanks of female nude mice. Approximately 8 days after tumor inoculation, tumors were measurable in two dimensions using an electronic caliper and ranged from 60-150 $mm^3$ (using the formula $V = (W^2 \times L)\overline{\pi}/6$; V, volume; W, width; L, length). Mice with tumors were randomly divided into treatment groups and received 5 intratumoral injections of Add/312, Ar6pAE2fF, Ar6pAE2fmGmF or Ar6pAE2f(E3+,mGm,Dg19b)F or PBS on a Monday, Wednesday, Friday, Monday and Wednesday schedule. Tumors were treated with $2 \times 10^{10}$ or $1 \times 10^{11}$ viral particles/injection. Tumor volumes were measured twice weekly for 34 days, group means were calculated and data analyzed by repeat-measures one way ANOVA (Figure 30). Mice were sacrificed when they became moribund or when the tumor volume exceeded 2000 $mm^3$.

10.5 Results and Discussion

The structure and cloning of pDR2(E3+,hGm,Dg19b)F and pDR2(E3+,mGm,Dg19b)F was described. Following cloning, orientation of donor plasmids was screened with SalI digestion and the structure was further confirmed by digestion with SpeI and FspI or SpeI and ClaI digestions (data not shown). The cloning scheme and the structure of the large plasmids are summarized in Figure 27 using pAr6pAE2f(E3+,hGm,Dg19b)F and pAr6pAE2f(E3+,mGm,Dg19b)F as examples. The integrity of pAr6pAE2f(E3+,hGm,Dg19b)F and pAr6pAE2f(E3+,mGm,Dg19b)F was confirmed by EcoRV, BsrGI, NotI and MluI digestion. Following transfection of S8 cells with SwaI digested large plasmid DNA, the dgp19 viral vectors were isolated and amplified. Genomic DNA from Ar6pAE2f(E3+,hGm,Dg19b)F and Ar6pAE2f(E3+, mGm,Dg19b)F was isolated and digested with restriction enzymes EcoRV, Sal I, BsrGI and NotI. The restriction enzyme digests showed the expected patterns. The integrity of the mouse GM-CSF cDNA insert and viral flanking sequences was confirmed by sequencing bp 28381 to 29550 of Ar6pAE2f(E3+,mGm,Dg19b)F. Similarly, the Ar6pAE2f(E3+,hGm, Dg19b)F vector was sequenced from bp 28381 to 29540. In the human GM-CSF vector, a single T to C mutation at bp position 29240 in the 3' untranslated sequence of GM-CSF was detected. This mutation does not affect the GM-CSF open reading frame or any viral coding sequence.

The Δgp19 viral vectors were shown to kill human tumor cell lines in vitro at LD50 values very close to those of the parental Ar6pAE2fF vector (Figure 28). Thus, the oncolytic function of the vectors was maintained following the modifications at the gp19 gene. In contrast, clear differences were evident in the ability of the various constructs to induce the secretion of GM-CSF by H460 cells. The differences were a function of the vector modifications and not the transgene such that for a given modification, similar levels of human and mouse GM-CSF were detected in the supernatants. Though the Δgp19a vectors were oncolytic as measured in the MTS assay, surprisingly, neither the human nor the mouse GM-CSF Δgp19a vectors produced detectable levels of GM-CSF in the culture supernatants. In contrast, the Δgp19b and Δgp19c vectors produced GM-CSF at levels that were comparable to the GM-CSF produced by the Ar6pAE2fGmF vector. The mouse GM-CSF secreting Δgp19d vector appeared to produce about half as much GM-CSF as Ar6pAE2fGmF (Figure 29).

The MTS cytotoxicity and GM-CSF production assays narrowed the viruses under consideration for further study to the Δgp19b and Δgp19c constructs. As depicted in Figure 26b, the Δgp19c vector restores the overlap between the E3-6.7 stop codon and the initial methionine of the immediate downstream gene. In order to accomplish this, it was necessary to mutate the 4th nucleotide of both human and mouse GM-CSF with subsequent mutations at the amino acid level as well. In the Δgp19b constructs, the E3-6.7 stop codon was altered to a TAA from the native TGA. The result of this change is that the final four nucleotides of the E3-6.7 gene are ATAA rather than the native ATGA and gene downstream of E3-6.7 (i.e., GM-CSF) fails to initiate translation at this site. To optimize GM-CSF expression, a concensus Kozak sequence was placed immediately upstream of the native GM-CSF sequence. Consequently, the GM-CSF produced by the Δgp19b viruses, in contrast to the Δgp19c viruses, is expected to have the native amino acid sequence. Thus, Δgp19b vectors (i.e., Ar6pAE2f(E3+,Gm,Dg19b)F) were chosen for further analysis.

The in vivo anti-tumor effects of the Ar6pAE2f(E3+,mGm,Dg19b)F vector were evaluated in pre-existing tumors using the H460 and Hep3B xenograft nude mouse models. Figure 30 shows the data from one Hep3B xenograft anti-tumor experiment. Significant anti-tumor activities of both Ar6pAE2fmGmF and Ar6pAE2f(E3+,mGm,Dg19b)F oncolytic vectors compared to PBS-treated tumors and the Add/312 control vector-treated tumors following five intratumoral vector injections were observed. At this vector dose (2x$10^9$ particles/injection) using the Hep3B tumor model, the Ar6pAE2fF unarmed vector control also demonstrated significant anti-tumor activity. However, it is important to note that in this study, the Hep3B tumors treated with the Ar6pAE2f(E3+,mGm,Dg19b)F vector were 290 $mm^3$ when the injections began, whereas the tumor treated with the other vectors averaged 175 $mm^3$ when vector injections began (or 65% larger). Thus, the Ar6pAE2f(E3+,mGm,Dg19b)F vector displayed anti-tumor activity that could inhibit the growth of relatively large tumors. Hep3B cells in this experiment were contaminated with a very low amount of E1A expressing cells. Though appropriate caution should be exercised in the interpretation of these data, it is apparent that the effects of the E1A deleted Add/312 control vector could be readily distinguished from the effects of the experimental vectors. Figure 31 shows data from one H460 xenograft anti-tumor experiment in which two vector doses were used to treat H460 tumors that were approximately equal in volume when the vector injections began (120-160 $mm^3$). The data in Figure 31, panel A show that H460 tumors treated with $1x10^{10}$ particles/injection of Ar6pAE2f(E3+,mGm,Dg19b)F significantly slowed the growth of this aggressive tumor compared to the PBS-treated tumors or control Add/312 treated tumors treated with $2x10^{10}$ particles/injection. H460 tumors treated with a five-fold higher dose of vectors demonstrated significant growth inhibition compared to PBS-treated tumors. As observed at the lower vector dose, significant differences were noted for the Ar6pAE2f(E3+,mGm,Dg19b)F vector compared to Add/312 control vector-treated tumors on day 29.

Example 11: Construction and Characterization of E3 Containing Oncolytic Adenoviruses with GM-CSF cDNA Inserted in the Position of the Adenoviral E3-gp19 Gene and with a restored E3-14.7 Gene

The construction of the Ar15pAE2fhGmF and Ar15pAE2fmGmF viral vectors is described. These viral vectors closely resemble Ar6pAE2f(E3+,hGm,Dg19b)F and Ar6pAE2f(E3+,mGm,Dg19b)F but have the E3-14.7 gene restored and the loxP site removed.

11.1.1 Construction of pAr15pAE2fhGmF and pAr15pAE2fmGmF

Generation of donor plasmids pDr5hGmF and pDr5mGmF

Donor plasmids pDr5hGmF and pDr5mGmF were generated from plasmids pDR2(E3+,hGm,Dg19b)F and pDR2(E3+,mGm,Dg19b)F, respectively, by removing the loxP site from the pDR2 plasmids. Figure 32 shows the structure of donor plasmids pDr5hGmF and pDr5mGmF.

The donor plasmids pDr5hGmF and pDr5mGmF were constructed as follows:

I. Preparation of Insert: The Ad5 genome DNA was digested with NotI/SphI, electrophoresed in a 0.8% agarose gel and the 1714 bp fragment was recovered and purified with a GeneClean II kit (BIO101, Inc., CA). The 1714 bp fragment was used as the insert DNA in the ligation reactions of step III.

II. Plasmids pDR2(E3+,hGm,Dg19b)F and pDR2(E3+,mGm,Dg19b)F were digested with NotI/SphI. The digests were electrophoresed in a 0.8% agarose gel and the 10960 bp fragment (for hGM-CSF) and 10987 bp fragment (for mGM-CSF) were isolated from the gel and purified using a GeneClean II kit. The purified DNA fragments were used as the vector DNAs in the ligation reactions of step III.

III. The insert and vector DNA were ligated and transformed into E. coli HB101 competent cells to generate donor plasmids pDr5mGmF and pDr5hGmF. Plasmid clones were screened using restriction enzyme digestion and plasmids demonstrating the correct patterns were use in the generation of large plasmids.

Generation of large plasmids pAr15pAE2fhGmF and pAr15pAE2fmGmF
The donor plasmids pDr5hGmF (Figure 32A) and pDr5mGmF (Figure 32B) were digested with FspI and SpeI. The large fragments containing the hGM-CSF or mGM-CSF cDNA were recovered from agarose gels and purified using a GeneClean II kit. Fifty to 100ng of the DNA fragments were co-transformed into E. coli BJ5183 competent cells with 100ng of SrfI/PacI digested pAr6pAE2fF plasmid DNA (Figures 33 & 34). Transformed BJ5183 cells were plated onto LB agar plates containing 100□g/ml ampicillin and allowed to grow at 37°C overnight. Colonies were screened and the correct plasmid was isolated as described in Example 8 (Section 8.1.1).

11.1.2 Generation of Ar15pAE2fhGmF & Ar15pAE2fmGmF Viral Vectors

Viral vector was produced by transfecting 2 μg of SwaI-digested large plasmid using the LipofectAMINE-PLUS reagent system (Life Technologies, Rockville, MD) into S8 cells and cultured in a 6-well plate. Virus was propagated and viral genomic DNA was isolated as described in example #8 (Section 8.1.2). The viral genomic DNA was digested with restriction enzymes EcoRV, BsrGI, NotI, or MluI and loaded on a 0.8% agarose gel. The restriction patterns were as expected and the sequences of the GM-CSF insert and the cloning junctions were confirmed.

11.2 In vitro cytotoxicity

To evaluate the ability of the Ar15pAE2fhGmF and Ar15pAE2fmGmF viral vectors to kill cells, MTS assays were performed as described in example #8 (Section 8.2). Human tumor cell lines H460 (non-small cell lung carcinoma, NCI-H460, ATCC #HTB-177) and Hep3B (hepatocellular carcinoma, Hep3B2.1-7, ATCC #HB-8064) were included in the MTS assay. Control viruses included Add/312 (E1A negative control), Add/327 (E1A positive control), Ar6pAE2fmGmF (E2F-1 promoted, E3 deleted backbone vector with mGM-CSF), Ar6pAE2f(E3+, mGm,Dg19b)F (described in example 10), and Ar15pAE2fF (Ar15 backbone lacking a GM-CSF transgene with E3-14.7 loxP correction and E3+ except for gp19).

The results (Figure 35) obtained from the MTS assays were subjected to sigmoidal dose-response curve fit analysis using Prism GraphPad software. The program was used to calculate LD50; the effective concentration at which 50% of the cells were dead compared to untreated control cells 7 days after infection. The Ar15 vectors retained the oncolytic capacity of the Ar6pAE2fF vector in all cell lines tested.

11.3 GM-CSF Transgene Expression

Mouse and human GM-CSF ELISAs were used as a screen to confirm expression of the human and mouse GM-CSF transgenes following infection of H460 and Hep3B cells with the Ar15pAE2fhGmF and Ar15pAE2fmGmF viral vectors. H460 and Hep3B cells were plated in 6-well plates using 2 ml/well of serum-free culture media at a density of $2.5x10^5$ cells/well. The next day, the media was removed and the cultured cells were transduced in duplicate with the viral vectors at 10, 100, and 1000 particles per cell in 500 μl serum-free medium. After two hours of incubation at 37°C in a 5% $CO_2$ incubator, virus was aspirated and 2 ml of fresh complete culture medium was added to each well. At 24 or 48 hours post infection, the supernatants were collected for ELISA. The results are shown if Figure 36.

11.4 Results and Discussion

Ar15pAE2fhGmF and Ar15pAE2fmGmF, GM-CSF armed oncolytic adenoviruses were constructed. The vectors have the same gp19 deletion as the Ar6pAE2f(E3+,Gm,Dgp19)F series of example 10, but with the loxP site removed and the E3-14.7 gene restored. The Ar15pAE2fhGmF and Ar15pAE2fmGmF vectors lack only the E3-gp19 gene and retain all the other E3 proteins, including E3-12.5, E3-6.7, E3-11.6 (ADP), E3-10.4 (RID□), E3-14.5 (RID□) and E3-14.7 proteins (E3 region reviewed in Wold et al., 1995). Restriction digestion and partial sequencing of the vectors confirmed the structure of the viruses. As shown by MTS assays, the Ar15pAE2fhGmF and Ar15pAE2fmGmF vectors retained the *in vitro* oncolytic properties of the parental vectors. Human and mouse GM-CSF specific ELISAs confirmed the production of substantial quantities of hGM-CSF or mGM-CSF following infection of tumor cells.

Example 12: Construction and Characterization of E3 Containing Oncolytic Adenoviruses with GM-CSF cDNA inserted in the Position of the Adenoviral E3-14.7 Gene

12.1 Construction of pAr16pAE2fhGmF

PCR amplifications were performed in order to precisely replace the adenoviral E3-14.7 open reading frame with the human GM-CSF cDNA from the start to the stop codon while keeping the stop codon for the upstream E3-14.5 gene intact. Three DNA fragments were generated and 3-fragment overlap PCR was employed to attach these fragments by site overlap extension, thus generating a single DNA fragment (Figure 37). The DNA fragment was digested with XhoI and SphI and used to replace the XhoI/SphI region containing the E3-14.7 gene of the pDR4F plasmid (the pDR4F plasmid closely resembles the pDR2F plasmid except that the entire wild-type Ad5 E3 region has been included).

In the native adenovirus, the reading-frame of the E3-14.5 gene overlaps the E3-14.7 gene (Figure 38a). The junction between the E3-14.5 gene and the inserted GM-CSF was designed to optimize GM-CSF expression while maintaining the integrity of the E3-14.5 gene (Figure 38b). A single T to C mutation in the original ATG start codon for the E3-14.7 gene was made such that the carboxy terminal amino acid sequence of the E3-14.5kDa protein was not altered and a Kozak sequence was inserted between the end of the E3-14.5 gene and the start of the GM-CSF cDNA in order to enhance the expression of GM-CSF. This strategy was previously successful in the insertion of GM-CSF into the E3-gp19 position, which also has an overlapping start/stop codon with the upstream E3-6.7 gene (see Example 10).

An adenovirus right donor plasmid, pDR6hGmF (Figure 39), was constructed for the Ar16pAE2fGmF vector (Tables 26 & 28). This donor plasmid contains the hGM-CSF cDNA in the position of the E3-14.7 gene of the pDR4F. Table 26 also shows the primer pairs used in generating each PCR product and the exact sequences of the primers are shown in Table 27.

The donor plasmids were constructed as follows:

I. PCR amplification of DNA fragments: As shown in Figure 37, three DNA fragments were PCR amplified for each donor plasmid using the primer pairs shown in Table 26 (only two fragments were generated for the control donor plasmid pDR6F that lacks the GM-CSF transgene). All PCR amplifications were carried out using Platinum Taq DNA Polymerase High Fidelity (Invitrogen Inc., Carlsbad, CA, CAT #11304-011) and conditions as suggested by the manufacturer.

II. Overlap PCR: The PCR products from step I were purified using a StrataPrep PCR Purification Kit (Stratagene Inc., La Jolla, CA, CAT #400771). An overlap PCR amplification was performed for each vector using the mixture of the 3 purified DNA fragments (2 for pDR6F plasmids) as template and Primer 1 (primer 147A) and Primer 6 (primer 147F) as primers. The overlap PCR generated a single DNA fragment in which the hGM-CSF cDNA is embedded within the E3-14.7 region. For the control plasmid, the fragment contains the E3 region with a deletion of the E3-14.7 gene. As in step I, all PCR amplifications were carried out using Platinum Taq DNA Polymerase High Fidelity and conditions as suggested by the manufacturer.

III. Preparation of Insert: The PCR product from step II was purified using a StrataPrep PCR Purification Kit and subjected to Xhol/Sphl double digestion. The digest was recovered from an agarose gel and purified with a GeneClean II kit (BIO101, Inc., CA), and was used as the insert DNA in the ligation reaction of step V.

IV. Plasmid pDR4F was digested with Xhol/Sphl and treated with CIP to remove 5' phosphate groups. The digest was electrophoresed in a 0.8% agarose gel and the 11635 bp fragment was purified using a GeneClean II kit. The purified DNA fragment was used as the vector DNA in ligation reaction of step V.

V. The insert and vector DNAs were ligated and transformed into E. coli HB101 competent cells to generate donor plasmid pDR6hGmF. Plasmid clones were screened using restriction enzyme digestion.

Figure 39 shows the structure of donor plasmid pDR6hGmF.

**Table 26**. Primers for construction of Ar16pAE2fGm viral vector

| Viral Vector (description) | Primer 1 / Primer 2 Template DNA | Primer 3 / Primer 4 Template DNA | Primer 5 / Primer 6 Template DNA |
|---|---|---|---|
| Ar16pAE2fF | 147A/Ar16.2 pDR4F | | Ar16.1/147F pDR4F |
| Ar16pAE2fhGmF | 147A/147BH pDR4F | 147CH/147DH pDR2hGmF | 147EH/147F pDR4F |

**Table 27.** Sequences of PCR primers

| Primer | Sequence | SEQ. ID NO: |
|---|---|---|
| 147A | 5'-CGGGTTCTATGTAAACTCCTTCATG-3' | 57 |
| 147BH | 5'-GCAGCCACATGGTCAGTCGTCTCCTCCTGTTAGATTAAAGTAGC-3' | 58 |
| 147CH | 5'-CTAACAGGAGGAGACGACTGACCATGTGGCTGCAGAGCCTGCTGC-3' | 59 |
| 147DH | 5'-GCTTTATTATTTTTTTTTATTACTCCTGGACTGGCTCCCAGCAG-3' | 60 |
| 147EH | 5'-CCAGGAGTAATAAAAAAAAATAATAAAGCATCAC-3' | 61 |
| 147F | 5'-GGCCGTTGCCCATTTTGAGCGCAAGC-3' | 62 |
| Ar16.1 | 5'-CTAACAGGAGGAGACGACTGATTAATAAAGCATCAC-3' | 63 |
| Ar16.2 | 5'-GCTTTATTATTTTTTTTTATCAGTCGTCTCCTCCTGTTAGATTAAAG-3' | 64 |

**Table 28.** Viral vectors, donor plasmids and large plasmids for Ar16pAE2fGmF constructs

| Viral Vector (Description) | Donor Plasmid | Large Plasmid |
|---|---|---|
| Ar16pAE2fF | pDR6F | PAr16pAE2fF |
| Ar16pAE2fhGmF | pDR6hGmF | PAr16pAE2fhGmF |

The donor plasmid was digested with FspI and SpeI; the large fragment containing the GM-CSF cDNA was recovered from an agarose gel and purified using a GeneClean II kit. Fifty to 100ng of the DNA fragment was co-transformed into E. coli BJ5183 competent cells with 100ng of Srfi/PacI digested pAr6pAE2fF plasmid DNA (generation of pAr16pAE2fhGmF shown in Figure 40). Transformed BJ5183 cells were plated onto LB agar plates containing 100□g/ml ampicillin and allowed to grow at 37°C overnight. Colonies were screened and the correct plasmid was isolated as described in Example 8 (Section 8.1.1). Table 28 shows the plasmids (donor and large) used in generating the Ar16pAE2fGmF viral vector.

12.2 Generation of Viral vector Ar16pAE2fhGmF

Generation of the adenoviral vector was performed as described in Example 10 using the plasmids detailed in Table 28. The AE1-2a clone S8 cells (S8 cells) were cultured in IMEM containing 10% heat inactivated FBS. Two □g of Swal-digested large plasmid was transfected into S8 cells and cultured in a 6-well plate using the LipofectAMINE-PLUS reagent system (Life Technologies, Rockville, MD). After 7 days incubation at 37°C, 5% $CO_2$, humidified, the viral vector was amplified and purified by CsCl gradient. Viral vector concentrations were determined by spectrophotometric analysis (Mittereder, et al., "Evaluation of the concentration and bioactivity of adenovirus vectors for gene therapy." J Virol 70:7498-7509 (1996)). To confirm the structure of Ar16pAE2fhGmF, viral genomic DNA was isolated with a Puregene DNA Isolation Kit from Gentra Systems as follows: 150□l viral vector solution was mixed with 300□l Cell Lysis Solution (supplied with Puregene kit) and 20□l of Proteinase K (10mg/ml, Promega, Inc.) at 56°C for 4 hours to overnight, with agitation. At the end of incubation the samples were cooled to room temperature by placing the tubes on ice for 1 minute. One hundred fifty □l of Protein Precipitation Solution (Puregene kit) was added to the samples, vortexed vigorously at high speed for 20 seconds and centrifuged at 14,000rpm for 1 minute. The supernatants containing the viral DNAs (the precipitated proteins form tight pellets at the bottom of tubes) were transferred into fresh 1.5ml microfuge tubes containing 450□l 100% Isopropanol (2-propanol). The samples were mixed by inverting gently 50 times, centrifuged at 14,000 rpm for 1 minute, the supernatant was discarded and the DNA pellets washed with 450□l of 70% ethanol followed by centrifugation at 14,000rpm for 1 minute. The supernatants were removed and the DNA pellets were air dried for 10 minutes. Fifty □l of DNA Hydration Solution (supplied with Puregene kit) was added to each tube and the DNAs were rehydrated at 65 °C for 1 hour. The viral genomic DNAs were digested with restriction enzymes (RE) EcoRV, BsrGI, NotI, and MluI, and electrophoresed on a 0.8% agarose gel. The restriction enzyme patterns of the Ar16pAE2fhGmF viral vector observed in the agarose gels matched the predicted patterns.

12.3 In vitro cytotoxicity assay (MTS assay)

To evaluate the ability of Ar16pAE2fhGmF to kill tumor cells, MTS assays were performed as described in Example 10 using human H460 tumor cells. Cytotoxicity due to Ar16pAE2fhGmF was compared to control viruses Ad*dl*/312, Ad*dl*/327, Ar6pAE2fF, Ar6pAE2fhGmF and Ar6pAE2f(E3+,hGm,□gp19)F. The results (Figure 41) obtained from the MTS assay screens were subjected to sigmoidal dose-response curve fit analysis using Prism GraphPad software. The program was used to calculate LD50; the effective concentration at which 50% of the cells were dead compared to untreated control cells 7 days after infection. The Ar16pAE2fhGmF vector retained the oncolytic capacity of the Ar6pAE2fF vector.

12.4 In vitro GM-CSF production induced by Ar16pAE2fhGmF

An ELISA for human GM-CSF was used to confirm expression of the hGM-CSF transgene following infection of H460 cells with the Ar16pAE2fhGmF viral vector using the same lots as in the MTS assay. The infection, media collection, ELISA and analysis were performed as described in Example 10 and the results are plotted in Figure 42. Human GM-CSF production following infection of H460 cells with Ar16pAE2fhGmF was very similar to GM-CSF production following infection with Ar6pAE2fhGmF and Ar6pAE2f(E3+,hGm,□g19)F.

12.5 Summary

In the Ar16pAE2fhGmF vector, the GM-CSF transgene was cloned using a scheme that was successful in the cloning of Ar6pAE2f(E3+,hGm,ðg19)F. The vector produced GM-CSF at levels similar to Ar6pAE2f(E3+,hGm, Δg19)F and retained the oncolytic capacity of the adenoviral backbone, thus illustrating the generality of the cloning scheme.

The combined Ar6pAE2f(E3+,hGm, Δg19)F and Ar16pAE2fhGmF data illustrate the concept that trangene expression levels can be fine-tuned according to the requirements of a particular application. Two different transgenes could be inserted into two different regions (e.g., into the E3-gp19 position and the E3-14.7 position) to create a vector that simultaneously expresses two different transgenes. This could be useful for the simultaneous production of immune activating cytokines or the production of an immune activating cytokine together with a suicide gene (eg, thymidine kinase or cytosine deaminase). With the appropriate prodrug, given following the stimulation of the immune system and production of the suicide gene, the infected cells could be ablated in an immunologically activated millieau. The resulting debris from ablated tumor cells would serve as an excellent source of antigen for the generation of anti-tumor immune responses that are additionally boosted by the presence of a cytokine (Albert ML, et al., "Dendritic Cells Acquire Antigen from Apoptotic Cells and Induce Class I-Restricted CTLs," Nature 392:86-89 (1998)). Additionally, it is likely that the placement of transgenes in other areas of the E3 region will result in a broader range of expression levels with varying kinetics (Wold et al., "E3 transcription unit of adenovirus," Curr Top Microbiol Immunol 199:237-274 (1995)). In particular, the addition of a transgene in the position of the adenoviral death protein (ADP) is expected to result in a vector that would express the transgene late in the viral cycle. This could be combined with the insertion of the same or a different transgene in the E3-gp19 or E3-14.7 position to create a vector with prolonged expression of a transgene or a vector that expresses two transgenes sequentially during the viral life cycle. If the transgenes are the same, the ORF may need to be recoded to minimize or eliminate homologous recombinations between the inserts. The recoded ORF would produce the same protein as the original ORF. Early expression of a cytokine (e.g., GM-CSF, flt-3, MIP1-α) that stimulates early phases of an immune response from the E3-gp19 or E3-14.7 position could be followed by delayed expression of a cytokine from the ADP position that stimulates later phases (e.g., IL-2 or IL-5) of immunity. Finally, vectors capable of the expression of three or more transgenes from the position of any of the E3 open reading frames can also be envisioned.

Example 13: Selective production of human GM-CSF in tumor cells by oncolytic vectors controlled by the E2F-1 promoter

13.1. Adenoviral vectors

Ar6pAE2fF is an oncolytic adenovirus vector in which the E1A promoter is replaced with the human E2F-1 promoter. Ar6pAE2fhGmF is an oncolytic vector generated with the human GM-CSF cDNA cloned into the deleted E3 region of Ar6pAE2fF. Ar6pAE2f(E3+,hGm,Dg19b)F is an oncolytic adenovirus vector that expresses most of the genes in the E3 region with the exception that the human GM-CSF gene has been cloned into the gp19 region and it contains a IoxP site that disrupts the E3-14.7 gene (see example #10). Ar15pAE2fF and Ar15pAE2fhGmF are similar to Ar6pAE2f (E3+,hGm,Dg19)F except that they lack the IoxP site and the E3-14.7 region has been restored.

13.2 Cell lines

Human embyro lung fibroblast cell line Wi38 (ATCC #CCL-75), and its SV40 transformed variant, Wi38-VA13 (ATCC #CCL-75.1) were cultured in EMEM containing 10% FBS, supplemented with 1 mM sodium pyruvate, 2mM L-glutamine, 0.1 mM NEAA and 1.5g/L sodium bicarbonate.

13.3 E2F-1 Northern analysis

Northern analysis was performed with 20μg of total RNA resolved on 6% formaldehyde-0.8% agarose gels along with RNA size markers, in 1x NorthernmaxTM Running Buffer (Ambion, Inc., Austin, TX; Sambrook et al., Molecular cloning: A laboratory manual, Second Editions, pp 9.31-9.52, 1989). The RNA was transferred to a HybondTM-N+ nylon membrane (Amersham Life Science, Amersham UK) as specified by the manufacturer. To generate a DNA probe specific for the E2F-1 transcript, full-length cDNA sequences of six members of the E2F gene family were retrieved from the GenBank database and aligned using Multiple Alignment software. The 3'-untranslated region (UTR) of the E2F-1 gene contains sequences that are not shared with other E2F gene family members. A pair of oligonucleotide primers forward:
5'-GTCCCTGAGCTGTTCTTCTGCCCCATAC-3' (SEQ ID NO:65) and reverse:
5'-AGCAGGAGGGAACAGAGCTGTTAGGAAGC-3') (SEQ ID NO:66) was designed. PCR was performed using human genomic DNA (Clontech, Palo Alto, CA) as template primed with the oligo primers. The PCR conditions were 1 minute at 95°C, 60°C and 72°C, for 35 thermal cycles to isolate a 350 bp DNA fragment from the E2F-1 3'-UTR located in exon 6. A total of 100ng probe DNA was labeled through random incorporation of 32P-dCTP. To hybridize the RNA with the specific DNA probes, the filter was prehybridized with NorthernmaxTM Prehybridization/hybridization Buffer (Ambion, Inc., Austin, TX) at 42°C for at least 2 hours. The radiolabeled probe was added and hybridization continued for approximately 16 hours. Blots were washed in 2x SSC/0.01% SDS at room temperature for 30 min, 0.1 x SSC/0.01% SDS at 55°C for 20 min and then 68°C for 30 min. The signals were visualized by autoradiography. As reported in the literature (Slansky et al. A protein synthesis-dependent increase in E2F1 mRNA correlates with growth regulation of the dihydrofolate reductase promoter. Mol Cell Biol. 1993 Mar;13(3):1610-8; Neuman et al. Structure and partial genomic sequence of the human E2F1 gene. Gene. 1996 Sep 16;173(2):163-9), the E2F-1 message was visualized as a 2.5kb transcript. A probe for the the housekeeping GAPDH gene was purchased from Ambion, Inc (Austin, TX), radiolabeled and used as an internal control for the amount of mRNA loaded in each lane.

13.3.1 Results: E2F-1 expression in RB-pathway defective Wi38-VA13 and Wi38 cells

Wi38-VA13 is the SV40 transformed variant of normal human fibroblast Wi38. The T-Ag from SV40 binds to and disrupts the normal pRb pathway and results in a higher level of free E2F, which then activates its own promoter. E2F expression in the cell lines was examined by Northern blot analysis. Upregulation of E2F-1 expression in Wi38-VA13 cells compared to the normal parental Wi38 cells was confirmed (data not shown).

13.4 Infection of cells

Wi38 or Wi38-VA13 cells were seeded in 6-well plates at $4x10^5$ per well one day prior to virus infection to obtain 50% to 60% confluence upon viral infection. To control the time for the initiation of E1A gene transcription, the infection was synchronized for viral internalization into the cells. Cells were infected with adenovirus vectors at 100 and 1000 ppc in 0.5 ml/well media with 2% FBS at 4°C for 1 hour with gentle rocking. The cold temperature allows the viruses to attach to the cytoplasmic membrane without entering the cells (Shenk T. Adenoviridae: The viruses and their replication. 1996; in Fields Virology, Fields BN, Knipe DM and Howley PM, eds. (Lippincott-Raven, Philadelphia) pp. 2111-2148). Virus containing media was removed and cells were washed with cold PBS then incubated at 37°C in growth media (basic media with 10% FBS) for variable times.

13.5 Determination of E1A transcript levels by real-time quantitative PCR

After infection with the viruses for one hour at 4°C, cells were cultured for 4 hours at 37°C in complete media to allow for viral internalization and gene transcription. At the end of the incubation, cells were washed with cold PBS, lysed in 1ml RNAzol B (Tel-Test Inc, Friendwood, TX) and stored at -70°C until the isolation of RNA. The cell lysates were extracted with 0.1 volume chloroform. RNA was precipitated with one volume isopropanol, washed with 75% ethanol, and resuspended in nuclease-free water. Each sample was treated with 5 Units DNase I (Life Technologies, Rockville, MD) for 15 minutes at room temperature. The DNase I was inactivated by the addition of EDTA (to a final concentration of 2.5 mM) and incubated at 65°C for 10 minutes. The RNA samples were re-precipitated with 2 volumes of Ethanol/0.1 volume 3M sodium acetate, washed in 75% ethanol and resuspended in nuclease-free water. RNA concentration was determined spectrophotometrically (A260 and A280) using the SPECTRAmax PLUS (Molecular Devices, Sunnyvale, CA). First strand cDNA was generated from 100ng of test sample RNA using Taqman Reverse Transcription Reagents (Applied Biosystems). The reverse transcription was performed in a 70μl reaction volume under the following conditions: 1X TaqMan RT Buffer, 5.5mM MgCl2, 3.8mM deoxyNTP mixture (0.96 mM of each deoxyNTP), 2.5μM random Hexamer, 1 Unit RNase Inhibitor and 2.5 Units of Multiscribe Reverse Transcriptase. The reactions were incubated for 10 minutes at 25°C, 30 minutes at 48°C, 5 minutes at 95°C and were held at 4°C. Primers specific for the adenoviral E1A sequences were designed using the Primer Express software v. 1.0 (Applied Biosystems, Foster City, CA). Primer and probe sequences were:

E1A Forward primer: 5'-AGCTGTGACTCCGGTCCTTCT-3' (SEQ ID NO:67)
E1A Reverse primer: 3'-GCTCGTTAAGCAAGTCCTCGA-3' (SEQ ID NO:68)
E1A Probe: 5'-FAM-TGGTCCCGCTGTGCCCCATTAAA -TAMRA-3' (SEQ ID NO:69)

Amplification was performed in a reaction volume of 50μl under the following conditions: 20μl of sample cDNA, 1X Taqman Universal PCR Master Mix (Applied Biosystems), 300nM forward primer, 900nM reverse primer and 100nM E1A probe. Thermal cycling conditions were: a 2 minute incubation at 50°C, a 10 minute 95°C activation step for the Amplitaq Gold, followed by 35 cycles of successive incubations at 95°C for 15 seconds and 60°C for 1 minute. Thermal cycling was carried out with the 7700 Sequence Detection System (Applied Biosystems). Data was collected and analyzed using the 7700 Sequence Detection System software v1.6.3 (Applied Biosystems). Relative levels of E1A were determined based on an E1A plasmid curve; with dilutions from 1,500,000 to 15 copies placed in a background RT reaction. Endogenous control: Input RNA was assessed by using a calibrator RNA sample on each plate. The threshold cycle (Ct) for each sample was then compared to the threshold cycle of the calibrator using the following equation: $2^{-\Delta Ct}$ where ACt = sample 18S Ct value minus calibrator Ct value. Reverse transcription reactions for E1A and 18S RNA were performed in duplicate and the average value for each was reported.

13.5.1 Results: E1A gene expression in Wi38-VA13 cells 4-hour post infection

E1A gene transcription begins shortly after host cell infection. A time course of E1A gene expression indicates that the E1A transcripts had reached a significant level 4-hours post infection (data not shown). Accordingly, Wi38-VA13 and Wi38 cells were infected with 1000 ppc Ar6pAE2fhGmF, Ar6pAE2f(E3+,hGm,Dg19b)F or Ar15pAE2fhGmF for 4 hours as described. The results were normalized to hexon DNA copy number 4 hours post-infection.
The E1A RNA copies in SV40 transformed Wi38-VA13 cells were higher than in normal Wi38 cells infected with different adenoviruses, indicating that E1A is selectively expressed in T-Ag transformed cells that have increased E2F levels.

13.6 Hexon DNA PCR

Adenovirus copies in the cells were measured by hexon DNA PCR. Multiple wells for each infection were plated to achieve the $3\times10^6$ cells required for the DNA PCR. Cells were washed and trypsinized after infection, centrifuged and the cell pellets were quickly frozen on dry ice and stored at -70°C for later extraction of DNA. DNA was isolated from approximately $3x10^6$ cells using Qiagen Qiamp Mini Columns (Qiagen Inc., Chatsworth, CA), according to the manufacturer's instructions. Elution of DNA was done in 250 to 300$\mu$l of water and concentrations were determined spectrophotometrically (A260 and A280) on the SPECTRAmax PLUS (Molecular Devices, Sunnyvale, CA). PCR primers and a Taqman probe specific to adenovirus hexon sequences were designed using Primer Express software v1.0 (Applied Biosystems, Foster City, CA). Primer and probe sequences were:

Hexon Forward primer: 5'-CTTCGATGATGCCGCAGTG-3' (SEQ ID NO:70)
Hexon Reverse primer: 3'-GGGCTCAGGTACTCCGAGG-3' (SEQ ID NO:71)
Hexon Probe: 5'-FAM-TTACATGCACATCTCGGGCCAGGAC-TAMRA-3' (SEQ ID NO:72)

Amplification was performed in a 50$\mu$l reaction volume under the following conditions: 10ng of sample DNA, 1X Taqman Universal PCR Master Mix (Applied Biosystems), 600nM forward primer, 900nM reverse primer and 100nM hexon probe. Thermal cycling conditions were: 2 minute incubation at 50°C, 10 minutes at 95°C, followed by 35 cycles of successive incubations at 95°C for 15 seconds and 60°C for 1 minute. Data was collected and analyzed using the 7700 Sequence Detection System software v.1.6.3 (Applied Biosystems). Quantification of adenovirus copy number was performed using a standard curve consisting of dilutions of adenovirus DNA from 1,500,000 to 15 copies in 10ng of cellular genomic DNA. The average number of total copies was normalized to copies per cell based on the input DNA amount and a genome size of $6x10^9$ bp.

13.6.1 Results: Adenoviral hexon DNA copy number 4 hours post infection

E1A gene transcription occurs following adenoviral entry into the host cells and lasts approximately 6-8 hours, after which viral DNA replication is first detected (Russell, et al., Update on adenovirus and its vectors, Gen.Virol., 81 (pt. 11 ):2573-2604 (2000)). Thus, four hours following infection, adenoviral DNA replication has yet to begin in the host cells such that detection of DNA copies per cell at this time point should reflect the adenovirus particles that entered the cells during the infection period (ie, the transduction efficiency). Hexon DNA PCR was performed 4 hours post infection to determine the number of adenoviral particles that had been internalized by Wi38 and Wi38-VA13 cells. As all known components of the adenoviral transduction mechanism are integral components of the vectors used here, transduction efficiency was not expected to vary between these vectors and an average transduction efficiency was determined for each cell type and ppc used. At 100 ppc, there were an average of 0.13 adenoviral DNA copies/cell in Wi38 cells and 0.2 DNA copies/cell in Wi38-VA13 cells. At 1000 ppc, an average of 0.8 DNA copies/cell in Wi38 cells and 1.9 DNA copies/cell in Wi38-VA13 cells were detected (Table 29). These averages were used as transduction efficiencies to normalize the differences observed in hGM-CSF production between Wi38 and Wi38-VA13.

**Table 29.** Hexon DNA copy per cell in Wi38-VA13 and Wi38 at 4-hour post infection

| Vectors | Wi38-VA13 | | Wi38 | |
|---|---|---|---|---|
| | Ppc=100 | ppc=1000 | ppc=100 | Ppc=1000 |
| Ar6pAE2fhGmF | 0.22 | 2.03 | 0.11 | 0.48 |
| Ar6pAE2f(E3+,hGm,Dgp19b)F | 0.14 | 1.91 | 0.17 | 1.17 |
| Ar15pAE2fhGmF | 0.26 | 1.86 | 0.10 | 0.77 |
| Averages: | 0.2 + 0.06 | 1.9 $\pm$ 0.09 | 0.13 $\pm$ 0.04 | 0.8 + 0.35 |

Cells were infected with 100 or 1000ppc adenoviral vectors at 4°C for 1 hour, moved to a 37°C incubator, 5% $CO_2$, humidified incubator and the hexon DNA copy number was measured by PCR. Averages are means $\pm$ SD of the three determinations shown.

13.7 Human GM-CSF ELISA

Conditioned media from Wi38 and Wi38-VA13 cells were collected following 24 hours of infection with adenoviruses. The amount of human GM-CSF in the supernatant was determined by ELISA, using kits purchased from R&D Systems (Quantikine HS Human GM-CSF Immunoassay Kit, Catalog #HSGM0 and Quantikine Human GM-CSF Immunoassay Kit, Catalog #DGM00). Briefly, 100μl of Assay Diluent was loaded into each well of the hGM-CSF 96-well microplates using a multichannel pipette. One hundred fifty μl of the samples or hGM-CSF standards was added to each well, which had previously been coated with anti-human GM-CSF monoclonal antibody. The plates were incubated at room temperature for 3 hours, the solutions were aspirated and the wells were washed five times with washing buffer. Two hundred μl of hGM-CSF conjugate solution was added to each well and incubated at room temperature for 2 hours. The plates were washed 5 times, 50μl of Substrate Solution was added to each well and the plates were incubated at room temperature for 1 hour. Fifty μl of the amplifier solution was added to each well and further incubated for 30 minutes. At the end of the incubation, 50μl of Stop Solution was added and the optical density was measured using a Molecular Devices SpectraMAX 190 microplate reader with the wavelength set to read the OD at 490nm. The concentrations of hGM-CSF in the diluted unknown samples were calculated from the standard curve based on the OD490 values. The concentrations of hGM-CSF in the original (neat) test samples were calculated by multiplying the hGM-CSF concentrations obtained as the read-out from the plate reader by the sample dilutions.

13.7.1 Results: Human GM-CSF levels in culture supernatant

The oncolytic vector Ar6pAE2fhGmF and its derivatives utilize the E2F-1 promoter to control the expression of E1A. E1A proteins then transactivate the E3 promoter to induce the expression of hGM-CSF. Wi38-VA13 cells are an SV40 transformed derivative of Wi38. In Wi38-VA13 cells, the T-Ag binds to the Rb/E2F complex and releases E2F protein. These free E2F proteins then bind the E2F binding site of the E2F promoter in Ar6pAE2fhGmF, leading to the transcription of E1A and in turn, hGM-CSF. Wi38-VA13 and Wi38 cells were infected with the oncolytic adenovirus vectors Ar6pAE2fhGmF, Ar6pAE2f(E3+,hGm,Dg19b)F or Ar15pAE2fhGmF at 100 and 1000 ppc. Culture supernatants collected 24 hours after infection were analyzed by ELISA for hGM-CSF expression. Since the number of viruses transducing the two cell lines could vary, differences in GM-CSF production could be a reflection of different transduction efficiencies. To exclude this possibility, hGM-CSF production detected by ELISA was normalized to the number of virus copies in the cells. A 4-hour post-infection hexon DNA PCR served this purpose. The normalized levels of hGM-CSF in Wi38-VA13 cells infected with all three adenovirus vectors were substantially higher compared to the levels produced by normal Wi38 cells. In addition, the amounts of hGM-CSF produced in Wi38-VA13 cells by the three vectors are at comparable levels, indicating that the structural differences between the vectors do not have obvious effects on the levels of GM-CSF transcription in vitro. These results suggest that the E2F promoter is substantially more active in Wi38-VA13 cells than in Wi38 cells, and the promoter is regulated by the level of E2F in the cells.

13.8 Human GM-CSF FACS analysis

13.8.1 Monoclonal Antibodies (mAbs):

GM-CSF specific monoclonal antibodies were purchased from BD/PharMingen (San Diego, CA). Phycoerythrin (PE) conjugated rat anti-human GM-CSF monoclonal antibody (IgG2a, catalogue #18595A, clone BVD2-21C11) was used to stain cells infected with adenovirus vectors. PE conjugated rat IgG2a, κ monoclonal antibody (catalogue #20625A, clone R35-95) was used as an isotype control.

13.8.2 Intracellular staining of human GM-CSF and FACS analysis

Cells were fixed and permeabilized in 250μl/tube of cytofix/cytoperm solution (BD/PharMingen, catalogue #554722) at 4°C for 20 minutes. Cells were washed and incubated with 2μl of anti-human GM-CSF monoclonal antibody at 4°C for 30 minutes in the dark. Cells were washed twice in 1x perm/wash solution (BD/PharMingen, catalogue #552723) and resuspended in PBS prior to flow cytometric analysis.

13.8.3 Data acquisition and analysis

Cells were acquired and analyzed with the use of CellQuest software on a Becton Dickinson FACSCalibur System. The photomultiplier tube (PMT) voltage was set using the isotype control stained cells. The homogeneous cell population was gated on the forward scatter (FSC) and side scatter (SSC) dot plot and the marker was set to the position where fewer than 2% of cells fall in the range of positive using the isotype control antibody stained cells. In most cases, 10,000 gated events were collected and analyzed. Data were displayed on histograms to determine the percentage of cells that express GM-CSF.

13.8.4 Results: Intracellular detection of human GM-CSF in Wi38-VA13 cells.

In addition to measuring bulk GM-CSF production in the supernatants of cells infected with hGM-CSF armed oncolytic adenoviral vectors, GM-CSF production was assessed at the single-cell level by flow cytometry. This provides information on the percentage of cells in the culture that are producing hGM-CSF. At 24 hours following infection, Wi38-VA13 cells infected with 1000 ppc hGM-CSF armed vectors were up to 40% positive for GM-CSF expression, while only about 2% (background levels) of Wi38 cells were positive. These data further demonstrate the correlation between cellular E2F-1 levels and regulation of GM-CSF expression.

13.9 Summary

In cancer therapy, replication competent adenoviruses have a major advantage over replication defective viruses because the therapeutic effect of the injected virus is expected to be augmented by viral replication within the tumor. The tumor selective E2F-1 promoter is used in the oncolytic adenovirus vector Ar6pAE2fhGmF to replace the wild-type adenoviral E1 promoter. The selectivity of the E2F-1 promoter is most likely based on the derepression of the E2F-1 promoter transactivator in pRb-pathway disrupted tumor cells.

The isogeneic Wi38-VA13 and Wi38 cell system was used in this study. Wi38 is a normal fibroblast cell line derived from embryonic lung tissue that has an intact Rb pathway. The Wi38-VA13 cell line is an SV40 transformed cell derived from Wi38 in which the SV40 T-Ag has disrupted the pRb-E2F pathway, resulting in high levels of free E2F transcription factor. This model was used to evaluate the influence of cellular E2F levels on E3 promoter driven GM-CSF production in oncolytic adenoviruses in which E1A expression is under the control of the E2F-1 promoter.

High hGM-CSF production was observed in infected Wi38-VA13 cells and minimal production was observed in Wi38 cells. These data suggest that the E2F-1 promoter is selectively activated in cells with abundant E2F levels, resulting in tumor selective production of GM-CSF. Limiting the production of GM-CSF to tumors should reduce possible toxicities as a result of systemic expression of GM-CSF (Emenens et al. "Chemotherapy: friend or foe to cancer vaccines?" Curr Opin Mol Ther, 3(1):77-84 (2001)). At the 24 hour time point analyzed, differences in GM-CSF production between Wi38 and Wi38-VA13 cells is the sum total of a cascade of molecular events initiated by differential activation of the E2F-1 promoter, resulting in transcription/translation of E1A, initiation of viral replication and activation of the E3 promoter. These events are selectively amplified in Wi38-VA13 cells such that GM-CSF production by the three viral vectors was 13 to 60 fold higher than in the E2F-1 low Wi38 cells.

In summary, we have provided evidence using the Wi38-VA13 model system that the human E2F-1 promoter in Ar6pAE2fhGmF and related vectors are capable of selectively regulating adenoviral E1A gene transcription and downstream E3 promoter regulated hGM-CSF expression in pRb-pathway defective cells.

Example 14: In Vivo Spread of Oncolytic Adenoviruses Through Tumors

Ar6pAE2fF is a replication-competent oncolytic adenovirus in which the E2F-1 tumor selective promoter regulates the expression of adenoviral E1A. To improve efficacy, another adenovirus, Ar6pAE2fE3F was constructed which contains the entire adenoviral E3 region. The E3 region has long been considered unnecessary for replication of adenovirus *in vitro* and has been frequently deleted from adenoviral gene therapy constructs. The E3 region is known to encode seven proteins designed to counteract host antiviral responses (Wold *et al.,* 1995; 1999). In addition to encoding immunoregulatory genes, the E3 region also contains the 11.6kDa adenovirus death protein (ADP), which mediates efficient lysis of infected cells and the release of the infectious virions. Similarly, Ar6pAE2fGmF and its E3-containing variant Ar6pAE2f(E3+,Gm,Dg19)F (lacking only the gp19 region), were constructed and have been tested. In this example, the spread of virus in solid tumors was estimated by detecting changes in the percentage of hexon positive cells following *in vivo* administration of viral vectors to H460 and Hep3B tumor xenografts.

14.1 Adenoviral Vectors

The oncolytic vector Ar6pAE2fF is an E3-deleted adenovirus vector in which the E1A promoter is replaced with the tumor selective human E2F-1 promoter to achieve selective viral replication in tumor cells. Ar6pAE2fE3F is an oncolytic adenoviral vector similar to Ar6pAE2fF except that it contains the majority of E3 genes. Ad*dl*312 is a replication-defective adenovirus that has a deletion of the E1A region and was used as a negative control vector. Ar6pAE2fhGmF is an oncolytic vector with the same E3 deletion as Ar6pAE2fF and carries the human GM-CSF cDNA under control of the E3 promoter. Ar6pAE2f(E3+,Gm,Dg19)F is an oncolytic adenoviral vector that carries the human GM-CSF cDNA in the E3-gp19 position and expresses most of the remaining E3 genes.

14.2 Tumor Cell Lines

Two tumor cell lines were used in nude mouse xenograft models. Human non-small cell lung carcinoma cell line H460 (NCI-H460, ATCC HTB-177) and human hepatocellular carcinoma cell line Hep3B (Hep3B2.1-7, ATCC HB-8064) were obtained from American Type Culture Collection (ATCC, Manassas, VA). H460 cells were cultured in RPMI1640 medium containing 10% fetal bovine serum (FBS) and 2mM L-glutamine. Hep3B cells were cultured in Earl's modified eagle media (EMEM) containing 10% FBS.

14.3 Xenograft Nude Mouse Tumor Models

Six to 8 week old female athymic outbred *nu*/*nu* mice (Harlan Sprague Dawley) were used in both models. All studies were conducted according to Genetic Therapy, Inc. animal facility regulations (AAALAC-accredited).

H460 tumor model:

Two million H460 tumor cells, resuspended in 0.1 ml of HBSS were implanted subcutaneously in the right flanks of the mice. When tumor volume reached -200 $mm^3$ (calculated by $V=(W^2xL)\pi /6$; V, volume; W, width; L, length), 7-9 days after tumor inoculation, the animals were distributed into groups of similar tumor sizes and intratumoral administration of Ar6pAE2fF, Ar6pAE2fE3F, Add/312 adenoviruses or HBSS was initiated. A single injection of $2x10^{10}$ viral particles in a volume of 30μl per mouse was administered. Tumors were excised at 1, 4 or 7 days after the injection for hexon FACS analyses.

Hep3B tumor model:

Ten million Hep3B tumor cells were resuspended in 0.1 ml of HBSS and implanted subcutaneously in the right flanks of the mice. When tumor volume reached - 200 $mm^3$, 12-14 days after tumor inoculation, intratumoral administration of Ar6pAE2fGmF, Ar6pAE2f(E3+,Gm,Dg19)F, Ad*dl*312 adenoviruses or HBSS was initiated. A single injection of $2x10^8$ viral particles per mouse was given. Tumors were excised at 1, 4 or 7 days after the injection for hexon FACS analyses.

14.4 Preparation of Single Cell Suspensions from Tumors

One, 4 or 7 days post intratumoral viral injection, 10 mice from each group were sacrificed and the subcutaneous tumors were excised. Tumors were cut into small pieces and dissociated with collagenase (Sigma, Catalog C5138) treatment at 37°C for 1 hour at 2 mg/ml/100mg tissue. Cells were then washed with PBS and passed through a 100μm nylon cell strainer (Becton Dickinson Labware, Catalog 352369), washed and resuspended in PBS.

14.5 Immunofluorescent Staining of Tumor Cells

Tumor cell surface MHC class I staining

The tumor cells from each mouse were spun down and incubated with purified rat anti-mouse CD16/CD32 (Fcγ III/II receptor) mAb (BD PharMingen 01240D, clone 2.4G2) to reduce the nonspecific background cause by Fc receptor binding. Cells were then stained with cy-chrome conjugated human HLA-A,B,C mAb (BD PharMingen 32298X, IgG1, κ, clone G46-2.6), a pan human MHC class I antibody, for 30 minutes at 4°C in the dark. A cy-chrome labeled mouse IgG1 mAb (BD PharMingen 33818X, IgG1, κ clone MOPC-21) was used as an isotype control mAb. Cells were washed with PBS prior to fixation and permeabilization.

Fixation and permeabilization of cells

Cells were resuspended in 250μl/tube of cytofix/cytoperm solution (BD PharMingen 554722, Lot M065585) and incubated at 4°C for 20 minutes. Cells were washed in 1ml of 1 x perm/wash buffer (BD PharMingen 552723, Lot M065585) and spun down.

Intracellular staining of adenoviral hexon

Cells were resuspended in 50μl of perm/wash solution. A FITC labeled anti-hexon mAb ($0.5\mu g/10^6$ cells, Chemicon International Inc., Mab8052, custom conjugated with FITC, clone 20/11, IgG1,κ) was added and staining was carried out for 30 minutes at 4°C in the dark. A FITC labeled mouse IgG1, κmAb (BD Pharmingen cat #20604A, clone MOPC-21) was used as an isotype control.

14.6 Flow cytometric data acquisition and statistical analysis

Cells were acquired and analyzed with the use of CellQuest software on a Becton Dickinson FACSCalibur System equipped with dual laser and four-color fluorescence capability. The photomultiplier tube (PMT) voltage was set using the unstained or isotype control cells such that fewer than 2% of the cells were positive. The gate was set to the large (tumor) cell population on the SSC vs FSC dot plot and the compensation was set using the single cy-chrome or single FITC positive cells. Usually 10,000 gated events were collected and analyzed. The data were displayed on cy-chrome/FITC two-color dot plots to determine hexon positive human tumor cells. Statistical analysis was performed using SigmaStat Software. The percentages of hexon positive cells between groups were compared with a one-way repeated measure analysis of variance (ANOVA) using the Student-Newman-Keuls (SNK) test.

14.7 Results and Discussion

14.7.1 Viral Spread in H460 Tumors

The oncolytic adenovirus Ar6pAE2fF, which lacks the E3 region, has been shown to selectively replicate in and lyse tumor cells *in vitro* and *in vivo* due to its E2F-1 tumor selective promoter. Ar6pAE2fE3F, a vector otherwise identical to Ar6pAE2fF, contains the majority of the E3 region, including the ADP protein for efficient host cell lysis. In this example, the spread of Ar6pAE2fF and Ar6pAE2fE3F in xenografted H460 tumors was compared. The subcutaneously grown human H460 tumors in nude mice were given one dose of the viruses ($2x10^{10}$ particles/mouse) by intratumoral injection. Mice (n=10) were sacrificed 1, 4 or 7 days after the viral injection. Tumors from each mouse were analyzed for intracellular hexon expression by FACS using hexon specific. Anti-human HLA-A,B,C mAbs were used to differentiate human tumor cells from mouse cells. Over 90% of the cells from the tumors were HLA-A,B,C positive. One day after the virus injection, hexon expression was detectable in tumors injected with Ar6pAE2fF and Ar6pAE2fE3F (Figure 43A). Four days after the injection, the percentage of hexon positive cells were significantly higher in Ar6pAE2fF and Ar6pAE2fE3F injected tumors than in the negative control groups (Figure 43B). In addition, there was a significantly higher percentage of hexon positive cells in the E3-containing vector Ar6pAE2fE3F infected tumors than in the Ar6pAE2fF infected tumors. Similarly, at day 7, hexon positive cells were significantly higher than control groups in both Ar6pAE2fF and Ar6pAE2fE3F injected tumors and there were significantly more hexon positive cells in Ar6pAE2fE3F infected tumors than in Ar6pAE2fF infected tumors (Figure 43C). These results suggest that the oncolytic adenoviruses effectively spread in the tumor, regardless of the E3 status of the vector. In addition, despite similar initial day 1 levels, the E3-containing adenovirus spread significantly more rapidly in the H460 solid tumor than the E3-deleted viruses. As the Ar6pAE2fE3F vector lacked the native E3-14.7 gene, this gene was not required for efficient spread through a tumor *in vivo.*

14.7.2 Viral Spread in Hep3B Tumors

Ar6pAE2fhGmF is an oncolytic adenovirus with the same E3 deletion as Ar6pAE2fF that carries the human GM-CSF cDNA under control of the E3 promoter. Ar6pAE2f(E3+,hGm,Dg19b)F is an oncolytic adenovirus vector that also carries human GM-CSF and expresses most of the genes of the E3 region with the exception of gp19 and E3-14.7. The addition of GM-CSF into the adenovirus vector has the potential of inducing specific antitumor immune responses (e.g., tumor-specific CTL) in tumor-bearing hosts. Human Hep3B tumors in nude mice were given a single viral dose ($2x10^8$ particles/mouse) by intratumoral injection. Mice (n=10) were sacrificed one day, four days and seven days after the injection and tumor cells from each mouse were analyzed for intracellular hexon expression by flow cytometry. Contrary to H460 tumors, Hep3B xenograft tumors taken from nude mice expressed low levels of MHC-Class I antigen although Hep3B expressed high levels of MHC-Class I antigens *in vitro* (data not shown). Tumor cells were discriminated from mouse cells by the unusually large size of the tumor cells. As in the H460 studies, the oncolytic adenoviruses effectively spread in the tumor, regardless of the E3 status of the vector and the E3-containing adenovirus spread significantly more rapidly in the Hep3B solid tumor than the E3-deleted viruses (Figure 44). Hexon staining in tumors treated with Add/312 failed to rise above the background staining following PBS treatment. As the Ar6pAE2f(E3+,hGm, Dg19b)F vector lacked the native E3-gp19 and E3-14.7 genes, these genes were not required for efficient oncolysis and spread through a tumor *in vivo.*

One limitation of nearly all current cancer gene therapy approaches is the low efficiency of therapeutic gene delivery *in vivo* to the tumor site. One approach to overcome this problem is to use replication competent adenoviruses. First, this approach takes advantage of the virus' lytic nature of infection to lyse the tumor cells and contribute to anti-tumor efficacy. Second, the therapeutic gene carried in the virus can be replicated and delivered to more tumor cells through the local spread of the virus. The oncolytic adenovirus Ar6pAE2fF uses a tumor selective promoter E2F-1 to achieve tumor selective replication. Since the E3 region is considered unnecessary for replication of adenovirus, it was deleted from Ar6pAE2fF to accommodate potential therapeutic genes. However, many genes encoded in the E3 region are immunoregulatory genes that assist immune evasion by the virus. With this feature, it may be possible to express therapeutic genes carried in the virus for a prolonged period of time. In addition, ADP encoded by the E3 region has been shown to facilitate the lysis of the infected cells and viral spread from cell to cell. The studies reported in this example demonstrate a surprisingly high level of transduction *in vivo* following a single administration of oncolytic adenoviruses that was not fully dependent on an intact E3 region such that most (as in Ar6pAE2fF and Ar6pAE2fhGmF) or specific parts (as in Ar6pAE2fE3F and Ar6pAE2f(E3+,hGm,Dg19b)F) of the region could be deleted.

Nevertheless, the E3 containing viruses Ar6pAE2fE3F and Ar6pAE2f(E3,Gm,Dg19)F spread more efficiently through tumors than their E3 deleted counterparts, Ar6pAE2fF and Ar6pAE2fGmF (Figures 43 & 44). The E3 region encodes several proteins that have immunoregulatory functions. The E3-gp19kDa protein has been shown to downregulate surface MHC Class I molecules and RID$\alpha$ and $\beta$ have been shown to downregulate TNF family receptors on the surfaces of infected cells (reviewed in Horwitz, MS. Adenovirus immunoregulatory genes and their cellular targets. Virology 279:1-8, 2001). However, how these immunoregulatory E3 functions relate to the tumor regressions seen in the xenograft models can not be discerned in the immunocompromised nude mice. The effects reported here in xenograft tumor models could best be explained by the effect of the addition of the ADP function to the vectors.

In summary, with the detection of intracellular hexon expression by flow cytometry, oncolytic adenoviruses effectively spread in the tumor, regardless of the E3 status of the vector. Nevertheless, the E3-containing adenoviruses spread significantly more rapidly in both the H460 and Hep3B solid tumors than the E3-deleted viruses. In the nude mice used in these studies, the increased spread of E3 containing adenoviruses may be due to the lytic function of the ADP protein.

Example 15: Construction of the dual promoter-controlled vector

15.1 Plasmid generation:

The entire cloning scheme is described below and is outlined in Figure 45. The diagrams of the shuttle plasmid, pDr17TrtexF, and the large plasmid containing the entire viral genome, pAr17pAE2fFTrtex, are depicted in Figure 46. Confirmatory restriction digests and sequencing were performed at each step.

15.1.1 Removal of E4 promoter sequences:

The deletion of the E4 promoter encompasses the Ad5 wild type sequence from 35575 to 35786 (all numbers refer to the wild type Ad5 sequences as assigned in the Genbank sequence accession number M73260; Fang B, Koch P, and Roth JA. Diminishing adenovirus gene expression and viral replication by promoter replacement. J. Virol 1997; 71:4798-4803). The starting plasmid for creating this was pDr2FE3 (equivalent region in the plasmid map at position 2286 to 2496). The plasmid pDr2FE3 is described in Example 10 (Section 10.1.1). There is a PacI site inserted at Ad bp 35575, that is not in the wild type Ad sequence but engineered into this plasmid previously. Using the primers OV20 and OV21 (Table 30), a fragment of DNA was PCR-amplified which corresponds to the Ad sequence from bp 35213 to 35574, plus the additional restriction sites BamHI and PacI to the end at 35574. The plasmid pDr2FE3 and the PCR-amplified fragment were digested with the enzymes BstEII and PacI. The large fragment of the plasmid and the expected PCR fragment were gel-purified and ligated together. The ligation was transformed and minipreps were grown and screened by restriction digests. The correct clone, pDr12FE3, has a deletion from Ad bp 35574 to 35786, deleting the E4 promoter, and adding a newly engineered BamHI site juxtaposed to a PacI site. Either or both of these sites can be used for inserting exogenous promoters and/or enhancers.

**Table 30**. Primers used to make E4 promoter deletion.

| Primer name | Sequence 5' to 3' | Corresponding Ad bases |
|---|---|---|
| OV20 | GGCGTGACCGTAAAAAAACTGGTCACCGTGAT (SEQ ID NO: 73) | 35213 to 35244 |
| OV21 | CGCCTTAATTAAGGATCCGAGTGGTGTTTTTTTAATAGG (SEQ ID NO:74) | PacI+BamHI+ 35574 to 35554 |

15.1.2 Cloning Trtex into the E4 promoter:

The Trtex promoter fragment was cut from the Geron plasmid, pGRN316 (received from Geron by MTA), using

the enzymes NheI and EcoRI which released a fragment of 252 bp. This fragment corresponds to 252 bases upstream of the translational start site for a Tert coding sequence (Takakura M, Kyo S, Kanaya T, Hirano H, Takeda J, Yutsudo M, and Inoue M. 1999; Cancer Research 59: 551-557). This was purified and the overhanging ends were filled in with Klenow. The plasmid generated above, pDr12FE3, was digested with BamHI, the ends were filled in and dephosphorylated, and then used for ligation with the promoter fragment. Colonies were generated and screened for insertion and orientation by restriction digestion and sequencing. This plasmid was called pDr12TrtexFE3.

15.1.3 Reconstitution of E3 in pDr12TrtexF:

While the plasmid pDr2FE3 does contain an E3 region, there is additional foreign sequence, a loxP site, inserted in frame in the N-terminus of the 14.7K gene that creates a change in the first six amino acids of the 14.7K protein. To create a vector with a completely wild type version of the E3 region, Ad5 viral DNA was digested with NotI and SphI (29510 to 31225) and the 1715 bp fragment was isolated. The plasmid pDr2FE3 was digested with SphI and NotI and the viral fragment was inserted into this region. The resulting plasmid, pDr4F, was screened by sequencing. PDr4F now contains the completely wild type E3 region and the packaging signal at the viral right end upstream of the ITR.

The next cloning step was performed to add the wild type E3 region into the plasmid pDr12TrtexFE3. PDr4F was digested with ClaI and StuI and an 8.4-kb fragment isolated. The pDr12TrtexFE3 was digested with ClaI and StuI and a 4.3-kb fragment was isolated. These fragments were ligated together and plasmids were generated and screened. The final construct shown in Figure 46 was sequenced in the E3 region and designated pDr17TrtexF; it contains the Trtex promoter driving the E4 transcription unit, the packaging signal at the right ITR, and a fully wild type E3 region.

15.1.4 Large plasmid generation:

The final plasmid used to make the virus was generated from plasmids pAr6pAE2fF and pDr17TrtexF by homologous recombination in bacteria as described above. The final large plasmid, designated pDr17pAE2fFTrtex (Figure 46) was confirmed by restriction digests and by sequencing relevant regions.

15.2 Oncolytic vector generation:

The oncolytic vector was generated as described above using the plasmid pAr17pAE2fFTrtex.

15.2.1 Restriction digests and sequence confirmation:

At each step in the cloning process, the DNA was digested and sequenced to confirm its structure. The plasmid used to make the virus, pAr17pAE2fFTrtex, was digested with the restriction enzymes EcoRI, XhoI, SwaI, and EcoRV. After generation of the virus, the viral DNA from Ar17pAE2fFTrtex was extracted and digested with the indicated enzymes. All digestions resulted in the predicted fragments. The virus was also sequenced for the critical regions, namely E3, Trtex·promoter, and packaging signal at the right end, and the E2F-1 promoter at the left end. Sequence of the right end is shown in Figure 47.

15.3 Summary:

The oncolytic vector, Ar17E2fFTrtex was designed and constructed as detailed above and is diagrammed in Figure 48.

This vector has the same left end as described for Ar6pAE2fF (see Figure 6). Briefly, the packaging signal has been relocated to the right end of the virus. An SV40 polyadenylation signal has been inserted just downstream of the left ITR. The E2F-1 promoter has been inserted upstream of the E1a transcription unit. In this vector, the E3 region has been reconstituted to be completely wild type. The endogenous E4 promoter and transcription start site has been deleted and Trtex (hTERT promoter fragment from -252 to +1, relative the hTERT translation start site) has been inserted. Remaining are 74 bp of endogenous Ad5 sequences upstream of the first E4 translational start site (E4 orf1) and now downstream of the Trtex promoter insertion. All plasmids and the final vector were confirmed to be correct by restriction digests and sequencing.

Example 16. *In vitro* activity of Ar17pAE2fFTrtex on relevant biological targets

Adenovirus infection is a complex biological process involving the interplay between host and viral processes (e.g. virus-cell binding, internalization, release from the endosome, trafficking to the nucleus, DNA replication, viral packaging, and lysis) (Russell WC. Update on adenovirus and its vectors. J. of Gen. Virol. 2000; 81:2573-2604). Cell lines can differ dramatically from one another in their permissiveness to Ad infection. The *in vitro* studies described here use a diverse panel of tumor cell lines with Rb-pathway defects and hTERT activation (Table 31). In addition, primary human cell cultures were used as normal cell controls. In order to focus on the relevant selective function of Ar17pAE2fFTrtex in each cell type, Ar17pAE2fFTrtex was directly compared to the Ad5 wildtype virus or normalized to the number of adenoviral genomes per cell. The key difference between Ad5 and Ar17pAE2fFTrtex is that the wildtype E1a and E4 promoters have been replaced with tumor-selective E2F-1 and hTERT promoters, respectively. Thus, Ad5 was used as a non-selective positive control to determine whether the replacement of the wildtype E1a and E4 promoters with E2F-1 and hTERT promoters was sufficient to confer tumor cell line-selective early gene expression, virus production, and cell killing.

**Table 31**. Rb-pathway and hTERT status of cell lines

| | Tissue origin | hTERT | Rb pathway |
|---|---|---|---|
| Hep3B | Hepatocellular carcinoma | -* | - |
| LNCaP | Prostate tumor | - | - |
| PC-3M2AC6 | Prostate tumor | - | - |
| SW480 | Colon tumor | - | ** |
| HCT116 | Colon tumor | - | - |
| WI-38 | Normal lung fibroblast | + | + |
| PHH | Normal hepatocytes | + | + |

The hTERT status was determined by RNA expression. +, indicates wildtype status. -, indicates defect in Rb-pathway or that hTERT is expressed. *, indicates that while the hTERT status of Hep3B is unknown it is likely to be expressed because c-myc, a known transcriptional activator of hTERT (Oh S, Song YH, Kim UJ, Yim J, Kim TK. In vivo and in vitro analyses of Myc for differential promoter activities of the human telomerase (hTERT) gene in normal and tumor cells. Biochem Biophys Res Commun. 1999 Sep 24;263(2):361-5), is upregulated. **SW480 cells have been reported to have higher phosphorylated Rb and less DNA binding, indicative of a potential p16 defect (Gope R, Gope ML. Abundance and state of phosphorylation of the retinoblastoma susceptibility gene product in human colon cancer. Mol Cell Biochem. 1992 Mar 25; 110(2):123-33).

16.1 Transduction of tumor and normal cells *in vitro*

16.1.1 Rationale

The ability to transduce human cells by oncolytic adenoviruses is required for oncolysis. We evaluated the transducibility of the cell lines used in our *in vitro* systems by measuring the viral copy number per cell following transduction.

16.1.2 Methods

When measured before the onset of replication, adenoviral DNA copy number per cell can be used as a measure of viral transduction. Average adenoviral hexon gene DNA copy numbers per cell four hours post-infection were calculated by real-time quantitative PCR, as described in Examples 4 and 13.

16.1.3 Results and conclusions

Efficient infection of cell lines by oncolytic vectors such as Ar17pAE2fFTrtex depends on levels of adenoviral receptors. Because these levels vary between cell types, the transduction efficiency of cell lines can differ. The cell cultures used here were all transducible by Ar17pAE2fFTrtex (Table 32). Hep3B, LNCaP and PC-3M2AC6 cells were the most susceptible to adenoviral infection, followed by SW480 and primary human hepatocytes. In this panel of cell lines, HCT116 and WI-38 cells were the least sensitive cells to infection.

**Table 32.** Viral DNA copy number per cell of Ar17pAE2fFTrtex

| Human cell cultures | Hexon copies per cell |
|---|---|
| Hep3B | 114.3 |
| LNCaP | 20.7 |
| PC3M2AC6 | 14.6 |
| SW480 | 8.1 |
| HCT116 | 2.7 |
| Wi38 | 2.9 |
| Primary hepatocytes (PHH) | 9.3 |

Transduction of each cell line was measured using real-time PCR for detection of the Ad hexon gene, as described in Examples 4 and 13. Cells were infected in duplicate at 100 particles per cell and DNA was isolated at four hours post-infection. Hexon copies per cell values represent the mean of duplicate samples.

16.2 Selective E1a and E4 expression

16.2.1 Rationale

The ability of oncolytic vectors to replicate in tumor cells is essential for their therapeutic effect. One of the parameters useful for measuring efficiency of viral replication is transcription of viral genes. Expression of E1a and E4 RNA levels were quantitated by real time reverse transcriptase PCR (real time RT-PCR) to determine selectivity of E2F-1 and hTERT promoter-mediated expression.

16.2.2 Methods

Selective E1a and E4 expression, parameters of oncolytic potential and selectivity, were measured by infecting human tumor cell lines and normal non-tumor cell cultures with Ar17pAE2fFTrtex. Briefly, a panel of tumor cell lines and normal cell cultures were infected with 100 particles per cell (ppc) of Ar17pAE2fFTrtex, Ad5 as a wildtype positive control, and Addl312, as a replication-defective negative control. RNA and DNA were isolated 4 hours after infection. The 4 hour time point allows sufficient time for detecting expression of viral E1a and E4 mRNA without viral DNA replication. A quantitative RT-PCR assay for E1a and E4 expression levels and a quantitative DNA PCR assay for adenoviral DNA copy number were used, as described in Examples 4 and 13.

16.2.3 Results

The mean values for E1 a and E4 expression levels at 4 hours post-transduction are shown in Table 33.

**Table 33**. E1a and E4 levels per viral genome

| | E1a RNA levels per Ad genome | | E4 RNA levels per Ad genome | |
|---|---|---|---|---|
| Cell line | Ar17pAE2fFTrtex | Ad5 | Ar17pAE2fFTrtex | Ad5 |
| PHH | 0.9 | 282.7 | 4.5 | 1137.3 |
| Hep3B | 46.1 | 4652.9 | 8.8 | 12341.7 |
| HCT116 | 166.1 | 1134.7 | 77.2 | 5293.7 |
| SW480 | 44.6 | 83.2 | 21.3 | 51.0 |
| LNCaP | 151.8 | 744.7 | 133.3 | 563.0 |

Mean E1 a and E4 transcript units per Ad genome were determined at four hours post-infection from duplicate samples, as described in Example 13. Wi38, not detectable.

In order to better quantify whether there was tumor cell line-selectivity, we calculated a "selectivity index" for early gene expression by Ar17pAE2fFTrtex. In evaluating the selectivity of an oncolytic vector *in vitro,* comparison with a wildtype control such as Ad5 can help control for differences in transduction efficiency between cell lines. Selectivity for tumor cell lines can be represented mathematically by a "selectivity index" value. A selectivity index value for a vector is simply expression of the particular early gene by an oncolytic vector relative to Ad5 on primary cells and tumor cells. Selectivity index values above "1" indicate tumor cell selectivity. For example, the selectivity index for E1a expression per viral genome ($SI_{E1a}$) can be calculated by the following equation:

$$SI_{E1a} = \frac{\text{E1a per Ar17pAE2fFTrtex genome}_{\text{tumor}}}{\text{E1a per Ar17pAE2fFTrtex genome}_{\text{primary}}}$$

The mean values for E1a and E4 expression levels and hexon DNA copy number were used to calculate the selectivity index (Table 34). In this case, the normal cell values used were from PHH.

**Table 34**. Selective expression of E1a and E4 in cells infected with Ar17pAE2fFTrtex.

| | Selectivity Index | |
|---|---|---|
| Cell line | E1a | E4 |
| Hep3B | 51.2 | 1.96 |
| HCT116 | 184.6 | 17.16 |
| SW480 | 49.6 | 4.73 |
| LNCaP | 168.7 | 29.62 |

Cells were infected with Ar17pAE2fFTrtex at 100 ppc and the RNA and DNA was harvested at four hr post-infection. The amount of E1a and E4 RNA was determined by RT-PCR and values were normalized to vector copy numbers per cell and then to Ad5 levels. This value was divided by the same value obtained for primary human hepatocytes (PHH) to give the selectivity index (SI). The vector is determined to be selective if the value is over one.

For Ar17pAE2fFTrtex, the selectivity in E1a expression for tumor cell lines was E2F-1 promoter-dependent since relative E1a expression was greater in tumor cells than in normal cells. The selectivity index ranged from 49.6 for SW480 cells to 184.6 for HCT116 cells. Selectivity was also seen with E4 expression. In all cases, the selectivity was above 1, ranging from 1.96 for Hep3B to 29.62 for LNCaP cells. The replacement of a tumor selective promoter for transcription of E4 is desirable since E4 can be expressed in the absence of E1a. We showed that in Addl312-infected cells, where the E1a transcription unit is deleted, there was still some expression of E4 RNA. Although this was at relatively low levels, this could result in unwanted toxicities *in vivo.*

Although there is selectivity for expression of the transcripts in tumor cells, the level of RNA from Ar17pAE2fFTrtex was still much lower than the level from Ad5 and its native promoters. These differences range from several fold to several logs difference between expression obtained from the oncolytic vector to that obtained by Ad5. This could reflect the relative strength of the promoters and may impact downstream viral processes such as production of progeny.

16.3 Selective production of virus

16.3.1 Rationale

The purpose of this assay is to measure the production of viral progeny using Ar17pAE2fFTrtex on various tumor and normal cells. The amount of vector produced will reflect the ability of a particular cell type to allow replication of the vector and is influenced by the expression of proteins encoded in regions E1a and E4. Since Ar17pAE2fFTrtex has tumor selective promoters driving the transcription of these genes, it is important to determine both the selectivity and the effect of these alterations on the normal viral life cycle.

16.3.2 Methods

Vector production was determined on five human tumor cell lines (Hep3B, HCT116, LNCaP, SW480, and PC-3M2AC6), one normal cell line (WI-38), and one primary cell culture (PHH). Initial infections were performed at a dose of 1 or 10 ppc with Ad5, Ar17pAE2fFTrtex, or Addl312 and were harvested on days three or six post-infection. The titer was then determined by limiting dilution. The procedure is described as follows:

Day -1: Plate set-up.

Cells in which adenovirus production was to be measured were counted and plated in 96 well plates at 1 x $10^4$ cells per well in a volume of 190μl of the appropriate cell culture media 24 hr prior to infection.

Day 0: Primary infection

Each adenovirus vector was diluted in the appropriate media to achieve the desired dose range in particles per cell (ppc) in a 10μl volume. For each vector on each cell line, the appropriate dose was applied in a 10μl volume to triplicate wells containing cells and 190μl of media for a total infection volume of 200μl. The infected cells were incubated at 37°C in a humidified 5% $CO_2$ incubator.

Day 3: Harvest of crude viral lysates and titer plate preparation

At day 3 or 6, the virus infection media was aspirated by using a multichannel pipette and 250μl of 1X THP buffer (200mM Tris, 50mM HEPES) was placed on the cells. To generate crude viral lysates (CVL), the 96-well plates were subjected to five freeze-thaw cycles by alternating between incubation of the plates on dry ice and at 37°C.
AE1-2a cells were used as the indicator cell line for adenovirus infection. They are derived from A549 cells and express the E1 a and E2a gene products under the control of glucocorticoid-responsive promoters (Gorziglia et al., 1996. Elimination of both E1 and E2a from adenovirus vectors further improves prospects for in vivo human gene therapy. J. Virol. 6,4173-4178). The expression of E1a and E2a is induced in the presence of 0.3μM dexamethasone. AE1-2A cells were plated in 96 well plates at 1 x $10^4$ cells per well in a volume of 200μl of Richter's, 5% FBS and 0.3μM dexamethosone 24 hr prior to the secondary infection with CVL. At least one 96 well plate is needed per virus replicate for the $TCID_{50}$ assay. The plates were incubated overnight at 37°C and 5% $CO_2$ until secondary infection.

Day 4: Secondary infection

The CVL was diluted either 1:10 or 1:100 in Richter's media supplemented with 5% FBS. A 20μl volume of diluted CVL was added to each well in the top row across the plate of AE1-2A cells. A multichannel pipette was used to do 10-fold serial dilutions down the 96 well plate by removing 22ul from each well in a row and adding it to the 200μl of volume in the row below. This was repeated for each row through Row G so that each row represents 12 cultures at each dilution. Row H was left uninfected as a control. The multichannel pipette tips were changed between each row of dilutions. The infected cells were incubated at 37°C and 5% $CO_2$ for 10 to 14 days.

Day 10 to 14: Scoring for cytopathic effect

Between days 10 and 14, the titer plates of AE1-2A cells were examined visually for cytopathic effect (cpe) caused by productive virus infections. A well is scored positive for cpe if any of the cells have rounded up or if a plaque/ foci has formed in any part of the well. The number of wells in a row that were positive for cpe was recorded.

$TCID_{50}$ calculation

The number of wells in a row that were positive for cpe was then entered into the $TCID_{50}$ calculation. A detailed description of the mathematics behind the $TCID_{50}$calculation is given in O'Reilly DR. Muller LK, Luckow VA. Appendix 6. In: Baculovirus Expression Vectors: A Laboratory Manual. Oxford: Oxford University Press, 1994:132-134.
A virus production Selectivity index ($SI_{Pro}$) was determined by the formula:

$$SI_{pro} = \frac{\text{Titer Ar17pAE2fFTrtex}_{\text{tumor}} \div \text{titer Ad5}_{\text{tumor}}}{\text{Titer Ar17pAE2fFTrtex}_{\text{primary}} \div \text{titer Ad5}_{\text{primary}}}$$

Values above one indicate that vector is selectively produced in tumor cells.

16.3.3 Results

An objective of this analysis was to assess vector production of the oncolytic vector on tumor and normal cells compared to that of Ad5. Virus production selectivity was determined and is represented in Table 36. This number is referred to as the virus production selectivity index ($SI_{pro}$) and the vector is considered selective if the number is above one. Since this normalizes the transduction efficiency of the vectors on each cell line or culture, $SI_{pro}$ can be compared among various cell lines, potentially reflecting the ability of that cancer type to support replication of the oncolytic vector. $SI_{pro}$ was determined relative to the normal cell cultures PHH or WI-38. Data shown was calculated from cells infected at 10 ppc and harvested at six days post-infection. Comparing these tumor cells to either PHH or WI-38 produced qualitatively similar results. In both cases, the SI was highest for LNCaP and Hep3B cells, with SW480 showing some selectivity when compared to WI-38. PC3M.2AC6 and HCT116 showed no selectivity.

**Table 36.** Selectivity index for vector production.

| | Selectivity Index | |
|---|---|---|
| Cell line | relative to PHH | relative to WI-38 |
| Hep3B | 2.42 | 134.97 |
| PC-3M2AC6 | 0.05 | 2.52 |
| SW480 | 0.67 | 37.26 |
| LNCaP | 3.99 | 222.3 |
| HCT116 | 0.01 | 0.29 |

Cell lines were infected with Ad5 and Ar17pAE2fFTrtex at an MOI of 10 and harvested at six days post-infection and vector production (pfu/ml) was determined. Selectivity index (SI) was calculated as described in the text. The normal cell was either primary human hepatocytes (PHH) or WI-38. Values above one indicate selectivity for tumor cells.

16.4 Selective *in vitro* killing

16.4.1 Rationale

The cytotoxicity of Ar17pAE2fFTrtex in tumor cell lines versus primary cell cultures *in vitro* was evaluated to determine the tumor-selectivity of cell killing.

16.4.2 Methods

The cytotoxicity assay was based on the Promega CellTiter 96® $AQ_{ueous}$ Non-Radioactive Cell Proliferation Assay and was performed as previously described in Example 8.

16.4.3 Results

Cytotoxicity is another measure of the potency of oncolytic vectors. A quantitative evaluation of the relative cytotoxicity of the adenoviral vectors can be performed by calculating the $LD_{50}$ values from the dose-response curves. The average $LD_{50}$ values for the dual promoter-controlled Ar17pAE2fFTrtex, the single promoter-controlled vector Ar13pAE2fF, and a positive control replication-competent virus Ad5 on each cell culture are reported in Table 37. Ar13pAE2fF is identical to Ar6pAE2fE3F described in Example 7 except that a loxP site present in the E3 region of Ar6pAE2fE3F has been removed. The $LD_{50}$ values for Ar17pAE2fFTrtex were similar to those of Ar13pAE2fF on Hep3B, LNCaP and SW480 tumor cell lines. On PC-3M2AC6 and HCT116 cells, the $LD_{50}$ values for Ar17pAE2fFTrtex were significantly higher than those of Ar13pAE2fF. In the primary cell cultures, the $LD_{50}$ value for Ar17pAE2fFTrtex was significantly higher on WI-38 cells. These results suggest that while the dual promoter-controlled Ar17pAE2fFTrtex has a potency equal to or less than that of the single promoter controlled Ar13pAE2fF on tumor cell lines, it also has equal or lower potency in the primary cell culture.

**Table 37.** $LD_{50}$ values on primary and tumor cell cultures: Ar17pAE2fFTrtex versus Ar13pAE2fF

| Cell line (no. experiments) | Ar17pAE2fFTrtex | | | Ar13pAE2fF | | | Ad5 | | |
|---|---|---|---|---|---|---|---|---|---|
| | mean | sd | n | mean | sd | n | mean | sd | n |
| Hep3B (4) | 0.06 | *0.05* | 12 | 0.01* | *0.01* | 13 | 0.01 * | *0.01* | 12 |
| LNCaP (3) | 14.0 | *11.6* | 9 | 4.4 | *3.5* | 8 | 0.3* | *0.4* | 8 |
| PC-3M2AC6 (3) | 4.2 | *1.5* | 8 | 1.5* | *0.6* | 8 | 0.1 * | *0.04* | 8 |
| SW480 (4) | 33.3 | *15.1* | 15 | 21.6 | *14* | 15 | 5.5* | *4.4* | 15 |
| HCT-116 (3) | 43.4 | *18.3* | 11 | 8.4* | *3.6* | 12 | 1.9* | *1.3* | 12 |
| Wi38 (4) | 2987 | *632* | 15 | 213* | *61* | 15 | 23* | *11* | 13 |

Abbreviations: n, number of replicates averaged. *, $p<0.05$ vs. Ar17pAE2fFTrtex (one-way ANOVA on $\log_{10}$ transformed data).

The $LD_{50}$ data indicate that although the Ar17pAE2fFTrtex and Ar13pAE2fF $LD_{50}$ values were higher than Ad5 for both tumor cell lines and primary cell cultures, the $LD_{50}$ values were closer to wildtype levels on tumor cell lines. In order to more easily compare tumor cell line-selectivity, a "selectivity index" was calculated for both Ar17pAE2fFTrtex and Ar13pAE2fF. In evaluating the selectivity of an oncolytic vector *in vitro,* comparison with a wildtype control such as Ad5 can be used to normalize for transduction efficiency differences between cell lines. Cytotoxicity selectivity for tumor cell lines can be represented mathematically by a "selectivity index" ($SI_{LD}$) value. An $SI_{LD}$ value for a vector is simply the $LD_{50}$ of an oncolytic vector relative to Ad5 on primary cells and tumor cells. The selectivity index has two components. First, the $LD_{50}$ ratio between the oncolytic vector and the wildtype virus on tumor cells gives a measure of the "potency" on tumor cells. Second, the $LD_{50}$ ratio between the oncolytic vector and the wildtype virus on primary cell cultures gives a measure of the "safety" on normal cells. For a tumor cell-selective vector, the ratio between the vector and the wildtype virus will be greater on the primary cell cultures than on the tumor cell lines. Dividing the $LD_{50}$ "potency" ratio by the "safety" ratio yields the $SI_{LD}$ value. $SI_{LD}$ values above "1" indicate tumor cell selectivity. The selectivity of a vector can be quantified by the following equation:

$$SI_{LD} = \frac{LD_{50}\,Ad5_{\,tumor} \div LD_{50}\,Ar17pAE2fFTrtex_{\,tumor}}{LD_{50}\,Ad5_{\,primary} \div LD_{50}\,Ar17pAE2fFTrtex_{\,primary}}$$

The selectivity index for Ar17pAE2fFTrtex and Ar13pAE2fF on the tumor cell lines versus the WI-38 primary cell cultures was calculated and is presented in Table 38. In all five tumor cell lines, the selectivity index for Ar17pAE2fFTrtex was higher than that of the single promoter-controlled Ar13pAE2fF. This suggests that the decrease in "potency" on tumor cells between Ar17pAE2fFTrtex and Ar13pAE2fF, was offset by an even greater increase in "safety".

**Table 38.** Cytotoxicity selectivity index ($SI_{LD}$) versus WI-38

| Cell line | Ar13pAE2fF | Ar17pAE2fFTrtex |
|---|---|---|
| Hep3B | 9.3 | 21.6 |
| LNCaP | 0.6 | 2.8 |
| PC-3M2AC6 | 0.6 | 3.1 |
| SW480 | 2.4 | 21.4 |
| HCT116 | 2.1 | 5.7 |

The $SI_{LD}$ is calculated for the indicated tumor cell lines versus WI-38 primary cells, according to the following formula: $LD_{50}$ Ad5 / $LD_{50}$ test vector on tumor cell line / $LD_{50}$ Ad5 / $LD_{50}$ test vector on Wi-38 primary cells.

16.5 Dependence of E4 expression on hTERT promoter

16.5.1 Rationale

E4 transcripts in Ar17pAE2fFTrtex-infected cells were characterized to determine whether replacement of the native E4 promoter with a hTERT promoter maintained the ability to express the E4 region in an hTERT promoter-dependent fashion.

16.5.2 Methods: Semi-quantitative RT-PCR

Semi-quantitative RT-PCR analysis was done on Hep3B cells infected with Ar17pAE2fFTrtex or wildtype Ad5. Primer sets were designed to distinguish transcripts initiating in the hTERT promoter of Ar17pAE2fFTrtex versus initiation from upstream cryptic start sites. The E4 transcriptional initiation sites for Ar17pAE2fFTertex were mapped by primer extension analysis.

Total RNA and gene specific oligonucleotide primers were used for E4 cDNA production in the SuperScript™ One-step RT-PCR with Platinum *Taq* system (Invitrogen, Carlsbad, CA. Cat. # 10928-034). RT-PCR was performed using the manufacturer's method with minor modification. The reaction was done using 0.4 to 0.6 ug of total RNA isolated from 10 ppc vector-infected Hep3B cells. In the presence of 0.2 uM specific primers and SuperScript/Platinum *Taq* mix in 1 x Reaction Buffer, reverse transcription and PCR was carried out at 50 °C for 30 min followed by 35 cycles of 94°C, 15 s; 60°C, 30 s; and 70°C, 30 s. Amplification products were analyzed on ethidium bromide-stained 1.2% agarose gels along with 100-bp DNA ladder (Invitrogen, Carlsbad, CA).

Oligonucleotide primers were synthesized by Sigma-Genosys (Woodlands, TX). Based upon Ar17pAE2fFTrtex and Ad5 vector genomes, the primers were designed and their sequences are listed in Table 39. The relative positions of each primer in the genomes are shown schematically in Fig. 49. The forward primers (PCR1.f and PCR2.f) contained different regions of E4 coding sequences and were used for both RT and PCR analysis. Reverse primer PCR 3.r contained 5' non-coding sequence of the E4 gene. PCR 4.r included 5' non-coding sequence of the hTERT transcript. This fragment is linked to the E4 transcript by cloning as schematically indicated in Fig. 49, and thus was specific for E4 transcription in Ar17pAE2fFTrtex. PCR 5.r contains non-coding sequence at 5' of hTERT or E4 promoter and 3' of right ITR sequences (and packaging signal if exists, Fig 49. Variable combinations of forward and reverse primers were used in RT-PCR analysis (Fig. 49), and the PCR products were used to evaluate specific E4 gene transcription and the origin of transcripts.

**Table 39.** Primer sequences

| Primer name | Property | Sequence 5' to 3' | SEQ ID NO: |
|---|---|---|---|
| PCR 1.f | Forward | GGAATACATACCCGCAGGCGTAGAGACAAC | 75 |
| PCR 2.f | Forward | CACATAAACACCTGAAAAACCCTCCTGCC | 76 |
| PCR 3.r | Reverse | TTTACTGGTAAGGCTGACTGTTAGGCTGC | 77 |
| PCR 4.r | Reverse | AGTTTCAGGCAGCGCTGCGTCCTGCTGC | 78 |
| PCR 5.r | Reverse | GGGCGGAGTAACTTGTATGTGTTGGGAATTG | 79 |
| ExtP 1 | Forward | ACAGCGCTTCACAGCGGCAGCCTAACAGTC | 80 |

16.5.3 Methods: Primer extension

Primer extension analysis was based on a method in Current Protocols in Molecular Biology (Ausubel et al., ed., Primer Extension Assay in Current Protocols in Molecular Biology, John Wiley & Sons, Inc., pp. 4.8.1 - 4.9.1, 1995) with modification to accommodate buffer components for SuperScript II reverse transcriptase (Invitrogen, Carlsbad, CA). ExtP1 contained 5'-noncoding sequence of the E4 gene and was used as a primer to map the 5' end of E4 transcript in the Ar17pAE2fFTrtex and Ad5-infected Hep3B RNA templates (Table 39, Fig. 51). The primers were labeled at their 5'-ends with [□-$^{32}$P] dATP to allow the detection of extension products. Thirty ug of total RNA isolated from 1 and 10 ppc viral-infected Hep3B cells was hybridized with 2x$10^5$ cpm radiolabeled ExtP1 in hybridization buffer (150 mM KCl; 10 mM Tris-HCl, pH 8.0; 1 mM EDTA) at 65 °C for 90 min. Primer annealing was carried out by gradually cooling the samples down to room temperature in a heating block. Primer extension was performed by the SuperScript II reverse transcriptase in RT buffer (50 mM Tris-HCl, pH8.0; 75 mM KCl; 13 mM $MgCl_2$; 10 mM DTT; 0.5 mM dNTP) at 42 °C for 2 hrs. Samples were then purified with phenol-chloroform extraction and ethanol precipitation. The primer extension products were isolated on a 7 M urea-6% polyacrylamide sequencing gel. The position of the 5'-end of the E4 transcript was determined by sequence analysis along with the vector sequence and was then defined as E4 transcription start site. As a negative control, the primer extension reaction was carried out in the absence of SuperScript II reverse transcriptase in the same condition and the reaction was analyzed along with the experiments as described above.

16.5.4 Results

E4 transcripts were readily detected in Ar17pAE2fFTrtex-infected Hep3B cells. PCR primers that were designed to detect read-through from cryptic transcriptional start sites failed to detect expression initiating upstream of this hTERT promoter. This suggests that E4 expression in Ar17pAE2fFTrtex is dependent on this hTERT promoter. To further verify this, the E4 transcriptional start sites in Ar17pAE2fFTrtex-infected cells were mapped by primer extension analysis. Three major transcription initiation sites were identified, all of which mapped to the hTERT promoter (Fig. 50).

Example 17. Efficacy of Ar17pAE2fFTrtex *in vivo*

A mouse xenograft model was used to evaluate both efficacy and selected toxicological endpoints following a single intravenous injection of Ar17pAE2fFTrtex at three doses, 1.5 x $10^{12}$, 3.0 x $10^{12}$ and 4.5 x $10^{12}$ particles/kg. Efficacy was assessed by measurements of tumor volume and survival. Analyses of vector content by hexon gene-specific quantitative PCR and gene expression by E1a and E4 region RT-PCR in tumor and normal tissues were made 3 days after vector administration. Ciinicai and anatomic pathology endpoints were evaluated one week and one month after vector administration.

17.1 Methods

17.1.1 Viruses and tumor cell line

Ar17pAE2fFTrtex, an oncolytic adenoviral vector with the native E1a promoter replaced with the E2F-1 promoter and the native E4 promoter replaced with the human telomerase promoter (hTrt), was prepared using standard cesium chloride gradient purification methods. Vector concentration was determined by optical particle titer. A non-replicating, E1a-deleted adenovirus, Add1312, was included in this study as a negative control vector (Young CSH, Shenk T, Ginsberg HS. The genetic system. In: Ginsberg HS, ed. The adenoviruses comprehensive virology, Vol. 4, New York: Plenum Press. 1984:125-172).

The human liver hepatocellular carcinoma line Hep3B (Hep 3B2.1-7; ATCC #HB-8064, batch number F-9462) was obtained from American Type Culture Collection (Manassas, VA) and found to be free of mycoplasma contamination. The Hep3B cells are cultured in Eagle's minimal essential media (EMEM) containing 10% fetal bovine serum (FBS).

17.1.2 Mouse tumor model study design

A Hep3B xenograft mouse tumor model was used to assess the effects of Ar17pAE2fFTrtex following a single intravenous injection at 3 doses (Table 40). Hep3B cells (1 x $10^7$ cells / 100 $\mu$l of HBSS) were implanted subcutaneously on the right flank of female athymic outbred nu/nu mice (Harlan, 6-8 weeks old). Tumor measurements were recorded twice weekly in two dimensions using calipers. Tumor volume was calculated using the formula Length x $Width^2$ x $\pi/6$. Body weights were recorded twice per week for the initial two weeks, then once per week for the duration of the study. An efficacy cohort of 89 mice (n/group = 17 -18; group mean tumor volume 147.73 - 151.93 $mm^3$) and a preliminary toxicity cohort of 60 mice (n/group = 15; group mean tumor volumes 147.45 - 148.19 $mm^3$) were selected and evenly distributed by tumor volume into five dose groups. Mice were dosed according to individual body weights collected on the day of vector administration. Mice were injected intravenously via the tail vein with Ar17pAE2fFhTrtex at 1.5x$10^{12}$ (n=33), 3.0x$10^{12}$ (n=32), or 4.5x$10^{12}$ (n=33) viral particles/kg in a final volume of 10 ml/kg. A replication deficient vector control group was injected with Addl312 at 4.5x$10^{12}$ (n=18, no preliminary toxicity) viral particles/kg in a final volume of 10 ml/kg, and a diluent control group was injected with HBSS (n=33), 10 ml/kg. Sub-sets of mice (n=3) were sacrificed three days after vector injection for preliminary analysis of vector distribution and expression of E1a and E4 genes. At one week and one month after vector injection, the remaining sub-sets of mice (n=5-6) were sacrificed for clinical and anatomic pathology evaluations.

**Table 40.** Study design

| Group No. | Test Material | Dose (vp/kg) | Dose Volume (mL/kg) | No. per Scheduled Sacrifice | | | |
|---|---|---|---|---|---|---|---|
| | | | | D4 | D8 | D29 | Efficacy |
| 1 | HBSS | - | 10 | 3 | 6 | 6 | 18 |
| 2 | Addl312 | 4.5e12 | 10 | - | - | - | 18 |

(continued)

| Group No. | Test Material | Dose (vp/kg) | Dose Volume (mL/kg) | No. per Scheduled Sacrifice | | | |
|---|---|---|---|---|---|---|---|
| | | | | D4 | D8 | D29 | Efficacy |
| 3 | Ar17pAE2fFTrtex | 1.5e12 | 10 | 3 | 6 | 6 | 18 |
| 4 | Ar17pAE2fFTrtex | 3.Oe12 | 10 | 3 | 6 | 6 | 18 8 |
| 5 | Ar17pAE2fFTrtex | 4.5e12 | 10 | 3 | 6 | 6 | 18 8 |

17.1.3. Survival criteria and analysis

Mice with tumors exceeding 2000 $mm^3$, mice that were found dead, and moribund mice that were humanely sacrificed were scored as study deaths. Deaths occurring less than 12 hours after intravenous injection were considered to be injection related and were excluded from the study (n=1). A Mantel-Haenszel logrank test of the data, which included both tumor-related and vector-related deaths, was performed.

17.1.4. Preliminary vector distribution and expression

Tumor and normal tissues, including liver, kidney, lung, bone marrow, brain, spleen, and ovary, were collected three days after vector administrations from 3 mice/group with the exception of Group 2. DNA was extracted from each tissue and quantitative PCR performed for the hexon gene. Tissues that were positive for hexon DNA were evaluated for E1a and E4 expression using RT-PCR methods.

17.1.5 DNA isolation from tissues

DNA from tissues was isolated using the Qiagen Blood and Cell Culture DNA Midi or Mini Kits (Qiagen Inc., Chatsworth, CA). Frozen tissues were partially thawed and minced using sterile disposable scalpels. Tissues were then lysed by incubation overnight at 55°C in Qiagen buffer G2 containing 0.2 mg/ml RNaseA and 0.1 mg/ml protease. Lysates were vortexed briefly and then applied to Qiagen-tip 100 or Qiagen-tip 25 columns. Columns were washed and DNAs were eluted as described in the manufacturer's instructions. After precipitation, DNAs were dissolved in water and the concentrations were spectrophotometrically determined ($A_{260}$ and $A_{280}$) on a DU-600 (Beckman Coulter, Inc.; Fullerton, CA) or a SPECTRAmax PLUS (Molecular Devices, Inc.; Sunnyvale, CA) spectrophotometer.

17.1 6 Hexon Taqman real-time PCR assay

PCR primers and a Taqman probe specific to adenovirus hexon sequences were designed using Primer Express software v. 1.0 (Applied Biosystems, Foster City, CA). Primer and probe sequences were:

Hexon Forward primer: 5'-CTTCGATGATGCCGCAGTG-3' (SEQ ID NO:81)

Hexon Reverse primer: 5'-GGGCTCAGGTACTCCGAGG-3' (SEQ ID NO:82)

Hexon Probe: 5'-FAM-TTACATGCACATCTCGGGCCAGGAC-TAMRA-3' (SEQ ID NO:83)

Amplification was performed in a reaction volume of 50 $\mu$l under the following conditions: 10 ng (tumor) or 1 $\mu$g (liver and lung) of sample DNA, 1X Taqman Universal PCR Master Mix (Applied Biosystems), 600 nM forward primer, 900 nM reverse primer and 100 nM hexon probe. Thermal cycling conditions were: 2 minute incubation at 50°C, 10 minutes at 95°C, followed by 35 cycles of successive incubation at 95°C for 15 seconds and 60°C for 1 minute.

Data was collected and analyzed using the 7700 Sequence. Detection System software v. 1.6.3 (Applied Biosystems). Quantification of adenovirus copy number was performed using a standard curve consisting of dilutions of adenovirus DNA from 1,500,000 copies to 15 copies in the appropriate background of cellular genomic DNA. For analysis of tumor tissues, a standard curve in a background of 10 ng human DNA was generated. For analysis of mouse liver and lung tissues, a standard curve consisting of the same adenovirus DNA dilutions in a background of 1 $\mu$g CD-1 mouse genomic DNA was generated. Samples were amplified in triplicate, and the average number of total copies was normalized to copies per cell based on the input DNA weight amount and a genome size of $6x10^9$ bp.

17.1.7 RNA isolation from tissue

Tissue samples were collected, directly placed into RNAlater™ (Ambion, Austin, TX) and stored at 4°C. Tissues stored for more that one week were placed at -20°C following an initial incubation at 4°C as per manufacturer's instructions. Tissues were cut into approximately 100-200mm$^3$ pieces using sterile scalpels. Each piece of tissue was placed in a BioPulverizer Green Tube (Bio101, Carlsbad, CA). One ml of RNAzol B (Tel-TEST, Friendswood, TX) was added and the tissue was immediately disrupted using the Fast Prep FP120 instrument (Bio101) according to manufacturer's instructions. Following disruption in RNAzol B, samples were extracted in 0.1 volume chloroform. The RNA was precipitated with one volume isopropanol, washed with 75% ethanol and resuspended in nuclease-free water. RNA samples were then treated with 10 Units DNase I (Life Technologies, Rockville, MD) at room temperature and purified using the RNeasy Mini Kit (Qiagen Inc., Chatsworth, CA). RNA concentration was determined spectrophotometrically ($A_{260}$ and $A_{280}$) on a DU-600 (Beckman Coulter, Inc.; Fullerton, CA) or the SPECTRAmax PLUS (Molecular Devices, Inc.; Sunnyvale, CA) spectrophotometer.

17.1.8 cDNA synthesis

First strand cDNA was generated from 100 ng of test sample RNA using Taqman Reverse Transcription Reagents (Applied Biosystems). The reverse transcription was performed in a 70 µl reaction volume at the following conditions: 1X TaqMan RT Buffer, 5.5 mM $MgCl_2$, 3.8 mM deoxyNTP mixture (0.96 mM of each deoxyNTP), 2.5 µM random Hexamer, 1 Unit Rnase Inhibitor and 2.5 Units of Multiscribe Reverse Transcriptase. The reactions incubated for 10 minutes at 25°C, 30 minutes at 48°C, 5 minutes at 95°C, and were then held at 4°C. A no-RT control reaction was performed for each test sample. The no-RT reaction mix was prepared by omitting the Multiscribe Reverse Transcriptase from the components listed above.

Primers specific for the adenovirus E1a and E4 sequences were designed using the Primer Express software v. 1.0 (Applied Biosystems, Foster City, CA). Primer and probe sequences are shown below. The numbers at the end of each oligonucleotide indicate the nucleotide positions found in plasmid pAr17pAE2fFTrtex (Fig. 46):

E1a Forward primer: 5'-AGCTGTGACTCCGGTCCTTCT-3' (1388-1408) (SEQ ID NO:84)
E1a Reverse primer: 5'-GCTCGTTAAGCAAGTCCTCGA-3' (1523-1503) (SEQ ID NO:85)
E1a Probe: 5'-FAM-TGGTCCCGCTGTGCCCCATTAAA-TAMRA-3' (1434-1456) (SEQ ID NO:86)
E4 orf63 Forward primer: 5'-TCTGTCTCAAAAGGAGGTAGACGA-3'(33993-34016) (SEQ ID NO:87)
E4 orf63 Reverse primer: 5'-GACCAACACGATCTCGGTTTGT-3' (34062-34042) (SEQ ID NO:88)
E4 orf63 Probe: 5'-FAM-CCCTACTGTACGGAGTGCGCCGA-TAMRA-3' (34018-34040) (SEQ ID NO:89)

The E4 probe and primer set is targeted to the E4 orf6 region. Amplification was performed in a reaction volume of 50 µl under the following conditions: 20 µl of sample cDNA, 1X Taqman Universal PCR Master Mix (Applied Biosystems), 300 nM forward primer, 900 nM reverse primer and 100 nM E1 a or E4 probe. Thermal cycling conditions were: a 2 minute incubation at 50°C, a 10 minute 95°C activation step for the Amplitaq Gold, followed by 35 cycles of successive incubation at 95°C for 15 seconds and 60°C for 1 minute. Thermal cycling was carried out with 7700 Sequence Detection System (Applied Biosystems). To assess RNA input (endogenous control), 10 µl of a 1:1000 dilution of each cDNA was amplified using a Pre-Developed Taqman Assay Reagent 18S kit (Applied Biosystems) as per manufacturer's instruction.

Data was collected and analyzed using the 7700 Sequence Detection System software v. 1.6.3 (Applied Biosystems). Relative levels of E1a and E4 were determined based on plasmid curve generated with dilutions from 1,500,000-15 copies. For endogenous control, input RNA was assessed by using an RNA standard curve. A standard curve consisting of tenfold dilutions from 1µg to 1 ng of cellular RNA (H460 was selected in this study) was reverse transcribed along with each set of test samples. Ten µl of a 1:1000 dilution of each standard curve point cDNA was amplified using Applied Biosystems Pre-Developed Taqman Assay Reagent 18S kit according to manufacturer's instructions. Amplifications of E1a, E4 and 18S RNA were performed in duplicate and the average value for each was reported.

17.1.9 Preliminary toxicity evaluations

17.1.10 In-life observations and measurements

Daily observations for morbidity and moribundity were performed. Body weights were collected one and three days after dosing (SD2 and SD4, respectively) to assess acute effects, then weekly for the duration of the study. Clinical pathology analyses were performed by AniLytics, Inc. (Gaithersburg, MD). Blood was collected by orbital bleed from 5 mice/group at SD2, 4, 8, 15 and 29 and processed to serum for measurement of ALT, AST, ALP, CPK, and Creatinine.

A complete blood cell count (CBC) was performed on whole, unclotted blood collected from 5 mice/group at SD4, 8, 15 and 29.

17.1.11 Post-mortem observations and measurements

Necropsies were performed on 5-6 mice/group on SD8 and SD29. Tissues were collected and placed in 10% neutral-buffered formalin, embedded in paraffin and processed to slides. Slides were stained with H&E and delivered to EPL (Herndon, VA) for microscopic evaluation by a pathologist. Tissues evaluated were tumor, liver, kidney, lung, brain, and spleen.

17.1.12 Statistical analyses

All quantitative data were tested for normality and variance. Using SigmaStat 2.03, one way analysis of variance (ANOVA) was used to determine whether statistically significant differences between treatment groups were present. If statistically significant differences were indicated, the Dunnett's t-test (parametric data) or Dunn's test (non-parametric data) was performed to distinguish significant differences from control groups. The level of significance was set at $p<0.05$ for all tests. For survival curves, a Mantel-Haenszel logrank test was performed using GraphPad Prism version 3.0.

17.2 Results

17.2.1 Tumor Volume

A xenograft model of hepatocellular carcinoma was used to assess the efficacy of Ar17pAE2fFhTrtex following systemic administration. A cohort of female nude mice formed tumors (91.6 - 218.5 $mm^3$) two weeks after subcutaneous injection of Hep3B cells into the right flank. A single intravenous injection of Ar17pAE2fFhTrtex at $1.5x10^{12}$ (n=18), $3.0x10^{12}$ (n=17), or $4.5x10^{12}$ (n=18) viral particles/kg in a final volume of 10 ml/kg showed a significant inhibition in tumor growth starting on study day 18 ($p < 0.05$) when compared to HBSS (n=18) injected controls (Figure 51). All three doses also showed a significant inhibition in tumor growth on study day 25 ($p < 0.05$) when compared to Addl312 (n=18) injected controls (Figure 51). However, no dose response was observed for Ar17pAE2fFTrtex over this relatively narrow dose range.

Tumor volume data expressed as the percent ratio of treated/control (%T/C) is shown in Table 49 for study days 18, 21, and 25. The lower tumor growth rate of the treated groups versus the HBSS control is reflected in the decreasing trend in %T/C values for all three treatment groups. Ar17pAE2fFhTrtex at $1.5x10^{12}$, $3.0x10^{12}$, and $4.5x10^{12}$ vp/kg have %T/C of about 55, 56, and 55, respectively, on SD25, the last day the HBSS group is intact.

**Table 49**. % T/C Values

| Treatment | SD 18 | SD 21 | SD 25 |
|---|---|---|---|
| Addl312 | 93 | 95 | 87 |
| Ar17pAE2fFTrtex 1.5e12 | 69 | 66 | 55 |
| Ar17pAE2fFTrtex 3.0e12 | 72 | 67 | 56 |
| Ar17pAE2fFTrtex 4.5e12 | 70 | 65 | 55 |

% T/C = mean tumor volume for treatment group divided by mean tumor volume for HBSS control group x 100.

17.2.2 Survival

The survival curves demonstrate significant survival enhancement for mice treated at each vector dose compared to HBSS control (Figure 52). Whereas median survival for the HBSS control group was 28 days, median survival for Ar17pAE2fFhTrtex at $1.5\times10^{12}$ vp/kg was 42 days, median survival for Ar17pAE2fFhTrtex at $3.0\times10^{12}$ vp/kg was 42 days, and median survival for Ar17pAE2fFhTrtex $4.5x10^{12}$ vp/kg was 35 days ($p<0.0001$, $p<0.0001$, and $p<0.0004$, respectively, versus HBSS control).

17.2.3 Preliminary vector distribution and expression

Vector was quantitated by PCR for the adenoviral hexon gene in selected tissues 3 days after intravenous dosing (SD4). Tissues from HBSS-treated control animals were negative for vector DNA. At all vector doses, tumors contained high levels of vector copies per cell (Table 50). Vector copy number in the tumors did not appear to be dose-dependent which is consistent with the observed lack of dose-dependence in the anti-tumor efficacy (Figure 51). Of the normal tissues tested, liver had the highest level of vector copies with a marked increase at the highest dose (Table 50). No statistical difference between vector copy number in the tumors and in the livers could be determined at any vector dose, possibly due to small sample size and high variability within groups. All other tissues analyzed contained low but measurable levels of vector DNA (Table 50).

**Table 17-3.** Vector copies per cell in selected tissues (Hexon PCR)

| | Vector Copies per Cell (±SD) | | | | | | | |
|---|---|---|---|---|---|---|---|---|
| **Treatment** | Tumor | Liver | Lung | Spleen | Kidney | Bone Marrow | Brain | Ovary |
| **HBSS** | 0.00 (0.00) | 0.00 (0.00) | 0.00 (0.00) | 0.00 (0.00) | 0.00 (0.00) | 0.00 (0.00) | 0.00 (0.00) | 0.00 (0.00) |
| **Low Dose** | 43.68 (34.15) | 1.85 (0.29) | 0.33 (0.11) | 1.01 (0.22) | 0.04 (0.01) | 0.17 (0.04) | 0.05 (0.05) | 0.04 (0.02) |
| **Mid Dose** | 45.75 (57.59) | 9.09 (2.27) | 0.80 (0.07) | 1.64 (0.33) | 0.08 (0.02) | 0.46 (0.08) | 0.01 (0.01) | 0.05 (0.03) |
| **High Dose** | 28.02 (21.9) | 91.64 (75.54) | 1.92 (0.68) | 1.71 (0.28) | 0.17 (0.02) | 1.16 (0.41) | 0.01 (na) | 0.19 (0.10) |

Vector DNA copies per cell in tissues collected from mice (n=3) after treatment with HBSS or Ar17pAE2fFTrtex at a Low Dose (1.5 x $10^{12}$ vp/kg), Mid Dose (3.0 x $10^{12}$ vp/kg), or High Dose (4.5 x $10^{12}$ vp/kg). HBSS treated mice were used as negative controls. Molecular analysis was done by PCR using primers specific for adenoviral Hexon DNA. Results are mean hexon copy number per cell (± SD). (na = not applicable).

Expression of vector E1a and E4 genes was analyzed in tumors and livers collected at SD4 (Table 51). E1 a and E4 expression in livers was highest at the high dose. There were no statistical differences in either E1a or E4 levels in tumors and livers, at any vector dose because of the high variability within groups.

**Table 51.** Mean E1a and E4 expression levels in tumor and liver

| | **RNA Levels (Normalized to 18S)** | | | |
|---|---|---|---|---|
| Treatment | E1a RT PCR | | E4 RT PCR | |
| | Tumor | Liver | Tumor | Liver |
| **HBSS** | 0 ± 0 | 0 ± 0 | 3 ± 1 | 0 ± na |
| **Low dose** | 472 ± na | 75 ± 90 | 4465 ± na | 85 ± 91 |
| **Mid dose** | 634 ±820 | 198 ±7 | 5333 ± 6698 | 224 ±56 |
| **High dose** | 885 ± 899 | 9722 ± 10938 | 7298 ± 6709 | 30259 ± 34925 |

Results are mean RNA level t SD. (na = not applicable).

<u>17.2.4 Preliminary toxicity assessments</u>

<u>17.2.5 Body weights</u>

There was a general trend of body weight loss one-day after injection in all treatment groups, but this was not statistically significant (Figure 53). At the highest dose of Ar17pAE2fFTrtex there was a 6% decrease in body weight. At the equivalent dose of a non-replicating control virus, AddI312, there was a 3% decrease in body weight. Body weights recovered in all groups after one week; after two weeks, all groups showed an increase from pre-treatment weights except the highest dose of Ar17pAE2fFTrtex. As the study progressed, there appeared to be a generalized body weight loss in all groups, possibly as a result of a general decline in condition due to tumor progression.

17.3 Repeat efficacy Efficacy of Ar17pAE2fFTrtex *in vivo*

In this study, we tested the ability of the oncolytic vector Ar17pAE2fFTrtex, delivered by systemic administration, to inhibit the growth and progression of pre-established human hepatocellular carcinoma (Hep3B) tumors in a subcutaneous xenograft model in nude mice. Results showed that a single intravenous injection of Ar17pAE2fFTrtex to nude mice bearing Hep3B xenograft tumors at doses of $3x10^{12}$ or $4.5x10^{12}$ particles/kg lead to a significant inhibition in tumor growth by study day 12. Tumor volume data on study day 19 expressed as percent treatment/control (T/C) show an anti-tumor response equal to 55% for the low dose and 46% for the high dose groups. In addition, Ar17pAE2fFTrtex at both doses significantly increased the median survival time of animals in both treatment groups when compared to the HBSS or Adl312-treated control groups. There were no significant differences in mean body weight change among any of the treatment or control groups and no mortality as a result of oncolytic vector treatment. However, there was a slight decrease in mean body weights at study day 16 for the dose group treated with $4.5x10^{12}$ particles/kg of Ar17pAE2fFTrtex. These results indicate that a single intravenous injection of Ar17pAE2fFTrtex leads to a significant inhibition in tumor growth.

17.3.1 Methods

17.3.1.2 Viruses and tumor cell line

Add/312 is a replication-defective adenovirus that has a deletion of the E1a region (bp 448-1349) and is used as a negative control (Young CSH, Shenk T, Ginsberg HS. The genetic system. In: Ginsberg HS, ed. The adenoviruses comprehensive virology. New York Plenum Press 1984; 4:125-172.). Add/312 was produced by the GTI Tissue Culture Core (4.0 x $10^{12}$ particles /ml). Ar17pAE2fFTrtex is an oncolytic adenoviral vector in which the E1a promoter is replaced with a human E2F-1 promoter and the E4 promoter is replaced with a human telomerase catalytic subunit promoter (hTERT) Ar17pAE2fFTrtex was produced (4.7 x $10^{12}$ particles/ml). Vector concentration was determined by optical particle titer (Mittereder et al., J. Virology 1996; 70:7498-7509). The human, hepatocellular carcinoma line Hep3B (Hep3B2.1-7; ATCC #HB-8064, batch number F-9462) was obtained from American Type Culture Collection (Manassas, VA) and found to be free of mycoplasma contamination. The Hep3B cells are cultured in Eagle minimal essential media (EMEM) containing 10% fetal bovine serum (FBS).

17.3.1.3 Mouse tumor model study

Female athymic outbred nu/nu mice (Harlan Sprague Dawley), 6-8 weeks of age, were implanted with 1 x $10^7$ Hep3B cells (resuspended in 0.1 ml of HBSS) subcutaneously in the right flank. Tumor measurements were recorded (in two dimensions) twice weekly using calipers. Tumor volume was calculated by the equation $L \times W^2 \times \pi/6$. Body weights were recorded once per week for the duration of the study. When tumor volume reached 100 - 200 $mm^3$, animals were randomly distributed into groups and intravenously injected with Ar17pAE2fFTrtex at $3x10^{12}$ (n=16) or $4.5x10^{12}$ (n=16) particles/kg. Dose volumes were adjusted to 10 ml/kg of body weight. Control groups were injected with HBSS (n=16) or Addl312 (n=16) at $4.5x10^{12}$ particles/kg. Animals were sacrificed when tumor volumes reached 2000 $mm^3$.

17.3.2 Results

17.3.2.1 Tumor Volumes

A subcutaneous xenograft model of hepatocellular carcinoma was used to assess the efficacy of the oncolytic adenovirus Ar17pAE2fFTrtex. Hep3B cells formed tumors of 100-200 $mm^3$ approximately two weeks after subcutaneous injection into nude mice. Intravenous injection of Ar17pAE2fFTrtex at doses of $3x10^{12}$(n=16), or $4.5\times10^{12}$ (n=16) particles/kg both showed a significant inhibition in tumor growth starting at study day 12 ($p<0.05$ by ANOVA) when compared to HBSS (n=16) or Addl312 (n=16) injected controls (Figure 54). There appeared to be a slight increase in efficacy with Ar17pAE2fFTrtex at a dose of $4.5x10^{12}$ particles/kg when compared to the $3x10^{12}$ particles/kg dose group, however the difference was not statistically significant.

Tumor volume data on study day 19 expressed as percent treatment/control (T/C) is shown in Table 52. These results show an anti-tumor response with T/C equal to 55 for the low dose and 46 for the high dose groups.

**Table 52.** T/C values

| Treatment Group | % T/C |
|---|---|
| HBSS | 100 |

(continued)

| Treatment Group | % T/C |
|---|---|
| AddI312: 4.5 x $10^{12}$ | 99 |
| Ar17pAE2fFTrtex: 3 x $10^{12}$ | 55 |
| Ar17pAE2fFTrtex: 4.5 x $10^{12}$ | 46 |
| Percent T/C = percent mean tumor volume for treatment group over HBSS control group; determined at study day 19. | |

17.3.2.2 Survival

Comparison of survival curves (Figure 55) indicates that treatment of tumors with Ar17pAE2fFTrtex at all doses significantly increased survival over HBSS and AddI312 treated control animals (p<0.01 by Mantel-Haenszel logrank test, for all groups). Median survival time was 26 days for the HBSS and AddI312 treated animals. Median survival time for Ar17pAE2fFTrtex treated animals was 43 days for the 3x$10^{12}$ particle/kg dose group and 56 days for the 4.5x$10^{12}$ particles/kg dose group. Surviving animals were observed until study day 58. There was no significant difference in survival between the Ar17pAE2fFTrtex treated groups. There was no significant difference in survival between the HBSS and AddI312 groups.

17.3.2.3 Body Weights

Although comparison of mean percent body weight change indicated no significant differences between all treatment and control groups (Figure 56), there was a transient decrease in the Ar17pAE2fFTrtex 4.5x$10^{12}$ part/kg dose group at day 16 when compared to the other groups.

17.4 Repeat Efficacy of Ar17pAE2fFTrtex *in vivo-Dose Response*

A subcutaneous xenograft model of hepatocellular carcinoma was used to assess the dose dependence of efficacy of four different doses of a single intravenous administration of the oncolytic adenovirus Ar17pAE2fFTrtex.

17.4.1 Methods

Human hepatocellular carcinoma Hep3B cells were inoculated into the flanks of female nude mice. When tumor volume reached 90 - 215 mm$^3$, animals were randomly distributed into groups (n=12 per group) and intraveneously injected with Ar17pAE2fFTrtex at $3\times10^{11}$, $6\times10^{11}$, $1\times10^{12}$, or $3\times10^{12}$ (n=12) particles/kg. Dose volumes were adjusted to 10 ml/kg. A control group was injected with 10 ml/kg HBSS (n=12). Tumor volumes were measure twice weekly.

17.4.2 Statistical analysis

Tumor volumes were analyzed for statistical significance by computer-based statistical programs, Excel with the XL*fit* module and SigmaStat, 2.03. A natural log transformation of tumor volumes for study days 3 to 22 resulted in a linear curve when plotted against time. Linear regression analysis was used to calculate slopes for individual mice and then group slopes were compared by Student's t-test (unpaired, 2 tail analysis). For comparison of survival curves, a Mantel-Haenszel logrank test was performed in GraphPad Prism 3.0. Kruskal-Wallis One-way analysis of variance on ranks was performed for body weight analysis. The level of significance was set at p<0.05 for all tests.

17.4.3 Results

17.4.3.1 Tumor volume

The dose response for Ar17pAE2fFhTrtex following systemic administration was assessed in a xenograft model of hepatocellular carcinoma. A cohort of 60 female nude mice formed tumors (90-215 mm$^3$) two weeks after subcutaneous injection of Hep3B cells into the right flank. A single intravenous injection of Ar17pAE2fFhTrtex at 3x$10^{11}$, 6x$10^{11}$, 1x$10^{12}$, or 3x$10^{12}$ viral particles/kg in a final volume of 10 ml/kg was administered on study day 1. The groups receiving the highest two doses showed significant inhibition in tumor progression on study day 22 (p < 0.05) when compared to HBSS (n=12) injected vehicle controls (Figure 57). The trend for the two lower doses was toward slower tumor progression, but the differences were not statistically significant.

Tumor volume data was expressed as the percent ratio of treated/control (%T/C) and is shown in Table 53 for study days 16 and 22. Ar17pAE2fFhTrtex at $3x10^{11}$, $6x10^{11}$, $1x10^{12}$, and $3x10^{12}$ vp/kg have %T/C of about 85, 68, 60, and 55, respectively, on SD22, the last day the HBSS group was intact. The trend toward a dose response was reflected in the rank order of the % T/C values for the four treatment groups.

**Table 53.** % T/C Values

| Ar17pAE2fFTrtex Dose (vp/kg) | SD16 | SD22 |
|---|---|---|
| $3 \times 10^{11}$ | 90 | 85 |
| $6 \times 10^{11}$ | 80 | 68 |
| $1 \times 10^{12}$ | 82 | 60 |
| $3 \times 10^{12}$ | 78 | 55 |

% T/C = mean tumor volume for treatment group divided by mean tumor volume for HBSS control group x 100.

The rank order of the efficacy response to vector dose observed at day 22 (Figure 57, Table 53) was consistent with a dose response since increased dose correlated with increased efficacy. While statistical analysis of mean tumor volumes at study day 22 was unable to identify a statistical difference between doses, plotting tumor volume progression for the individual mice contributing to the tumor volume means in Figure 58 strongly suggested a dose response was occurring. Therefore, the kinetics of tumor progression were analyzed. Tumor volumes underwent natural log transformation and then were plotted as a function of study day to produce tumor progression curves that more closely fit a linear equation than the untransformed data (Figure 58). HBSS (Fig. 58A) and Ar17pAE2fFTrtex at $3x10^{11}$ vp/kg (Fig. 58B) groups have individual tumor progression curves that show steady increases in tumor volume over time. !n contrast, the three higher doses of Ar17pAE2fFTrtex, $6x10^{11}$ vp/kg (Fig. 58C), $1 \times 10^{12}$ vp/kg (Fig. 58D), and $3x10^{12}$ vp/kg (Fig. 58E), have greater variability in tumor progression and multiple tumors with a decreasing rate of progression. Linear regression analysis was used to determine the slope for tumor growth between study day 3 and study day 22 (Table 54). Slopes were compared by Student's t-test and a statistical difference was observed for Ar17pAE2fFTrtex at $3x10^{11}$ vp/kg (Fig. 58B) versus $1x10^{12}$ (Fig. 58D) vp/kg.

**Table 54.** Average tumor volume slopes

| Treatment (Dose vp/kg) | HBSS | Ar17pAE2fFTrt ($3 \times 10^{11}$) | Ar17pAE2fF Trt ($6 \times 10^{11}$) | Ar17pAE2fF Trt ($1 \times 10^{12}$) | Ar17pAE2fF Trt ($3 \times 10^{12}$) |
|---|---|---|---|---|---|
| Average slope | $7.955 \times 10^{-2}$ | $7.871 \times 10^{-2}$ | $6.812 \times 10^{-2}$ | $5.776 \times 10^{-2*}$ | $5.177 \times 10^{-2}$ |
| Std Dev | $2.587 \times 10^{-2}$ | $1.465 \times 10^{-2}$ | $2.851 \times 10^{-2}$ | $2.165 \times 10^{-2}$ | $4.604 \times 10^{-2}$ |

Natural log (Ln) transformed tumor volumes were plotted as a function of time. Linear regression analysis in Excel was used to determine the slope of the line for individual mice between SD3 and SD22. Mean slopes for each group were compared by Student's t-test (*p=0.044 vs HBSS, 0.014 vs Ar17pAE2fFTrtex $3x10^{11}$ vp/kg).

17.4.3.2 Survival

There was a significant enhancement of survival for all vector doses compared to the HBSS control (Figure 59). Whereas median survival for the HBSS control group was 25 days, median survival for Ar17pAE2fFhTrtex at $3 \times 10^{12}$ vp/kg was 28 days, median survival for Ar17pAE2fFhTrtex at $6 \times 10^{12}$ vp/kg was 29.5 days, median survival for Ar17pAE2fFhTrtex $1 \times 10^{12}$ vp/kg was 41 days, and median survival for Ar17pAE2fFhTrtex $3 \times 10^{12}$ vp/kg was 37.5 days (p= 0.010, 0.016, 0.002, and 0.001, respectively). Although there was a trend toward increased survival with increased dose, a statistically significant dose response relationship was not demonstrated.

17.4.3.3 Body Weights

Comparison of the percent body weight change among the treatment groups indicated a significant decline for

HBSS and Ar17pAE2fFTrtex $3x10^{11}$ vp/kg treated mice on study day 15, 18 and 22 (Figure 60). None of the other Ar17pAE2fFTrtex treatment groups had a statistically significant change in body weight following vector administration. The body weights of mice treated with Ar17pAE2fFTrtex at 6 x $10^{11}$ vp/kg were relatively unchanged from study 1 to 22. Mice treated with Ar17pAE2fFTrtex at 1 x $10^{12}$ vp/kg lost a small amount of weight by study day 4, maintained that weight up to study day 18, and then experienced another weight loss by study day 22. Finally, mice treated with Ar17pAE2fFTrtex at 3 x $10^{12}$ vp/kg lost a small amount of weight by study day 4, consistently gained weight up to study day 18, and then experienced another weight loss by study day 22. In previous studies using SCID mice, dramatic declines in body weight have been observed when mice are given an oncolytic vector at or above the acute tolerated dose. In this study, the rate and scale of body weight loss are very different from the observations in the SCID studies, indicating no overt toxicity is associated with any of these vector doses. In fact, there is an inverse correlation between dose and body weight because the highest dose maintained weight best while the HBSS control experienced the greatest weight loss.

Example 18: Level and duration of Ar17pAE2fFTrtex DNA replication following a single intravenous injection

This study evaluated both the level and duration of Ar17pAE2fFTrtex DNA in xenograft tumors and mouse livers over a 28 day time course. Animals bearing human xenograft tumors received a single intravenous injection of Ar17pAE3fFTrtex. At various times after vector administration, animals were sacrificed and tumors and livers collected for quantitative molecular analysis of vector DNA. Molecular evaluation indicated that significant vector DNA replication occurs in tumors as the level of vector increased from an average of 0.25 vector copies per cell 4 hrs after injection to an average of 666.8 vector copies per cell 14 days after injection. An elevated level of vector DNA persisted at 28 days after injection. The kinetics of Ar17pAE2fFTrtex level and duration in tumors suggest that significant DNA replication is occurring, consistent with previous investigations demonstrating both efficacy and tolerability of a single intravenous injection of Ar17pAE2fFTrtex at a dose of 3 x $10^{12}$ vp/kg. Molecular evaluation also indicated that there was a significant loss of vector DNA in livers from an initial level of 73.6 vector copies per liver cell 4 hrs after vector administration with a decline to approximately 10 vector copies per cell 24 hours after injection. This level of vector DNA persisted in livers for the remainder of the study. The kinetics of Ar17pAE2fFTrtex level and duration in livers suggest that vector DNA replication is not occurring at a significant level, consistent with the tolerability previous observed at this same dose.

18.1 Methods

18.1.1 Virus and tumor cell line

Ar17pAE2fFTrtex, an oncolytic adenoviral vector with the native E1 a promoter replaced with the E2F-1 promoter and the native E4 promoter replaced with the human telomerase promoter (hTrt) was prepared using standard cesium chloride gradient purification methods. Vector concentration was determined by optical particle titer (Mittereder et al., J. Virology 1996; 70:7498-7509).

The human hepatocellular carcinoma line Hep3B (Hep 3B2.1-7; ATCC #HB-8064, batch number F-9462) was obtained from American Type Culture Collection (Manassas, VA) and found to be free of pathogens (IMPACT II PCR Profile, Missouri University Research Animal Diagnostic and Investigative Laboratory, Accession Number 2845-2001). The Hep3B cells are cultured in Eagle's minimal essential media (EMEM) containing 10% fetal bovine serum (FBS).

18.1.2. Study Design

Hep3B cells are a human hepatocellular carcinoma tumor cell line that can be killed *in vitro* and *in vivo* by administration of Ar17pAE2fFTrtex. Hep3B cells (1 x $10^7$ cells / 100 $\mu$l of HBSS) were implanted subcutaneously on the right flank of female athymic outbred *nu*/*nu* mice (Harlan, 6-8 weeks old). Study design is shown in Table 55. Thirty-three mice with subcutaneous Hep3B tumors (mean tumor volume of 149.1 $mm^3$; range 89.6 - 212.5 $mm^3$) were selected and distributed to sample collection timepoints on the basis of tumor volume to eliminate bias. Vector was diluted to the appropriate concentration with HBSS immediately prior to dosing and then administered intravenously via lateral tail vein.

**Table 55.** Study Design

| Test Material | Dose (particles/kg) | Dose Volume (mL/kg) | No. per Sample Collection Timepoint | | | | | | |
|---|---|---|---|---|---|---|---|---|---|
| | | | Pre Test | 4hr | 24hr | D4 | D8 | D15 | D29 |
| Ar17pAE2fFTrtex | $3x10^{12}$ | 10 | 3 | 5 | 5 | 5 | 5 | 5 | 5 |

18.1.3. Vector level and duration

Tumor and liver were collected from 3 mice without vector injection (pretest). Tumor and liver were collected from 5 mice at 4 hours, 24 hours, 3 days (D4), 7 days (D8), 14 days (D15), and 28 days (D29) after vector administration. DNA was extracted from each tissue and quantitative PCR performed for the vector hexon gene.

18.1.3.1 DNA isolation from tissues

DNA from tissues was isolated using the Qiagen Blood and Cell Culture DNA Midi or Mini Kits (Qiagen Inc., Chatsworth, CA). Frozen tissues were partially thawed and minced using sterile disposable scalpels. Tissues were then lysed by incubation overnight at 55°C in Qiagen buffer G2 containing 0.2 mg/ml RNaseA and 0.1 mg/ml protease. Lysates were vortexed briefly and then applied to Qiagen-tip 100 or Qiagen-tip 25 columns. Columns were washed and DNAs were eluted as described in the manufacturer's instructions. After precipitation, DNAs were dissolved in water and the concentrations were spectrophotometrically determined ($A_{260}$ and $A_{280}$) on a DU-600 (Beckman Coulter, Inc.; Fullerton, CA) or a SPECTRAmax PLUS (Molecular Devices, Inc.; Sunnyvale, CA) spectrophotometer.

18.1.3.2 Hexon Taqman real-time PCR assay

PCR primers and a Taqman probe specific to adenovirus hexon sequences were designed using Primer Express software v. 1.0 (Applied Biosystems, Foster City, CA). Primer and probe sequences were:

Hexon Forward primer: 5'-CTTCGATGATGCCGCAGTG-3' (SEQ ID NO:90)

Hexon Reverse primer: 5'-GGGCTCAGGTACTCCGAGG-3' (SEQ ID NO:91)

Hexon Probe: 5'-FAM-TTACATGCACATCTCGGGCCAGGAC-TAMRA-3' (SEQ ID NO:92)

Amplification was performed in a reaction volume of 50 $\mu$l under the following conditions: 10 ng (tumor) or 1 $\mu$g (liver and lung) of sample DNA, 1X Taqman Universal PCR Master Mix (Applied Biosystems), 600 nM forward primer, 900 nM reverse primer and 100 nM hexon probe. Thermal cycling conditions were: 2 minute incubation at 50°C, 10 minutes at 95°C, followed by 35 cycles of successive incubation at 95°C for 15 seconds and 60°C for 1 minute.

Data was collected and analyzed using the 7700 Sequence Detection System software v. 1.6.3 (Applied Biosystems). Quantification of adenovirus copy number was performed using a standard curve consisting of dilutions of adenovirus DNA from 1,500,000 copies to 15 copies in the appropriate background of cellular genomic DNA. For analysis of tumor tissues, a standard curve in a background of 10 ng human DNA was generated. For analysis of mouse liver and lung tissues, a standard curve consisting of the same adenovirus DNA dilutions in a background of 1 $\mu$g CD-1 mouse genomic DNA was generated. Samples were amplified in triplicate, and the average number of total copies was normalized to copies per cell based on the input DNA weight amount and a genome size of $6 \times 10^9$ bp.

18.1.4 Statistical analyses

All quantitative data were tested for normality and variance. Using SigmaStat 2.03, Student's t-test was used to assess the statistical significance of treatment group differences. The level of significance was set at $p<0.05$.

18.2 Results

18.2.1 Vector Copy Number

**[0320]** Ar17pAE2fFTrtex vector copy number in tumors and livers was quantitated by hexon DNA PCR at various timepoints after intravenous dosing. Pretest samples from untreated animals were negative for vector DNA as expected. In tumors the number of vector copies was low but detectable at the early timepoints and then increases significantly after the 24 hour timepoint, suggesting vector DNA replication in the tumor (Table 56, Figure 61). While the increase in vector DNA from 4 hours (0.25 $\pm$ 0.07 vector copies per cell) to 24 hours (1.31 $\pm$ 1.01 vector copies per cell) was statistically significant (p=0.032), the dramatic differences between 24 hours and either D4 (118.5 $\pm$ 118.0 vector copies per cell) or D8 (168.5 $\pm$ 181.6 vector copies per cell) were not statistically different due to the high individual variability at these timepoints. The peak observed at D15 (666.8 $\pm$ 199.3 vector copies per cell) was statistically different from D8 (p= 0.006), but the slight decline to D29 (429.9 $\pm$ 127.4 vector copies per cell) from the D15 peak was not significant.

In livers the highest vector copy number is detected at 4 hours (73.6 $\pm$ 17.7 vector copies per cell), the earliest timepoint assessed. Vector copy number in liver declined 7 fold by 24 hours (10.7 ± vector copies per cell, p= <0.001). After 24 hours, vector copy number in liver at D4, D8, D15, and D29 persists at levels that were statistically indistinguishable from the 24 hour level.

The relative levels of vector in tumor and liver changed dramatically over the course of the study. There was higher vector copy number in liver versus tumor at 4 and 24 hours (p= <0.001), >10 fold higher levels in tumor versus liver at D4 and D8 that were not statistically different, and >25 fold higher levels in tumor at D15 and D29 that were statistically significant (p= 0.015 and <0.001, respectively).

**Table 56.** Mean vector copies in tumor and liver

| | Vector Copies / Cell (hexon PCR) | | | | | | |
|---|---|---|---|---|---|---|---|
| | PreTest | 4 hr | 24 hr | D4 | D8 | D15 | D29 |
| *Tumor* | 0.00 ± 0.00 | 0.25* ± 0.07 | 1.31*± 1.01 | 118.5 ± 118.0 | 168.5 ± 181.6 | 666.8*± 199.3 | 429.9± 127.4 |
| Liver | 0.00 ± 0.00 | 73.6* ± 17.7 | 10.7 ± 0.81 | 10.6 ± 4.7 | 10.5 ± 4.7 | 8.2 ± 4.2 | 160 ± 19.3 |

Vector DNA copies per cell in tumors and livers collected from mice prior to treatment (n=3) and at indicated times and after intravenous injection of Ar17pAE2fFTrtex at 3.0 × $10^{12}$ vp/kg (n=5). Molecular analysis was done by PCR using primers specific for adenoviral hexon DNA. Results are mean hexon copy number per cell ± SD. *p< 0.05 compared to matched tissue at previous timepoint by Student's t-test.

## Example 19: In vivo SCID model for evaluation of Ar17pAE2fFTrtex

### 19.1 Rationale.

The SCID mouse model has been described previously as a screen for oncolytic vectors (see example 3). The rationale for choosing this model is based on the toxicity associated with expression of the viral E1a region. The SCID screening model was originally developed to distinguish between oncolytic vectors in which only E1a expression was controlled and used vector doses of 6.25x$10^{11}$ particles/kg, as in Example 3. As oncolytic vectors were constructed with improved tumor-selective promoters, candidates could no longer be distinguished from each other or negative controls since they produced no overt signs of toxicity. Therefore, the model was modified for this example by administering higher vector doses such that effects on selected parameters, such as body weight and liver enzyme levels, could be used to distinguish between more selective vectors. In addition, E1a and E4 mRNA levels and adenoviral DNA copy number can be used as endpoints for replication of oncolytic vectors in non-tumor cells *in vivo.* We compared the dual promoter-controlled Ar17pAE2fFTrtex, the single promoter-controlled Ar6pAE2fE3F (described in Example 7) and the E1a-deleted Addl312 to determine the effect of E1 a and E4 transcription on control of DNA replication in a normal tissue *in vivo.*

### 19.2 Methods

Each animal was weighed on the morning of dosing, study day SD1. The dose volume was adjusted based on individual body weight to achieve a dose of 4x$10^{12}$ particles/kg of each test vector in a dose volume of 10 ml/kg. The dose was administered intravenously into the lateral tail vein (n=10/group). A control group of animals (n=10) was injected with an equivalent dose volume (10 ml/kg) of HBSS. Clinical observations and evaluations of selected clinical pathology parameters were performed. In addition, livers were collected at SD4 and SD15 scheduled necropsies and portions were frozen on dry ice for subsequent adenoviral hexon DNA PCR, E1a RT-PCR, and E4 RT-PCR, as described in Examples 4 and 13.

### 19.3 Results and conclusions

The effect on body weight after a single administration of the indicated vector at the dose of 4 x $10^{12}$ particles/kg is shown in Fig. 62. After an initial loss of body weight at SD2 in all treatment groups, the HBSS and Ad*dl*312-treated animals gained weight over the course of the study. Ar6pAEF2fE3F-treated animals lost weight steadily between SD1 and SD4, at which time they became moribund and were sacrificed. The mean body weight change in this group was significantly different than that of the HBSS groups at SD2, SD3 and SD4 (p<0.05, one-way ANOVA). In contrast, the Ar17pAE2fFTrtex treatment group maintained mean body weight over the course of the study. The difference in body weight change between the Ar17pAE2fFTrtex and the HBSS-treated groups was not significant at any point during the study.

Serum levels of ALT, AST, CPK and CREAT were measured on SD4 and SD15. CPK and CREAT levels were not different between any treatment group at SD4 or SD15, indicating that there was little toxicity in skeletal muscle, cardiac muscle, brain or kidney. While the Ar17pAE2fFTrtex treated group had elevated levels of AST and ALT on SD4 relative to HBSS-treated mice ($p<0.05$, t-test), the Ar17pAE2fFTrtex treated group had significantly lower levels than the single promoter controlled vector Ar6pAE2fE3F ($p < 0.05$, one-way ANOVA). Table 57 shows the data for AST levels; results for ALT levels were similar. Thus, relative to the single promoter-controlled Ar6pAE2fE3F, additional control of E4 expression in the dual promoter-controlled Ar17pAE2fFTrtex was associated with decreased toxicity.

**Table 57.** Serum AST levels in SCID mice

| Treatment | SD4 | SD15 |
|---|---|---|
| HBSS | 103 | 126 |
| Addl312 | 114 | 172 |
| Ar6pAE2fE3F | 14604 | ND |
| Ar17pAE2fFTrtex | 1383 | 1331 |

Animals were dosed on SD1 with HBSS or with $4x10^{12}$ particles/kg of the indicated vector. Serum was collected on SD4 (n=10) and SD15 (n=5). The mean AST values in each treatment group (+SD) are shown. SD15 for Ar6pAE2fE3F not determined since animals in this group were sacrificed before SD15. **p*<0.05 versus HBSS (t-test on $log_{10}$-transformed data). †*p*<0.05 versus Ar6pAE2fE3F (t-test on $log_{10}$-transformed data). ND, not done.

Mice treated with the replication-defective vector Addl312 averaged 19 copies of vector DNA per liver cell 3 days after a single intravenous injection. In contrast, mice treated with the single promoter-controlled vector Ar6pAE2fE3F averaged 1522 copies per cell (Table 58). This 80-fold difference in vector copy number suggests that vector replication can occur in the livers of mice treated with this dose of Ar6pAE2fE3F. However, the Ar17pAE2fFTrtex treatment group had a significantly lower mean of 232 copies per cell ($p<0.05$, one-way ANOVA). Thus, despite similar input doses, there was a 7-fold drop in viral DNA levels between the vector with E1 a control versus the vector with both E1 a and E4 transcriptional control.

To determine whether the differences that we saw in the DNA copy number between Ar6pAE2fE3F and Ar17pAE2fFTrtex were associated with hTERT promoter control in the E4 region, we evaluated the early gene expression levels in these same treatment groups (Table 58). The levels of E1a on a per Ad genome basis were similar in both Ar6pAE2fE3F and Ar17pAE2fFTrtex-treated mice, within 1.5-fold. Since the E1a region in both vectors is under the control of the E2F-1 promoter, this result was expected.

This similarity in E1a RNA levels between Ar17pAE2fFTrtex and Ar6pAE2fE3F-treated mice suggested that the differences that we saw in the DNA copy number may be associated with hTERT promoter control in the E4 region. Therefore, E4 expression levels were measured in mice treated with Ar17pAE2fFTrtex, Ar6pAE2fE3F and Addl312 (Table 58). Even in the absence of E1a, like in Addl312, there is residual activation of the E4 promoter (Gorziglia MI, Lapcevich C, Roy S, Kang Q, Kadan M, Wu V, Pechan P, Kaleko M. Generation of an adenovirus vector lacking E1, e2a, E3, and all of E4 except open reading frame 3. J Virol. 1999 Jul;73(7):6048-55). Ar6pAE2fE3F and Addl312 both have wildtype E4 promoters; mice treated with these vectors showed no difference in E4 expression levels per genome. However, mice treated with Ar17pAE2fFTrtex in which E4 is under the control of a hTERT promoter showed a significant reduction in E4 RNA levels. This reduction is associated with the decrease in viral DNA copy number. These data suggest that placing the E4 region under the control of a second tumor-selective promoter has led to a decrease in DNA replication in a normal tissue relative to the single promoter controlled Ar6pAE2fE3F.

**Table 58.** Levels of Ad DNA, and E1a/E4 RNA in SCID livers following iv administration, SD4

| Treatment | No. Ad genomes per cell | E1a RNA levels per Ad genome | E4 RNA levels per Ad genome |
|---|---|---|---|
| Addl312 | 19 ±2 | 3 ±2† | 2169 ±548† |
| Ar6pAE2fE3F | 1522±244 | 106 ±32 | 1787±443† |
| Ar17pAE2fFTrtex | 232 ±244* | 155 ±142 | 272 ±246 |

Liver was collected from five mice per group on SD4 for extraction of total RNA. Molecular analysis was done to determine adenoviral genome copy number per cell by quantitative DNA PCR for the hexon gene. Expression of E1a and E4 genes was measured by quantitative RT-PCR and normalized on a per adenoviral genome basis. Results are expressed as mean values ± SD. *, $p<0.05$ versus Addl312 and Ar6pAE2fE3F (one-way ANOVA). †, $p<0.05$ versus Ar17pAE2fFTrtex (one-way ANOVA).

These investigations showed that Ar17pAE2fFTrtex is associated with significantly lower levels of E1a-related hepatoxicity, E4 expression and hexon DNA copy number relative to the single promoter-controlled Ar6pAE2fE3F. The operative difference between these two vectors is the transcriptional control of the E4 region. These results suggest that the attenuation of replication in normal liver achieved with single promoter control of E1 a has been enhanced by additional control of the E4 region. While E1a RNA levels in the mouse livers remained similar between Ar17pAE2fFTrtex and Ar6pAE2fE3F, we observed lower levels of E4 expression when the wildtype E4 promoter was replaced with a hTERT promoter in Ar17pAE2fFTrtex. This was associated with lower Ad DNA copy number and reduced hepatotoxicity. The low levels of E4 expression by a hTERT promoter in Ar17pAE2fFTrtex-treated mice was not attributable to the inability of a hTERT promoter to use the mouse transcriptional machinery since expression of an hTERT promoter LacZ reporter construct has been seen in the mouse lung carcinoma cell line M109 (Gu J, Kagawa S, Takakura M, Kyo S, Inoue M, Roth JA, Fang B. Tumor-specific Transgene Expression from the Human Telomerase Reverse Transcriptase Promoter Enables Targeting of the Therapeutic Effects of the Bax Gene to Cancers. Cancer Res. 2000 60: 5359-5364).

Example 20: Synergistic effect of combination oncolytic adenovirus with chemotherapy

The synergistic effect of combining the oncolytic adenoviral vector Ar17pAE2fFTrtex with chemotherapeutics was examined in vitro and in vivo. Chemotherapy agents with different functions, doxorubicin, paclitaxel and Epothilone B, were tested in combination with Ar17pAE2fFTrtex against liver and prostate carcinoma cells by cell killing assay. The combination effect is analyzed by isobologram. Strong synergistic effects are shown in vitro at certain combinations. In vivo, two formulations of doxorubicin, free doxorubicin and doxorubicin entrapped in long-circulation liposomes (Doxil®), were tested in combination with Ar17pAE2fFTrtex in a hepatocellular carcinoma xenograft model by assessing tumor growth. Both combinations had higher efficacy to regress tumor growth than oncolytic vector or chemotherapy alone. The Doxil® combination showed stronger synergism than the doxorubicin combination in vivo.

20.1 Combination with chemotherapy in vitro

20.1.1 Methods

20.1.1.1 Cell culture and virus

Prostate cancer cell line PC3M.2AC6 (also known as PC-3M2AC6) was obtained from the PC-3M human prostatic cancer cell line by transfection with a firefly luciferase expression vector, pGL-3 (Promega, Madison, WI). A clone, designated PC-3M2AC6 was selected based on high light output and retention of *in vitro* sensitivity to antiproliferative activity of cytotoxic drugs. The PC-3M2AC6 cell line was generated at Xenogen Corporation (Alameda, CA). The cell line is maintained in RPMI 1640 media containing 10% FBS. Hepatocellular carcinoma cell line Hep3B (Hep3B2.1-7 ATCC #HB-8064) is cultured in EMEM containing 10% FBS. Virus Ar17pAE2fFTrtex was prepared as described in example 15.

20.1.1.2 Chemotherapy drugs

Taxol® (Paclitaxel) and doxorubicin were purchased from Washington Wholesale Drug (Savage, MD). Taxol is a product of Mead Johnson Oncology (a Bristol-Myers Squibb Co. Princeton, NJ 08543). Each ml contains 6 mg paclitaxel, 527 mg purified Cremophor@ EL (polyoxyethylated castor oil) and 49.7% (V/V) dehydrated alcohol, USP.

Doxorubicin is a product of Gensia Sicor Pharmaceuticals, Inc. (Irvine, CA). Each ml contains Doxorubicin HCl 2 mg, Sodium chloride 0.9%, pH is 2.5-4.5.

Epothilone B (WO 99/43320), also called CGP 47906 or EPO906, was from Novartis AG (Basel, Switzerland). Its molecular structure and physical characteristics are described by Altmann K, Wartmann M and O'Reilly T, 1998, Biochimica et Biophysica Acta, 1470: M79-M91.

20.1.1.3 Combination cell killing assay in vitro

The isobologram method used to evaluate synergy between combinations of chemotherapy drugs is described in detail (Tallarida RJ. 1992; Pain, 49:93-97). This method was adapted to evaluate in vitro synergy between combinations of oncolytic vectors and chemotherapy with the following modifications. Briefly, 5000 cells per well are plated into 96-well plate one day before virus infection. One day after virus infection, chemotherapy drugs are added to the cells. Each 96-well plate can be used to test dose-response curves of two single drugs and four combinations at a fixed mixture ratio of virus to chemotherapy drug per row. Seven days after virus infection (6 days after the treatment with chemotherapy drugs), MTS assay is used to determine percent cell death. Sigmoidal dose-response curves are fitted by GraphPad Prism program and effective concentration (EC) at different percent effect or response levels are calculated with output parameters from the Prism program. Combination index $CI=d_A/D_A+d_B/D_B$. $D_A$ is $EC_{50}$ of virus alone treatment; $D_B$ is $EC_{50}$ of chemotherapy drug alone treatment; $d_A$ is $EC_{50}$ of virus in combination treatment; $d_B$ is $EC_{50}$ of chemotherapy drug in combination treatment. Similarly, CI at 30%, 70%, and 90% cell death ($CI_{30}$, $CI_{70}$ and $CI_{90}$) are calculated with appropriate $EC_{30}$, $EC_{70}$, and $EC_{90}$, respectively.

Improved isobologram with envelope of additivity was constructed at 50% cell death to better distinguish synergism and antagonism from additivity (Steel GG and Peckham MJ.1979; Int. J. Radiation Oncology Biol. Phys., 5: 85-91.). Mode I represents heteroaddition, in which the two agents act additively by independent mechanisms. Mode II represents isoaddition, in which the two agents act additively by similar mechanisms. Mode IIa indicates that agent A acts first and Mode IIb indicates that agent B acts first.

If the CI is less than 1 or the isoeffective points of combination fall to the left of envelope of additivity, the combination is synergistic. If the CI is equal to 1 or the isoeffective points of the combination fall inside the envelope of additivity, the combination is additive. If the CI is greater than 1 or the isoeffective points of combination fall to the right of the envelope of additivity, the combination is antagonistic.

20.1.2 Results

20.1.2.1 Combination of Ar17pAE2fFTrtex and Taxol

Results of combinations of Ar17pAE2fFTrtex and Taxol tested in Hep3B cells and PC3M.2AC6 cells are shown in Tables 64 and 65, respectively. For Hep3B cells, $CI_{50-90}$ were less than 1 at all tested mixture ratios. Only $CI_{30}$ at mixture ratios of $3.33x10^{-4}$ ppc/nM and $5.33x10^{-3}$ ppc/nM were greater than 1. When Ar17pAE2fFTrtex and Taxol were mixed at a ratio of $3.33x10^{-4}$ ppc/nM, the $CI_{90}$ was as low as 0.08 and the $CI_{50}$ was as low as 0.238. At all tested ratios, CI decreased with increase in response level (% cell death) (Table 59). This indicates that the synergy is stronger at higher response levels than at lower levels. For PC3M.2AC6 cells, when Ar17pAE2fFTrtex and Taxol were mixed at ratios of 0.2 and 2 ppc/nM, $CI_{30-90}$ were less than 1 and again decreased with increase in response level. When mixture ratios were 0.02 or 20, $CI_{30}$-$_{70}$ and $CI_{70-90}$ were greater than 1 indicating antagonism (Table 60).

An improved isobologram method using envelope of additivity confirmed the synergistic combinations of Ar17pAE2fFTrtex and Taxol at all tested mixture ratios against Hep3B cells, and of Ar17pAE2fFTrtex and Taxol at mixture ratios of 0.2 and 2 against PC3M.2AC6 cells (Figure 63). All the points of combinations were closer to the X-axis than to the Y-axis in Figure 63, which indicates chemosensitization by the virus is predominant. In the synergistic combinations, Ar17pAE2fFTrtex had 1.1 to 4.3-fold reduction in $EC_{50}$ and Taxol had 232 to 3753-fold reduction in $EC_{50}$ against Hep3B cells. Similarly, when PC3M.2AC6 cells were treated, Ar17pAE2fFTrtex had 1.3 to 1.4-fold reduction and Taxol had 35 to 2592-fold reduction in $EC_{50}$. These data show that the synergistic effect leads to greater reduction in Taxol dosage than in Ar17pAE2fFTrtex dosage. This implicates the oncolytic vector in sensitizing both cell lines to Taxol at the tested mixture ratios.

**Table 59**. Combination of Ar17pAE2fFTrtex and Taxol on Hep3B cells.

| MR (ppc/nM) | $CI_{30}$ | $CI_{30}$ | $CI_{70}$ | $CI_{90}$ |
|---|---|---|---|---|
| 8.33E-05 | **0.454** | **0.238** | **0.131** | **0.080** |
| 3.33E-04 | 1.010 | **0.532** | **0.284** | **0.121** |
| 1.33E-03 | **0.605** | **0.400** | **0.266** | **0.144** |

(continued)

| MR (ppc/nM) | $CI_{30}$ | $CI_{30}$ | $CI_{70}$ | $CI_{90}$ |
|---|---|---|---|---|
| 5.33E-03 | 1.395 | **0.927** | **0.617** | **0.326** |

**Table 60**. Combination of Ar17pAE2fFTrtex and Taxol on PC3M.2AC6 cells.

| MR (ppc/nM) | $CI_{30}$ | $CI_{50}$ | $CI_{70}$ | $CI_{90}$ |
|---|---|---|---|---|
| 0.02 | 11.115 | 4.728 | 2.195 | **0.802** |
| 0.2 | **0.930** | **0.739** | **0.597** | **0.454** |
| **2** | **0.834** | **0.753** | **0.681** | **0.585** |
| 20 | **0.797** | **0.973** | 1.188 | 1.633 |

20.1.2.2 Combination of Ar17pAE2fFTrtex and Doxorubicin

Results of combinations of Ar17pAE2fFTrtex and Doxorubicin tested in Hep3B cells are shown in Table 61. $CI_{30-90}$ were less than 1 when mixture ratios were 1.25x10$^{-5}$ to 8x10$^{-4}$ ppc/nM. Again, the CI decreased with increase in effect at the tested mixture ratios except at 1.25x10$^{-5}$ ppc/nM. In the other words, the synergy is stronger at higher level of cell killing than at lower levels. Furthermore, the improved isobologram (Figure 64) confirmed that the combinations at mixture ratios of 1.25x10$^{-5}$ to 8x10$^{-4}$ were synergistic. The reduction of virus $EC_{50}$ (9 to 358-fold) were much greater than the reduction in doxorubicin $EC_{50}$ (1.7 to 2.8-fold) in the tested combinations. This suggests that, unlike Taxol, doxorubicin sensitizes Hep3B cells to oncolytic vectors at the tested mixture ratios.

When PC3M.2AC6 cells were treated, $CI_{30-70}$were less than 1 and $CI_{90}$ were greater than 1 for Ar17pAE2fFTrtex and doxorubicin mixed at 10, 100, and 1000 ppc/nM (Table 62). For the mixture ratio of 1 ppc/nM, $CI_{30-50}$ were greater than 1 and $CI_{70-90}$ were less than 1. Improved isobologram agreed that the combinations at mixture ratios of 10-1000 were synergistic and the mixture ratio of 1 was antagonistic (Figure 64). The 1.1 to 3.2-fold reduction in $EC_{50}$ for Ar17pAE2fFTrtex and the 68 to 470,000-fold reduction in $EC_{50}$ for doxorubicin indicate that Ar17pAE2fFTrtex sensitizes PC3M.2AC6 cells to doxorubicin at the tested mixture ratios.

**Table 61.** Combination of Ar17pAE2fFTrtex and Doxorubicin on Hep3B cells.

| MR (ppc/nM) | $CI_{30}$ | $CI_{50}$ | $CI_{70}$ | $CI_{90}$ |
|---|---|---|---|---|
| 7.81 E-07 | 2.426 | 1.469 | **0.890** | **0.401** |
| 1.25E-05 | **0.579** | **0.600** | **0.622** | **0.658** |
| 5.00E-05 | **0.473** | **0.426** | **0.383** | **0.324** |
| 2.00E-04 | **0.732** | **0.419** | **0.240** | **0.099** |
| 8.00E-04 | **0.930** | **0.464** | **0.232** | **0.078** |

**Table 62.** Combination of Ar17pAE2fFTrtex and Doxorubicin on PC3M.2AC6 cells.

| MR (ppc/nM) | $CI_{30}$ | $CI_{50}$ | $CI_{70}$ | $CI_{90}$ |
|---|---|---|---|---|
| 1 | 5.246 | 2.232 | **0.974** | **0.378** |
| 10 | **0.854** | **0.920** | **0.995** | 1.180 |
| 100 | **0.138** | **0.336** | **0.820** | 3.402 |
| 1000 | **0.107** | **0.317** | **0.944** | 5.368 |

20.1.2.3 Combination of Ar17pAE2fFTrtex and Epothilone B

Epothilone B has a function similar to Taxol, i.e. inhibiting the assembly of microtubules, but it is more potent. When Ar17pAE2fFTrtex and Epothilone B were mixed at ratios of 3.13x10-6, or 1.25x10-5 ppc/nM, the combinations were synergistic at all levels of effect (Table 63). The CI30-50 were less than 1 at mixture ratios of 5x10-5 and 1.28x10-2 ppc/nM. Improved isobologram analysis confirmed that the combinations at mixture ratio of 3.13x10-6, 1.25x10-5 and 5x10-5 ppc/nM were synergistic at the 50% response level, but the combination at a mixture ratio of 1.28x10-2 ppc/nM was only additive (Figure 65). The positions of the synergistic combinations were closer to the Y-axis than to the X-axis suggesting Epothilone B sensitizes the cells to the virus. The reduction of Ar17pAE2fFTrtex EC50 in the synergistic combinations was 119 to 2851-fold while the reduction of Epothilone B EC50 was only 1.1 to 1.6-fold. This is consistent with the conclusion that Epothilone B sensitizes Hep3B cells to Ar17pAE2fFTrtex at the tested mixture ratios.

**Table 63.** Combination of Ar17pAE2fFTrtex and Epothilone B on Hep3B cells.

| MR (ppc/nM) | $CI_{30}$ | $CI_{50}$ | $CI_{70}$ | $CI_{90}$ |
|---|---|---|---|---|
| 3.13E-06 | **0.665** | **0.632** | **0.601** | **0.555** |
| 1.25E-05 | **0.749** | **0.802** | **0.859** | **0.961** |
| 5.00E-05 | **0.840** | **0.955** | 1.088 | 1.346 |
| 2.00E-04 | 1.036 | 1.219 | 1.455 | 1.955 |
| 8.00E-04 | 1.227 | 1.819 | 2.847 | 6.121 |
| 3.20E-03 | 1.128 | 1.675 | 2.817 | 7.550 |
| 1.28E-02 | **0.766** | **0.881** | 1.158 | 2.388 |
| 5.12E-02 | 1.080 | 1.237 | 1.513 | 2.641 |

20.2 Combination with chemotherapy *in vivo*

Two in vivo combination studies are presented here. Both showed that chemotherapy enhanced efficacy of Ar17pAE2fFTrtex. The first study was Ar17pAE2fFTrtex ($3X10^{12}$ particle/kg) combined with doxorubicin. The second study was Ar17pAE2fFTrtex ($1X10^{12}$ and $6X10^{11}$ particle/kg) combined with Doxil®. Doxil® is doxorubicin HCl encapsulated in long-circulating STEALTH® liposomes. The stealth liposome contains polyethylene glycol (PEG) on the surface that can shield liposomes from fast take-up by macrophages in the circulation. Hence, liposome formulations stay in the circulation for a relatively long period of time. More importantly, these long-circulating liposomes show significantly greater accumulation in solid tumors compared with conventional liposomes (no PEG) and free drug.

20.2.1 Methods

20.2.1.1 Materials

Doxil® (doxorubicin HCl liposome injection) is product of ALZA Pharmaceuticals (Mountain View, CA). Each ml contains 2 mg of doxorubicin HCl, 3.19 mg of N-(carbonyl-methoxypolyethyleneglycol 2000)-1,2,distearoyl-sn-glycero-3-phosphoethanolamine sodium salt, 9.58 mg of fully hydrogenated soy phosphatidylcholine, 3.19 mg of cholesterol, 2 mg of ammonium sulfate, sucrose, histidine, hydrochloric acid and/or sodium hydroxide. Doxil® was diluted appropriately in 5% Glucose before administration to mice.

20.2.1.2 Combination of Ar17pAE2fFTrtex with Doxorubicin

Hep3B cells ($1 \times 10^7$ cells / 100 $\mu$l of HBSS) were implanted subcutaneously on the right flank of female athymic outbred nu/nu mice (Harlan Sprague Dawley, 6-8 weeks old). Tumor measurements were recorded twice weekly in two dimensions using calipers. Tumor volume was calculated using the formula Length x $Width^2 \times \pi/6$. When tumor volumes reached between 89 and 280 $mm^3$, mice were selected and evenly distributed into four groups (n=10), Ar17pAE2fFTrtex only, doxorubicin only, Ar17pAE2fFTrtex combined with doxorubicin, and negative control HBSS. Mean tumor volume on study day 1 was 183 $\pm$18.3 $mm^3$.
On study day 1, mice were injected intravenously (i.v.) via the tail vein with Ar17pAE2fFTrtex alone at $3.0x10^{12}$ particle/kg, intraperitoneal (i.p.) with doxorubicin alone at 10 mg/kg, or with both Ar17pAE2fFTrtex, $3.0x10^{12}$ particle/kg i.v. and doxorubicin, 10 mg/kg, i.p. A negative control group was injected intravenously with HBSS, 10 ml/kg.

20.2.1.3 Combination of Ar17pAE2fFTrtex with Doxil® (ON1-029y)

When subcutaneously implanted Hep3B tumors reached a volume between 173 and 321 $mm^3$, mice were selected and evenly distributed into four treatment groups (n=10) and one vehicle control group (HBSS, n=9). The four treatment groups were Ar17pAE2fFTrtex only ($3x10^{12}$ particle/kg), Doxil® only, Ar17pAE2fFTrtex ($1x10^{12}$ particle/kg) combined with Doxil®, Ar17pAE2fFTrtex ($6x10^{11}$ particles/kg) combined with Doxil®. Mean tumor volume on study day 0 was 250 $\pm$13 $mm^3$.

On study day 1, mice were injected intravenously (i.v.) via the tail vein with Doxil® at 9 mg/kg. On study day 2, mice were injected i.v. with Ar17pAE2fFTrtex at $1x10^{12}$ particle/kg or at $6x10^{11}$ particle/kg. Injection volume was 10 ml/kg. The control group was injected i.v. with HBSS, 10 ml/kg, on study day 1.

20.2.1.4 Statistical Analyses

Differences between treatment groups were analysed for statistical significance by computer-based statistical programs, SigmaStat, version 2.0 or GraphPad Prism version 3.0. For tumor volumes, t-tests were performed by Student t-test using Microsoft Excel.

**20.2.2** Results

20.2.2.1 Efficacy of Doxorubicin Combination

A xenograft model of hepatocellular carcinoma was used to assess the efficacy of Ar17pAE2fFTrtex in combination with the chemotherapeutic compound doxorubicin following systemic administration. A single intravenous injection of Ar17pAE2fFTrtex at $3.0x10^{12}$ viral particles/kg in a final volume of 10 ml/kg (n=10) showed a significant inhibition in tumor growth starting on study day 16 ($p < 0.001$, Student t-test) when compared to HBSS injected controls (Figure 66). Doxorubicin treatment alone showed significant tumor inhibition compared to HBSS on study day 13 ($p< 0.001$ by Student t-test). Combining doxorubicin with oncolytic vector treatment improved efficacy significantly over treatment with either agent alone ($p < 0.001$ by Student t-test on study day 20). In this study, one complete tumor regression was noted in the vector only treated group.

Tumor volume data expressed as the percent ratio of treated / control (T/C) is shown in Table 64 for study days 13, 16, and 20. The lower tumor growth rate of the treated groups versus the HBSS control is reflected in the decreasing trend in T/C values for all three treatment groups. The combination group has the lowest value, 20% on study day 20; the last day all groups were intact.

**Table 64.** Doxorubicin Combination Study % T/C Values

| Treatment | Day 13 | Day 16 | Day 20 |
|---|---|---|---|
| Ar17pAE2fFTrtex 3.0e12 vp/kg | 68 | 55 | 42 |
| Doxorubicin 10 mg/kg | 55 | 55 | 50 |
| Ar17pAE2fFTrtex 3.0e12 vp/kg & Doxorubicin 10 mg/kg | 41 | 28 | 20 |

% T/C = mean tumor volume for treatment group divided by mean tumor volume for HBSS control group x 100.

20.2.2.2 Efficacy of Doxil® Combination

Treatment with Doxil® alone lead to significant efficacy ($p< 0.001$ on study day 21) with a T/C value of 37% on study day 21 (Figure 69, Table 70). Treatment with vector only at 1 x $10^{12}$ vp/kg also lead to significant tumor inhibition compared to HBSS control (Figure 20.5, $p<0.005$ on study day 21). Vector only was less efficacious in this study with T/C = 65% on study day 21 (Figure 67, Table 65). The starting average tumor volumes were large (250 $\pm$ 13 $mm^3$) which may account for the more moderate effect following vector treatment only. Efficacy in combination with Doxil® was significantly improved compared to either agent alone ($p<0.01$ by t-test on study day 21). The low dose of vector, $6x10^{11}$ vp/kg, in combination with Doxil® gave similar efficacy as the high dose, $1x10^{12}$ vp/kg, in combination with Doxil® with T/C values on study day 21 of 17% and 18% respectively (Table 65). In the high dose vector plus Doxil® combination treated group, 5 of 10 mice had complete tumor regressions by study day 28. In the low dose plus Doxil® combination group, 3 of 10 had complete regressions by study day 28. No other treated or control groups had complete tumor regressions in this study.

**Table 65.** Doxil® Combination Study % T/C Values

| Treatment | Day 14 | Day 16 | Day 21 |
|---|---|---|---|
| Ar17pAE2fFTrtex 1 e12 vp/kg | 80 | 77 | 65 |
| Doxil® 9 mg/kg (Doxil® only) | 40 | 35 | 37 |
| Ar17pAE2fFTrtex 1 e12 vp/kg, Doxil® 9 mg/kg | 32 | 26 | 18 |
| Ar17pAE2fFTrtex 6e11 vp/kg, Doxil® 9 mg/kg | 26 | 26 | 17 |

% T/C = mean tumor volume for treatment group divided by mean tumor volume for HBSS control group x 100.

20.2.2.3 Body weights

Tolerability of the doxorubicin combination treatment was monitored by weekly body weight measurements (Table 66). Vector treatment alone did not result in decreased body weight. Doxorubicin treatment lead to a maximal body weight loss of 4.8% on study day 27 and the combination treatment group lead to 8.4% maximal body weight loss on study day 13. There was one death noted on day 27 in the doxorubicin combination treatment group. In the second study, Doxil® treatment alone lead to a maximal body weights loss of 7.5% on study day 7. Vector treatment alone at the high dose (1 x $10^{12}$ vp/kg) resulted in a 4.2% decrease in body weight on study day 7. The combination treatment of Doxil® with high dose vector caused a 6.3% body weight loss on study day 7 and the low dose vector combination a 6.4% decrease in body weight (Table 67).

**Table 66.** Effect of Doxorubicin Combination on Mean Body Weight Change (%)

| | Study day | | | | |
|---|---|---|---|---|---|
| | 3 | 6 | 13 | 20 | 27 |
| HBSS | 101±2.5 | 101±2.6 | 101±2.3 | 98.9±1.6 | 99.3±2.8 |
| Ar17pAE2fFTrtex3e12P/Kg | 101±2.4 | 102±2.2 | 105±1.9 | 106±1.8 | 106±1.6 |
| Doxorubicin 10 mg/Kg | 96.8±2.4 | 97.1±2.3 | 96.7±2.3 | 96.1±2.5 | 95.2±5.4 |
| Ar17pAE2fFTrtex+ Doxorubicin | 92.4±1.9 | 95.3±2.3 | 91.6±2.7 | 93.3±3.6 | nd |

Analysis of mean % body weight change after treatment. The body weight of each mouse at indicated study day was compared to its weight at study day 1 that was termed as 100%. Body weights were measured once per week. Data is expressed as mean percent of body weight on SD1 ± SEM. The nd means mean body weight not determined because one mouse died before weighing.

**Table 67.** Effect of Doxorubicin Combination on Mean Body Weight Change (%)

| | Study day | | | | | | |
|---|---|---|---|---|---|---|---|
| | 2 | 4 | 7 | 14 | 21 | 28 | 35 |
| HBSS | nd | 102±1.4 | 100±1.5 | 98.6t2 | nd | nd | nd |
| Ar17pAE2fFTrtex 1e12P/Kg | nd | 98±1.6 | 95.8±2 | 96.1±0 | nd | nd | nd |
| Doxil® 9 mg/Kg | 101±3.1 | 97.1 ±3 | 92.5±3 | 95±3.3 | 101±3.4 | 95.6±3 | 95.3±3 |
| Ar17pAE2fFTrtex 1e12+Doxil® | 102t2.8 | 96.9t3 | 93.7±3 | 98±2.6 | 105±2.7 | 99.5±3 | 101t3.2 |
| Ar17pAE2fFTrtex 6e11 +Doxil® | 101±5.3 | 96.9±5 | 93.6±5 | 96.3± | 104±6 | 101±5.5 | 103±6 |

Analysis of mean % body weight change after treatment. The body weight of each mouse at indicated study day was compared to its weight at study day 0 that was termed as 100%. Body weights were measured once per week. Data is expressed as mean percent of body weight on SD1 ± SEM. The nd means no measurement.

20.2.2.4. *In vivo* combination effect

Our studies *in vitro* have measured a strong synergistic relationship between Ar17pAE2fFhTrtex and paclitaxel, Epothilone B, and doxorubicin against the prostate cancer cell line PC3M.2AC6 and the hepatocellular carcinoma cell line Hep3B. This *in vitro* method uses isobolographic analysis to quantitate the extent of synergy. Quantitation of the *in vivo* synergistic effect is more problematic but one method is to compare the expected and the observed T/C values in the combinations. The expected T/C value is determined by multiplying the T/C value observed for each agent alone. The ratio of expected / observed T/C indicates synergy if it is greater than 1 and antagonism if it is less than 1. Based on this type of analysis, as shown in Table 68, the anti-tumor effect of combining doxorubicin with Ar17pAE2fFhTrtex is marginally synergistic. In the second study, the evidence for synergy is stronger with a ratio of expected/observed equal to 1.34 on study day 21 (Table 74). This analysis was done for the combination of Doxil® with the high dose of vector because the low dose of vector only was not tested in this experiment. Nevertheless, the effect of combining a low dose of vector, $6 \times 10^{11}$ vp/kg, with Doxil® was just as efficacious as combination with the high dose of vector, $1 \times 10^{12}$ vp/kg. We can only speculate that the synergistic effect calculated for the low vector dose would be even greater.

**Table 68.** Expected and Observed T/C values for Doxorubicin Combination Treatment

| Study Day | Ar17pAE2fFTrtex | Dox only | Combination treatment | | Ratio[b] of expected/ observed | Effect |
|---|---|---|---|---|---|---|
| | | | Expected[a] | Observed | | |
| 16 | 0.55 | 0.55 | 0.31 | 0.28 | 1.08 | synergistic |
| 20 | 0.42 | 0.50 | 0.21 | 0.20 | 1.03 | synergistic |

T/C is mean tumor size of treated group divided by HBSS control group. [a]Expected T/C obtained by multiplying the mean T/C of Ar17pAE2fFTrtex by mean T/C of Doxorubicin. [b]Ratio determined by dividing the expected T/C by the observed T/C. A ratio of >1 indicates a synergistic effect; a ratio of <1 indicates a antagonistic effect; and a ratio of =1 indicates an additive effect.

**Table 69.** Expected and Observed T/C values for Doxil® Combination Treatment

| Study Day | Ar17pAE2fFTrtex | Doxil® | Combination treatment | | Ratio[b] of expected/ observed | Effect |
|---|---|---|---|---|---|---|
| | | | Expected[a] | Observed | | |
| 14 | 0.80 | 0.40 | 0.32 | 0.32 | 1.00 | additive |
| 16 | 0.77 | 0.35 | 0.27 | 0.26 | 1.04 | synergistic |
| 21 | 0.65 | 0.37 | 0.24 | 0.18 | 1.34 | synergistic |

T/C is mean tumor size of treated group divided by HBSS control group. [a]Expected T/C obtained by multiplying the mean T/C of Ar17pAE2fFTrtex ($1 \times 10^{12}$ vp/kg) by mean T/C of Doxil®. [b]Ratio determined by dividing the expected T/C by the observed T/C. A ratio of >1 indicates a synergistic effect; a ratio of <1 indicates a antagonistic effect; and a ratio of =1 indicates an additive effect. Combination group analyzed is Ar17pAE2fFTrtex, $1 \times 10^{12}$ vp/kg with Doxil®, 9 mg/kg.

Example 21: *In vitro* toxicity assessment of Ar17pAE2fFTrtex using primary human hepatocytes

The current study was to determine the *in vitro* cytotoxicity of the oncolytic vector Ar17pAE2fFTrtex (E2F-1 promoter control of E1a and hTert promoter control of E4 transcription) in the primary human hepatocyte (PHH) system. The reduction in the cytotoxicity by this vector was evaluated by comparing to the cytotoxicity from a single promoter-controlled vector Ar13pAE2fF (E2F-1 promoter control of E1a transcription). Our results showed that no obvious cytotoxicity was detected from Ar17pAE2fFTrtex and Ar 13pAE2fF at 5 days post transduction of PHH. However, at 7 days post transduction, Ar13pAE2fF had twice the cytotoxicity as that of Ar17pAE2fFTrtex and Addl312 vectors. These results suggest that the E2F-1 single promoter control of E1a gene has effectively reduced the cytotoxicity and the E2F-1/hTERT dual promoter control of E1a and E4 genes further reduced this cytotoxicity.

21.1 Methods

21.1.1 Primary human hepatocyte culture

Primary human hepatocytes were purchased from In Vitro Technologies (Baltimore, MD). The growth media was Hepatocyte Culture Media (HCM™, BioWhittaker/Clonetics Inc., San Diego, CA). Amphotericin B and Penicillin/Streptomycin were added to the HCM at final concentrations of 250 ng/ml, 10 unit/ml & 10 □g/ml respectively to prevent contamination. Upon arrival of cells, media was aspirated from the wells and replaced with fresh HCM. Cells were maintained at 37°C with 5% $CO_2$ in a humidified incubator, and media was replaced every 2 days.

21.1.2 Cell infection and LDH analysis

Primary human hepatocytes in 12-well collagen-coated plates at a concentration of approximately 1.5-1.8 x $10^5$ cells/well were infected with Ar17pAE2fFTrtex, Ar13pAE2fF, Ad5 or Addl312 adenoviral vectors. Cells were treated with adenoviral doses of 0.1, 1.0, 10, and 50 ppc in 200 □l infection media (HCM containing 2% FBS). For infection, culture plates were rocked at 37°C for 1 hour. Then 2 ml growth media was added to each well and the cells were incubated under 37°C with 5% $CO_2$ in a humidified incubator. On days 5 and 7 post infection, growth medium was collected and LDH in the supernatant was measured to determine the amount of LDH released from the cells. Cell lysates for determination of maximal cellular LDH were simultaneously collected by freeze-thaw for three cycles.

The CytoTox® 96 Non-Radioactive Cytotoxicity Assay (Promega, Cat No. G1780) was used for the quantitation of LDH released by cells. Cytotoxicity was defined by the level of LDH released from the cells and was calculated by the following formula:

$$\%\ \text{Cytotoxicity} = \frac{\text{LDH units in culture supernatant x100}}{\text{Sum of LDH units in supernatant and cell lysate}}$$

The cytotoxicity at each dose and time point was determined in triplicate wells except where indicated. Statistical analysis was performed using t test.

21.2 Results and Discussion

PHH were transduced with 1, 10 and 50 ppc of the indicated vectors and cytotoxicity was measured at five and seven days post-infection by an LDH release assay (Figure 70). At the 1 and 10 ppc doses, the level of cytotoxicity associated with Ar17pAE2fFTrtex or Ar13pAE2fF transduction remained low and comparable to the level in Addl312-transduced PHH. In contrast, at a dose of 50 ppc, the single promoter-controlled vector, Ar13pAE2fF, elicited 14% cytotoxicity, which is significantly higher than the 8% cytotoxicity measured in Ar17pAE2fFTrtex transduced cells.

The difference in vector-mediated cytotoxicity between the single and dual promoter-controlled oncolytic vectors was more pronounced at 7 days post infection. At lower doses, the level of cytotoxicity with Ar13pAE2fF, the single-promoter-controlled vector, increased to 16-25%. This greater degree of cytotoxicity at 7 versus 5 days post-infection may be the result of vector replication. In contrast, cytotoxicity with Ar17pAE2fTrtex (8-11%) remained at levels seen 5 days post-infection and was comparable to the level in Addl312-transduced PHH. Interestingly, at the high dose of 50 ppc, cytotoxicity associated with Addl312 was 30% and with Ar17pAE2fFTrtex was 16%. That an E1a-deleted vector such as Addl312 can have lower toxicity than Ar17pAE2fFTrtex indicates that other viral genes, such as E4, can contribute to cytotoxicity in this system *in vitro.* By controlling expression of both E1a and E4 genes, as in the case of Ar17pAE2fFTrtex, *in vitro* cytotoxicity can be significantly reduced.

Example 22: Construction and generation of the Ad35 oncolytic vectors, Ar350scE1A and Ar35E2FE1A

Ad5 genomic DNA was isolated by standard methods from Ad35 virus (ATCC:Catalog #VR-718, Designation: Holden, Stalder H., et al., 1977, J. Clin. Microbiol., 6:257-265). The left (nt 1-985) and the right (nt 29387-34794) terminal restriction enzyme fragments generated by PstI digestion of Ad35 genomic DNA were first cloned into a modified pGEM-3Z (Promega) to generate pGEM3S-Ad35LTF and pGEM3S-Ad35RTF respectively. The modified pGEM-3Z was generated from pGEM-3Z by insertion of a restriction enzyme recognition site for I-SceI between the SmaI and KpnI recognition sites. Next, the cloned left and the right terminal fragments were combined into a single plasmid, pBluescript (Stratagene) to generate pBSMAd35L&RTF. The terminal fragments were combined such that the left and the right ITRs were separated from each other by plasmid sequences. Cotransformation of the PstI-linearized pBSMAd35L&RTF and Ad35 genomic DNA into *E.coli* BJ5183 *recBC sbcBC* resulted in a plasmid, pFLAd35, containing the total Ad35 genome. In pFLAd35, there are two unique I-Scel sites immediately upstream of the left ITR and downstream of the right ITR. Since the I-Scel recognition site is absent in Ad35 genomic DNA, I-Scel digestion allowed the liberation of the full-length Ad35 genome from the pFLAd35. The infectivity of pFLAd35-derived Ad35 genome was demonstrated by LipofectAMINE (Invitrogen-Life Technologies) transfection of PER.C6 cells. The resulting recovered virus was amplified on PER.C6 cells and the genomic DNA was analyzed by restriction enzyme analyses. By restriction enzyme analysis, pFLAd35 derived Ad35 was indistinguishable from wild-type Ad35.

The left (nt 1-3098) and the right (nt 32683-34794) terminal restriction enzyme fragments generated by SphI digestion were subcloned from pFLAd35 to generate the plasmid pAd35L&RSph. The E1A promoter region between nts 339 and 542 was deleted using a PCR approach and a unique PmeI site was engineered into the region to create pAd35L&RSphdelE1 P(L). A 872 bp fragment containing SV40 poly (A) signal and the mouse osteocalcin promoter obtained from pDL6pAOsc was inserted into the PmeI site of pAd35L&RsphdeIE1 P(L) to generate pAd350SOCP(L). The SV40 poly (A) signal was inserted upstream of the osteocalcin promoter to prevent potential transcriptional read through from promoter-like elements present in the left ITR and packaging signal and thus allowing tight regulation of osteocalcin promoter activity in specific tissue. Cotransformation of the SphI-linearized pAd350SOCP(L) and Ad35 genomic DNA into *E.coli* BJ5183 resulted in the plasmid, pFLAd35OstpE1A(L).

The desired Ad35 recombinant virus, Ar350scE1A was generated following transfection of I-Scel digested pFLAD35OstpE1A(L) into PER.C6 cells. The recovered progeny virus was amplified and viral DNA analyzed by restriction enzyme digestions and sequence determination of the left end of the viral DNA.

The Ad35 oncolytic vector, Ad35E2FE1A, was generated in a similar manner to that of Ar35OscE1A. However, the osteocalcin promoter was replaced with the E2F promoter. The viral DNA of Ar35OscE1a was analyzed by restriction digest and the digestion pattern was as expected. A schematic diagram of Ad35OscE1A and Ad35E2FE1A is displayed in Figure 69.

Example23: Evaluation of oncolytic potential of Ar35OscE1a in a PC3 xenograft tumor model in nude mice

The objective of this study was to evaluate the oncolytic activity of Ar35OscE1A, compared to the Ad5-based vector, Ar6pAOscE3F, in a PC3 xenograft model. Groups of 10 animals each were treated with vehicle (HBSS), Ad350.5 (an E1a deficient Ad35 based vector), Ar6pAOscE3F (the Ad5- based oncolytic vector containing the osteocalcin promoter driving expression of E1a), Ad35 (wt virus), and Ar350scE1a (the Ad35-based oncolytic vector containing the osteocalcin promoter driving expression of E1a). All vectors were delivered intratumorally (IT), using a single dose of 2 x $10^{11}$ particles/mouse (1 x $10^{13}$ particles/kg).

Animals with tumors ranging in size from 100-200 $mm^3$ at study day 0, were treated by IT delivery of the indicated vector, or HBSS. Following vector delivery, tumor size was monitored twice weekly, up to day 56, at which time the study was terminated. Tumor measurements were taken in two dimensions and tumor volume calculated as W x $(L)^2\pi/6$. Animals with tumors larger than 2000 $mm^3$ and moribund animals were sacrificed prior to study termination.

The mean tumor sizes for each group are displayed in Figure 70. These data demonstrate that the Ad35OscE1A vector, as well as the Ar6pAOscE3F vector displayed a significant inhibition of tumor growth compared to the HBSS-treated cohorts at time points between day 10 and day 38.

However, the effect of Ar35OscE1A was apparent from days 10- 38, while the effect of Ar6pAOscE3F was apparent from days 17 to 31. These data demonstrate the oncolytic potential of the Ad35-based vector system.

Statistical analyses (ANOVA, and Newman-Keuls Multiple Comparison Test) revealed at day 10 a difference between the HBSS and Ar35OscE1A cohorts (P<0.001), and between Ar35OscE1A and Ad350.5 (P<0.001), and Ad35 wt and HBSS (P<0.05), and Ad35 wt and Ad350.5 (P<0.05). At day 14 a difference was detected between HBSS and Ar35OscE1A cohorts (P<0.01), and between Ar35OscE1A and Ad350.5 (P<0.05), and Ad35 wt and Ar35OscE1A (P<0.05). At day 17 a difference was detected between HBSS and Ar35OscE1A cohorts (P<0.001), and between Ar35OscE1A and Ad350.5 (P<0.01), and HBSS and Ar6pAOscE3F (P<0.01), and Ar6pAOscE3F and Ad350.5 (P<0.05), and HBSS and Ad35 wt (P<0.01). At day 24 a difference was detected between HBSS and Ar350scE1A cohorts (P<0.0001), and HBSS and Ar6pAOscE3F (P<0.01), and Ar6pAOscE3F and Ad350.5 (P<0.05), and HBSS and Ad35 wt (P<0.05). At day 28 a difference was detected between HBSS and Ar35OscE1A cohorts (P<0.001), and between Ar35OscE1A and Ad350.5 (P<0.01), and HBSS and Ar6pAOscE3F (P<0.05), and HBSS and Ad35 wt (P<0.01). At day 31 a difference was detected between HBSS and Ar35OscE1A cohorts (P<0.01), and between Ar35OscE1A and Ad350.5 (P<0.01), and HBSS and Ar6pAOscE3F (P<0.05). At day 35 a difference was detected between HBSS and Ar35OscE1A cohorts (P<0.01), and between Ar35OscE1A and Ad350.5 (P<0.01). At day 38 a difference was detected between HBSS and Ar35OscE1A cohorts (P<0.01), and HBSS and Ar6pAOscE3F (P<0.05), and HBSS and Ad35 wt (P<0.05).

The disclosures of all patents, patent applications, publications (including published patent applications), and database accession numbers referred to in this specification are specifically incorporated herein by reference in their entirety to the same extent as if each such individual patent, patent application, publication, and database number were specifically and individually indicated to be incorporated by reference in its entirety.

**Claims**

1. A recombinant oncolytic adenoviral vector comprising in sequential order in the 5' to 3' direction: a left ITR, an adenoviral packaging signal, a termination signal sequence, an E2F responsive promoter which is operably linked to an adenoviral gene essential for replication of the recombinant viral vector, and a right ITR.

2. A recombinant oncolytic adenoviral vector comprising in sequential order in the 5' to 3' direction: a left ITR, a termination signal sequence, an E2F responsive promoter which is operably linked to an adenoviral gene essential for replication of the recombinant viral vector, an adenoviral packaging signal, and a right ITR.

3. The adenoviral vector of Claim 1 or Claim 2, wherein the termination signal sequence is a polyadenylation signal sequence.

4. The adenoviral vector of Claim 3, wherein the polyadenylation signal sequence is the SV40 early polyadenylation signal sequence.

5. The adenoviral vector of Claim 3, wherein the polyadenylation signal sequence is the SV40 late polyadenylation signal sequence.

6. The adenoviral vector of Claim 1 or Claim 2, wherein the E2F responsive promoter is the human E2F-1 promoter.

7. The adenoviral vector of Claim I or Claim 2, wherein the adenoviral nucleic acid backbone is derived from adenovirus serotype 5 (Ad5) or serotype 35 (Ad35).

8. The adenoviral vector of Claim 1 or Claim 2, wherein the gene essential for replication is the E1a gene.

9. The adenoviral vector of Claim 1 or Claim 2, further comprising a deletion upstream of the termination signal sequence.

10. The adenoviral vector of Claim 1 or Claim 2, wherein the adenoviral nucleic acid backbone comprises an E3 region comprising a mutation or deletion.

11. The adenoviral vector of Claim 10, wherein the E3 region has been deleted from said backbone.

12. The adenoviral vector of Claim 8, wherein the adenoviral nucleic acid backbone comprises an E4 region that is operably linked to a tissue-specific promoter.

13. The adenoviral vector of Claim 12, wherein said tissue-specific promoter is a human telomerase reverse transcriptase promoter.

14. The adenoviral vector of Claim 13, wherein said tissue-specific promoter is the Trtex promoter of SEQ ID NO: 94 or the TERT promoter of SEQ ID NO:93.

15. The adenoviral vector of Claim 12, wherein said tissue-specific promoter is an osteocalcin promoter.

16. The adenoviral vector of Claim 1 or Claim 2, wherein the adenoviral nucleic acid backbone comprises an E1b gene comprising a mutation or deletion.

17. The adenoviral vector of Claim 16, wherein said mutation or deletion results in the loss of an active 19kD protein expressed by the wild-type E1b gene

18. The adenoviral vector of any of Claims 1-17, further comprising a therapeutic gene.

19. The adenoviral vector of Claim 18, wherein the therapeutic gene is inserted in the E3 region.

20. The adenoviral vector of Claim 19, wherein said therapeutic gene is inserted in place of the 19kD or 14.7kD E3 gene.

**21.** The adenoviral vector of Claim 18, wherein said therapeutic gene encodes an immunostimulatory protein.

**22.** The adenoviral vector of Claim 21, wherein said immunostimulatory protein is a cytokine.

**23.** The adenoviral vector of Claim 21, wherein the immunostimulatory protein is selected from the group consisting of GM-CSF, interleukin-1 (IL1), IL2, IL4, IL5, interferon-alpha (IFNα), IFNγ, tumor necrosis factor-alpha (TNFα), IL12, IL18, and flt3.

**24.** The adenoviral vector of Claim 21, wherein said immunostimulatory protein is selected from the group consisting of MIP1α, MIP3α, CCR7 ligand, calreticulin, B7, CD28, MHC class I, MHC class II, and TAPs.

**25.** The adenoviral vector of Claim 21, wherein said immunostimulatory protein is a tumor associated antigen.

**26.** The adenoviral vector of Claim 25, wherein said tumor associated antigen is selected from the group consisting of MART-1, gp100(pmel-17), tyrosinase, tyrosinase-related protein 1, tyrosinase-related protein 2, a melanocyte-stimulating hormone receptor, MAGE1, MAGE2, MAGE3, MAGE12, BAGE, GAGE, NY-ESO-1, β-catenin, MUM-1, CDK-4, caspase 8, KIA 0205, HLA-A2R1701, α-fetoprotein, telomerase catalytic protein, G-250, MUC-1, carcinoembryonic protein, p53, Her2/neu, triosephosphate isomerase, CDC-27, and LDLR-FUT.

**27.** The adenoviral vector of Claim 21, wherein said immunostimulatory protein is an antibody that blocks inhibitory signals.

**28.** The adenoviral vector of Claim 27, wherein the inhibitory signal is due to expression of CTLA4.

**29.** The adenoviral vector of Claim 18, wherein the therapeutic gene encodes an anti-angiogenic protein.

**30.** The adenoviral vector of Claim 29, wherein said anti-angiogenic protein is selected from the group consisting of a VEGF/VEGFR antagonist, an angiopoietin/Tie antagonist, an Ephrin/Eph antagonist, and an FGF/FGFR antagonist.

**31.** The adenoviral vector of Claim 29, wherein said anti-angiogenic protein is an inhibitor of PDGF, TGFß, or IGF-1.

**32.** The adenoviral vector of Claim 29, wherein said anti-angiogenic protein is a fragment of an extracellular matrix protein.

**33.** The adenoviral vector of Claim 32, wherein said extracellular matrix protein is selected from the group consisting of angiostatin, endostatin, kininostatin, fibrinogen-E, thrombospondin, tumstatin, canstatin, and restin.

**34.** The adenoviral vector of Claim 29, wherein the anti-angiogenic protein is a fragment of TrpRS.

**35.** The adenoviral vector of Claim 29, wherein the anti-angiogenic protein is selected from the group consisting of sFlt-1, sFlk, sNRP1, sTie-2, IP-10, PF-4, Gro-beta, IFN-gamma (Mig), sEphB4, sephrinB2, vasostatin, PEDF, prolactin fragment, proliferin-related protein, METH-1, and METH-2.

**36.** The adenoviral vector of Claim 18, wherein said therapeutic gene encodes a protein that leads to cell death.

**37.** The adenoviral vector of Claim 36, wherein said protein that leads to cell death is selected from the group consisting of carboxypeptidase G2 (CPG2), carboxylesterase (CA), cytosine deaminase (CD), cytochrome P450 (cyt-450), deoxycytidine kinase (dCK), herpes simplex virus thymidine kinase (HSV-TK), nitroreductase (NR), purine nucleoside phosphorylase (PNP), thymidine phosphorylase (TP), varicella zoster virus thymidine kinase (VZV-TK), and xanthine-guanine phosphoribosyl transferase (XGPRT).

**38.** An adenoviral vector particle comprising the adenoviral vector of any one of Claims 1-37.

**39.** The adenoviral vector particle of Claim 38, further comprising a targeting ligand included in a capsid protein of said particle.

**40.** The adenoviral vector particle of Claim 39, wherein said capsid protein is a fiber protein.

**41.** The adenoviral vector particle of Claim 40, wherein said ligand is in the H1 loop of said fiber protein.

**42.** A eukaryotic cell transfected with the adenoviral vector particle of Claim 38.

**43.** A pharmaceutical composition comprising the adenoviral vector particle of Claims 38-41 and a pharmaceutically acceptable carrier.

**44.** Use of an effective amount of the adenoviral vector particle of any one of claims 38-41 in the manufacture of a medicament for selectively killing a neoplastic cell in a cell population, wherein upon administration of the medicament the recombinant viral vector can transduce the cells of said cell population.

**45.** The use of Claim 44, wherein the neoplastic cell has a defect in the Rb-pathway.

**46.** Use of the adenoviral vector particle of any one of claims 38-41 in the manufacture of a medicament for treating a host organism having a neoplastic condition.

**47.** The use of Claim 46, wherein the host organism is a human patient.

**48.** The use of Claim 46, wherein the neoplastic condition is lung, breast, prostate or colon cancer.

## Patentansprüche

**1.** Rekombinanter, onkolytischer, adenoviraler Vektor, umfassend in sequentieller Ordnung in 5' - 3' Richtung: eine linke terminale invertierte Sequenzwiederholung (ITR), ein adenovirales Verpackungssignal, eine Terminationssignalsequenz, einen E2F-responsiven Promotor, der funktionell mit einem adenoviralen Gen verbunden ist, das essentiell für die Replikation des rekombinanten viralen Vektors ist, und eine rechte terminale invertierte Wiederholungssequenz (ITR).

**2.** Rekombinanter, onkolytischer, adenoviraler Vektor, umfassend in sequentieller Ordnung in 5' - 3' Richtung: eine linke terminale invertierte Wiederholungssequenz (ITR), eine Terminationssignalsequenz, einen E2F-responsiven Promotor, der funktionell mit einem adenoviralen Gen verbunden ist, das essentiell für die Replikation des rekombinanten viralen Vektors ist, ein adenovirales Verpakkungssignal und eine rechte terminale invertierte Wiederholungssequenz (ITR).

**3.** Adenoviraler Vektor nach Anspruch 1 oder 2, wobei die Terminationssignalsequenz eine Polyadenylierungssignalsequenz ist.

**4.** Adenoviraler Vektor nach Anspruch 3, wobei die Polyadenylierungssignalsequenz die frühe SV40-Polyadenylierungssignalsequenz ist.

**5.** Adenoviraler Vektor nach Anspruch 3, wobei die Polyadenylierungssignalsequenz die späte SV40-Polyadenylierungssignalsequenz ist.

**6.** Adenoviraler Vektor nach Anspruch 1 oder Anspruch 2, wobei der E2F-responsive Promotor der humane E2F-1 Promotor ist.

**7.** Adenoviraler Vektor nach Anspruch 1 oder Anspruch 2, wobei das adenovirale Nukleinsäurerückgrat von Adenovirus Serotyp 5 (Ad5) oder Serotyp 35 (Ad35) stammt.

**8.** Adenoviraler Vektor nach Anspruch 1 oder Anspruch 2, wobei das für die Replikation essentielle Gen das E1a-Gen ist.

**9.** Adenoviraler Vektor nach Anspruch 1 oder Anspruch 2, weiter eine der Terminationssignalsequenz vorgelagerte Deletion umfassend.

**10.** Adenoviraler Vektor nach Anspruch 1 oder Anspruch 2, wobei das adenovirale Nukleinsäurerückgrat eine E3-Region umfaßt, die eine Mutation oder Deletion umfaßt.

**11.** Adenoviraler Vektor nach Anspruch 10, wobei die E3-Region aus dem Rückgrat deletiert wurde.

**12.** Adenoviraler Vektor nach Anspruch 8, wobei das adenovirale Nukleinsäurerückgrat eine E4-Region umfaßt, die funktionell mit einem gewebsspezifischen Promotor verbunden ist.

**13.** Adenoviraler Vektor nach Anspruch 12, wobei der gewebsspezifische Promotor ein humaner Telomerase Reverse Transkriptase-Promotor ist.

**14.** Adenoviraler Vektor nach Anspruch 13, wobei der gewebsspezifische Promotor der Trtex-Promotor nach SEQ ID NR: 94, oder der TERT-Promotor nach SEQ ID NR: 93 ist.

**15.** Adenoviraler Vektor nach Anspruch 12, wobei der gewebsspezifische Promotor ein Osteocalcin-Promotor ist.

**16.** Adenoviraler Vektor nach Anspruch 1 oder Anspruch 2, wobei das adenovirale Nukleinsäurerückgrat ein E1 b-Gen umfaßt, das eine Mutation oder Deletion umfaßt.

**17.** Adenoviraler Vektor nach Anspruch 16, wobei die Mutation oder Deletion zum Verlust eines aktiven 19 kDa Proteins führt, das vom Wildtyp E1b-Gen exprimiert wird.

**18.** Adenoviraler Vektor nach einem der Ansprüche 1 bis 17, weiterhin ein therapeutisches Gen umfassend.

**19.** Adenoviraler Vektor nach Anspruch 18, wobei das therapeutische Gen in die E3-Region insertiert ist.

**20.** Adenoviraler Vektor nach Anspruch 19, wobei das therapeutische Gen an Stelle des 19 kDa oder 14,7 kDa E3-Gens insertiert ist.

**21.** Adenoviraler Vektor nach Anspruch 18, wobei das therapeutische Gen ein immunstimulatorisches Protein kodiert.

**22.** Adenoviraler Vektor nach Anspruch 21, wobei das immunstimulatorische Protein ein Cytokin ist.

**23.** Adenoviraler Vektor nach Anspruch 21, wobei das immunstimulatorische Protein ausgewählt ist aus der Gruppe, bestehend aus GM-CSF, Interleukin-1 (IL1), 1L2, IL4, 1L5, Interferon-alpha (IFN$\alpha$), IFN$\gamma$, Tumornekrosefaktor-alpha (TNF$\alpha$), IL12, IL18 und flt3.

**24.** Adenoviraler Vektor nach Anspruch 21, wobei das immunstimulatorische Protein ausgewählt ist aus der Gruppe, bestehend aus MIP1$\alpha$, MIP3$\alpha$, CCR7-Ligand, Calreticulin, B7, CD28, MHC Klasse I, MHC Klasse II und TAPs.

**25.** Adenoviraler Vektor nach Anspruch 21, wobei das immunstimulatorische Protein ein Tumor-assoziiertes Antigen ist.

**26.** Adenoviraler Vektor nach Anspruch 25, wobei das Tumor-assoziierte Antigen ausgewählt ist aus der Gruppe, bestehend aus MART-1, gp100 (pmel-17), Tyrosinase, Tyrosinase-assoziiertem Protein 1, Tyrosinase-assoziiertem Protein 2, einem Melanocyten-stimulierenden Hormonrezeptor, MAGE1, MAGE2, MAGE3, MAGE12, BAGE, GAGE, NY-ESO-1, $\beta$-Catenin, MUM-1, CDK-4, Caspase 8, KIA 0205, HLA-A2R1701, $\alpha$-Fetoprotein, Telomerase Katalytischem Protein, G-250, MUC-1, carcinoembryonalem Protein, p53, Her2/neu, Triosephosphatisomerase, CDC-27 und LDLR-FUT.

**27.** Adenoviraler Vektor nach Anspruch 21, wobei das immunstimulatorische Protein ein Antikörper ist, der inhibitorische Signale blockiert.

**28.** Adenoviraler Vektor nach Anspruch 27, wobei das inhibitorische Signal von der Expression von CTLA4 herrührt.

**29.** Adenoviraler Vektor nach Anspruch 18, wobei das therapeutische Gen ein antiangiogenes Protein kodiert.

**30.** Adenoviraler Vektor nach Anspruch 29, wobei das anti-angiogene Protein ausgewählt ist aus der Gruppe, bestehend aus einem VEGF/VEGFR-Antagonisten, einem Angiopoietin/Tie-Antagonisten, einem Ephrin/Eph-Antagonisten und einem FGF/FGFR-Antagonisten.

**31.** Adenoviraler Vektor nach Anspruch 29, wobei das anti-angiogene Protein ein Inhibitor von PDGF, TGF$\beta$ oder IGF-1 ist.

**32.** Adenoviraler Vektor nach Anspruch 29, wobei das anti-angiogene Protein ein Fragment eines Proteins der extrazellulären Matrix ist.

**33.** Adenoviraler Vektor nach Anspruch 32, wobei das Protein der extrazellulären Matrix ausgewählt ist aus der Gruppe, bestehend aus Angiostatin, Endostatin, Kininostatin, Fibrinogen-E, Thrombospondin, Tumstatin, Canstatin und Restin.

**34.** Adenoviraler Vektor nach Anspruch 29, wobei das anti-angiogene Protein ein Fragment von TrpRS ist.

**35.** Adenoviraler Vektor nach Anspruch 29, wobei das anti-angiogene Protein ausgewählt ist aus der Gruppe, bestehend aus sFlt-1, sFlk, sNRP1, sTie-2, IP-10, PF-4, Gro-beta, IFN-gamma (Mig), sEphB4, sephrinB2, Vasostatin, PEDF, Prolactinfragment, Proliferin-verwandtem Protein, METH-1 und METH-2.

**36.** Adenoviraler Vektor nach Anspruch 18, wobei das therapeutische Gen ein Protein kodiert, das zum Zelltod führt.

**37.** Adenoviraler Vektor nach Anspruch 36, wobei das Protein, das zum Zelltod führt, ausgewählt ist aus der Gruppe, bestehend aus Carboxypeptidase G2 (CPG2), Carboxylesterase (CA), Cytosindeaminase (CD), Cytochrom P450 (cyt-450), Deoxycytidinkinase (dCK), Herpes simplex-Virus Thymidinkinase (HSV-TK), Nitroreduktase (NR), Purinnukleosidphosphorylase (PNP), Thymidinphosphorylase (TP), Varicella zoster-Virus Thymidinkinase (VZV-TK) und Xanthin-Guanin-Phosphoribosyltransferase (XGPRT).

**38.** Adenovirales Vektor-Teilchen, umfassend den adenoviralen Vektor nach einem der Ansprüche 1 bis 37.

**39.** Adenovirales Vektor-Teilchen nach Anspruch 38, weiter einen Targeting-Liganden umfassend, der im Capsidprotein des Teilchens beinhaltet ist.

**40.** Adenovirales Vektor-Teilchen nach Anspruch 39, wobei das Capsidprotein ein Faserprotein ist.

**41.** Adenovirales Vektor-Teilchen nach Anspruch 40, wobei der Ligand in der H1-Schleife des Faserproteins liegt.

**42.** Eukaryote Zelle, die mit dem adenoviralen Vektor-Teilchen nach Anspruch 38 transfiziert ist.

**43.** Pharmazeutische Zusammensetzung, das adenovirale Vektor-Teilchen nach den Ansprüchen 38 bis 41 und ein pharmazeutisch verträgliches Trägermaterial umfassend.

**44.** Verwendung einer wirksamen Menge des adenoviralen Vektorteilchens nach einem der Ansprüche 38 bis 41 in der Herstellung eines Medikaments zur selektiven Abtötung einer neoplastischen Zelle in einer Zellpopulation, wobei der rekombinante, virale Vektor bei Verabreichen des Medikaments die Zellen der Zellpopulation transduzieren kann.

**45.** Verwendung nach Anspruch 44, wobei die neoplastische Zelle einen Defekt im Rb-Signalweg aufweist.

**46.** Verwendung des adenoviralen Vektorteilchens nach einem der Ansprüche 38 bis 41 in der Herstellung eines Medikaments zur Behandlung eines Wirtsorganismus, der einen neoplastischen Zustand aufweist.

**47.** Verwendung nach Anspruch 46, wobei der Wirtsorganismus ein menschlicher Patient ist.

**48.** Verwendung nach Anspruch 46, wobei der neoplastische Zustand Lungen-, Brust-, Prostata- oder Kolonkrebs ist.

## Revendications

**1.** Vecteur adénoviral oncolytique recombinant comprenant dans un ordre séquentiel dans la direction 5’ vers 3’ : une ITR gauche, un signal d’encapsidation adénoviral, une séquence signal de terminaison, un promoteur sensible à E2F qui est en liaison fonctionnelle avec un gène adénoviral essentiel à la réplication du vecteur viral recombinant, et une ITR droite.

**2.** Vecteur adénoviral oncolytique recombinant comprenant dans un ordre séquentiel dans la direction 5’ vers 3’ : une ITR gauche, une séquence signal de terminaison, un promoteur sensible à E2F qui est en liaison fonctionnelle avec un gène adénoviral essentiel à la réplication du vecteur viral recombinant, un signal d'encapsidation adénoviral, et une ITR droite.

3. Vecteur adénoviral selon la revendication 1 ou la revendication 2, dans lequel la séquence signal de terminaison est une séquence signal de polyadénylation.

4. Vecteur adénoviral selon la revendication 3, dans lequel la séquence signal de polyadénylation est la séquence signal de polyadénylation précoce de SV40.

5. Vecteur adénoviral selon la revendication 3, dans lequel la séquence signal de polyadénylation est la séquence signal de polyadénylation tardive de SV40.

6. Vecteur adénoviral selon la revendication 1 ou la revendication 2, dans lequel le promoteur sensible à E2F est le promoteur E2F-1 humain.

7. Vecteur adénoviral selon la revendication 1 ou la revendication 2, dans lequel le squelette d'acide nucléique adénoviral est dérivé de l'adénovirus de sérotype 5 (Ad5) ou de sérotype 35 (Ad35).

8. Vecteur adénoviral selon la revendication 1 ou la revendication 2, dans lequel le gène essentiel à la réplication est le gène E1a.

9. Vecteur adénoviral selon la revendication 1 ou la revendication 2, comprenant en outre une délétion en amont de la séquence signal de terminaison.

10. Vecteur adénoviral selon la revendication 1 ou la revendication 2, dans lequel le squelette d'acide nucléique adénoviral comprend une région E3 comprenant une mutation ou une délétion.

11. Vecteur adénoviral selon la revendication 10, dans lequel la région E3 a été délétée dudit squelette.

12. Vecteur adénoviral selon la revendication 8, dans lequel le squelette d'acide nucléique adénoviral comprend une région E4 qui est en liaison fonctionnelle avec un promoteur spécifique de tissu.

13. Vecteur adénoviral selon la revendication 12, dans lequel ledit promoteur spécifique de tissu est un promoteur de la transcriptase inverse de la télomérase humaine.

14. Vecteur adénoviral selon la revendication 13, dans lequel ledit promoteur spécifique de tissu est le promoteur Trtex de SEQ ID n° 94 ou le promoteur TERT de SEQ ID n° 93.

15. Vecteur adénoviral selon la revendication 12, dans lequel ledit promoteur spécifique de tissu est un promoteur de l'ostéocalcine.

16. Vecteur adénoviral selon la revendication 1 ou la revendication 2, dans lequel le squelette d'acide nucléique adénoviral comprend un gène E1b comprenant une mutation ou une délétion.

17. Vecteur adénoviral selon la revendication 16, dans lequel ladite mutation ou délétion entraîne la perte d'une protéine active de 19 kD exprimée par le gène E1b de type sauvage.

18. Vecteur adénoviral selon l'une quelconque des revendications 1 à 17, comprenant en outre un gène thérapeutique.

19. Vecteur adénoviral selon la revendication 18, dans lequel le gène thérapeutique est inséré dans la région E3.

20. Vecteur adénoviral selon la revendication 19, dans lequel ledit gène thérapeutique est inséré à la place du gène E3 de 19 kD ou 14,7 kD.

21. Vecteur adénoviral selon la revendication 18, dans lequel ledit gène thérapeutique code pour une protéine immunostimulatrice.

22. Vecteur adénoviral selon la revendication 21, dans lequel ladite protéine immunostimulatrice est une cytokine.

**23.** Vecteur adénoviral selon la revendication 21, dans lequel la protéine immunostimulatrice est choisie dans le groupe constitué par GM-CSF, l'interleukine-1 (IL1), IL2, IL4, IL5, l'interféron-alpha (IFN$\alpha$), IFN$\gamma$, le facteur de nécrose tumorale alpha (TNF$\alpha$), IL12, IL18 et flt3.

**24.** Vecteur adénoviral selon la revendication 21, dans lequel ladite protéine immunostimulatrice est choisie dans le groupe constitué par MIP1$\alpha$, MIP3$\alpha$, le ligand du CCR7, la calréticuline, B7, CD28, le CMH de classe I, le CMH de classe II, et les TAP.

**25.** Vecteur adénoviral selon la revendication 21, dans lequel ladite protéine immunostimulatrice est un antigène tumoral.

**26.** Vecteur adénoviral selon la revendication 25, dans lequel ledit antigène tumoral est choisi dans le groupe constitué par MART-1, gp100(pmel-17), la tyrosinase, la protéine 1 liée à la tyrosinase, la protéine 2 liée à la tyrosinase, un récepteur de l'hormone stimulant les mélanocytes, MAGE1, MAGE2, MAGE3, MAGE12, BAGE, GAGE, NY-ESO-1, la $\beta$-caténine, MUM-1, CDK-4, la caspase 8, KIA 0205, HLA-A2R1701, l'$\alpha$-foetoprotéine, la protéine catalytique de la télomérase, G-250, MUC-1, la protéine carcinoembryonnaire, p53, Her2/neu, la triosephosphate isomérase, CDC-27 et LDLR-FUT.

**27.** Vecteur adénoviral selon la revendication 21, dans lequel ladite protéine immunostimulatrice est un anticorps qui bloque les signaux inhibiteurs.

**28.** Vecteur adénoviral selon la revendication 27, dans lequel le signal inhibiteur est dû à l'expression de CTLA4.

**29.** Vecteur adénoviral selon la revendication 18, dans lequel le gène thérapeutique code pour une protéine anti-angiogénique.

**30.** Vecteur adénoviral selon la revendication 29, dans lequel ladite protéine anti-angiogénique est choisie dans le groupe constitué par un antagoniste de VEGF/VEGFR, un antagoniste de l'angiopoïétine/Tie, un antagoniste de Ephrin/Eph, et un antagoniste de FGF/FGFR.

**31.** Vecteur adénoviral selon la revendication 29, dans lequel ladite protéine anti-angiogénique est un inhibiteur du PDGF, TGF$\beta$, ou IGF-1.

**32.** Vecteur adénoviral selon la revendication 29, dans lequel ladite protéine anti-angiogénique est un fragment d'une protéine de la matrice extracellulaire.

**33.** Vecteur adénoviral selon la revendication 32, dans lequel ladite protéine de la matrice extracellulaire est choisie dans le groupe constitué par l'angiostatine, l'endostatine, la kininostatine, le fibrinogène-E, la thrombospondine, la tumstatine, la cantastine, et la restine.

**34.** Vecteur adénoviral selon la revendication 29, dans lequel la protéine anti-angiogénique est un fragment de TrpRS.

**35.** Vecteur adénoviral selon la revendication 29, dans lequel la protéine anti-angiogénique est choisie dans le groupe constitué par sFlt-1, sFlk, sNRP1, sTie-2, IP-10, PF-4, Gro-bêta, IFN-gamma (Mig), sEphB4, sephrinB2, la vasostatine, PEDF, un fragment de prolactine, la protéine liée à la proliférine, METH-1 et METH-2.

**36.** Vecteur adénoviral selon la revendication 18, dans lequel ledit gène thérapeutique code pour une protéine qui conduit à la mort cellulaire.

**37.** Vecteur adénoviral selon la revendication 36, dans lequel ladite protéine qui conduit à la mort cellulaire est choisie dans le groupe constitué par la carboxypeptidase G2 (CPG2), la carboxylestérase (CA), la cytosine désaminase (CD), le cytochrome P450 (cyt-450), la désoxycytidine kinase (dCK), la thymidine kinase du virus herpès simplex (HSV-TK), la nitroréductase (NR), la purine nucléoside phosphorylase (PNP), la thymidine phosphorylase (TP), la thymidine kinase du virus de la varicelle et du zona (VZV-TK), et la xanthine-guanine phosphoribosyl transférase (XGPRT).

**38.** Particule de vecteur adénoviral comprenant le vecteur adénoviral selon l'une quelconque des revendications 1 à 37.

**39.** Particule de vecteur adénoviral selon la revendication 38, comprenant en outre un ligand de ciblage inclus dans une protéine capsidique de ladite particule.

**40.** Particule de vecteur adénoviral selon la revendication 39, dans laquelle ladite protéine capsidique est une protéine fibreuse.

**41.** Particule de vecteur adénoviral selon la revendication 40, dans laquelle ledit ligand est dans la boucle H1 de ladite protéine fibreuse.

**42.** Cellule eucaryote transfectée avec la particule de vecteur adénoviral selon la revendication 38.

**43.** Composition pharmaceutique comprenant la particule de vecteur adénoviral selon les revendications 38 à 41 et un véhicule pharmaceutiquement acceptable.

**44.** Utilisation d'une quantité efficace de la particule de vecteur adénoviral selon l'une quelconque des revendications 38 à 41 dans la fabrication d'un médicament destiné à tuer de manière sélective une cellule néoplasique dans une population de cellules, dans laquelle, après administration du médicament, le vecteur viral recombinant peut transduire les cellules de ladite population de cellules.

**45.** Utilisation selon la revendication 44, dans laquelle la cellule néoplasique a une anomalie dans la voie Rb.

**46.** Utilisation de la particule de vecteur adénoviral selon l'une quelconque des revendications 38 à 41 dans la fabrication d'un médicament destiné à traiter un organisme hôte ayant une pathologie néoplasique.

**47.** Utilisation selon la revendication 46, dans laquelle l'organisme hôte est un patient humain.

**48.** Utilisation selon la revendication 46, dans laquelle la pathologie néoplasique est le cancer du poumon, du sein, de la prostate ou du côlon.

Fig. 1 Cleavage and Polyadenylation Process For The SV40 early Poly(A) site

**A.** CTTATCGATACCGTCGAAACTTGTTTATTGCAGCTTATAATGGTTACAAATAAAGCAATAGCAT

CACAAATTTCACAAATAAAGCATTTTTTTCACTGCATTCTAGTTGTGGTTTGTCCAAACTCATCA

++++++ (under AATAAA) ↑ Cleavage site (between A and T of GCATT) +++++++++++ (under downstream element)

ATGTATCTTATCATGTC **(Seq ID NO:1)**

**B.** AAUAAA ++++++ ——— GCA

**C.** ——— GCAaaaaaaaaaaaaaaaaaaaa **(Seq ID NO:18)**

\+ Upstream and downstream cleavage-polyadenylation elements

**(Seq ID NO:2)**

# Fig 2 E1A transcription control region

ITR

CATCATCAAT AATATACCTT ATTTTGGATT GAAGCCAATA TGATAATGAG GGGGTGGAGT 60

ITR

TTGTGACGTG GCGCGGGGCG TGGGAACGGG GCGGGTGACG TAGTAGTGTG GCGGAAGTGT 120

AP3 DNA BS

GATGTTGCAA GTGTGGCGGA ACACATGTAA GCGACGGATG TGGCAAAAGT GAC G TTTTTG 180

Ψ

GTGTGCGCCG GTGTACACAG GAAGTGACAA TTTTCGCGCG GTTTTAGGCG GATGTTGTAG 240

TAAATTTGGG CGTAACCGAG TAAGATTTGG CCATTTTCGC GGGAAAACTG AATAAGAGGA 300

Ψ

AGTGAAATCT GAATAATTTT GTGTTACTCA TAGCGCGTAA TATTTGTCTA GGGCCGCGGG 360

GACTTTGACC GTTTACGTGG AGACTCGCCC AGGTGTTTTT CTCAGGTGTT TTC CGC GTTC 420

E1a TATA Box

CGGGTCAAAG TTGGCGTTTT ATTATTATAG TCAGCTGACG TGTAGTGTAT TTA TAC CCGG 480

+1

TGAGTTCCTC AAGAGGCCAC TCTTGAGTGC CAGCGAGTAG AGTTTTCTCC TCC GAG CCGC 540

TCCGACACCG GGACTGAAA A TGAGACATAT TATCTGCCAC GGAGGTGTTA TTACCGAAGA 600

- • Enhancer elements
- X E2F-motif
- \+ Packaging elements

dl 103-551 **Ar6**

dl 189-551

dl 357-551 **Ar5**

**Figure 3**. Sequence of Ar6pAE2fF from left and right ends of viral DNA

A. Nucleotides 1-1802 containing ITR, polyA, E2F-1 promoter, E1a and a portion of the E1b gene (Seq ID NO:3)

```
  1 CATCATCAATAATATACCTTATTTTGGATTGAAGCCAATATGATAATGAGGGGGTGGAGT
    +-------------------------ITR-------------------------------

 61 TTGTGACGTGGCGCGGGGCGTGGGAACGGGGCGGGTGACGTAGGGCGCGATCAAGCTTAT
    +-------------------------ITR------------+             +----

121 CGATACCGTCGAAACTTGTTTATTGCAGCTTATAATGGTTACAAATAAAGCAATAGCATC
    -------------------------polyA------------------------------

181 ACAAATTTCACAAATAAAGCATTTTTTTCACTGCATTCTAGTTGTGGTTTGTCCAAACTC
    -------------------------polyA------------------------------

241 ATCAATGTATCTTATCATGTCTGGATCCGCGCCGCTAGCGATCATCCGGACAAAGCCTGC
    -------------------+                       +----------------

301 GCGCGCCCCGCCCCGCCATTGGCCGTACCGCCCCGCGCCGCCGCCCCATCTCGCCCCTCG
    ------------------------E2F-1 promoter----------------------

361 CCGCCGGGTCCGGCGCGTTAAAGCCAATAGGAACCGCCGCCGTTGTTCCCGTCACGGCCG
    ------------------------E2F-1 promoter----------------------

421 GGGCAGCCAATTGTGGCGGCGCTCGGCGGCTCGTGGCTCTTTCGCGGCAAAAAGGATTTG
    ------------------------E2f-1 promoter----------------------

481 GCGCGTAAAAGTGGCCGGGACTTTGCAGGCAGCGGCGGCCGGGGGCGGAGCGGGATCGAG
    ------------------------E2f-1 promoter----------------------

541 CCCTCGATGATATCAGATCATCGGATCCCGGTCGACTGAAAATGAGACATATTATCTGCC
    --------------+                    +------------------------

601 ACGGAGGTGTTATTACCGAAGAAATGGCCGCCAGTCTTTTGGACCAGCTGATCGAAGAGG
    -------------------------E1a gene---------------------------

661 TACTGGCTGATAATCTTCCACCTCCTAGCCATTTTGAACCACCTACCCTTCACGAACTGT
    -------------------------E1a gene---------------------------

721 ATGATTTAGACGTGACGGCCCCCGAAGATCCCAACGAGGAGGCGGTTTCGCAGATTTTTC
    -------------------------E1a gene---------------------------

781 CCGACTCTGTAATGTTGGCGGTGCAGGAAGGGATTGACTTACTCACTTTTCCGCCGGCGC
    -------------------------E1a gene---------------------------

841 CCGGTTCTCCGGAGCCGCCTCACCTTTCCCGGCAGCCCGAGCAGCCGGAGCAGAGAGCCT
    -------------------------E1a gene---------------------------

901 TGGGTCCGGTTTCTATGCCAAACCTTGTACCGGAGGTGATCGATCTTACCTGCCACGAGG
    -------------------------E1a gene---------------------------
```

```
961  CTGGCTTTCCACCCAGTGACGACGAGGATGAAGAGGGTGAGGAGTTTGTGTTAGATTATG
     -------------------------E1a gene--------------------------

1021 TGGAGCACCCCGGGCACGGTTGCAGGTCTTGTCATTATCACCGGAGGAATACGGGGGACC
     -------------------------E1a gene--------------------------

1081 CAGATATTATGTGTTCGCTTTGCTATATGAGGACCTGTGGCATGTTTGTCTACAGTAAGT
     -------------------------E1a gene--------------------------

1141 GAAAATTATGGGCAGTGGGTGATAGAGTGGTGGGTTTGGTGTGGTAATTTTTTTTTTAAT
     -------------------------E1a gene--------------------------

1201 TTTTACAGTTTTGTGGTTTAAAGAATTTTGTATTGTGATTTTTTTAAAAGGTCCTGTGTC
     -------------------------E1a gene--------------------------

1261 TGAACCTGAGCCTGAGCCCGAGCCAGAACCGGAGCCTGCAAGACCTACCCGCCGTCCTAA
     -------------------------E1a gene--------------------------

1321 AATGGCGCCTGCTATCCTGAGACGCCCGACATCACCTGTGTCTAGAGAATGCAATAGTAG
     -------------------------E1a gene--------------------------

1381 TACGGATAGCTGTGACTCCGGTCCTTCTAACACACCTCCTGAGATACACCCGGTGGTCCC
     -------------------------E1a gene--------------------------

1441 GCTGTGCCCCATTAAACCAGTTGCCGTGAGAGTTGGTGGGCGTCGCCAGGCTGTGGAATG
     -------------------------E1a gene--------------------------

1501 TATCGAGGACTTGCTTAACGAGCCTGGGCAACCTTTGGACTTGAGCTGTAAACGCCCCAG
     -------------------------E1a gene--------------------------

1561 GCCATAAGGTGTAAACCTGTGATTGCGTGTGTGGTTAACGCCTTTGTTTGCTGAATGAGT
     -------------------------E1a gene--------------------------

1621 TGATGTAAGTTTAATAAAGGGTGAGATAATGTTTAACTTGCATGGCGTGTTAAATGGGGC
     ---------------------------+--------------------------------

1681 GGGGCTTAAAGGGTATATAATGCGCCGTGGGCTAATCTTGGTTACATCTGACCTCATGGA
     -------------------------E1b gene--------------------------

1741 GGCTTGGGAGTGTTTGGAAGATTTTTCTGCTGTGCGTAACTTGCTGGAACAGAGCTCTAA
     -------------------------E1b gene--------------------------

1801 CA
     --
```

B. Nucleotides 33881-34412 containing packaging signal and ITR (Seq ID NO:4)

```
33881 AACCTACGCCCAGAAACGAAAGCCAAAAAACCCACAACTTCCTCAAATCGTCACTTCCGT

33941 TTTCCCACGTTACGTCACTTCCCATTTTAATTAAGAATTCTACAATTCCCAACACATACA
```

```
34001 AGTTACTCCGCCCTAAAACCCTGGGCGAGTCTCCACGTAAACGGTCAAAGTCCCCGCGGC
                                  +-packaging signal----------------------

34061 CCTAGACAAATATTACGCGCTATGAGTAACACAAAATTATTCAGATTTCACTTCCTCTTA
      --------------------packaging signal----------------------

34121 TTCAGTTTTCCCGCGAAAATGGCCAAATCTTACTCGGTTACGCCCAAATTTACTACAACA
      --------------------packaging signal----------------------

34181 TCCGCCTAAAACCGCGCGAAAATTGTCACTTCCTGTGTACACCGGCGCACACCAAAAACG
      -----------------------------------+

34241 TCACTTTTGCCACATCCGTCGCTTACATGTGTTCCGCCACACTTGCAACATCACACTTCC

34301 GCCACACTACTACGTCACCCGCCCCGTTCCCACGCCCCGCGCCACGTCACAAACTCCACC
               +-----------------ITR-----------------------------

34361 CCCTCATTATCATATTGGCTTCAATCCAAAATAAGGTATATTATTGATGATG
      --------------------------ITR--------------------+
```

**Figure 4**. Sequence of Ar6F from left end of viral DNA (Seq ID NO:5)

```
  1 CATCATCAATAATATACCTTATTTTGGATTGAAGCCAATATGATAATGAGGGGGTGGAGT
      +-------------------------ITR----------------------------------

 61 TTGTGACGTGGCGCGGGGCGTGGGAACGGGGCGGGTGACGTAGGGCGCGCCGCTAGCGAT
    -------------------------ITR--------------++------MCS--------

121 ATCGGATCCCGGTCGACTGAAAATGAGACATATTATCTGCCACGGAGGTGTTATTACCGA
    --------------+------------E1a-------------------------------

181 AGAAATGGCCGCCAGTCTTTTGGACCAGCTGATCGAAGAGGTACTGGCTGATAATCTTCC
    ---------------------------E1a-------------------------------

241 ACCTCCTAGCCATTTTGAACCACCTACCCTTCACGAACTGTATGATTTAGACGTGACGGC
    ---------------------------E1a-------------------------------

301 CCCCGAAGATCCCAACGAGGAGGCGGTTTCGCAGATTTTTCCCGACTCTGTAATGTTGGC
    ---------------------------E1a-------------------------------

361 GGTGCAGGAAGGGATTGACTTACTCACTTTTCCGCCGGCGCCCGGTTCTCCGGAGCCGCC
    ---------------------------E1a-------------------------------

421 TCACCTTTCCCGGCAGCCCGAGCAGCCGGAGCAGAGAGCCTTGGGTCCGGTTTCTATGCC
    ---------------------------E1a-------------------------------

481 AAACCTTGTACCGGAGGTGATCGATCTTACCTGCCACGAGGCTGGCTTTCCACCCAGTGA
    ---------------------------E1a-------------------------------

541 CGACGAGGATGAAGAGGGTGAGGAGTTTGTGTTAGATTATGTGGAGCACCCCGGGCACGG
    ---------------------------E1a-------------------------------

601 TTGCAGGTCTTGTCATTATCACCGGAGGAATACGGGGGACCCAGATATTATGTGTTCGCT
    ---------------------------E1a-------------------------------
```

**Figure 5.** Sequence of Ar6pAF from left end of viral DNA (Seq ID NO:6)

```
  1 CATCATCAATAATATACCTTATTTTGGATTGAAGCCAATATGATAATGAGGGGGTGGAGT
    +-------------------------ITR---------------------------------

 61 TTGTGACGTGGCGCGGGGCGTGGGAACGGGGCGGGTGACGTAGGGCGCGATCAAGCTTAT
    --------------------------ITR---------------+           +----

121 CGATACCGTCGAAACTTGTTTATTGCAGCTTATAATGGTTACAAATAAAGCAATAGCATC
    --------------------------polyA-------------------------------

181 ACAAATTTCACAAATAAAGCATTTTTTTCACTGCATTCTAGTTGTGGTTTGTCCAAACTC
    --------------------------polyA-------------------------------

241 ATCAATGTATCTTATCATGTCTGGATCCGCGCCGCTAGCGATATCGGATCCCGGTCGACT
    --------------------+                                     +--

301 GAAAATGAGACATATTATCTGCCACGGAGGTGTTATTACCGAAGAAATGGCCGCCAGTCT
   ---------------------------E1a-------------------------------

361 TTTGGACCAGCTGATCGAAGAGGTACTGGCTGATAATCTTCCACCTCCTAGCCATTTTGA
   ---------------------------E1a-------------------------------

421 ACCACCTACCCTTCACGAACTGTATGATTTAGACGTGACGGCCCCCGAAGATCCCAACGA
   ---------------------------E1a-------------------------------

481 GGAGGCGGTTTCGCAGATTTTTCCCGACTCTGTAATGTTGGCGGTGCAGGAAGGGATTGA
   ---------------------------E1a-------------------------------

541 CTTACTCACTTTTCCGCCGGCGCCCGGTTCTCCGGAGCCGCCTCACCTTTCCCGGCAGCC
   ---------------------------E1a-------------------------------

601 CGAGCAGCCGGAGCAGAGAGCCTTGGGTCCGGTTTCTATGCCAAACCTTGTACCGGAGGT
   ---------------------------E1a-------------------------------
```

**Figure 6.** Schematic diagram of Ar6pAF and Ar6pAE2fF vectors

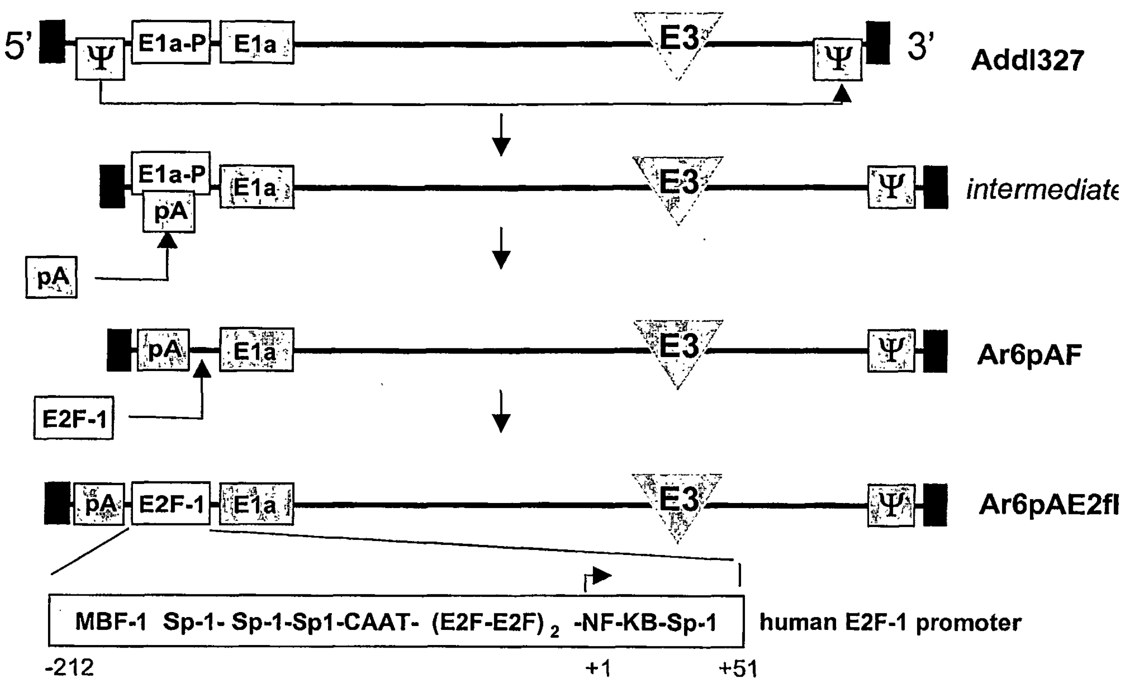

# Fig. 7 Body weight change

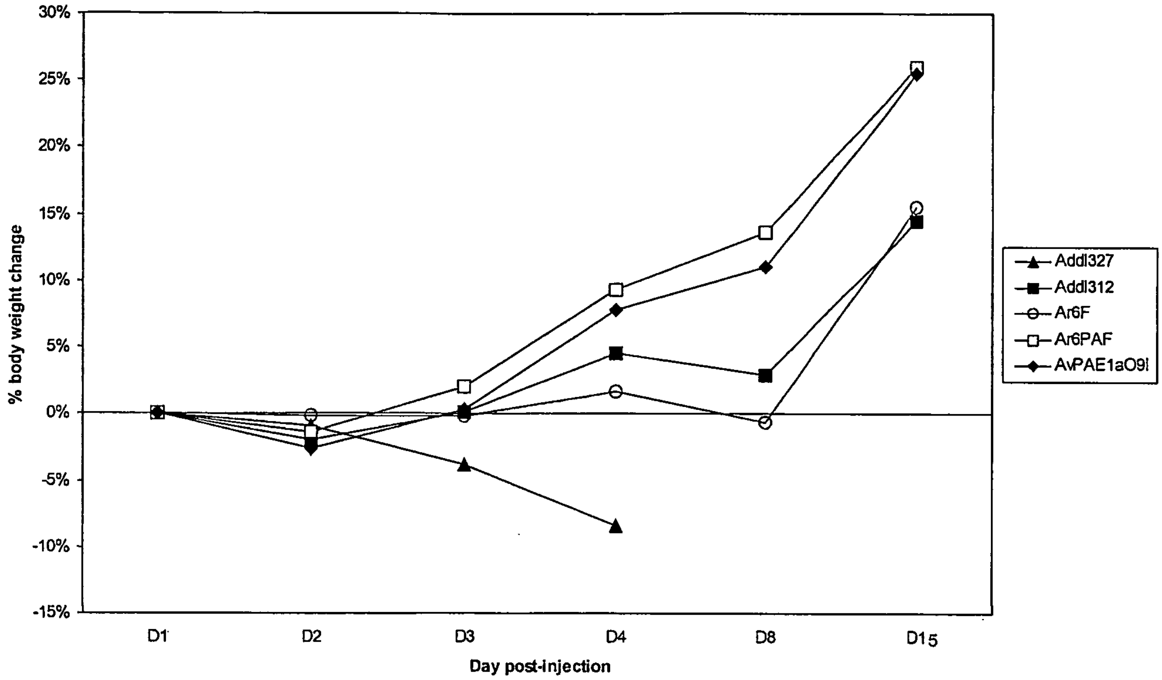

# Fig. 8 Minimizing nonspecific transactivation of E1a gene

Backbones generated:

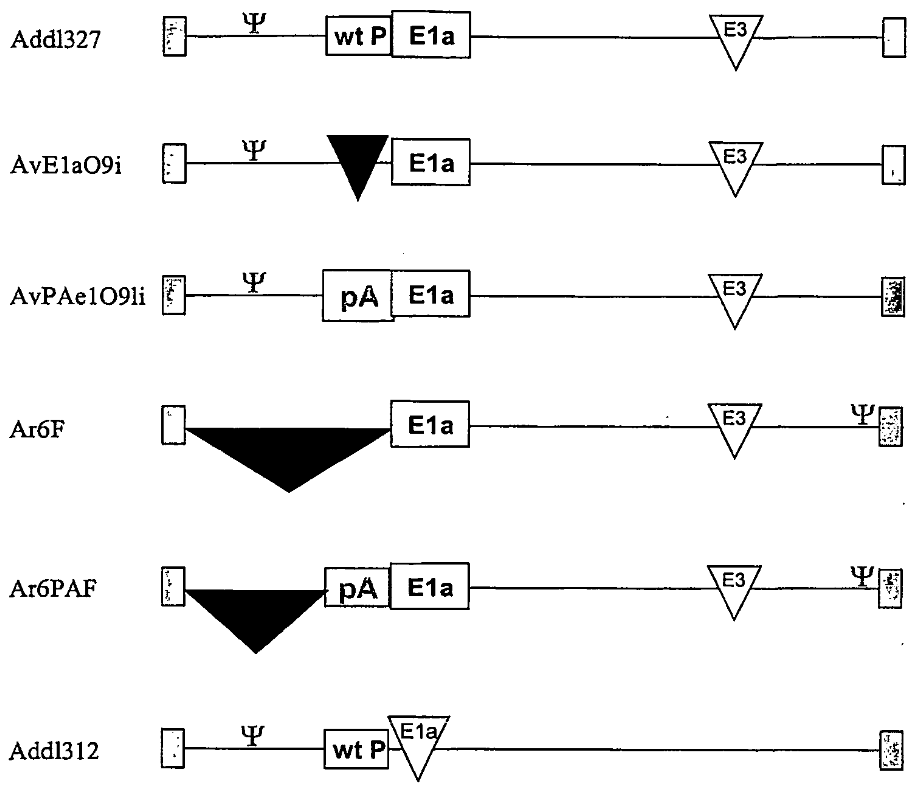

**Figure 9.** Mean H460 tumor volume

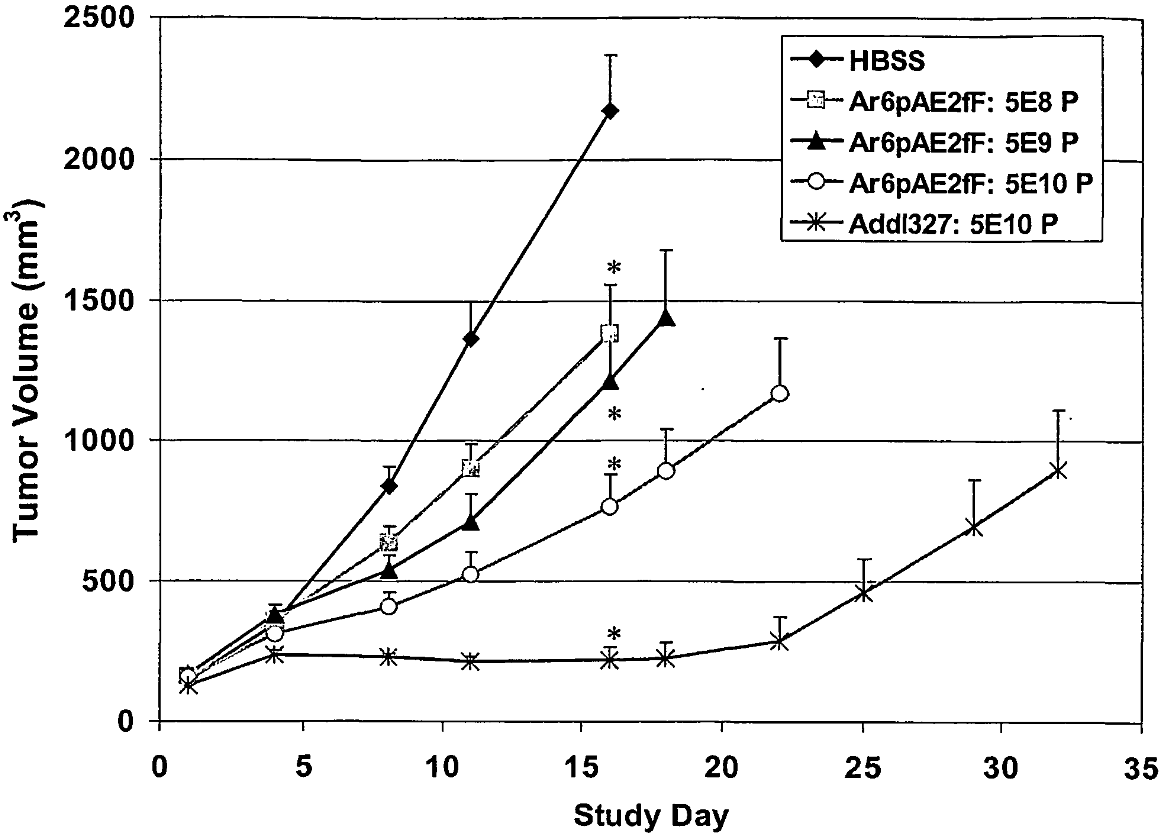

**Figure 10**. Survival following intratumoral administration of vectors to H460 tumors

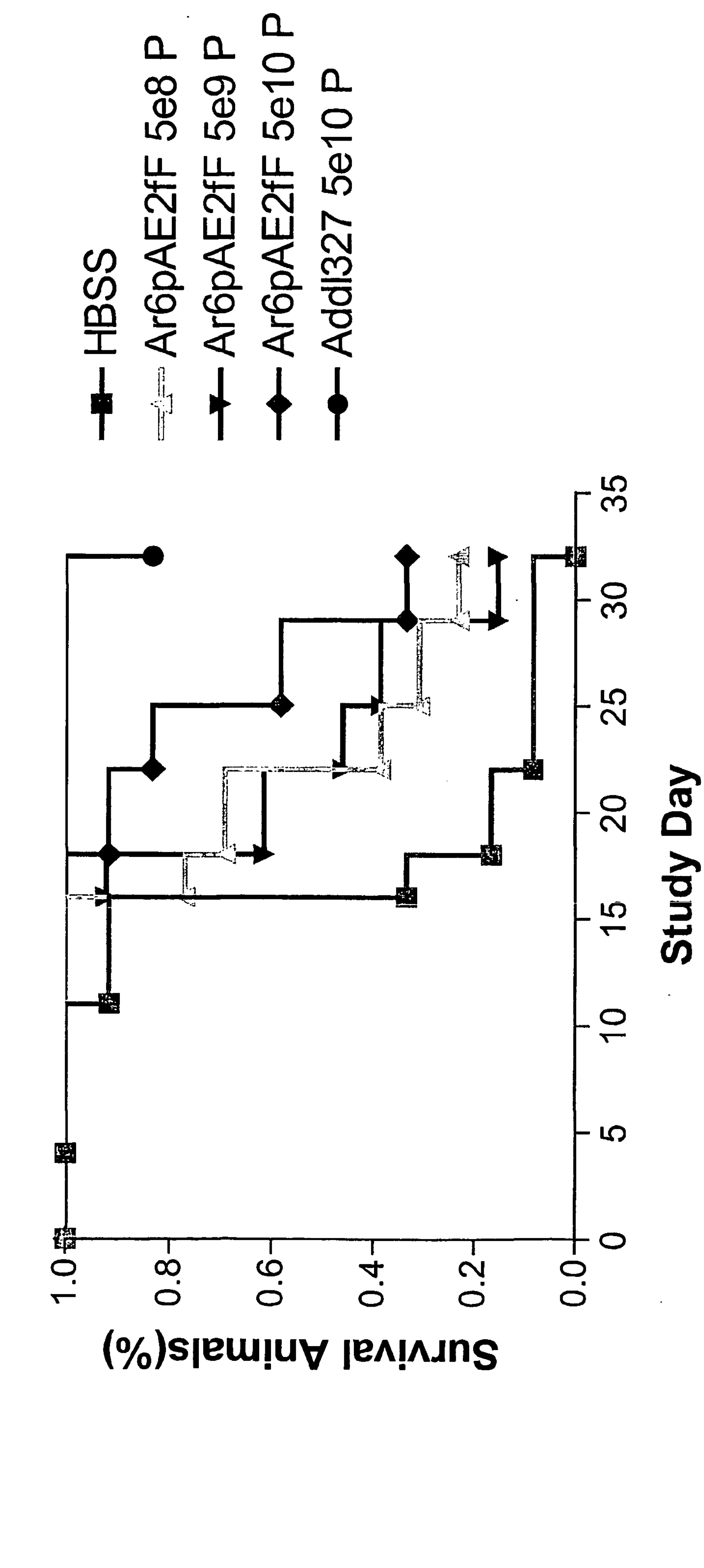

Figure 11. Mean Hep3B tumor volumes

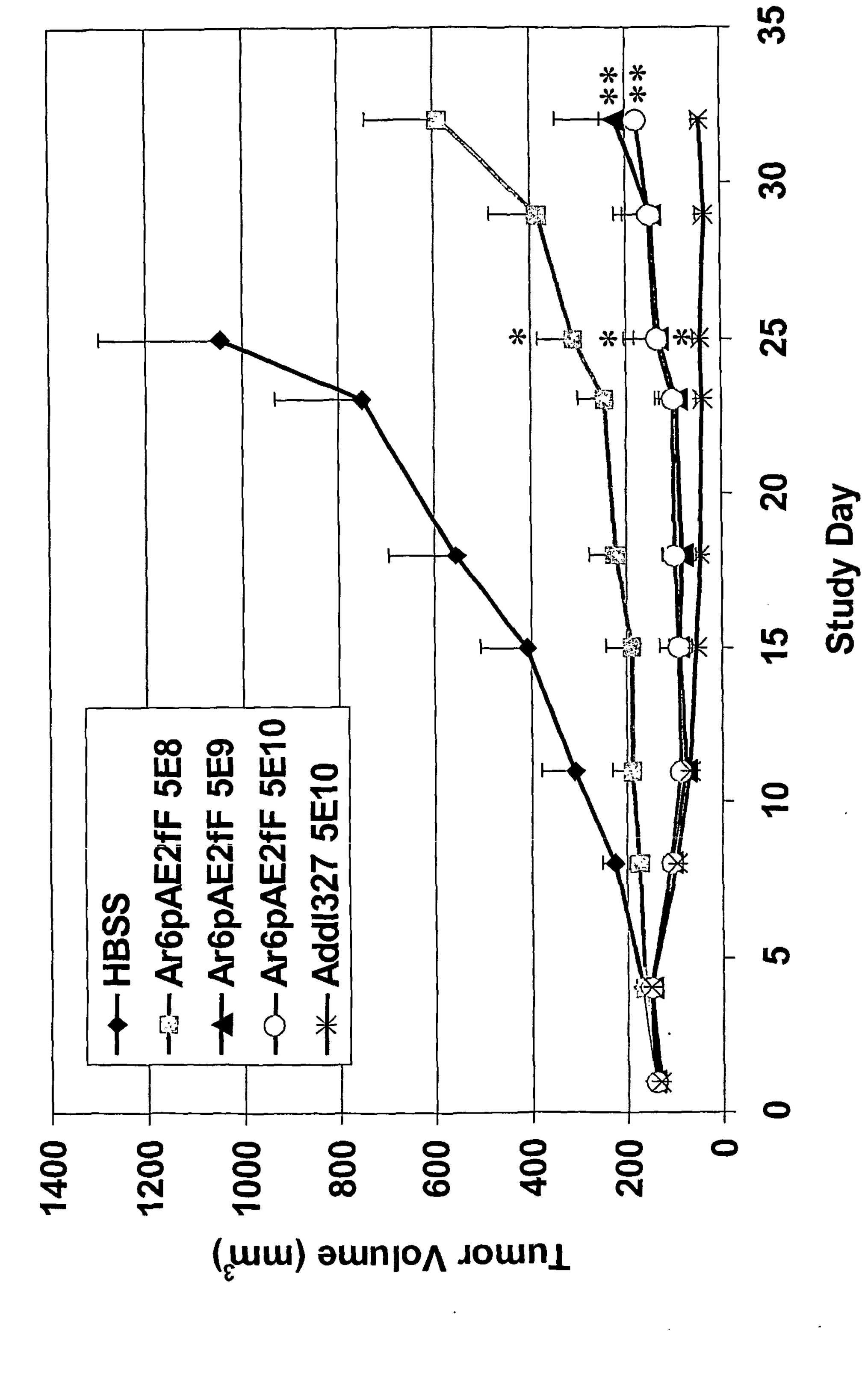

**Figure 12**. Survival following intratumoral administration of vector to Hep3B tumors

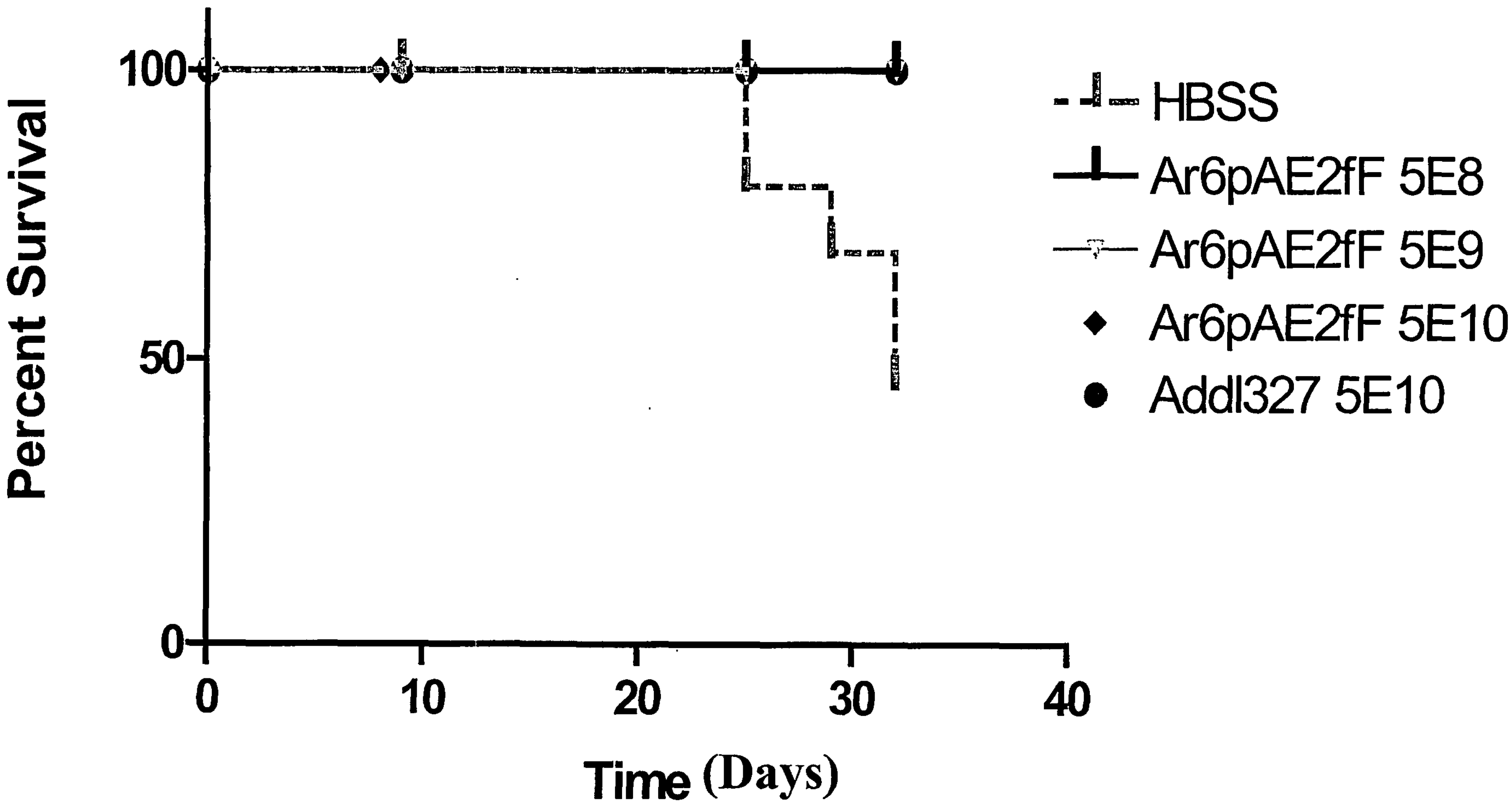

**Figure 13. Schematic diagram of adenovirus right donor plasmid pDR2F.**

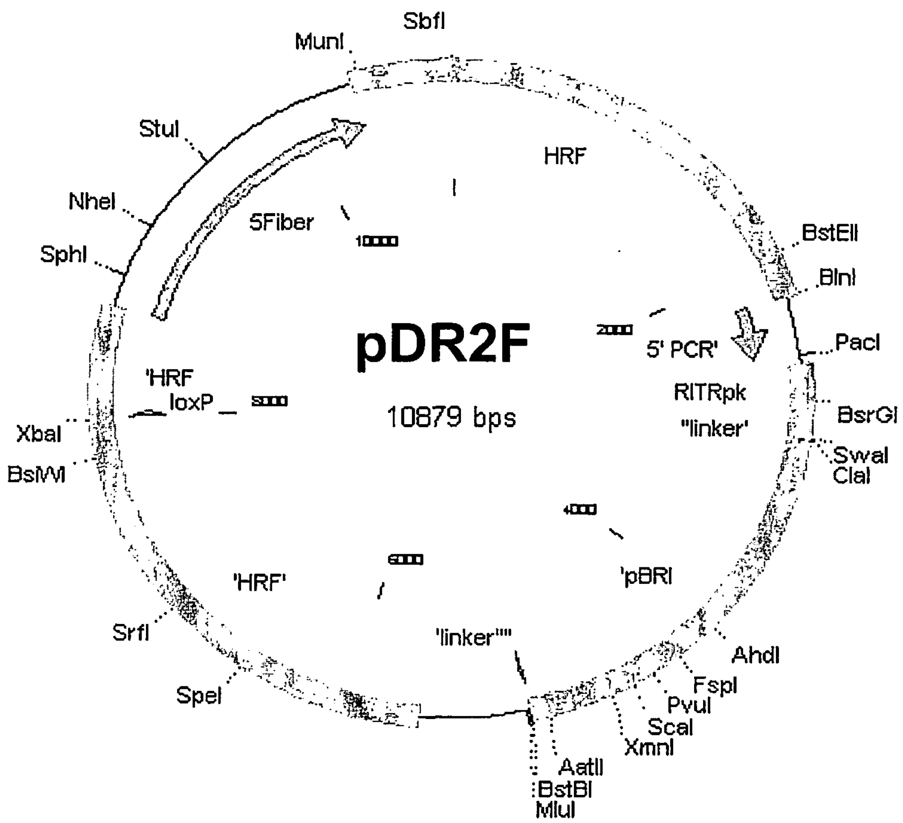

**Figure 14.** Schematic diagram of adenovirus right donor plasmid pDR2mGmF.

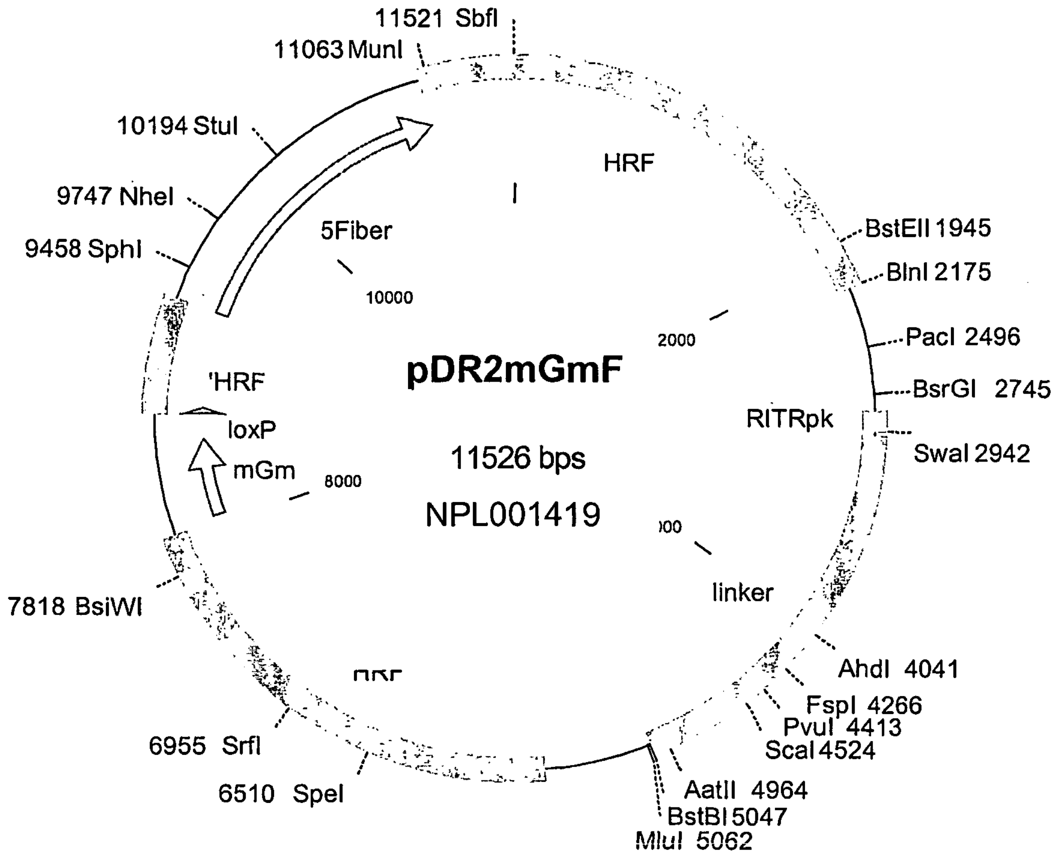

**Figure 15.** Schematic diagram of plasmid pG1mGmSvNa.

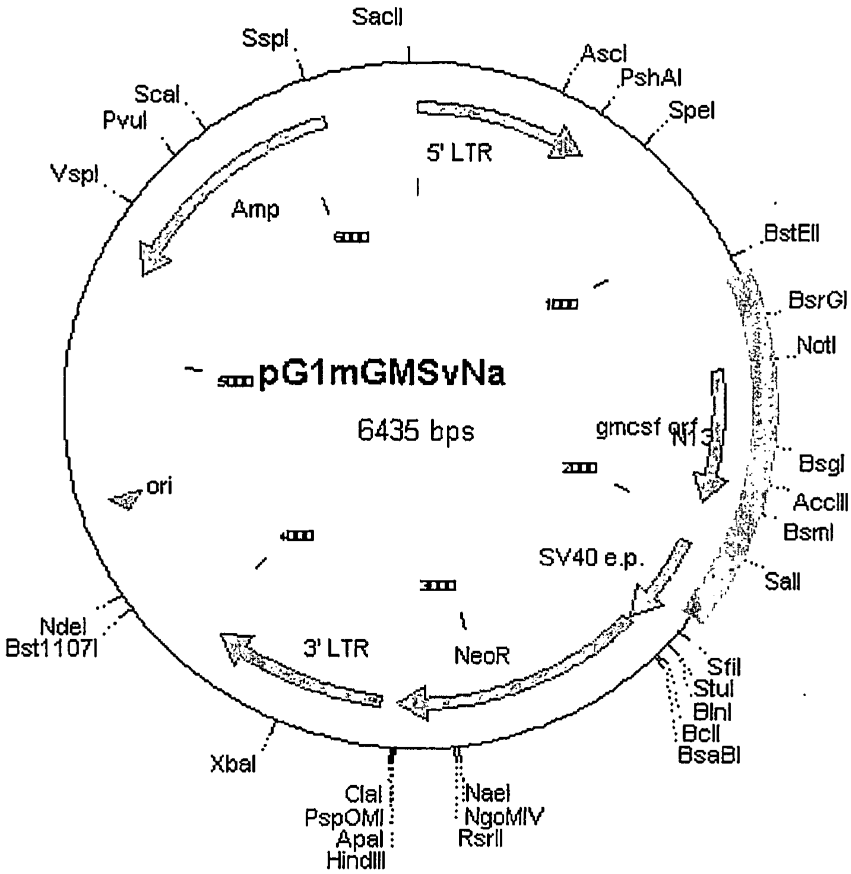

**Figure 16.** Schematic diagram of plasmid pG1NaSvBg.

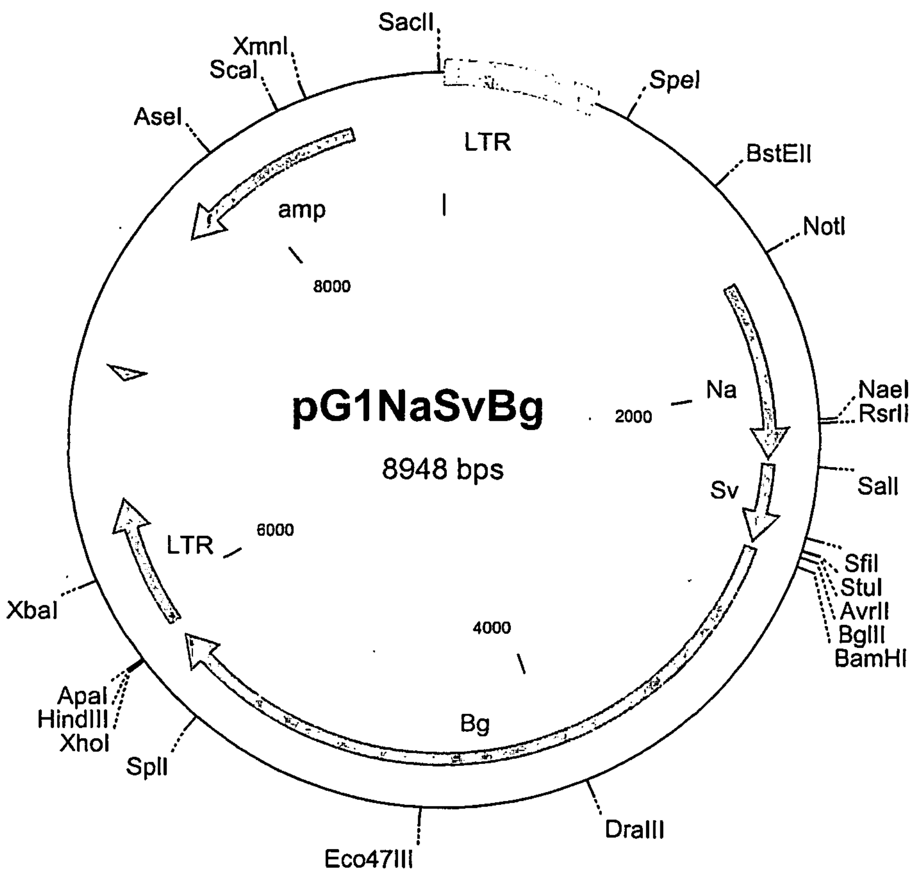

**Figure 17.** Sequence of the murine GM-CSF cDNA (Seq ID NO:7) and protein (Seq ID NO:8).

```
7878 TTCCGGACAG ACCTCAATAA CTCTGTTTAC CAGAACAGGA GGTGAGCTTA
7928 GAAAACCCTT AGGGTATTAG GCCAAAGGCG CAGCTACTGT GGGGTTTATG
7978 AACAATTCAA GCAACTCTAC GGGCTATTCT AATTCAGGTT TCTCTAGCCG
8028 GGCTGCAGGA ATTCGATGGC CGCTACCTAC AATGGCCCAC GAGAGAAAGG
                                      M  A  H   E  R  K
8078 CTAAGGTCCT GAGGAGGATG TGGCTGCAGA ATTTACTTTT CCTGGGCATT
       A  K  V   L  R  R  M   W  L  Q   N  L  L   F  L  G  I
8128 GTGGTCTACA GCCTCTCAGC ACCCACCCGC TCACCCATCA CTGTCACCCG
       V  V  Y   S  L  S   A  P  T  R   S  P  I   T  V  T
8178 GCCTTGGAAG CATGTAGAGG CCATCAAAGA AGCCCTGAAC CTCCTGGATG
     R  P  W  K   H  V  E   A  I  K   E  A  L  N   L  L  D
8228 ACATGCCTGT CACATTGAAT GAAGAGGTAG AAGTCGTCTC TAACGAGTTC
       D  M  P   V  T  L  N   E  E  V   E  V  V   S  N  E  F
8278 TCCTTCAAGA AGCTAACATG TGTGCAGACC CGCCTGAAGA TATTCGAGCA
       S  F  K   K  L  T   C  V  Q  T   R  L  K   I  F  E
8328 GGGTCTACGG GGCAATTTCA CCAAACTCAA GGGCGCCTTG AACATGACAG
     Q  G  L  R   G  N  F   T  K  L   K  G  A  L   N  M  T
8378 CCAGCTACTA CCAGACATAC TGCCCCCCAA CTCCGGAAAC GGACTGTGAA
       A  S  Y   Y  Q  T  Y   C  P  P   T  P  E   T  D  C  E
8428 ACACAAGTTA CCACCTATGC GGATTTCATA GACAGCCTTA AAACCTTTCT
       T  Q  V   T  T  Y   A  D  F  I   D  S  L   K  T  F
8478 GACTGATATC CCCTTTGAAT GCAAAAAACC AGTCCAAAAA TGAGGAAGCC
     L  T  D  I   P  F  E   C  K  K   P  V  Q  K   -
8528 CAGGCCAGCT CTGAATCCAG CTTCTCAGAC TGCTGCTTTT GTGCCTGCGT
8578 AATGAGCCAG GAACTCGGAA TTTCTGCCTT AAAGGGACCA AGAGATGTGG
8628 CACAGGTAGT CGAATCAAGC TTATCGATAC CGTCGACCTC GACTAGATAA
8678 CTTCGTATAA TGTATGCTAT ACGAAGTTAT GCTAGAAATG GACGGAATTA
8728 TTACAGAGCA GCGCCTGCTA GAAAGACGCA GGGCAGCGGC CGAGCAACAG
8778 CGCATGAATC AAGAGCTCCA AGACATGGTT AACTTGCACC AGTGCAAAA 8826
```

Figure 18. Pathway used to generate pAr6pAE2fmGmF plasmid.

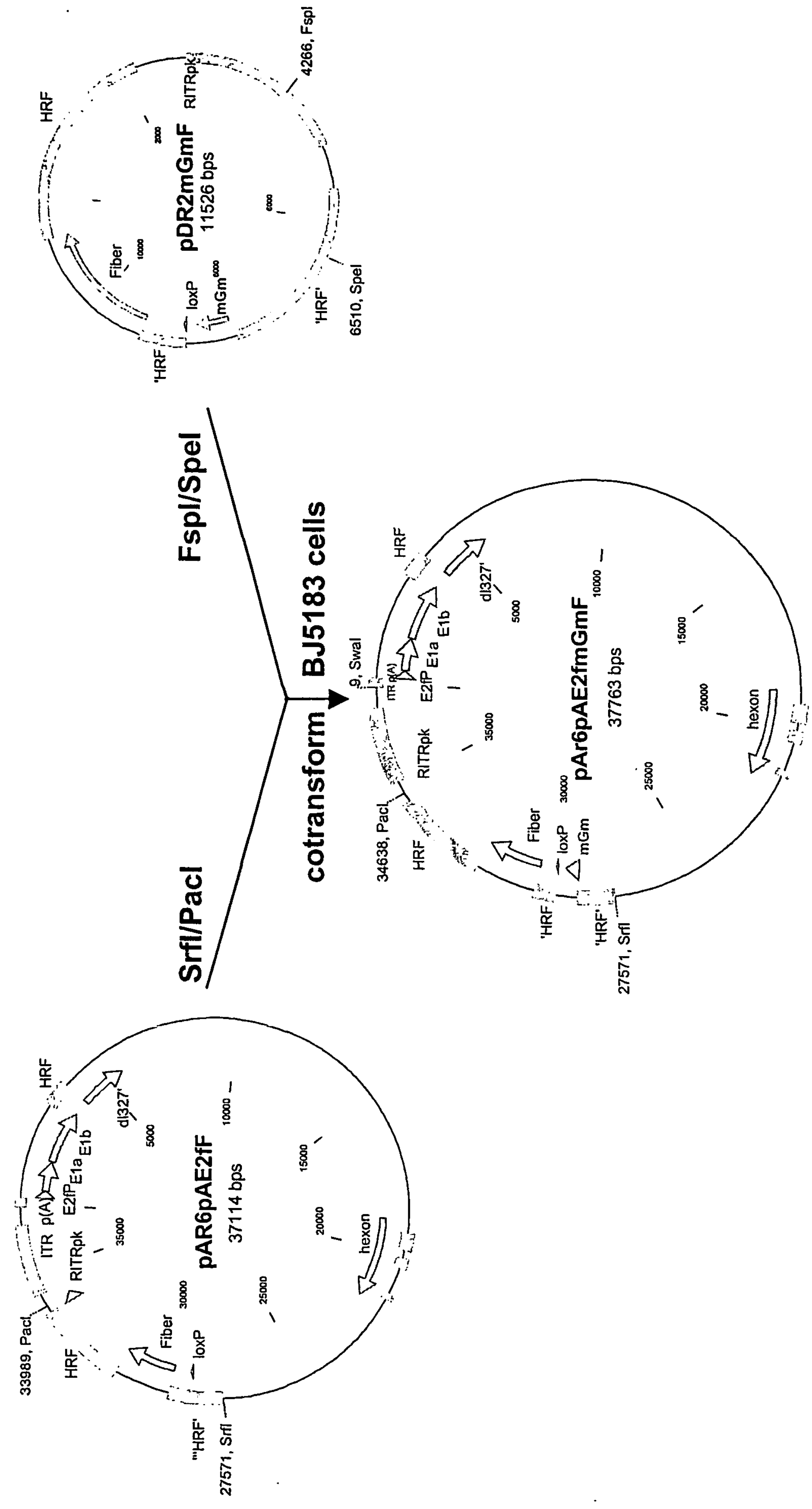

Figure 19. MTS assay of oncolytic vectors on different tumor cell lines.

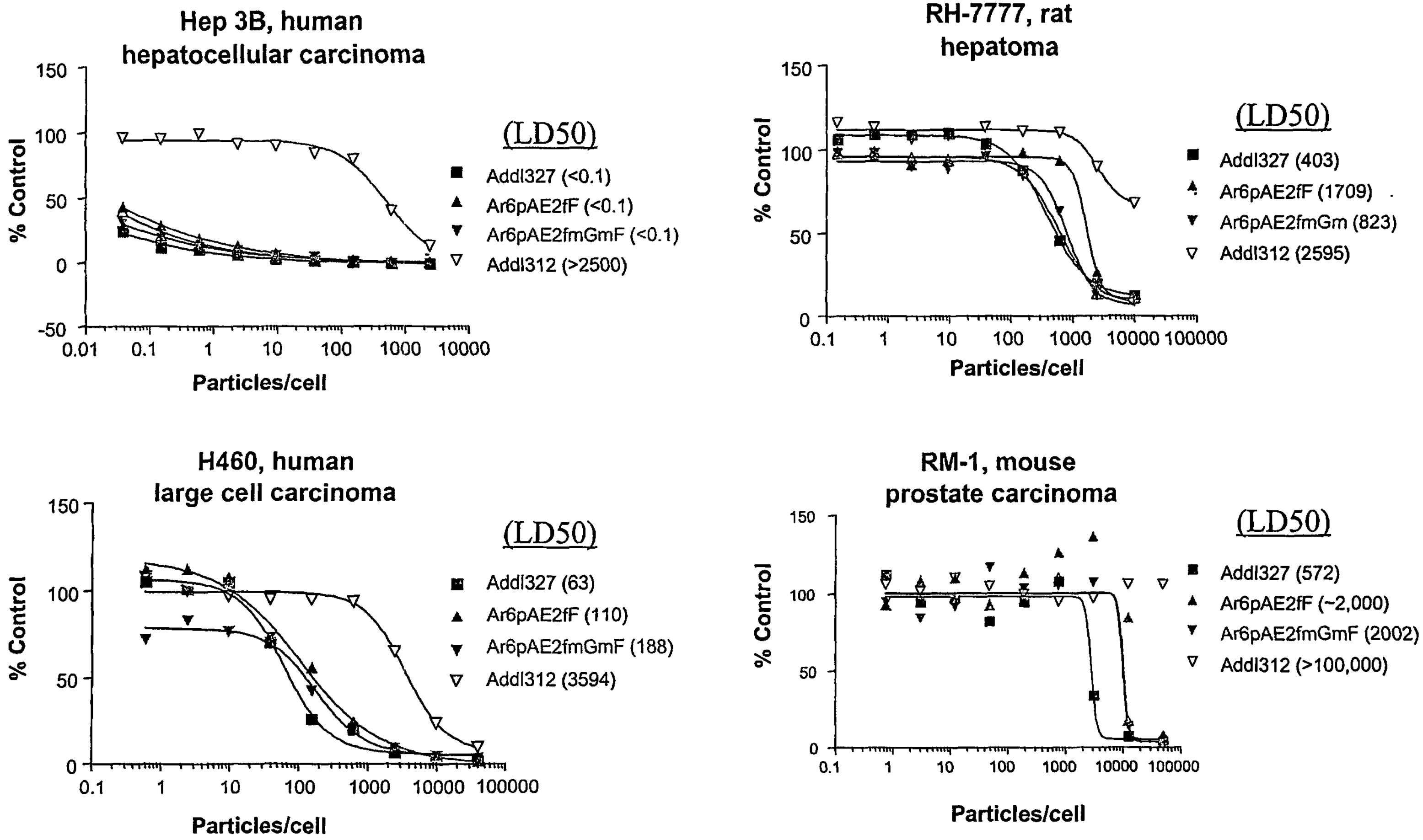

**Figure 20. Sequence of the human GM-CSF cDNA (Seq ID NO:19) and protein (Seq ID NO:20).**

```
28536  TATTAGGCCA AAGGCGCAGC TACTGTGGGG TTTATGAACA ATTCAAGCAA
28586  CTCTACGGGC TATTCTAATT CAGGTTTCTC TAGGATCTTT CCGCAGCAGC

28636  CGCCACCATG TGGCTGCAGA GCCTGCTGCT CTTGGGCACT GTGGCCTGCA
                M   W  L  Q   S  L  L   L  L  G  T   V  A  C

28686  GCATCTCTGC ACCCGCCCGC TCGCCCAGCC CCAGCACGCA GCCCTGGGAG
        S  I  S   A  P  A  R   S  P  S   P  S  T   Q  P  W  E

28736  CATGTGAATG CCATCCAGGA GGCCCGGCGT CTCCTGAACC TGAGTAGAGA
        H  V  N   A  I  Q   E  A  R  R   L  L  N   L  S  R

28786  CACTGCTGCT GAGATGAATG AAACAGTAGA AGTCATCTCA GAAATGTTTG
       D  T  A  A   E  M  N   E  T  V   E  V  I  S   E  M  F

28836  ACCTCCAGGA GCCGACCTGC CTACAGACCC GCCTGGAGCT GTACAAGCAG
        D  L  Q   E  P  T  C   L  Q  T   R  L  E   L  Y  K  Q

28886  GGCCTGCGGG GCAGCCTCAC CAAGCTCAAG GGCCCCTTGA CCATGATGGC
        G  L  R   G  S  L   T  K  L  K   G  P  L   T  M  M

28936  CAGCCACTAC AAGCAGCACT GCCCTCCAAC CCCGGAAACT TCCTGTGCAA
       A  S  H  Y   K  Q  H   C  P  P   T  P  E  T   S  C  A

28986  CCCAGACTAT CACCTTTGAA AGTTTCAAAG AGAACCTGAA GGACTTTCTG
        T  Q  T   I  T  F  E   S  F  K   E  N  L   K  D  F  L

29036  CTTGTCATCC CCTTTGACTG CTGGGAGCCA GTCCAGGAGT GAGTCGACAA
        L  V  I   P  F  D   C  W  E  P   V  Q  E   -

29086  GCTCTAGATA ACTTCGTATA ATGTATGCTA TACGAAGTTA TGCTAGAAAT
29136  GGACGGAATT ATTACAGAGC AGCGCCTGCT AGAAAGACGC AGGGCAGCGG
29186  CCGAGCAACA GCGCATGAAT CAAGAGCTCC AAGACATGGT TAACTTGCAC
29236  CAGTGCAAAA GGGGTATCTT TTGTCTGGTA AAGCAGG  29273
```

**Figure 21. Pathway used to generate pAr6pAE2fhGmF plasmid.**

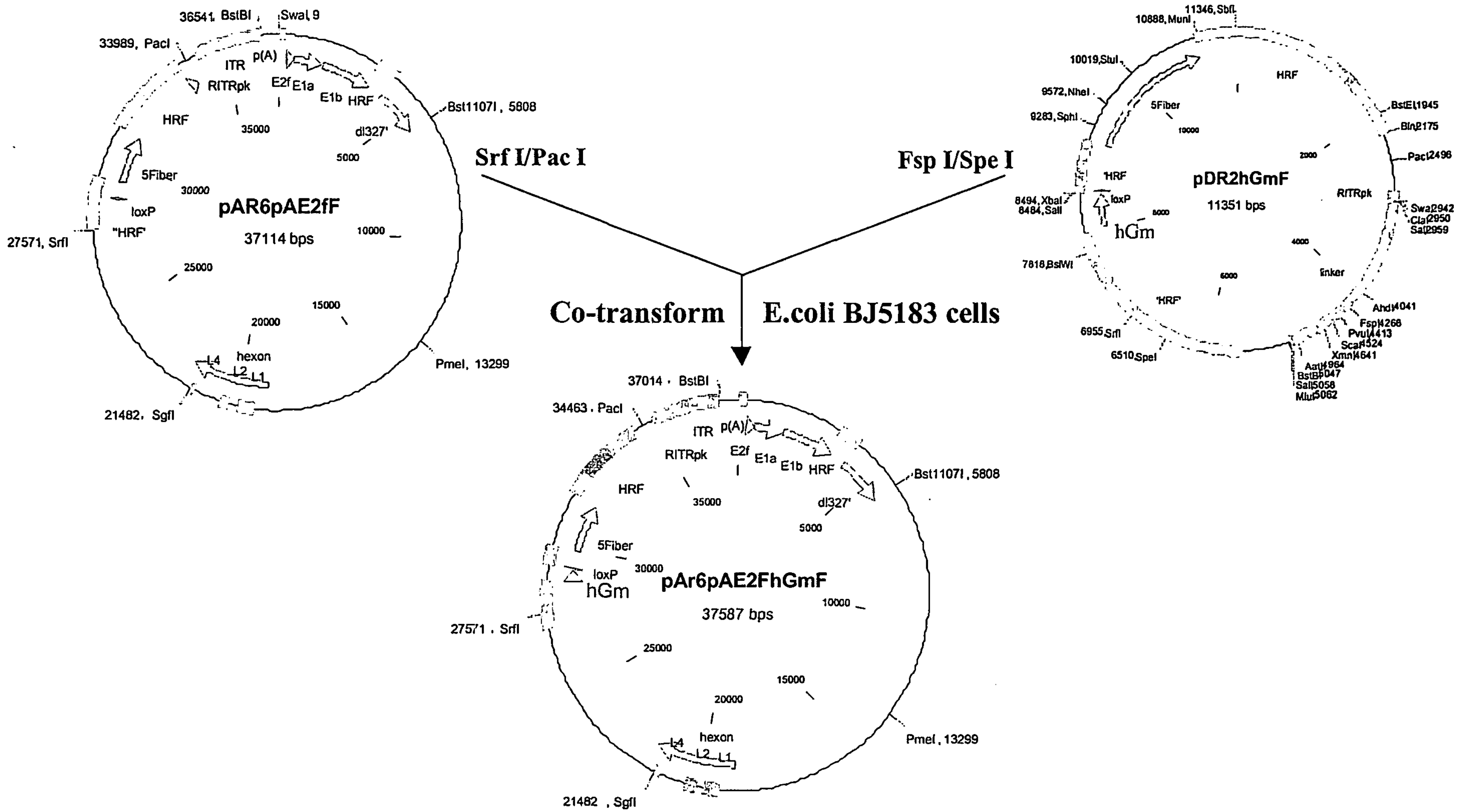

Figure 22. MTS assay of oncolytic vectors on different tumor cell lines.

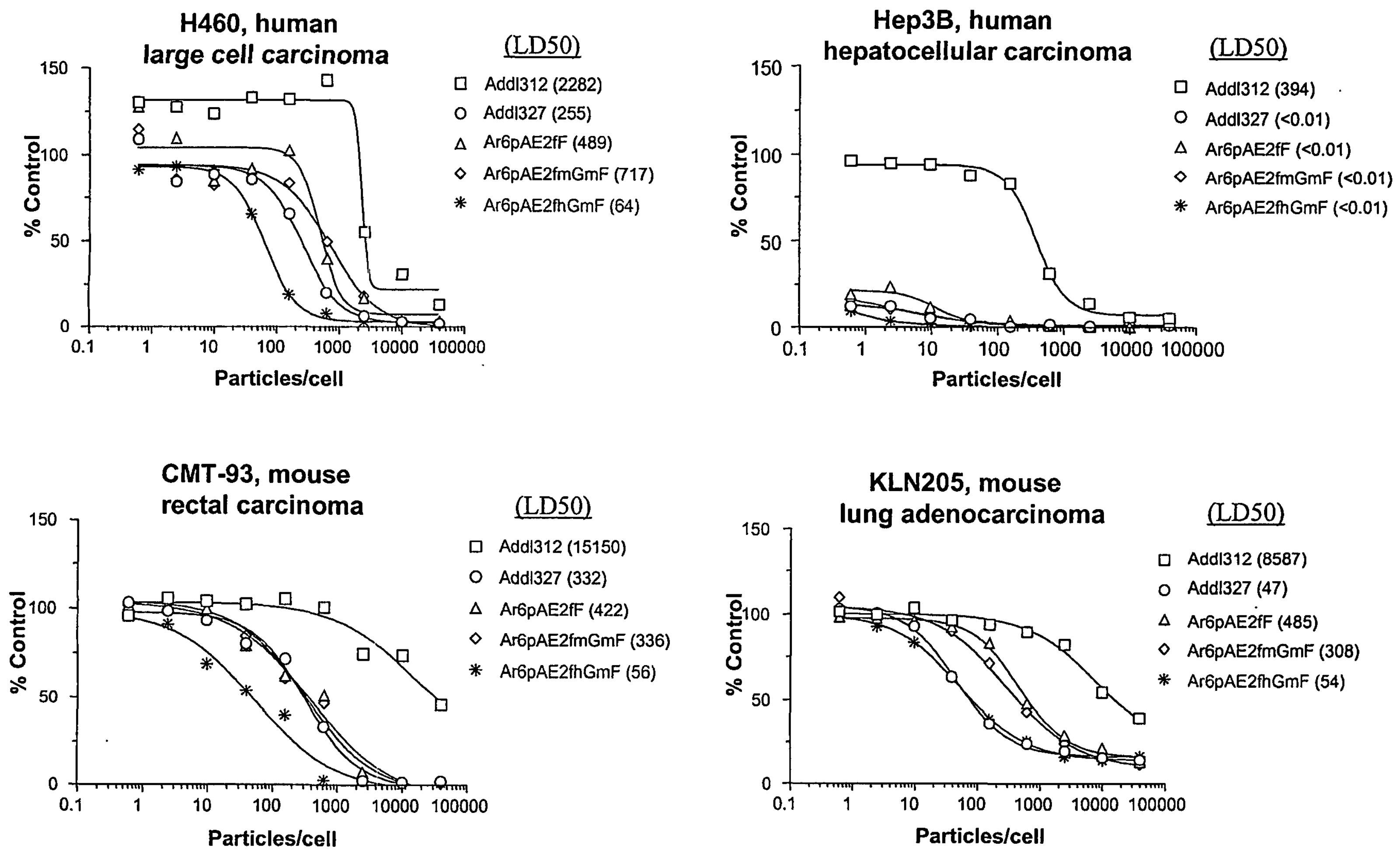

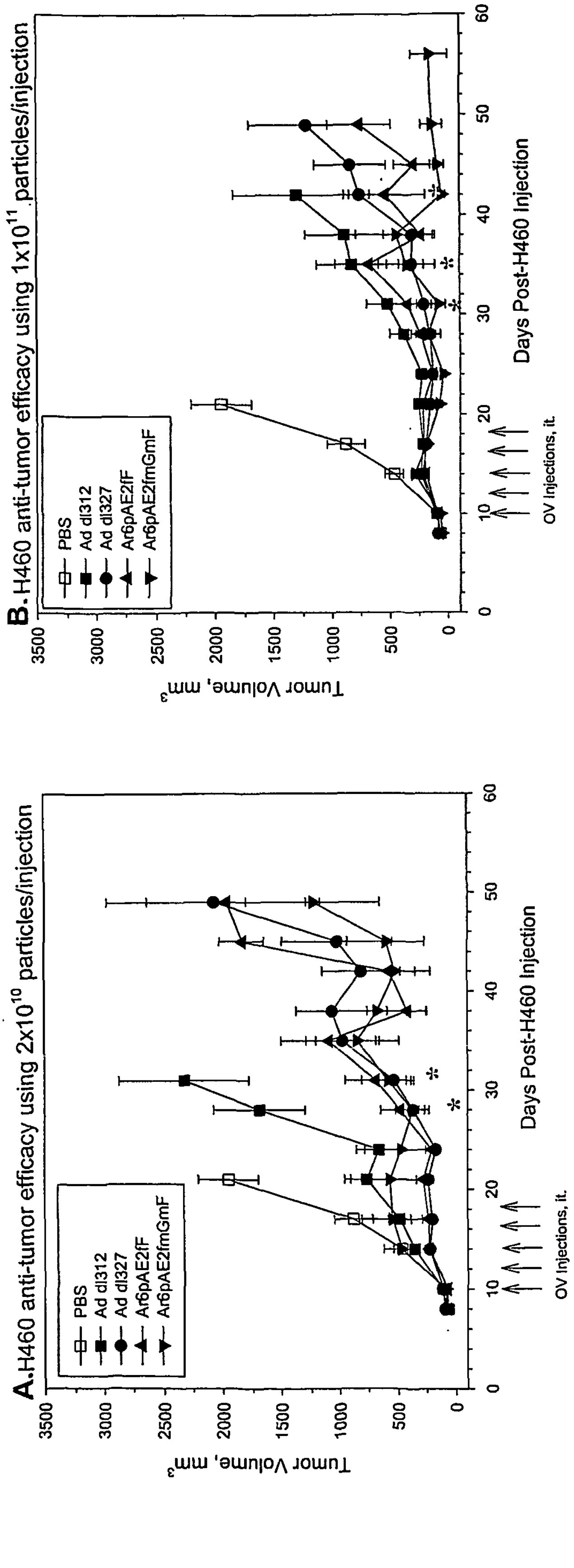


Figure 23. Efficacy of GM-CSF armed oncolytic vectors in H460 tumor model

# Figure 24. Efficacy of GM-CSF armed oncolytic vectors in Hep3B tumor model

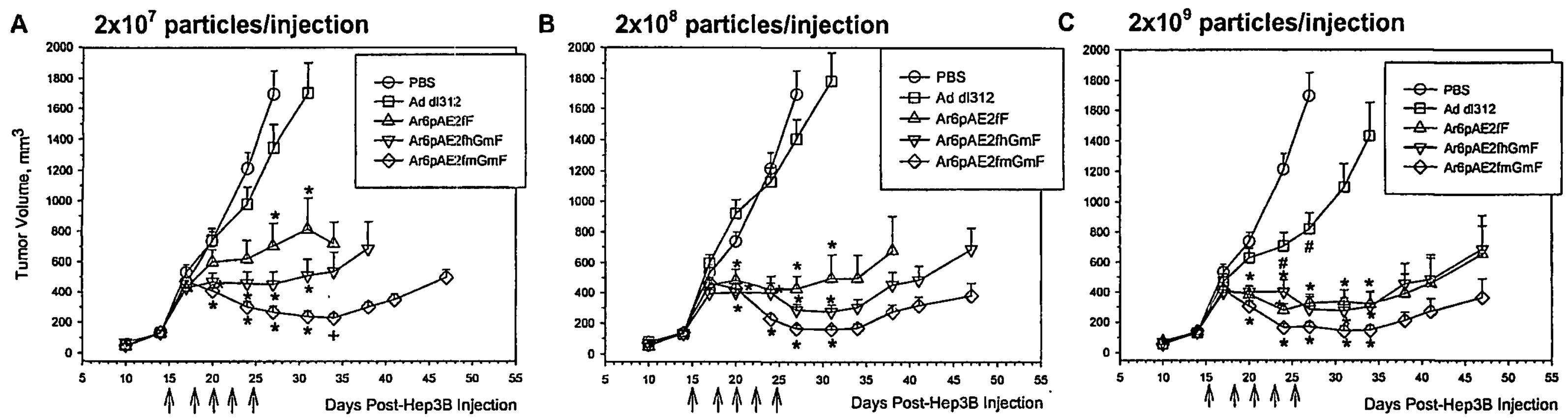

Figure 25. Schematic Diagram of PCR and Overlap PCR for Δgp19 Donor Plasmids

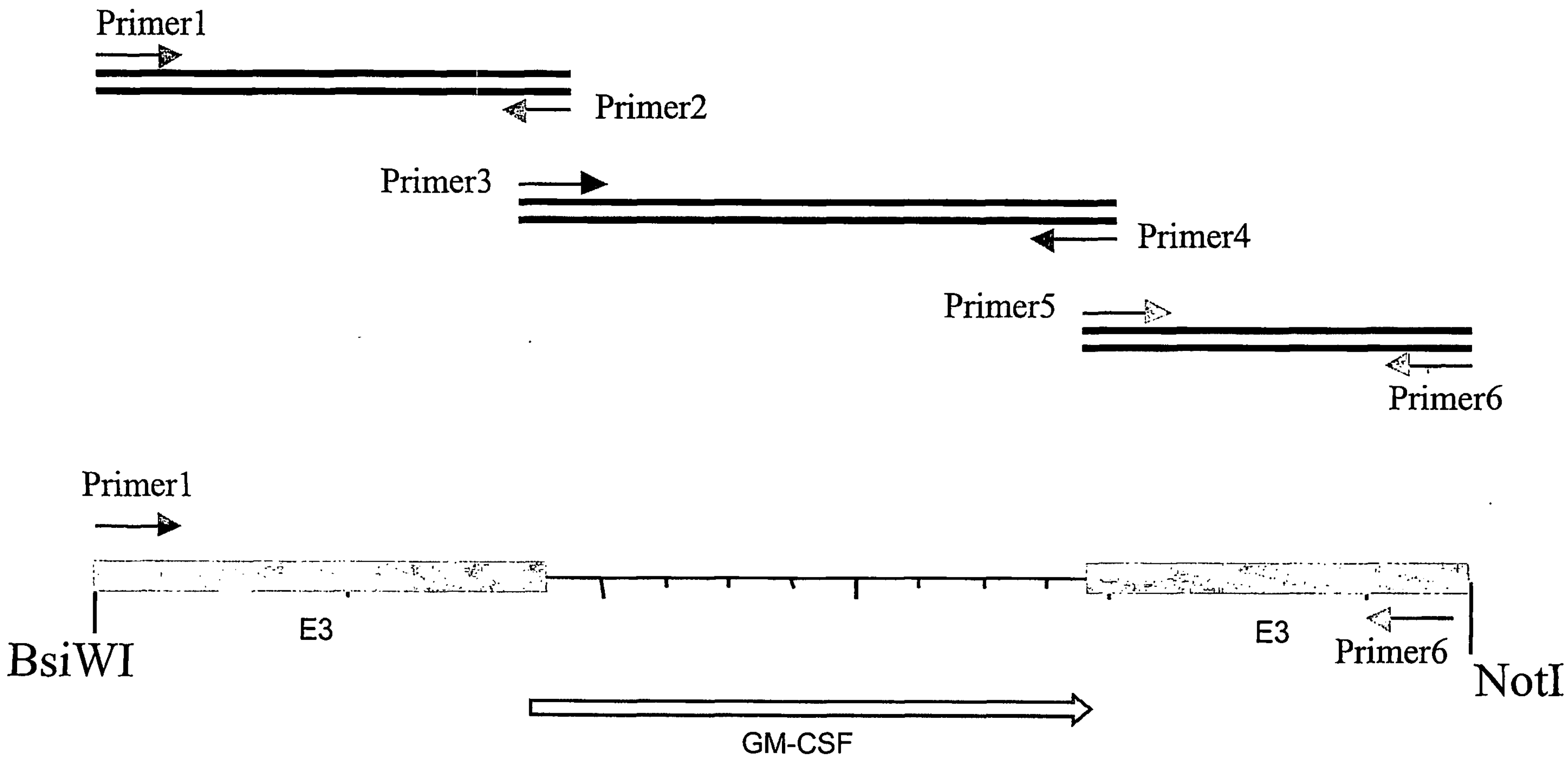

## Figure 26. Schematic Diagram of □gp19 Vectors

a. Sequence of native E3 region:

cgccacccaagATGAttaggtac (Seq ID NO:9)
6.7kDa---------R H P R STOP (Seq ID NO:13)
M------gp19kDa

b. Sequence comparison of Δgp19 vectors at the junction between E3-6.7 and GM-CSF:

1. Δgp19a: atgg
6.7kDa-----plus 9aa to STOP M------mouse GMCSF
atgt
6.7kDa---plus 33aa to STOP M------human GMCSF

2. Δgp19b: cgccacccaag ATAACCatg.........
6.7kDa-------R H P R STOP M------GMCSF

3. Δgp19c: cgccacccaag atgA......... (Seq ID NO:10)
6.7kDa-------R H P R STOP
M------GMCSF

4. Δgp19d: cgccacccaag ATGACC atg.........(Seq ID NO:11)
6.7kDa---------R H P R STOP
M T M------GMCSF

5. Δgp19b/IRES: cgccacccaagatga CAATTC...IRES...atg.........(Seq ID NO:12)
6.7kDa--------R H P R STOP M------GMCSF

Figure 27a. Pathway Used to Generate the pAr6pAE2f(E3+,mGm,Dg19b)F Large Plasmid

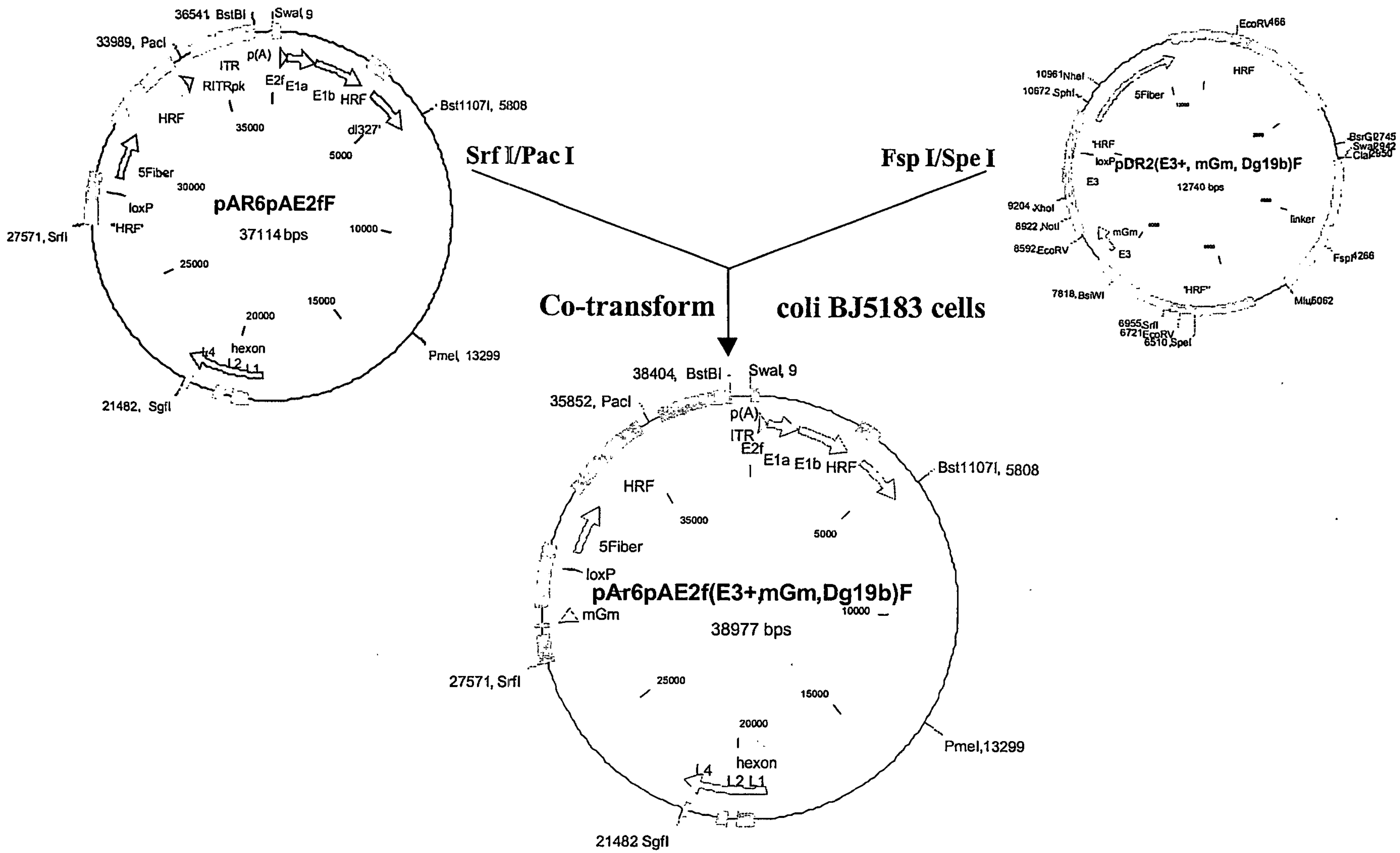

Figure 27b. Pathway Used to Generate the pAr6pAE2f(E3+,hGm,Dg19b)F Large Plasmid

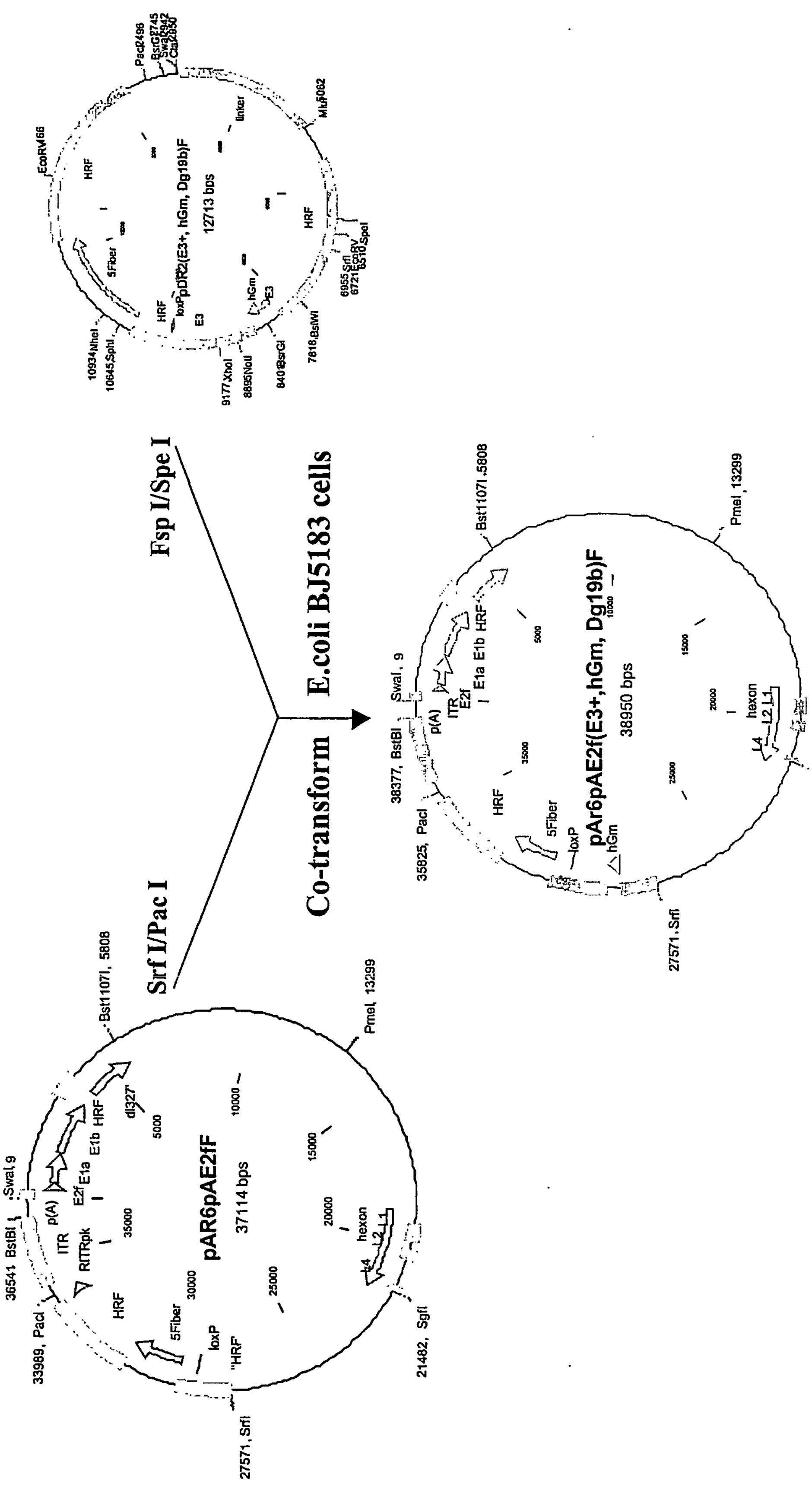

Figure 28. MTS Assay of Δgp19 mGM-CSF Vectors on H460 and Hep3B Tumor Cell Lines

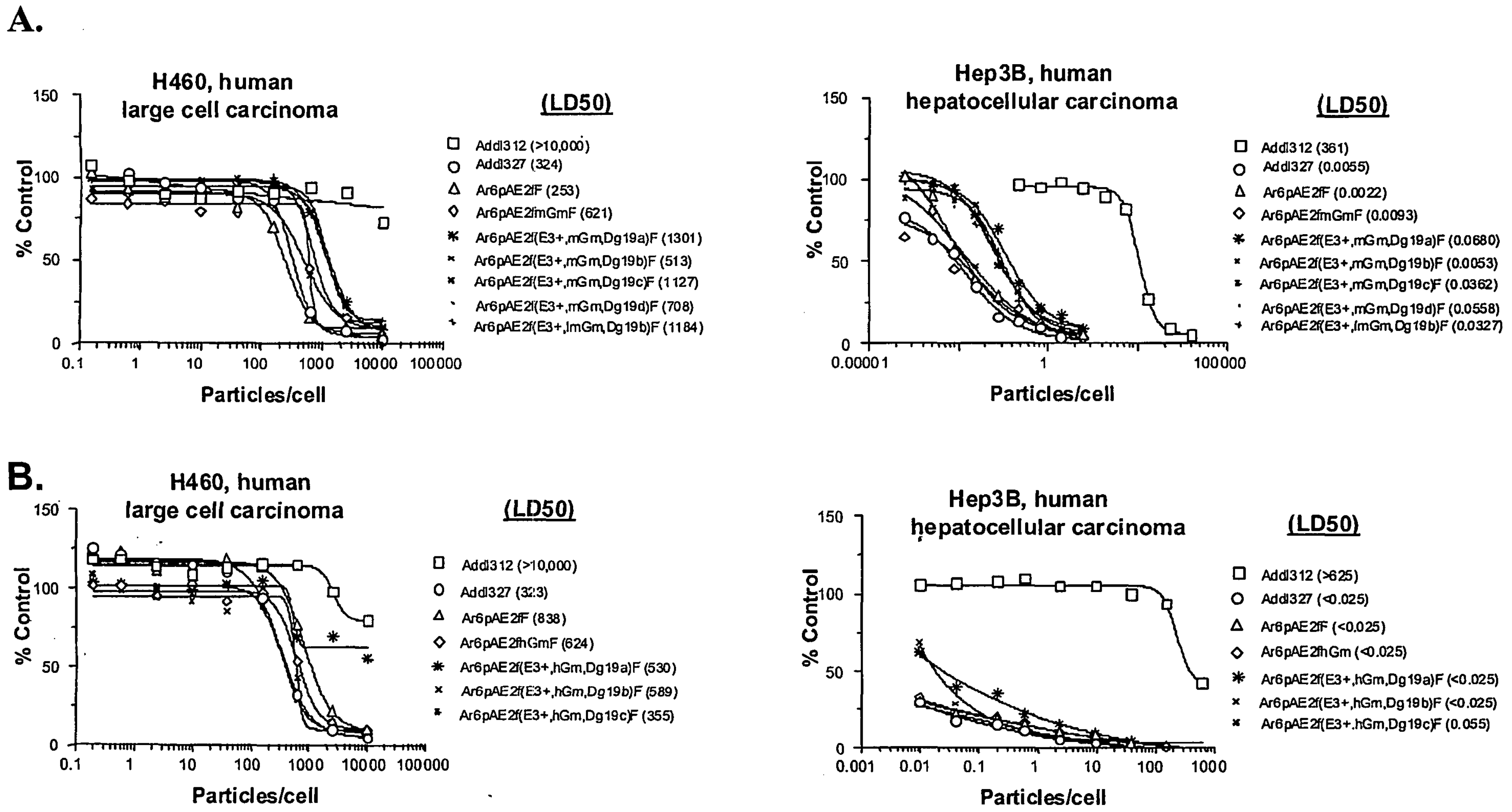

## Figure 29. GM-CSF Expression Mediated by Δgp19 GM-CSF Vectors in Infected H460 Cells Detected by ELISA

**a. Mouse GM-CSF expression in H460 cells**

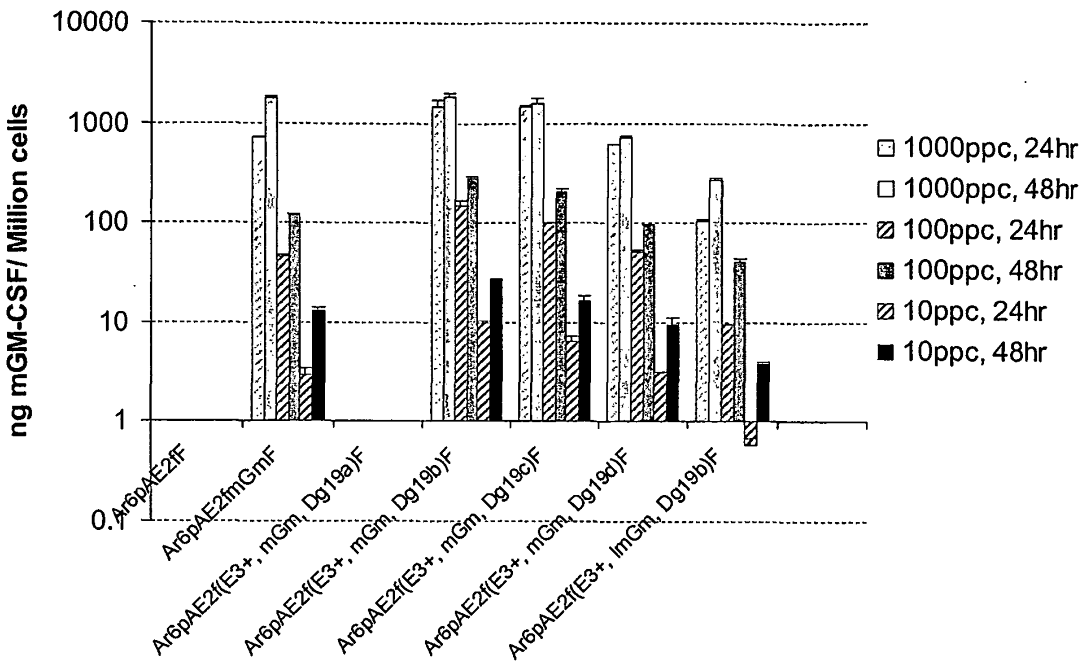


**b. Human GM-CSF expression in H460 cells**

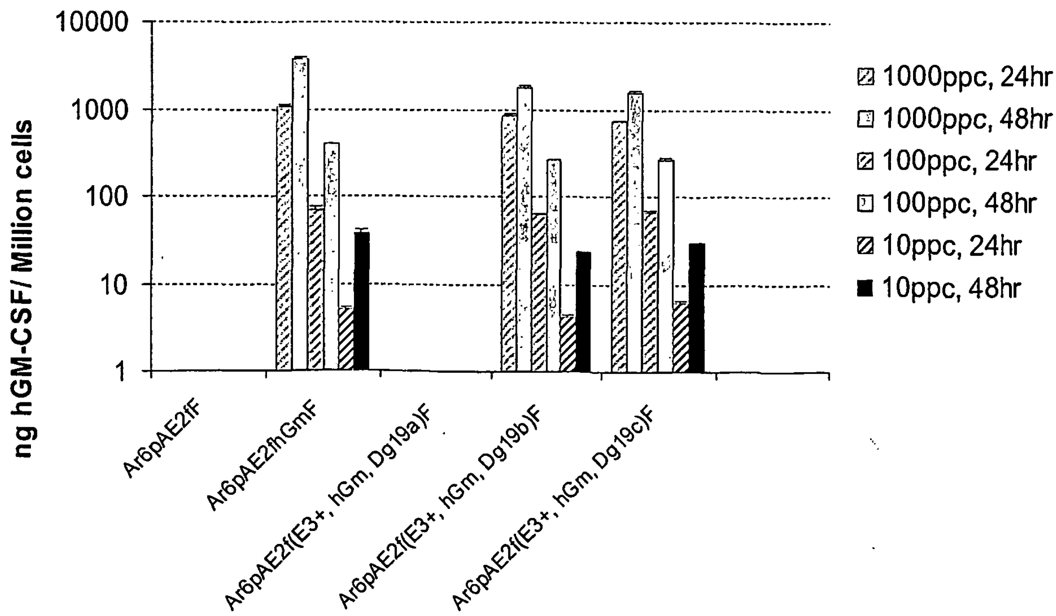

**Figure 30. Anti-Tumor Activity of Oncolytic Adenoviruses (2x10$^9$ particles/injection) in the Hep3B Xenograft Subcutaneous Tumor Model**

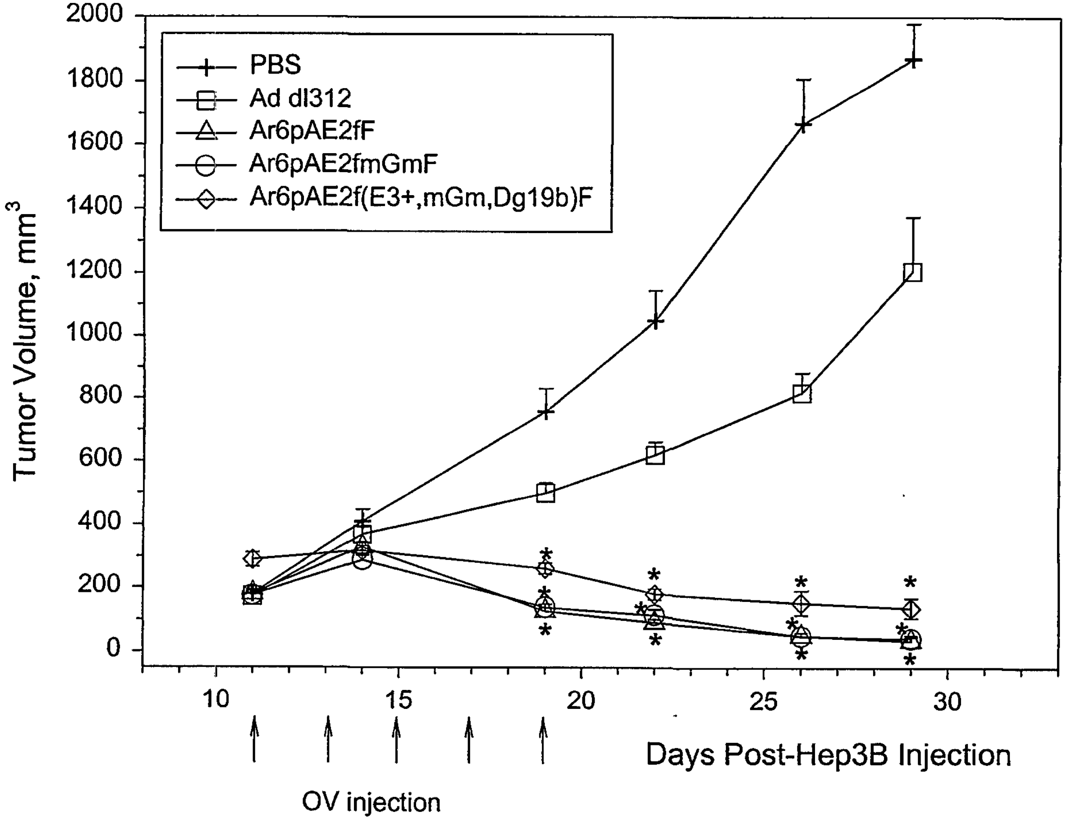

Figure 31. Anti-Tumor Activity of Oncolytic Adenoviruses in the H460 Xenograft Subcutaneous Tumor Model

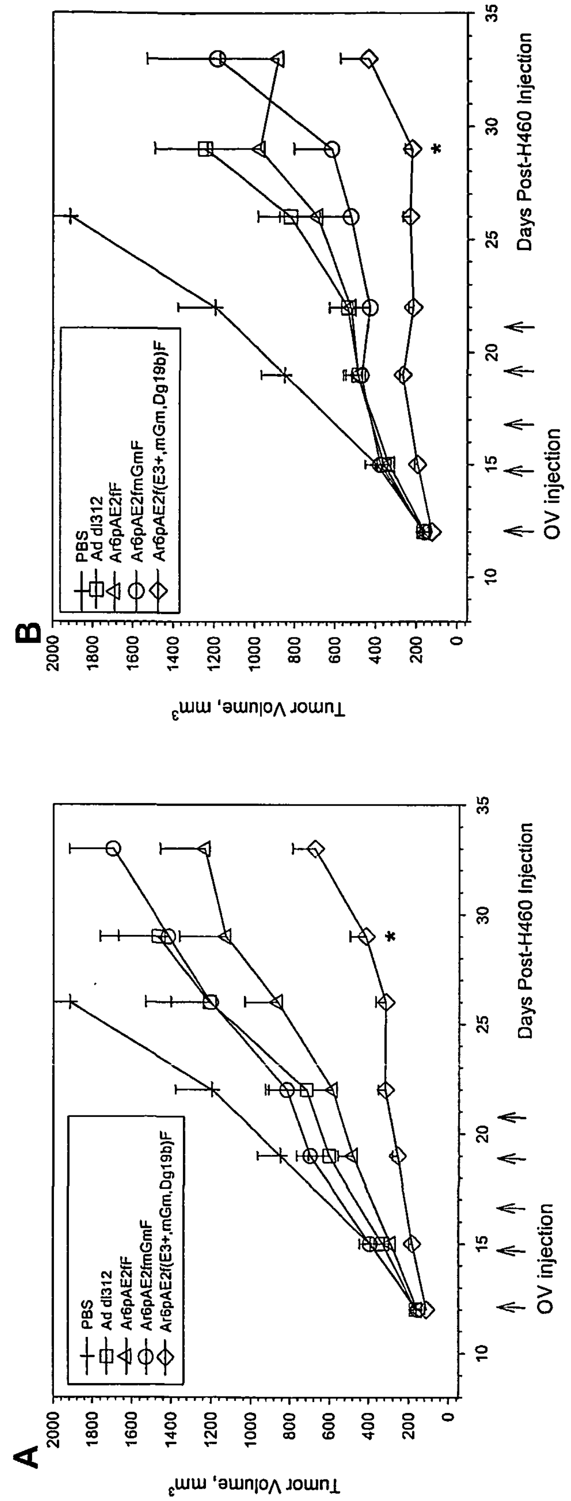

**Figure 32. Schematic diagram of adenovirus pDr5hGmF and pDr5mGmF right donor plasmids.**

A. pDr5hGmF

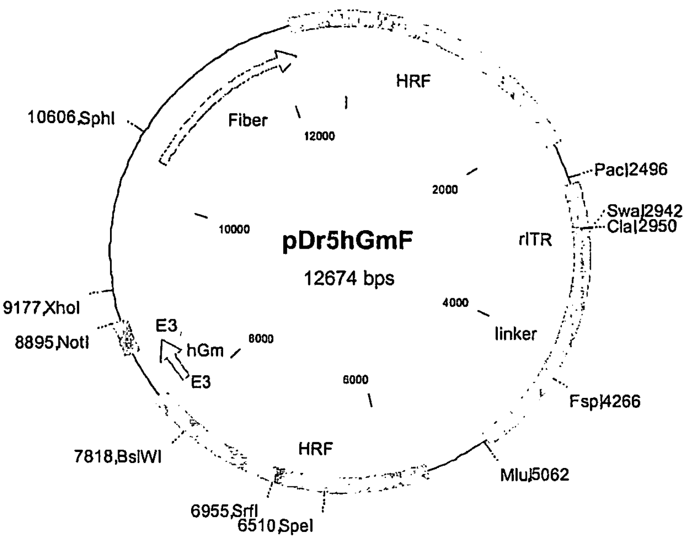


B. pDr5mGmF

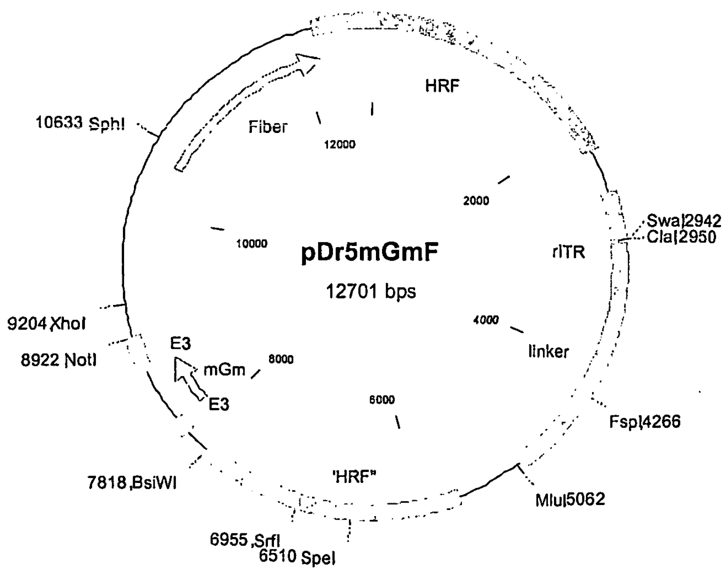

Figure 33. Pathway used to generate the pAr15pAE2fhGmF plasmid.

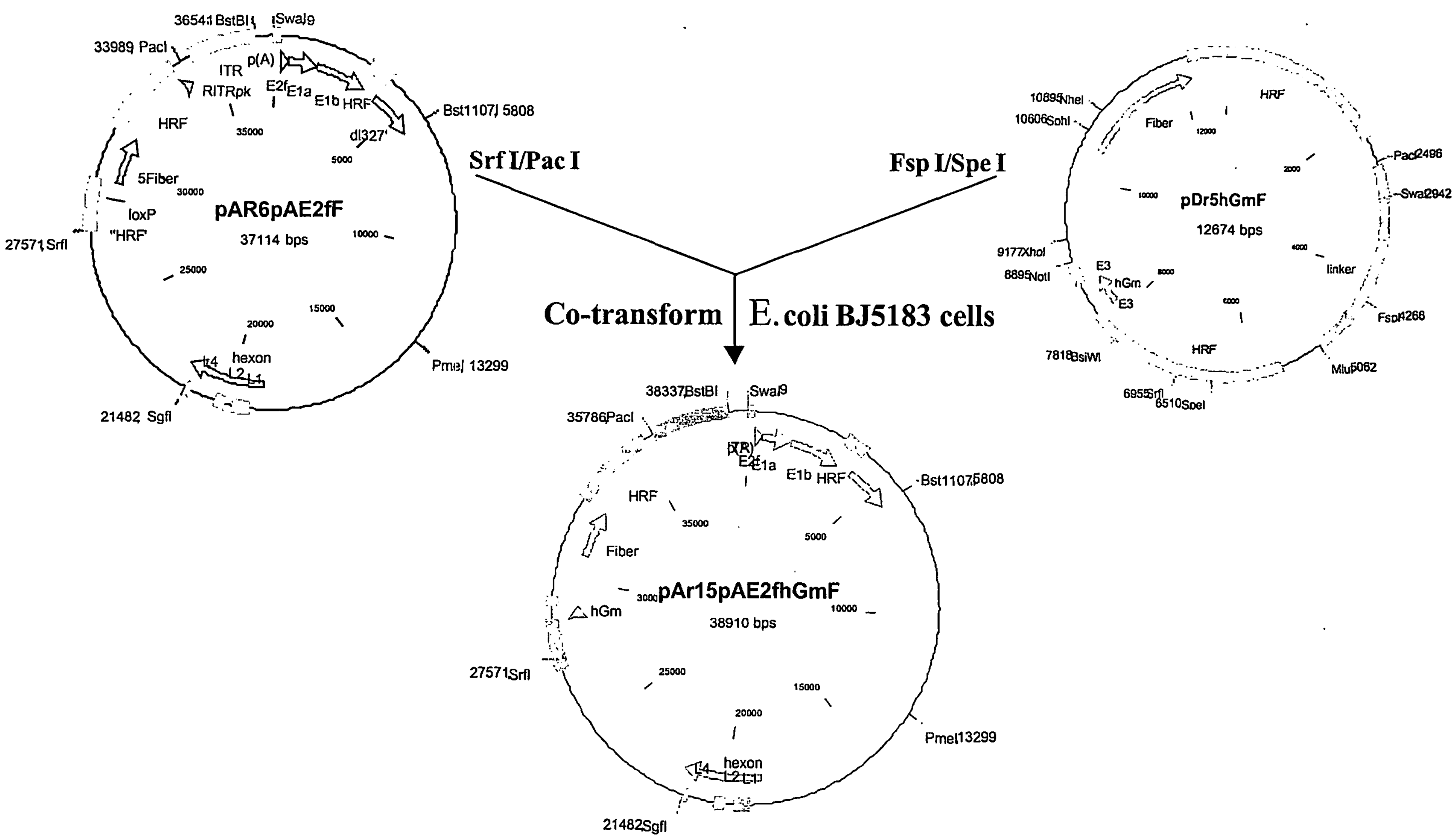

Figure 34. Pathway used to generate the pAr15pAE2fmGmF plasmid.

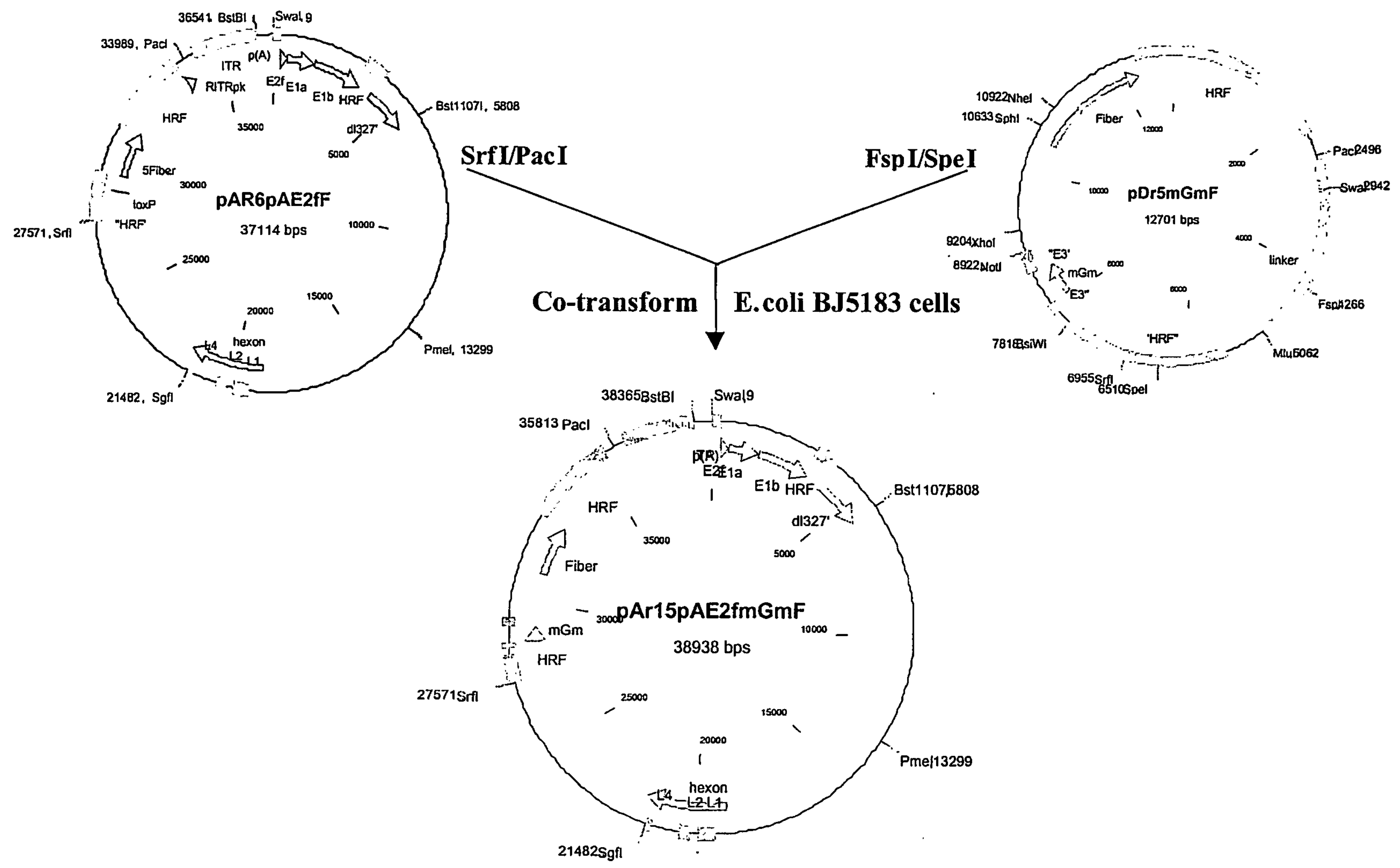

Figure 35. MTS assay of Ar15pAE2fhGmF and Ar15pAE2fmGmF vectors on H460 and Hep3B tumor cell lines.

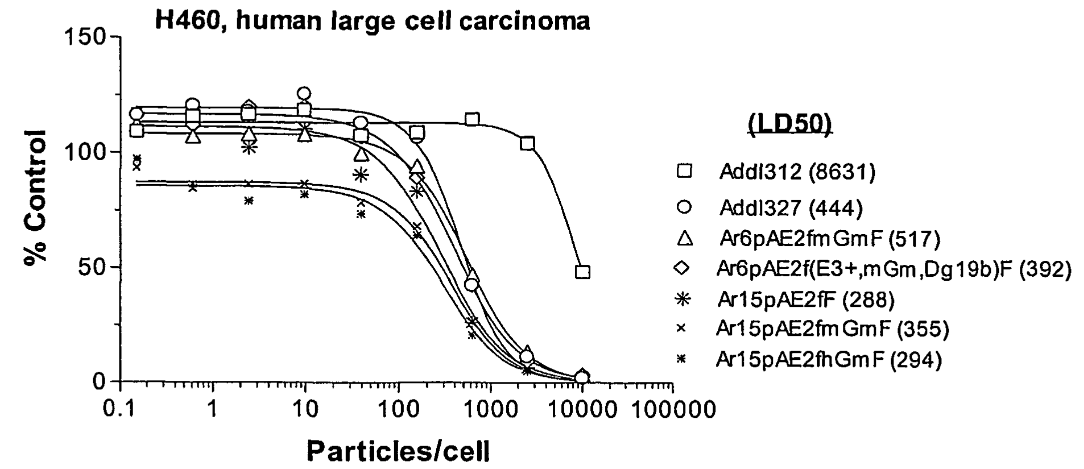


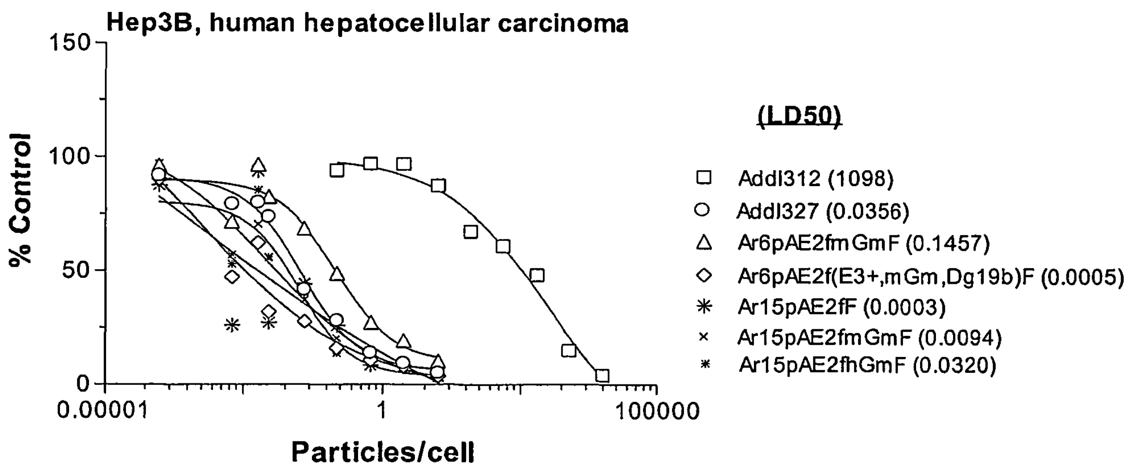

**Figure 36. GM-CSF expression mediated by Ar15pAE2fhGmF and Ar15pAE2fmGmF vectors in infected H460 cells detected by ELISA.**

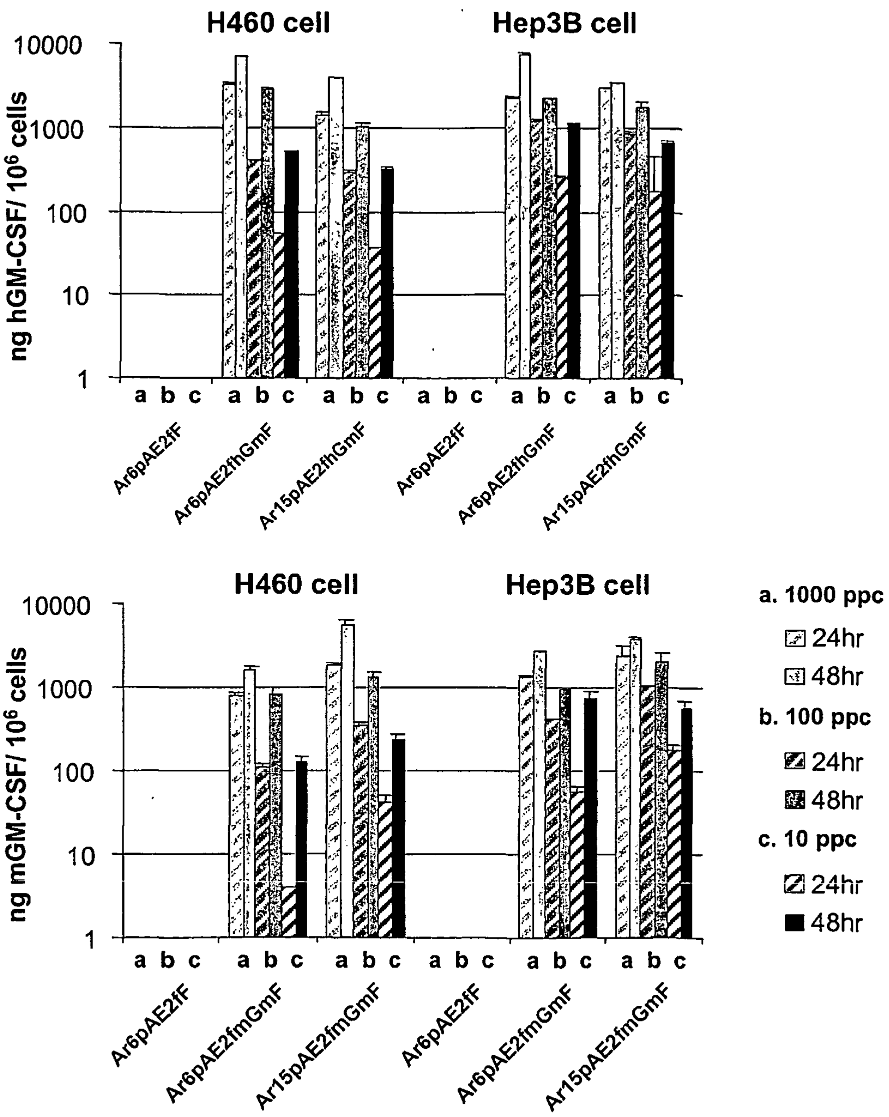

**Figure 37. Schematic Diagram of PCR and Overlap PCR for ΔE3-14.7 plasmids**

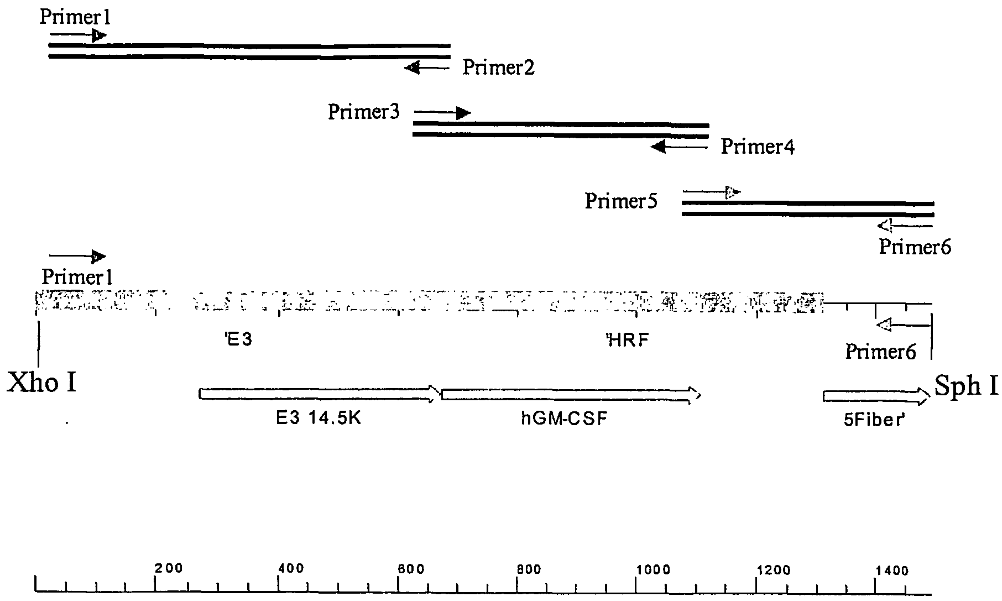


**E3-hGm/XhoI + SphI** (1491 bps)

**Figure 38. Schematic Diagram of ΔE3-14.7 Vectors**

a. Sequence of native E3-14.5/E3-14.7 junction:

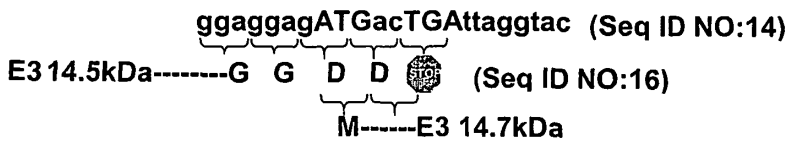


b. Sequence of the Ar16pAE2fhGm vector at the junction engineered between the E3-14.5 gene and human GM-CSF cDNA:

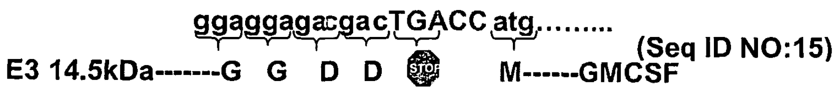

**Figure 39. Pathway Used to Generate the pAr16pAE2fhGmF Large Plasmid**

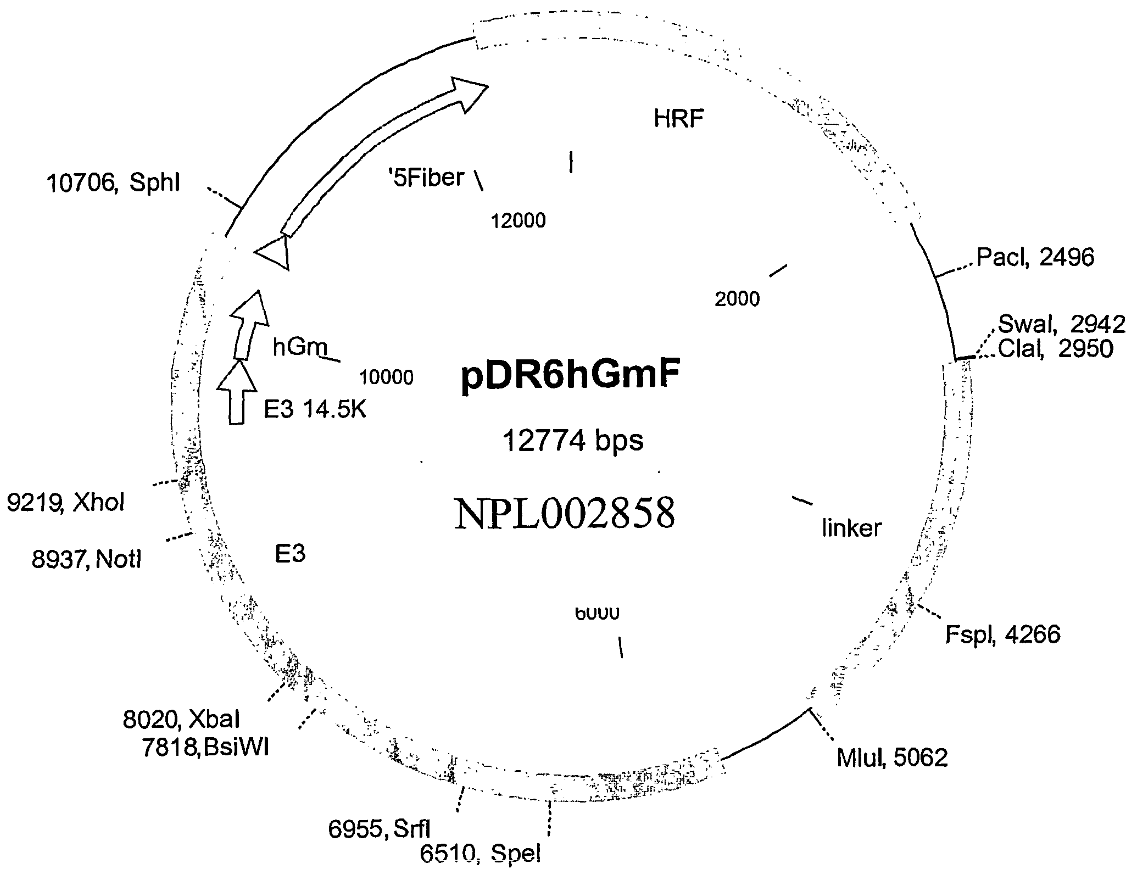

Figure 40. Pathway Used to Generate the pAr16pAE2fhGmF Large Plasmid

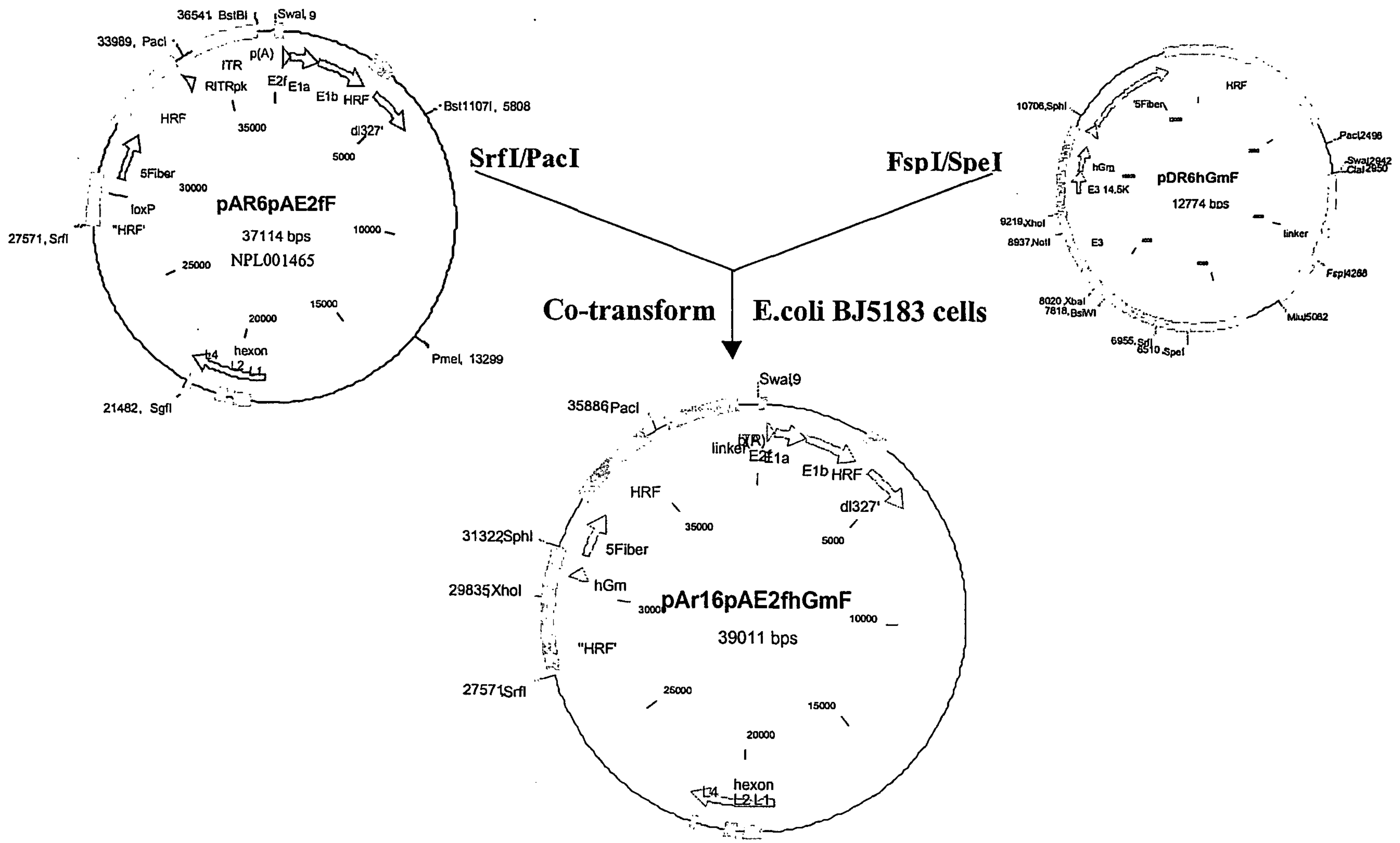

Figure 41. MTS Assay of ΔE3-14.7 hGM-CSF Vector on H460 Tumor Cell Line

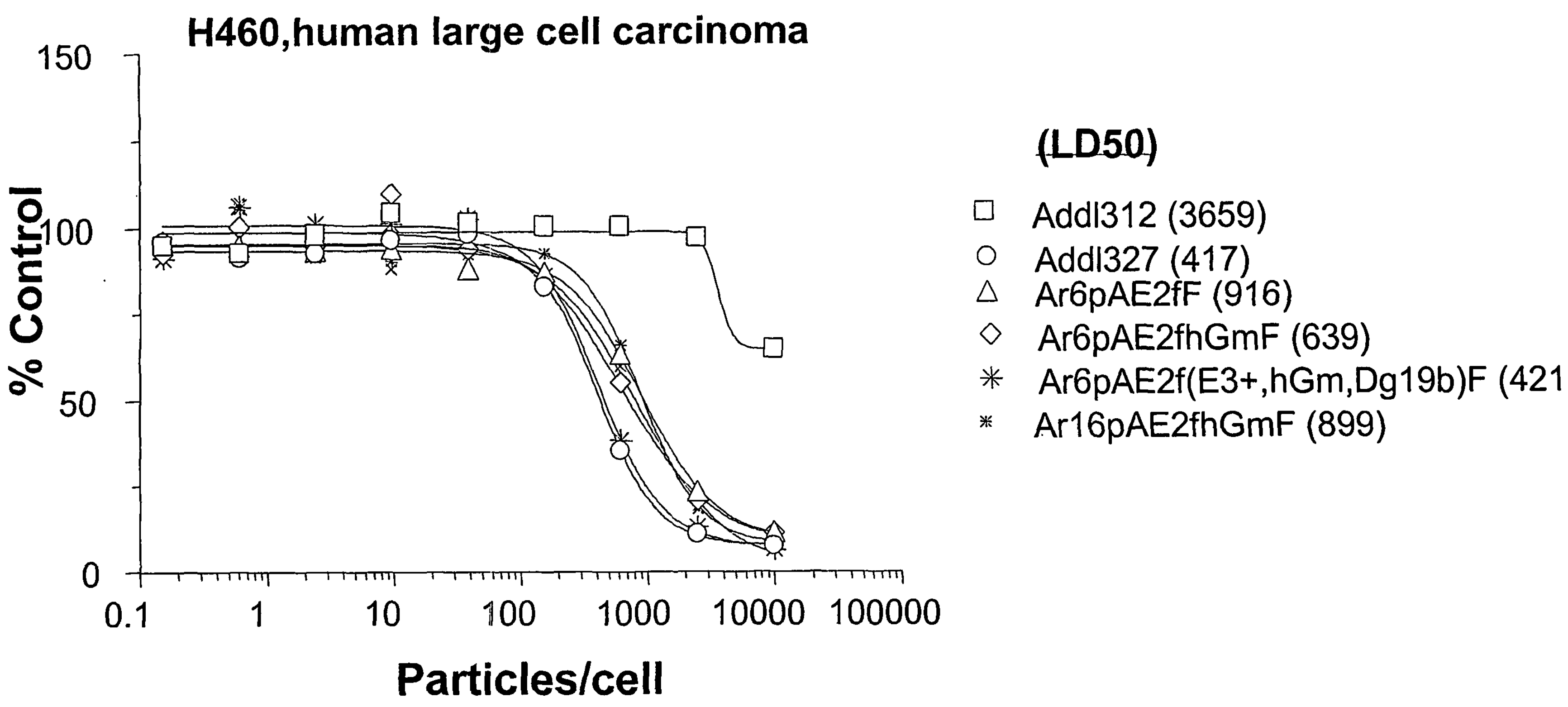

Figure 42. GM-CSF Expression Mediated by ΔE3-14.7 hGM-CSF Vector (Ar16pAE2fhGmF) compared to Ar6pAE2fF, Ar6pAE2fhGmF and Ar6pAE2f(E3+,hGm,Dg19)F in Infected H460 Cells 24 Hours Post-Infection

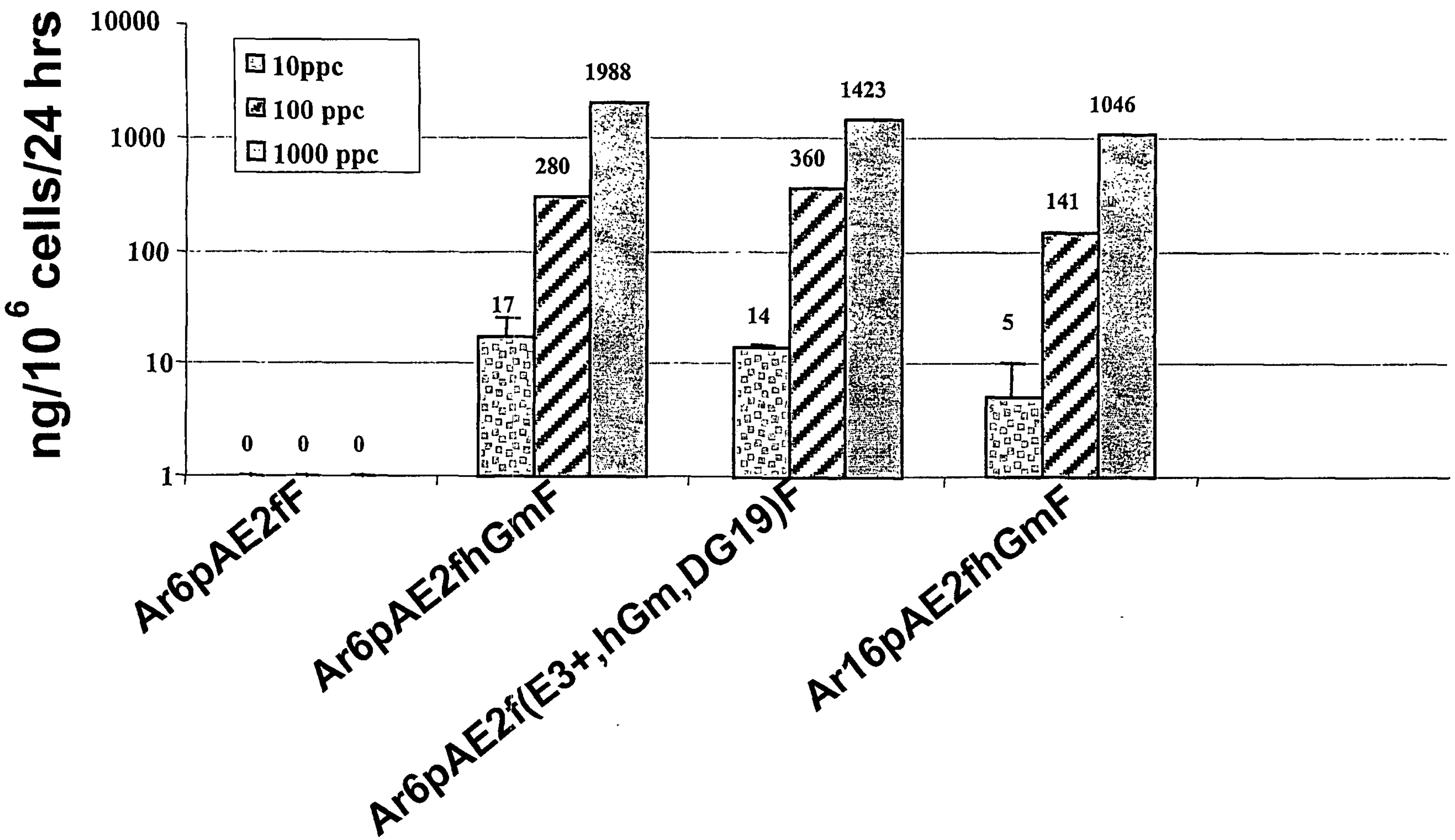

Figure 43. Spread of adenoviruses in H460 xenograft tumors detected by FACS.

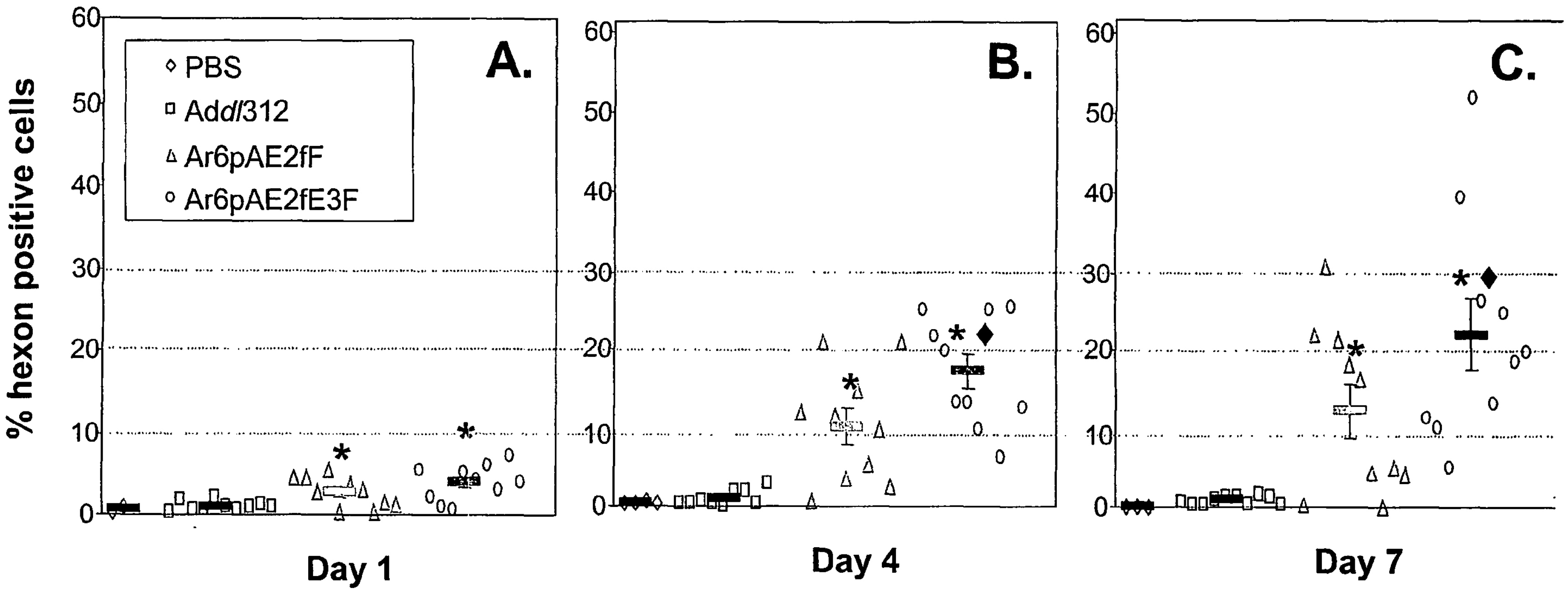


*: $p<0.05$ between Ar6pAE2fF or Ar6pAE2fE3F and Add*l*312, ANOVA

♦: $p<0.05$ between Ar6pAE2fF and Ar6pAE2fE3F vectors, ANOVA

Figure 44. Spread of adenoviruses in Hep3B xenograft tumors detected by FACS.

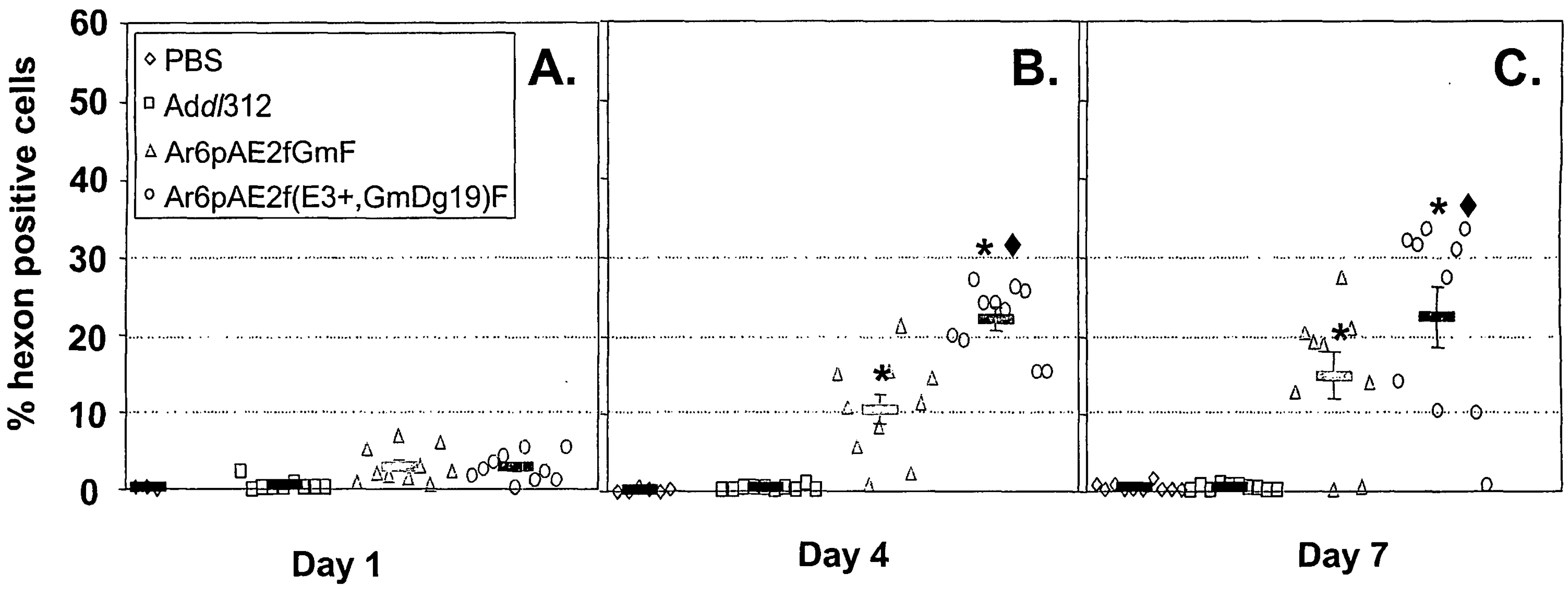


*: $p<0.05$ between Ar6pAE2fhGmF or Ar6pAE2f(E3+,hGm,Dg19)F and Ad*dl*312, ANOVA
♦: $p<0.05$ between Ar6pAE2fhGmF and Ar6pAE2f(E3+,hGm,Dg19)F vectors, ANOVA

## Figure 45: Flowchart for construction of pAR17pAE2fFTrtex:

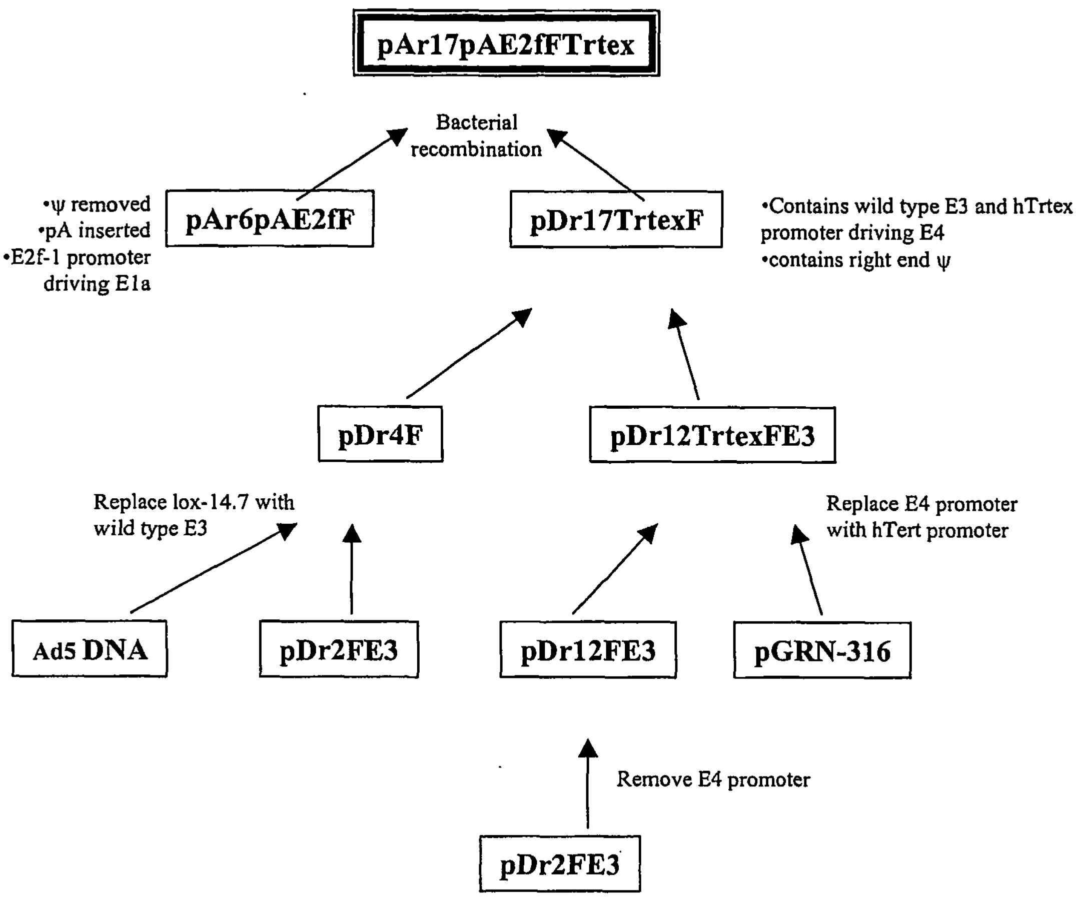

Figure 46: Plasmids used to create oncolytic vector Ar17pAE2fFTrtex

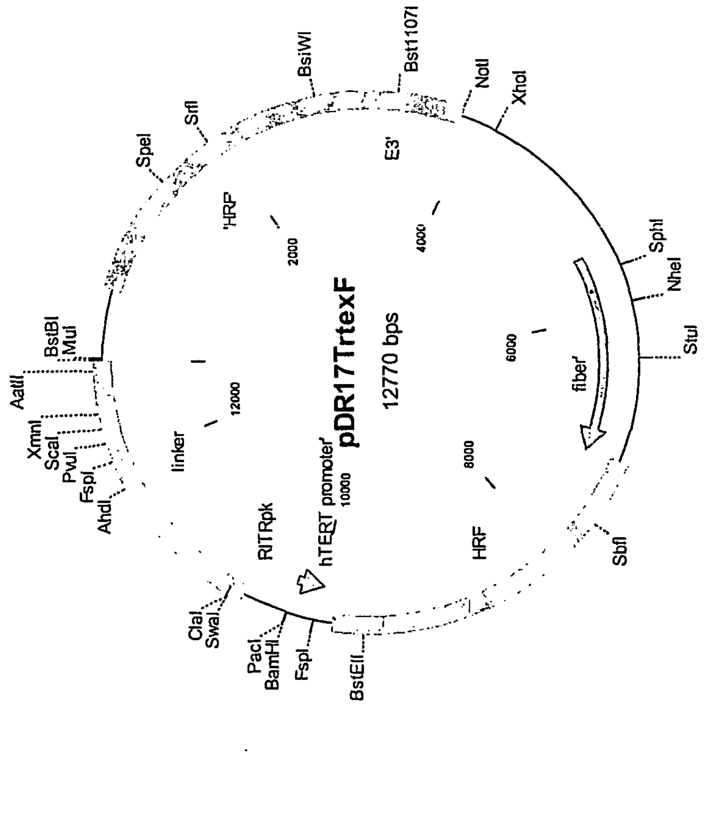


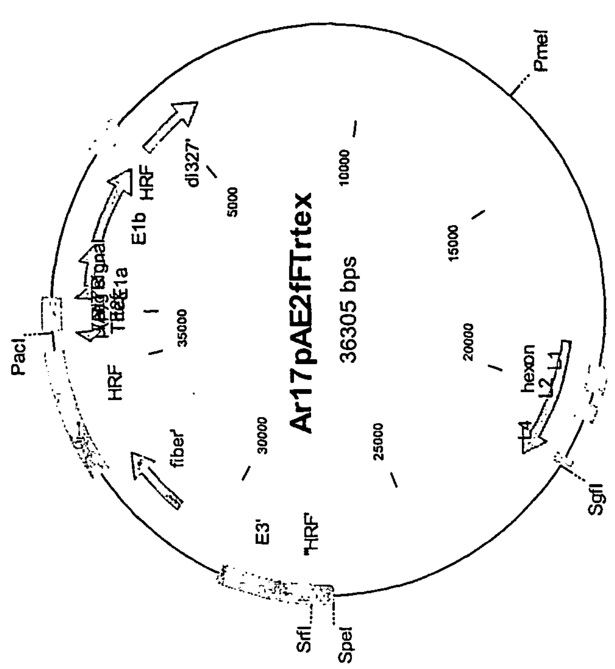

## Figure 47: Sequence of the right end of Ar17pAE2fFTrtex (Seq ID NO:17).

```
35351  agtgctaaaa agcgaccgaa atagcccggg ggaatacata cccgcaggcg
35401  tagagacaac attacagccc ccataggagg tataacaaaa ttaataggag
35451  agaaaaacac ataaacacct gaaaaaccct cctgcctagg caaaatagca
35501  ccctcccgct ccagaacaac atacagcgct tcacagcggc agcctaacag
35551  tcagccttac cagtaaaaaa gaaaacctat taaaaaaaca ccactcggat
35601  caattcgcgg gggtggccgg ggccagggct tcccacgtgc gcagcaggac
35651  gcagcgctgc ctgaaactcg cgccgcgagg agagggcggg gccgcggaaa
35701  ggaaggggag gggctgggag ggcccggagg gggctgggcc ggggacccgg
35751  gaggggtcgg gacggggcgg ggtccgcgcg gaggaggcgg agctggaagg
35801  tgaaggggca ggacgggtgc ccgggtcccc agtccctccg ccacgtgggg
35851  ctaggatcct taattaagaa ttctacaatt cccaacacat acaagttact
35901  ccgccctaaa accctgggcg agtctccacg taaacggtca aagtccccgc
35951  ggccctagac aaatattacg cgctatgagt aacacaaaat tattcagatt
36001  tcacttcctc ttattcagtt ttcccgcgaa aatggccaaa tcttactcgg
36051  ttacgcccaa atttactaca acatccgcct aaaaccgcgc gaaaattgtc
36101  acttcctgtg tacaccggcg cacaccaaaa acgtcacttt tgccacatcc
36151  gtcgcttaca tgtgttccgc cacacttgca acatcacact tccgccacac
36201  tactacgtca cccgccccgt tcccacgccc cgcgccacgt cacaaactcc
36251  accccctcat tatcatattg gcttcaatcc aaaataaggt atattattga
36301  tgatg
```

Figure 48: Diagram of Ar17pAE2fFTrtex.

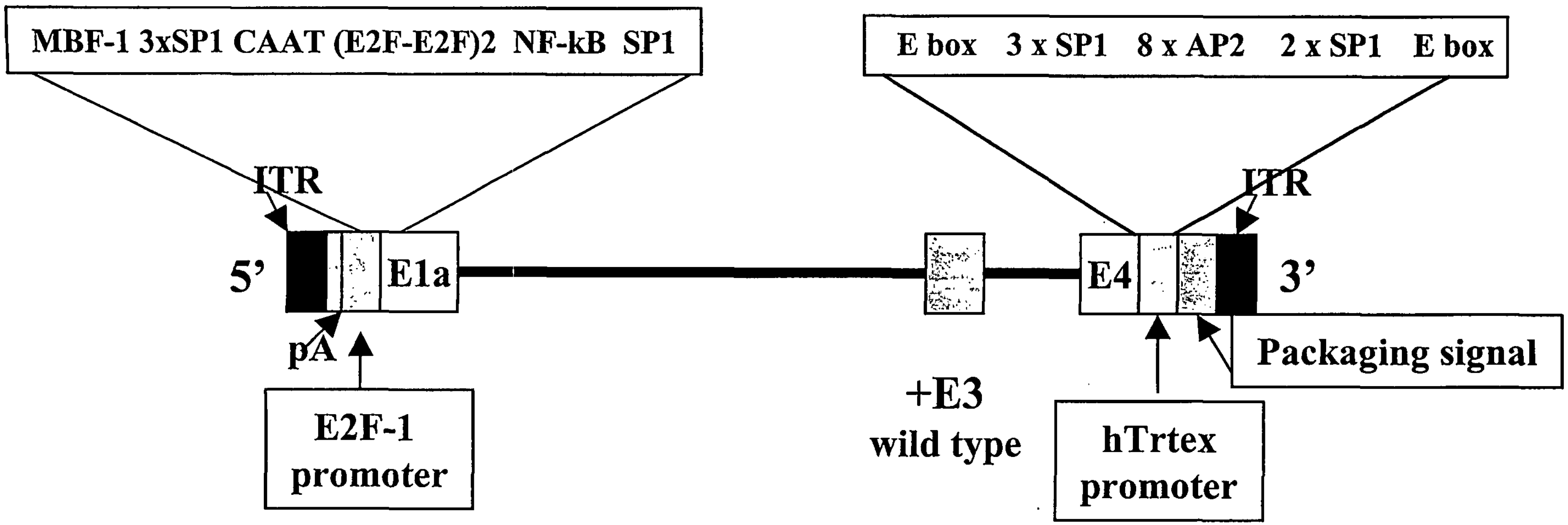

Figure 49. E4 expression is dependent on the hTERT promoter

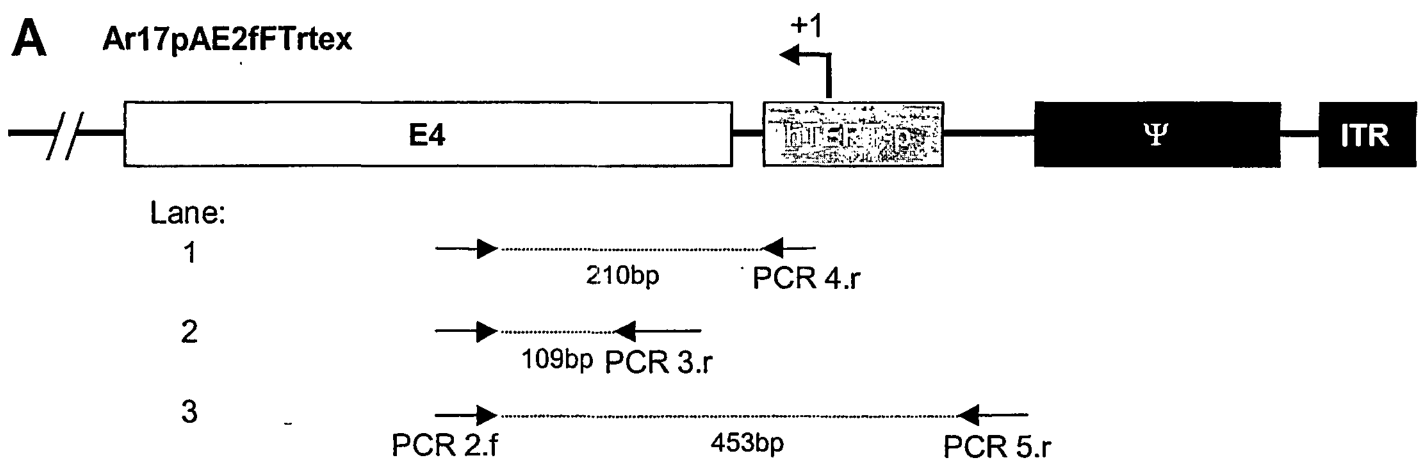

## Figure 50. E4 transcription start sites in Ar17pAE2fFTrtex (Seq ID NO:21)

```
35521   ATACAGCGCT TCACAGCGGC AGCCTAACAG TCAGCCTTAC CAGTAAAAAA GAAAACCTAT
                 ExtP1                 ←
35581   TAAAAAAACA CCACTCGGAT CAATTCGCGG GGGTGGCCGG GGCCAGGGCT TCCCACGTGC
                   ←         ←                ←
35641   GCAGCAGGAC GCAGCGCTGC CTGAAACTCG CGCCGCGAGG AGAGGGCGGG GCCGCGGAAA
                                               ←
35701   AGGAACGGGA CGGGCTGGGA TGGCCCGGAA GGGGCTGGGC CGGGGACCCG GGAAGGGTTC

35761   GGGACGGGGC GGGGTTCCGC GGGGACGAGG CGGAGCTGGA AGGTGAAGGG GCAGGACCGG

35821   TGCCCGGGTC CCCAGTCCCT CCGCCACGTG GGGCTAGGAT CCTTAATTAA GAATTCTACA

35881   ATTCCCAACA CATACAAGTT ACTCCGCCCT AAAACCCTGG GCG
```

Figure 51. Efficacy of Ar17pAE2fFTrtex in Hep3B model.

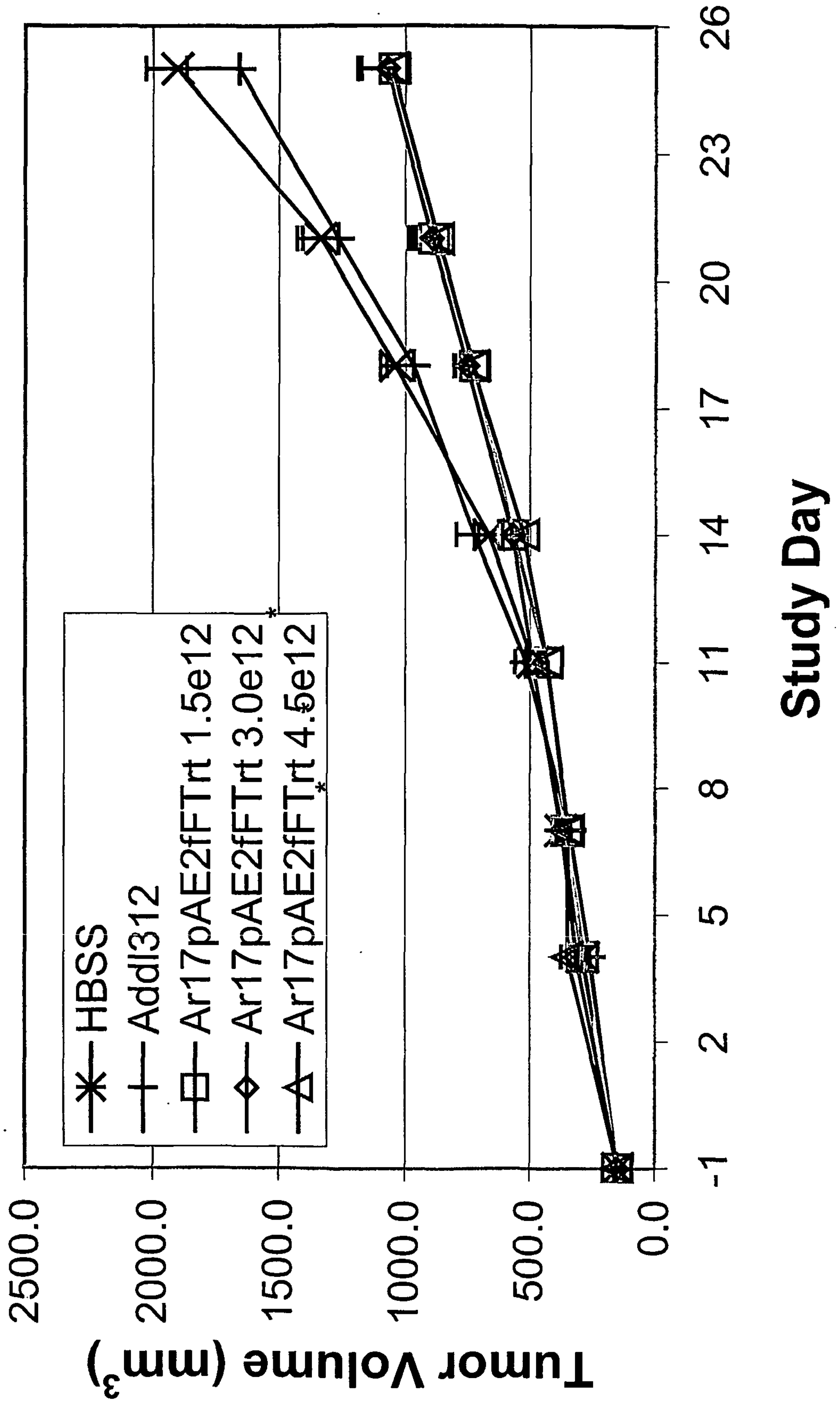

Figure 52. Effect of Ar17pAE2fFTrtex on survival.

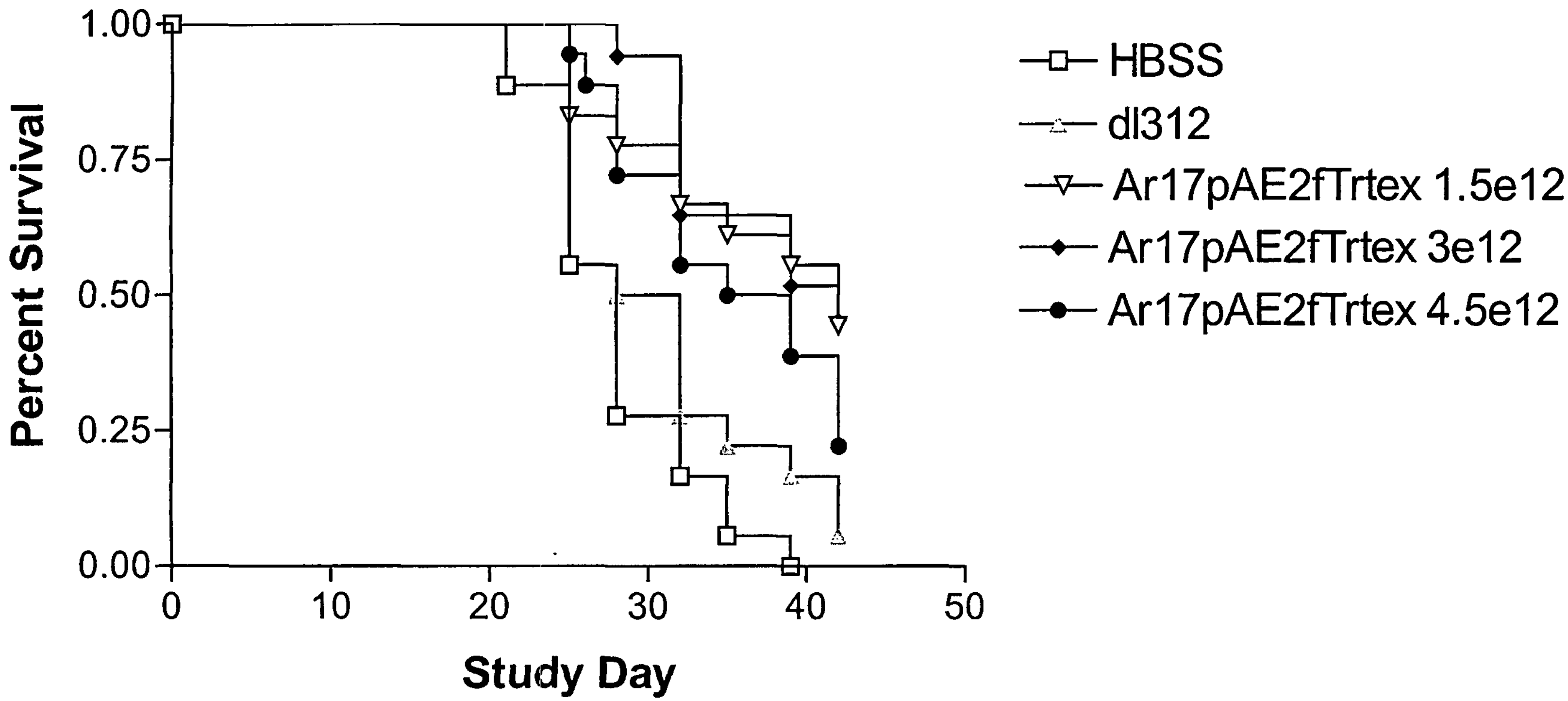

Figure 53. Body weight changes

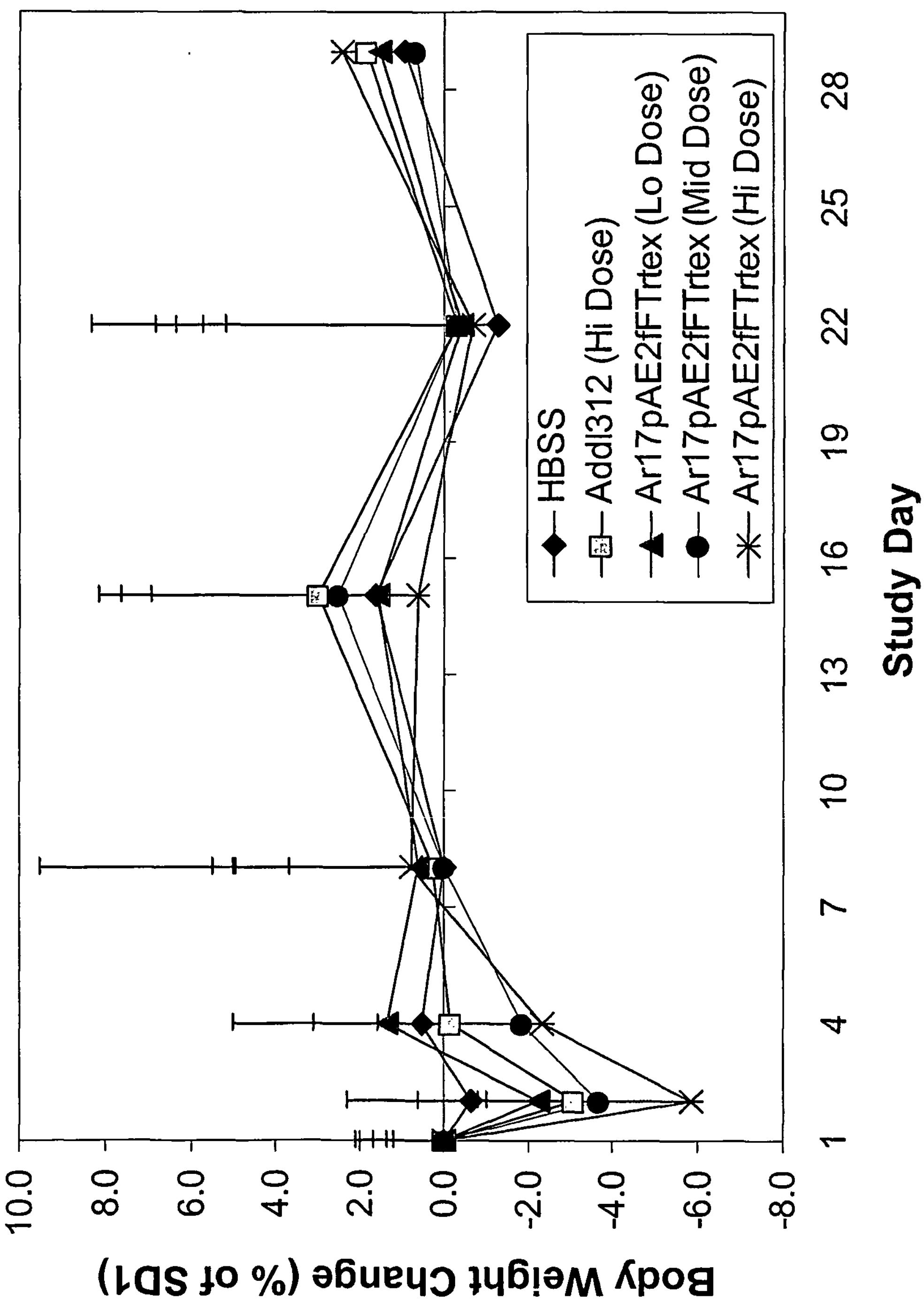

Figure 54. Efficacy of Ar17pAE2fFTrtex in Hep3B model.

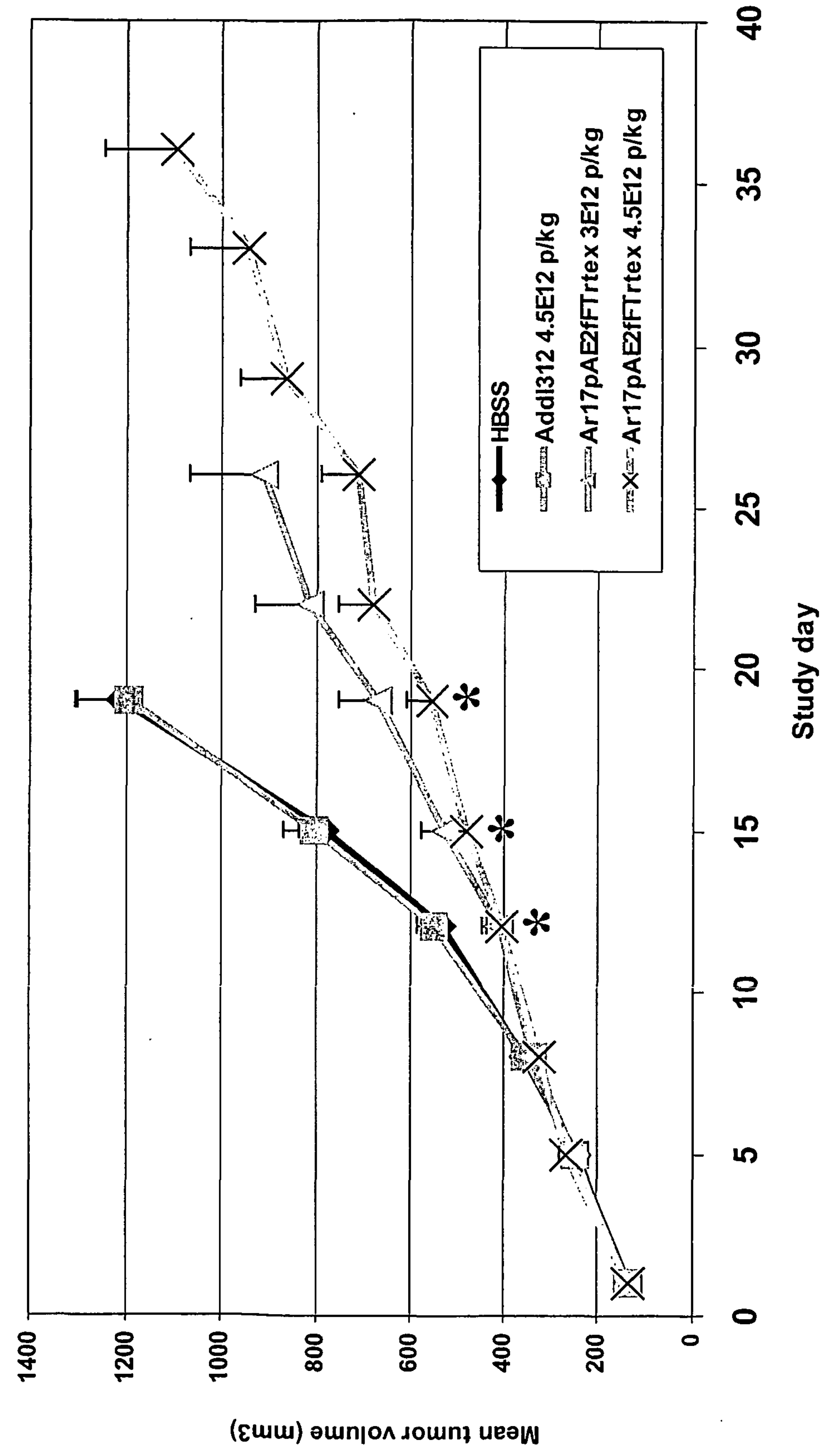

Figure 55. Effect of Ar17pAE2fFTrtex on survival.

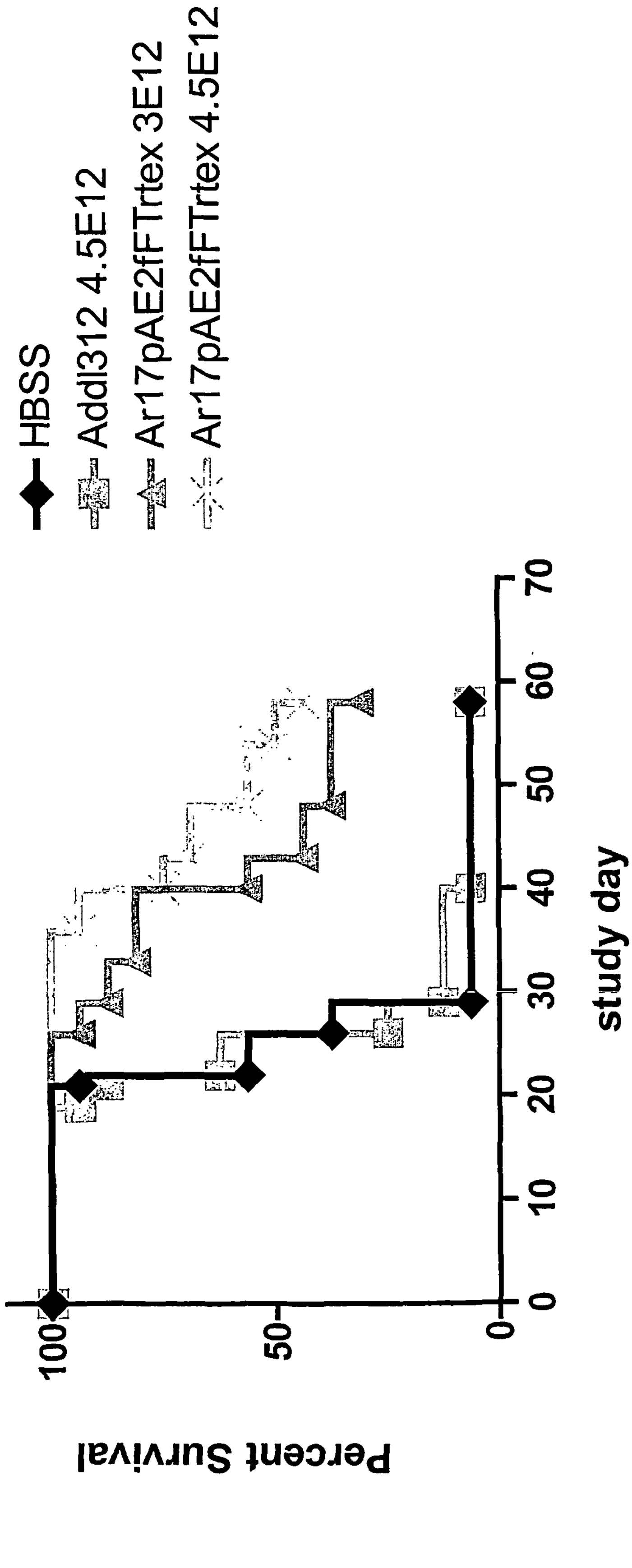

Figure 56. Body weight changes

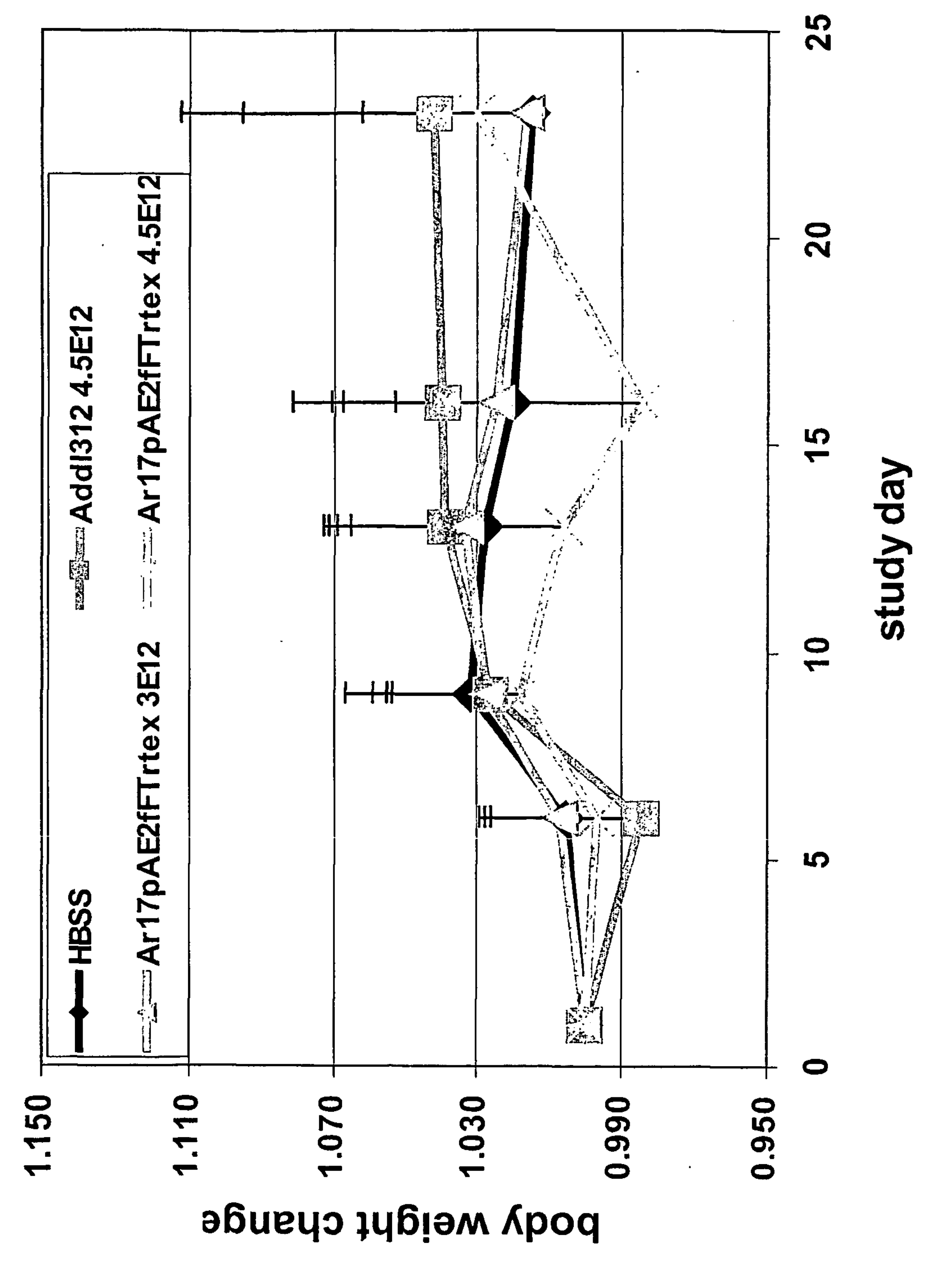

Figure 57 Dose-dependent anti-tumor efficacy

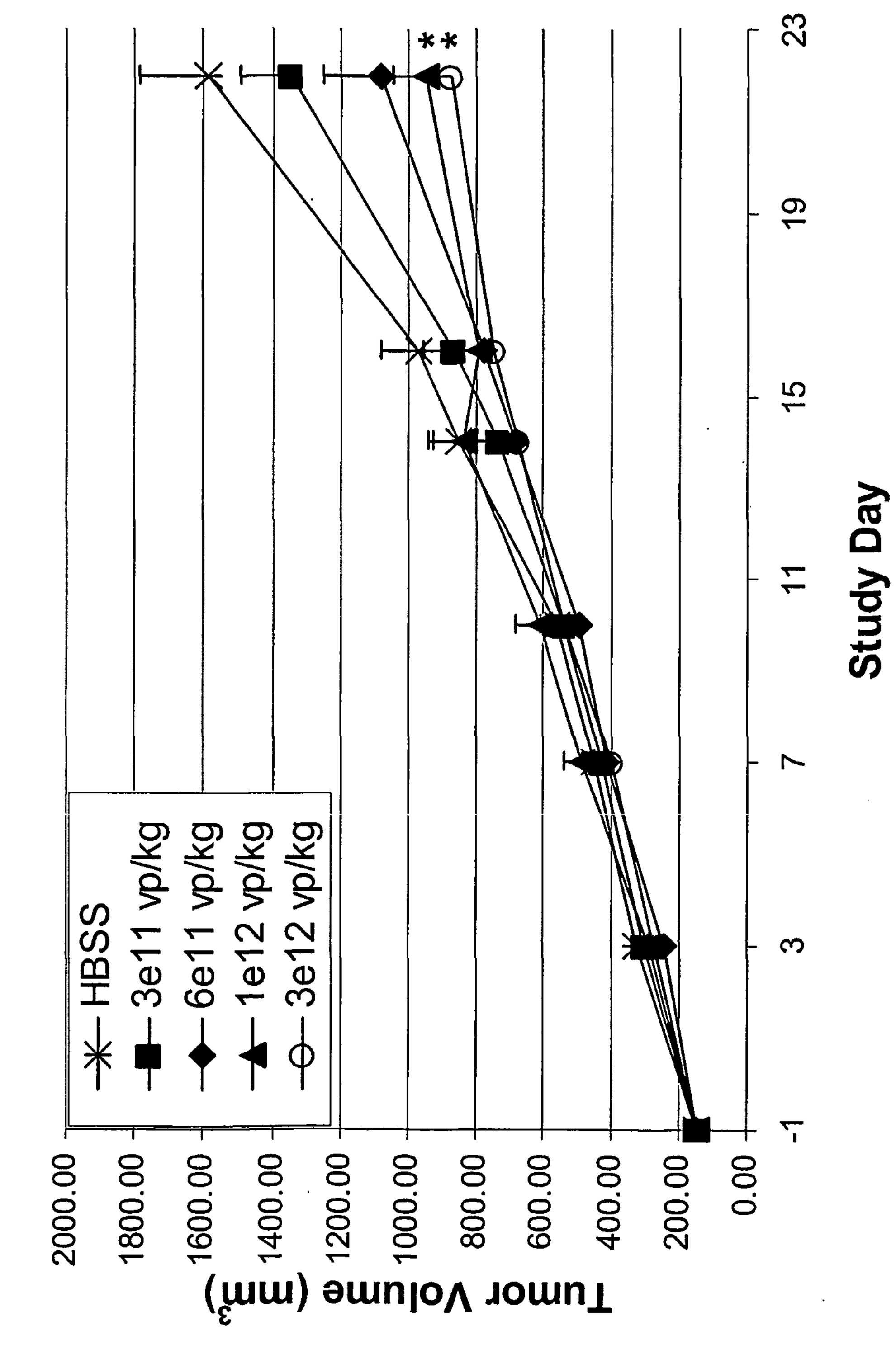

**Figure 58. Individual tumor volumes following intravenous administration of Ar17pAE2fFTrtex**

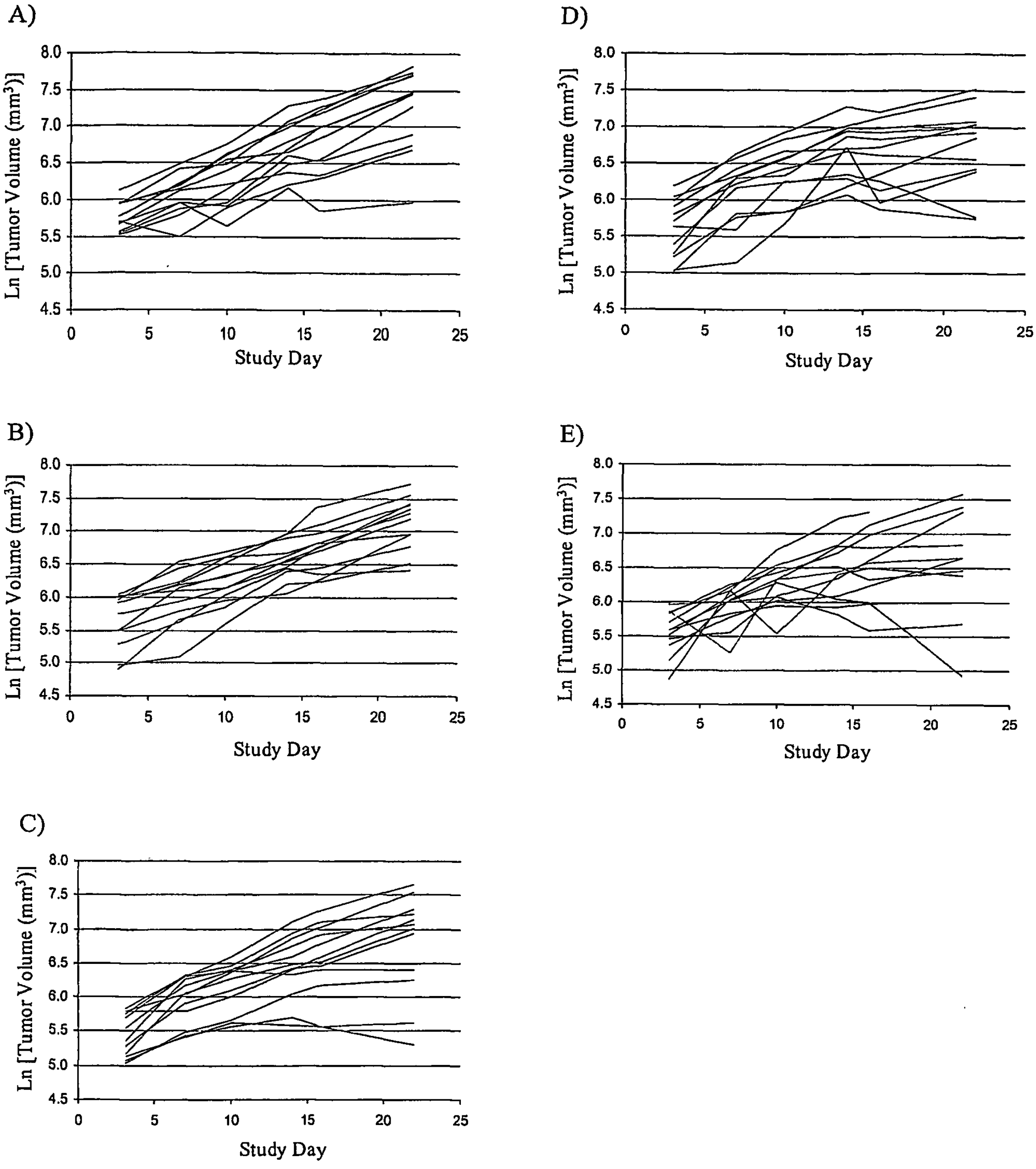

Figure 59. Effect of Ar17pAE2fFTrtex on survival.

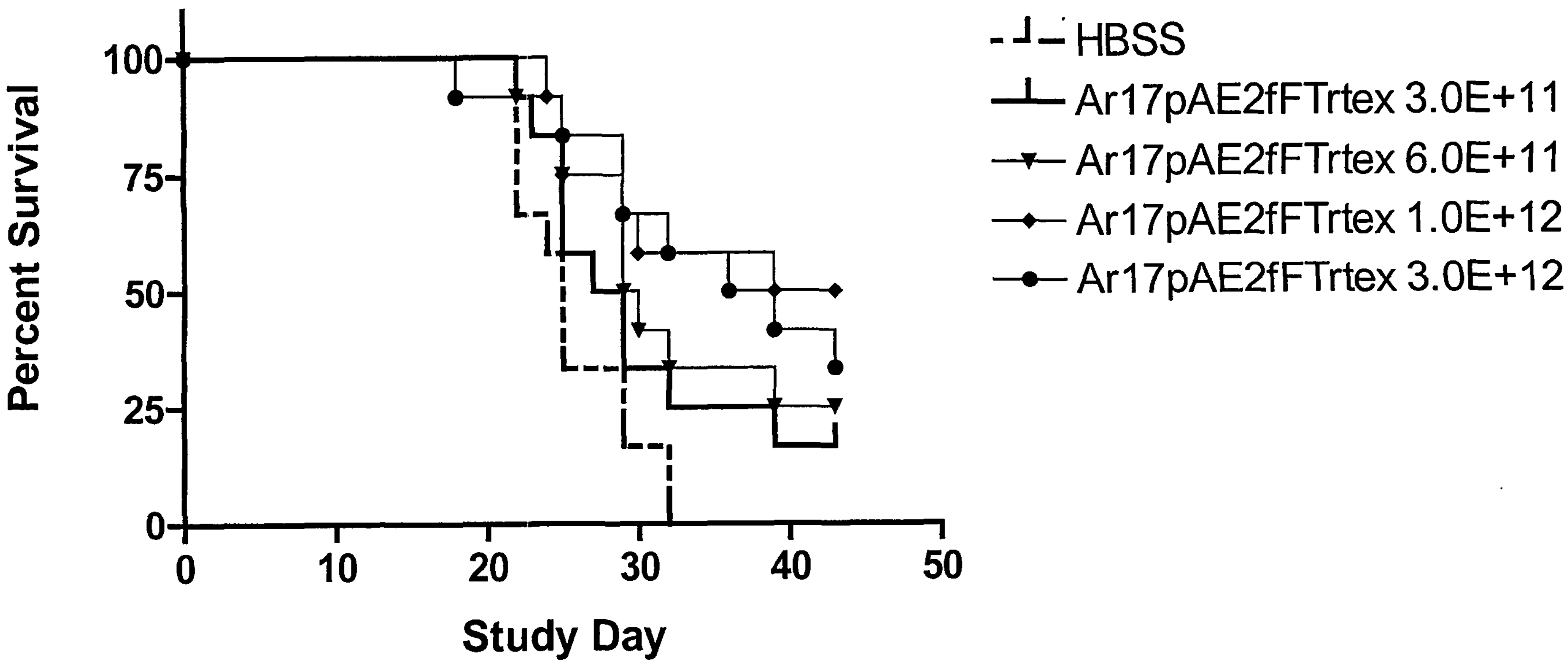

Figure 60. Body weight (% change)

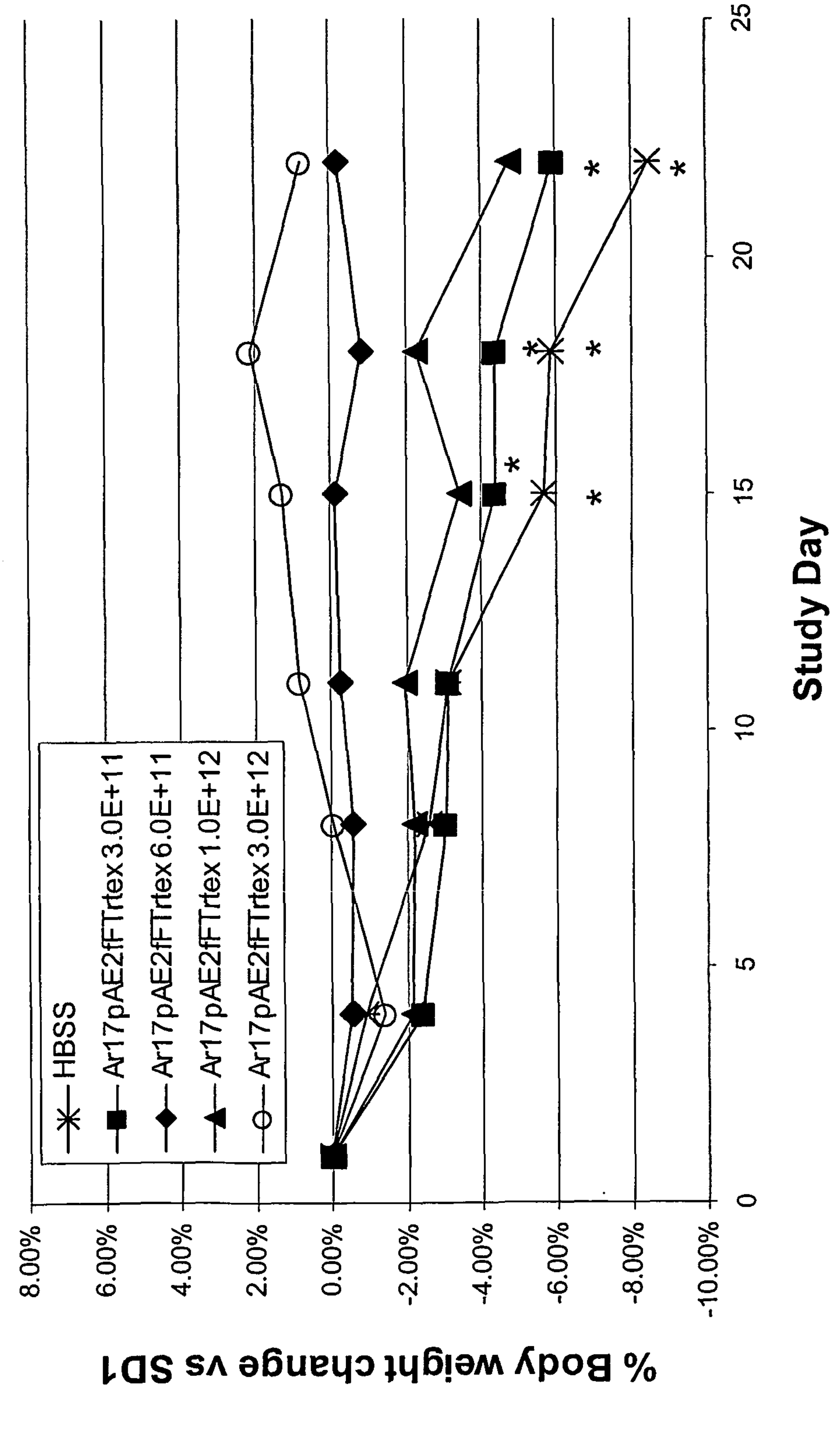

Figure 61.

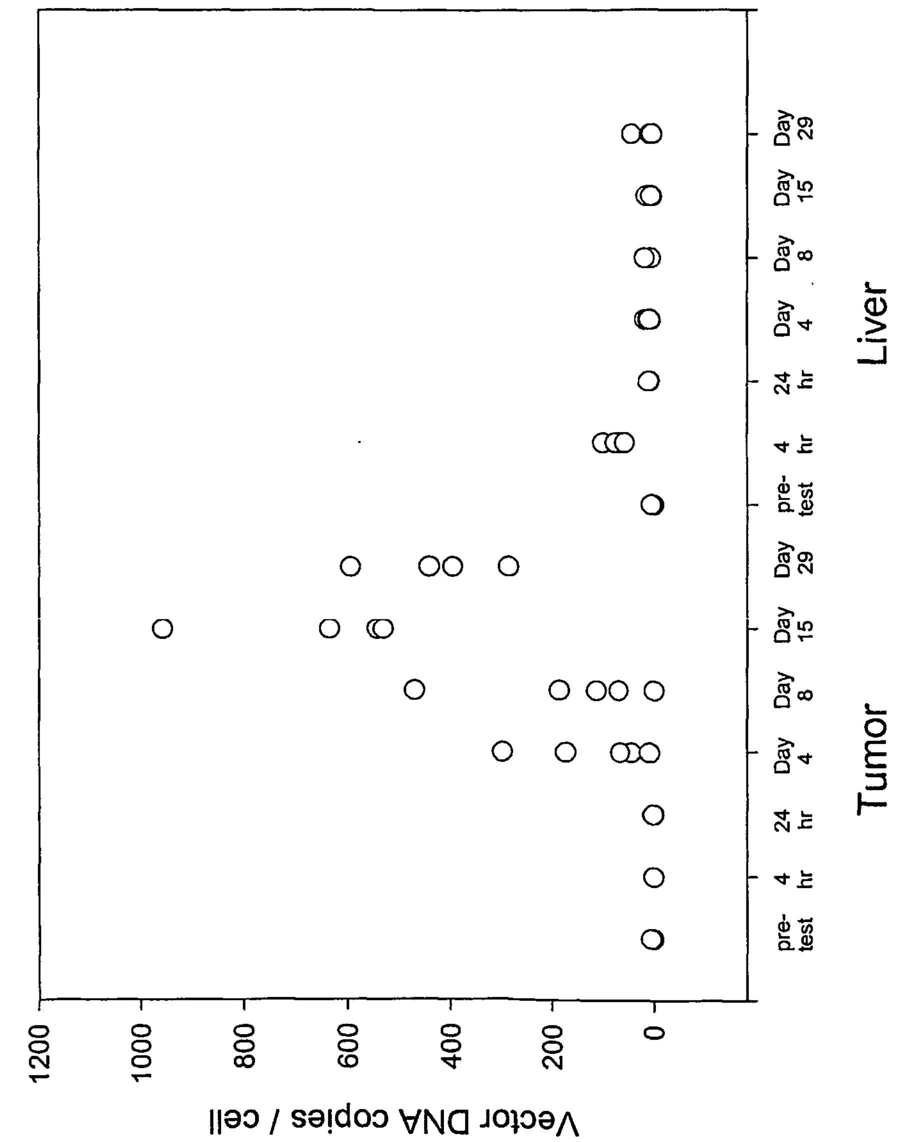
Vector DNA copies / cell
1200
1000
800
600
400
200
0
pre-test
4 hr
24 hr
Day 4
Day 8
Day 15
Day 29
pre-test
4 hr
24 hr
Day 4
Day 8
Day 15
Day 29
Tumor
Liver

Figure 62. Effect on body weight in SCID mice

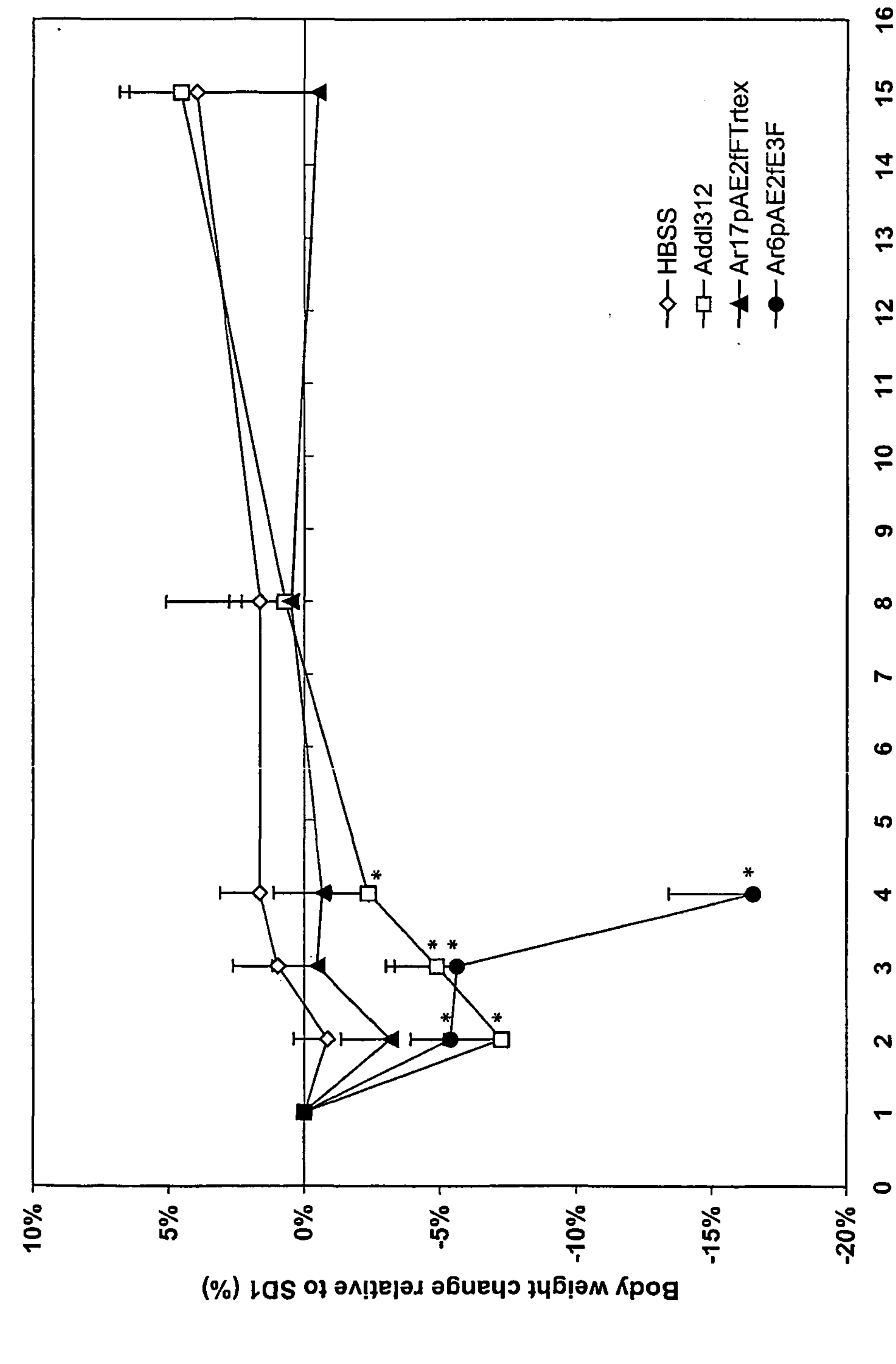

**Figure 63. Improved isobologram with additivity envelope for Ar17pAE2fFTrtex and Taxol against Hep 3B and PC3M.2AC6 cells.**

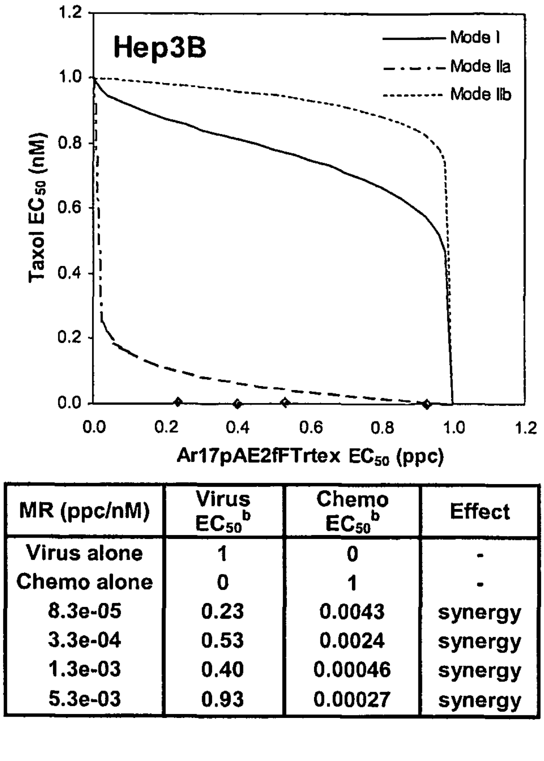


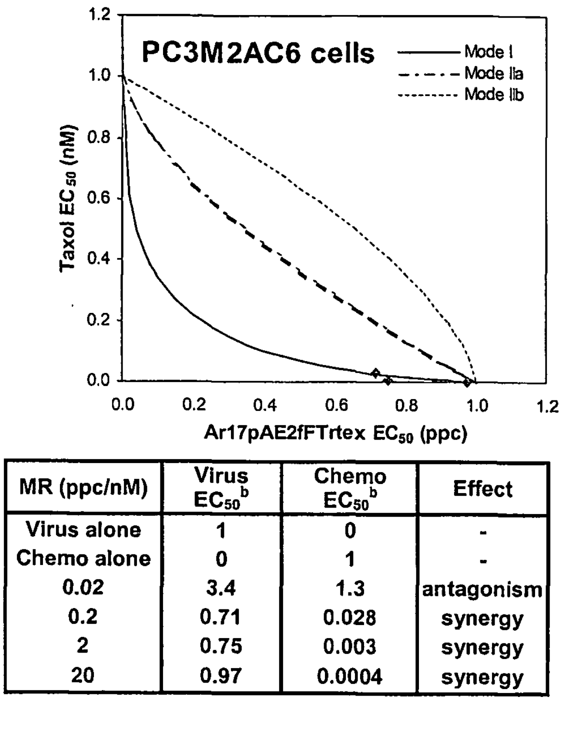


| MR (ppc/nM) | Virus $EC_{50}^b$ | Chemo $EC_{50}^b$ | Effect |
|---|---|---|---|
| Virus alone | 1 | 0 | - |
| Chemo alone | 0 | 1 | - |
| 8.3e-05 | 0.23 | 0.0043 | synergy |
| 3.3e-04 | 0.53 | 0.0024 | synergy |
| 1.3e-03 | 0.40 | 0.00046 | synergy |
| 5.3e-03 | 0.93 | 0.00027 | synergy |

| MR (ppc/nM) | Virus $EC_{50}^b$ | Chemo $EC_{50}^b$ | Effect |
|---|---|---|---|
| Virus alone | 1 | 0 | - |
| Chemo alone | 0 | 1 | - |
| 0.02 | 3.4 | 1.3 | antagonism |
| 0.2 | 0.71 | 0.028 | synergy |
| 2 | 0.75 | 0.003 | synergy |
| 20 | 0.97 | 0.0004 | synergy |

**Figure 64. Improved isobologram with additivity envelope for Ar17pAE2fFTrtex and Doxorubicin against Hep 3B and PC3M.2AC6 cells.**

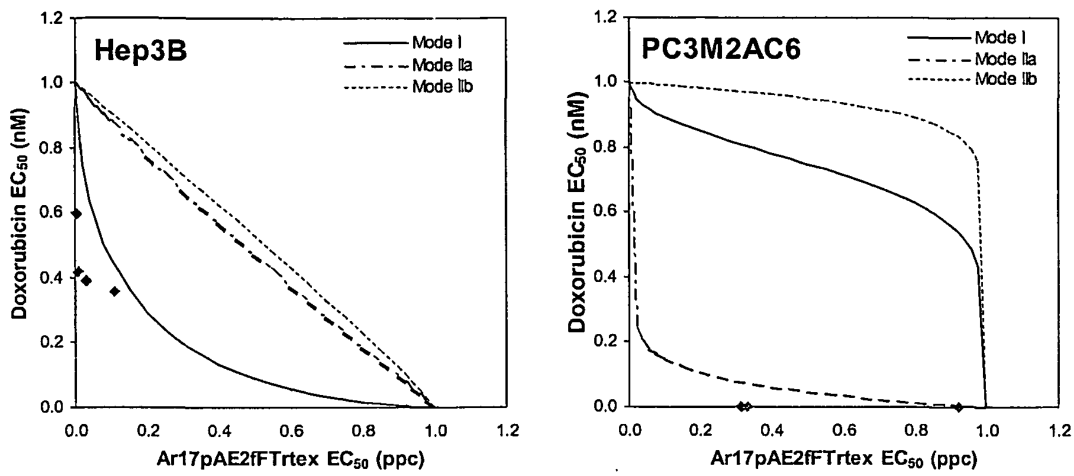


| MR (ppc/nM) | Virus $EC_{50}^{b}$ | Chemo $EC_{50}^{b}$ | Effect |
|---|---|---|---|
| Virus alone | 1 | 0 | - |
| Chemo alone | 0 | 1 | - |
| 1.3e-05 | 0.0028 | 0.60 | synergy |
| 5.0e-05 | 0.0078 | 0.42 | synergy |
| 2.0e-04 | 0.029 | 0.39 | synergy |
| 8.0e-04 | 0.11 | 0.36 | synergy |

| MR (ppc/nM) | Virus $EC_{50}^{b}$ | Chemo $EC_{50}^{b}$ | Effect |
|---|---|---|---|
| Virus alone | 1 | 0 | - |
| Chemo alone | 0 | 1 | - |
| 1 | 2.2 | 0.015 | antagonism |
| 10 | 0.92 | 6.1e-4 | synergy |
| 100 | 0.34 | 2.2e-5 | synergy |
| 1000 | 0.32 | 2.1e-6 | synergy |

**Figure 65. Improved isobologram with additivity envelope for Ar17pAE2fFTrtex and Epothilone B against Hep 3B cells.**

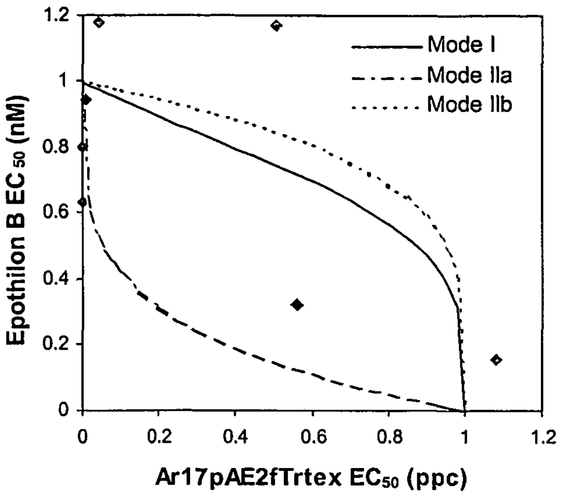


| | Virus $EC_{50}$[b] | Chemo $EC_{50}$[b] | Effect |
|---|---|---|---|
| Virus alone | 1 | 0 | - |
| Chemo alone | 0 | 1 | - |
| 3.1e-06 | 0.00045 | 0.63 | synergy |
| 1.3e-05 | 0.0018 | 0.80 | synergy |
| 5.0e-05 | 0.0084 | 0.95 | synergy |
| 2.0e-04 | 0.042 | 1.2 | antagonism |
| 8.0e-04 | 0.18 | 1.6 | antagonism |
| 3.2e-03 | 0.51 | 1.2 | antagonism |
| 1.3e-02 | 0.56 | 0.32 | additivity |
| 5.1e-02 | 1.1 | 0.06 | antagonism |

Figure 66. Doxorubicin Combination: Mean Tumor Volumes

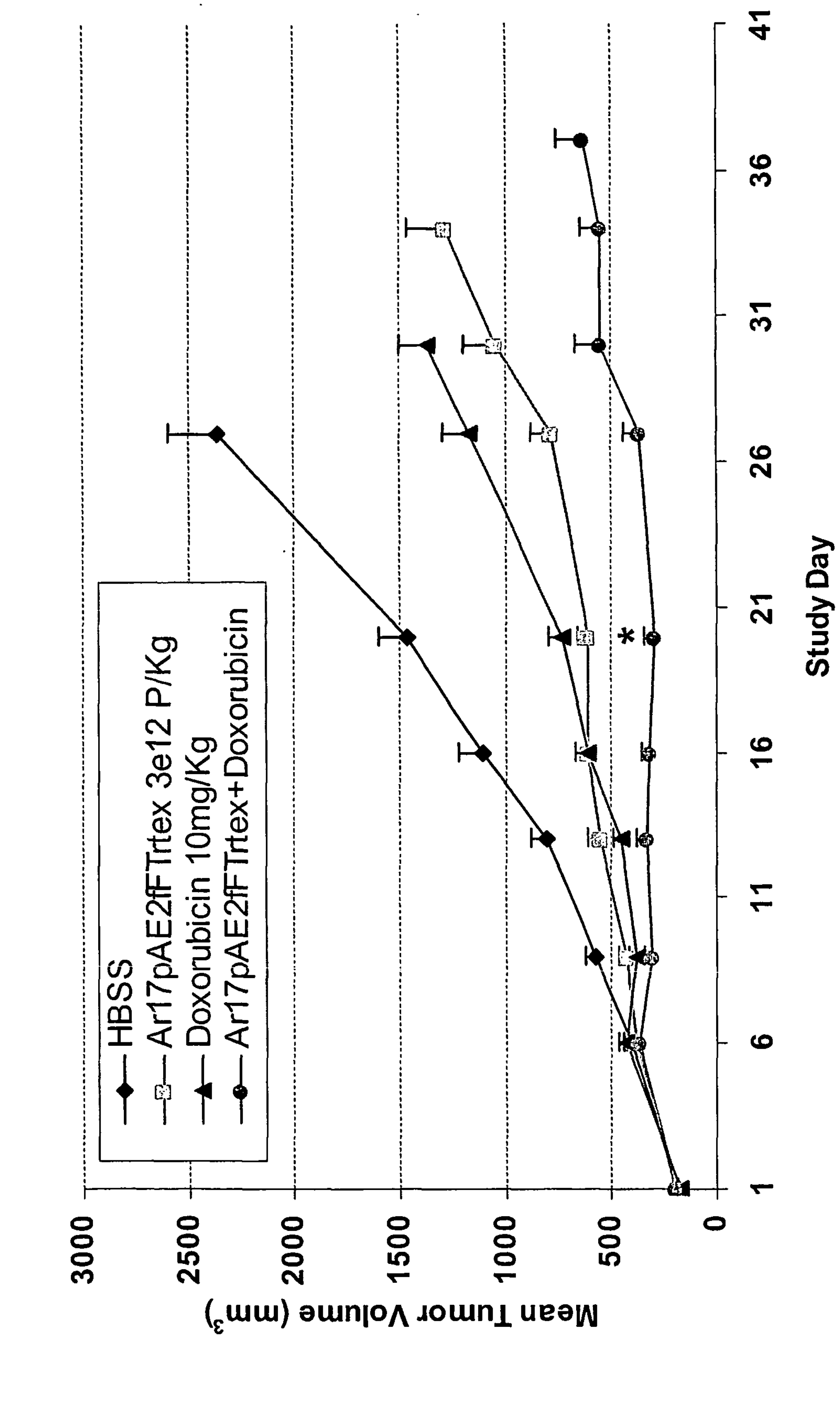

Figure 67. Doxil® Combination Mean Tumor Volumes

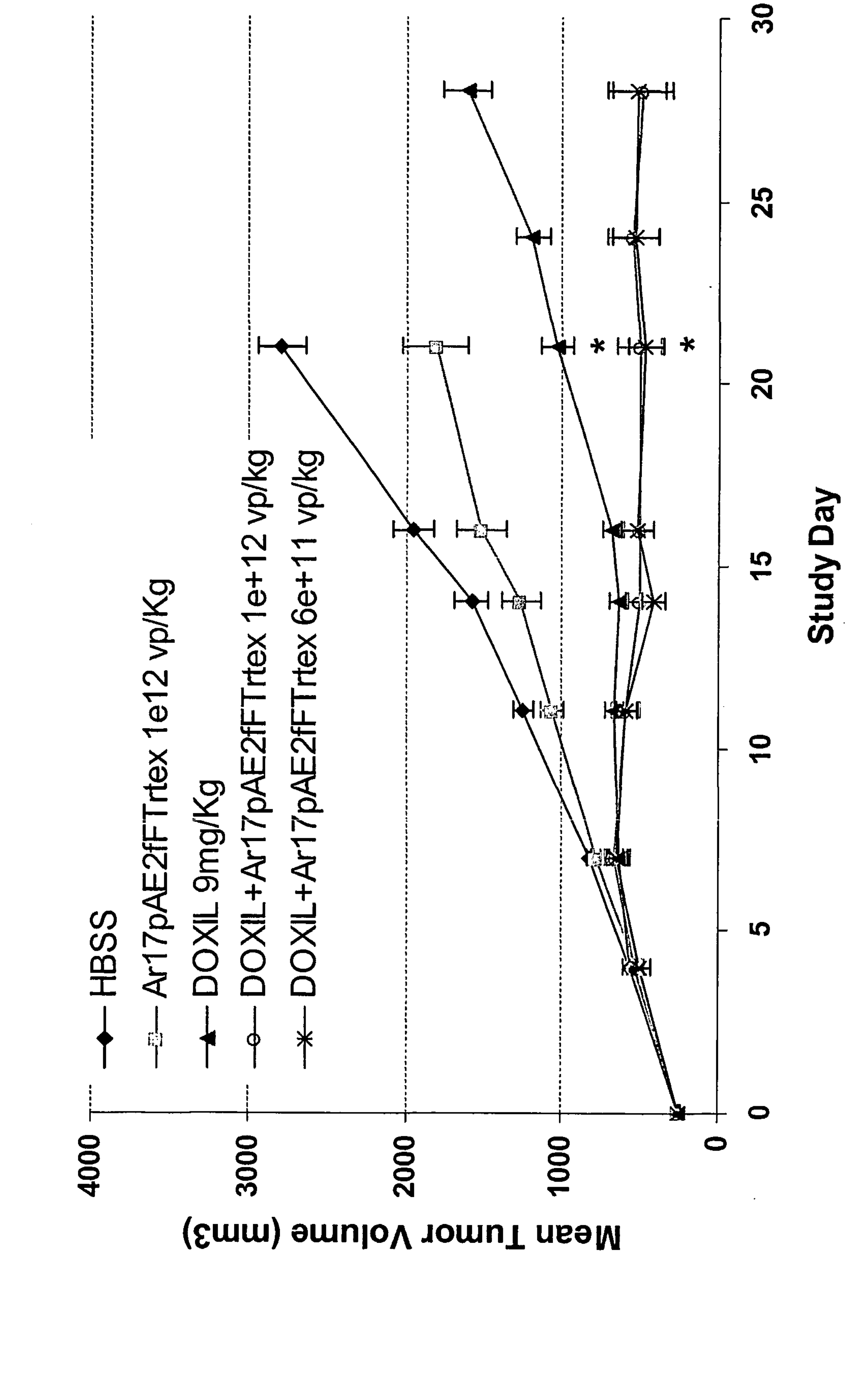

**Figure 68. Cytotoxicity assessed in primary human hepatocytes**

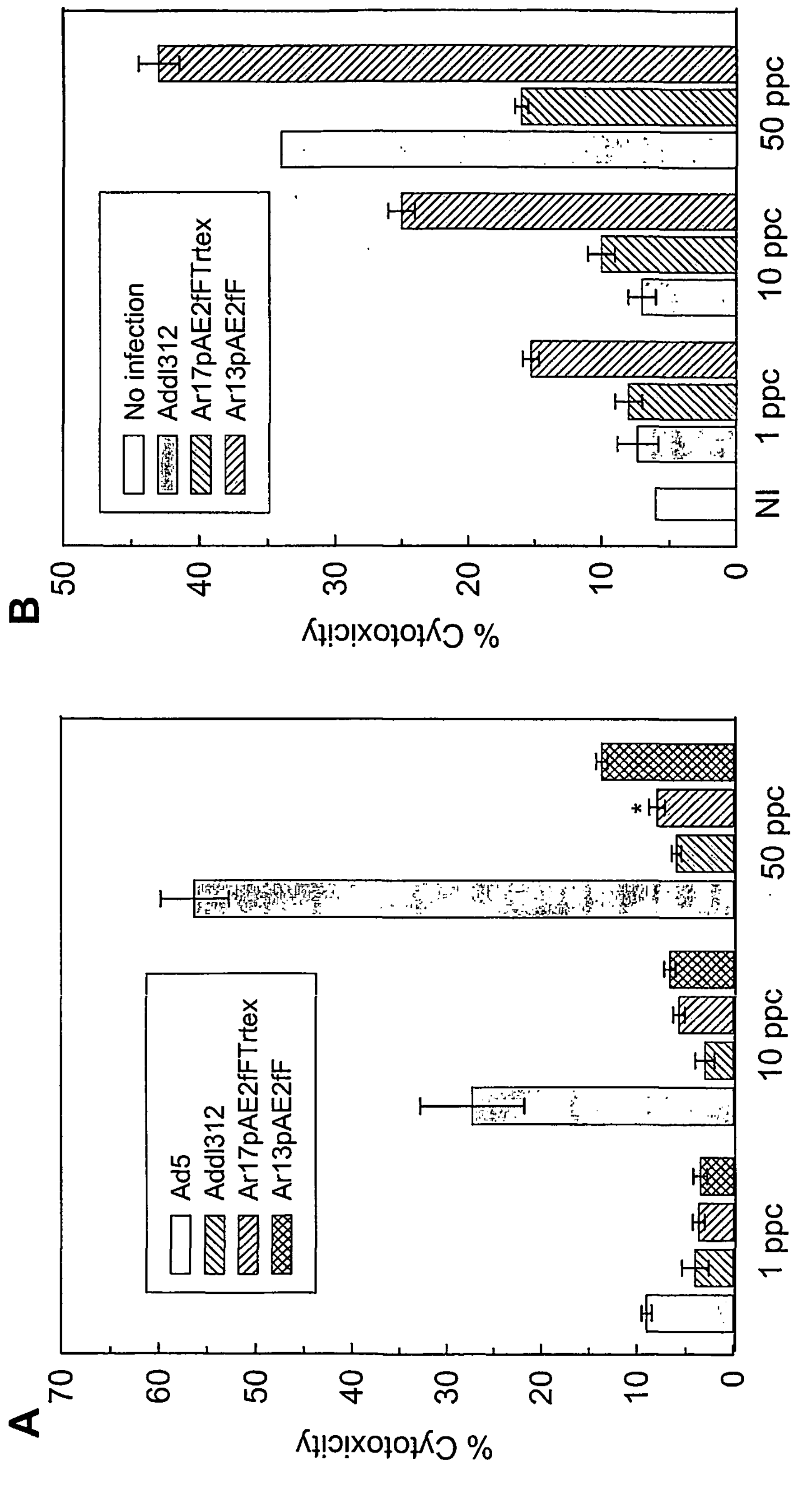

Figure 69

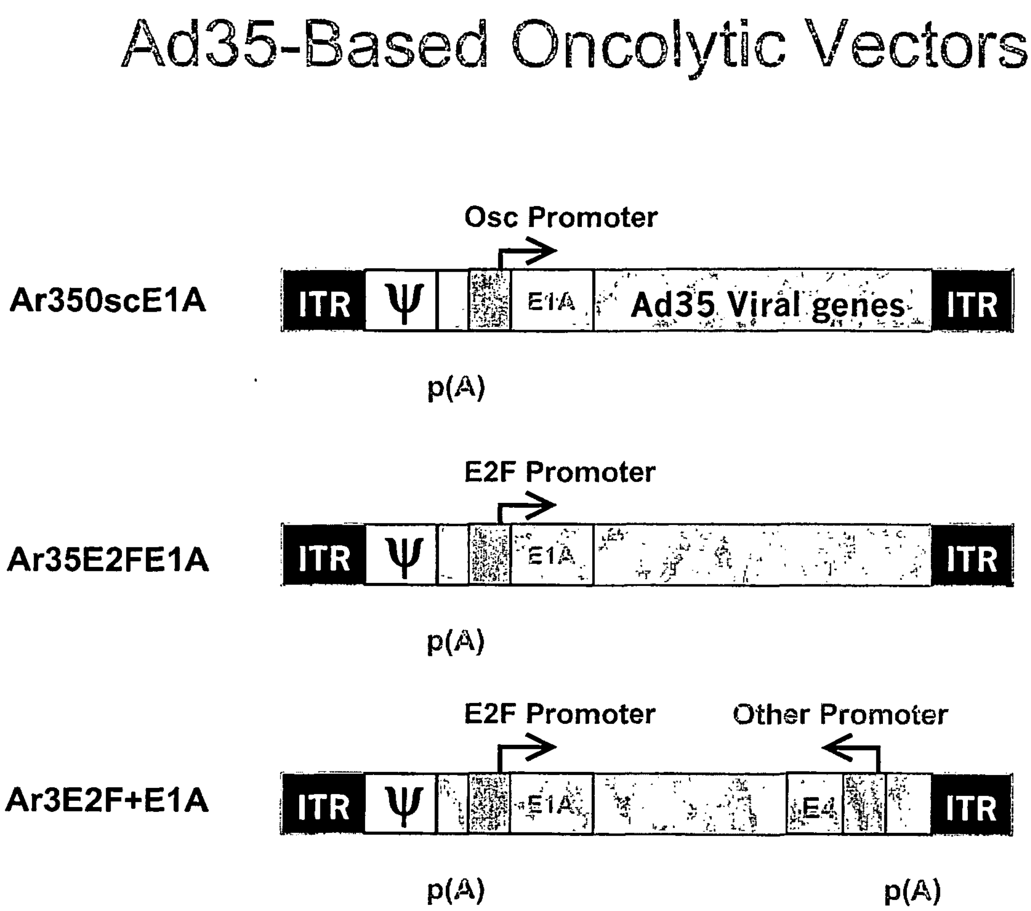
Ad35-Based Oncolytic Vectors
Osc Promoter
Ar350scE1A
ITR
Ψ
E1A
Ad35 Viral genes
ITR
p(A)
E2F Promoter
Ar35E2FE1A
ITR
Ψ
E1A
ITR
p(A)
E2F Promoter
Other Promoter
Ar3E2F+E1A
ITR
Ψ
E1A
E4
ITR
p(A)
p(A)

Figure 70

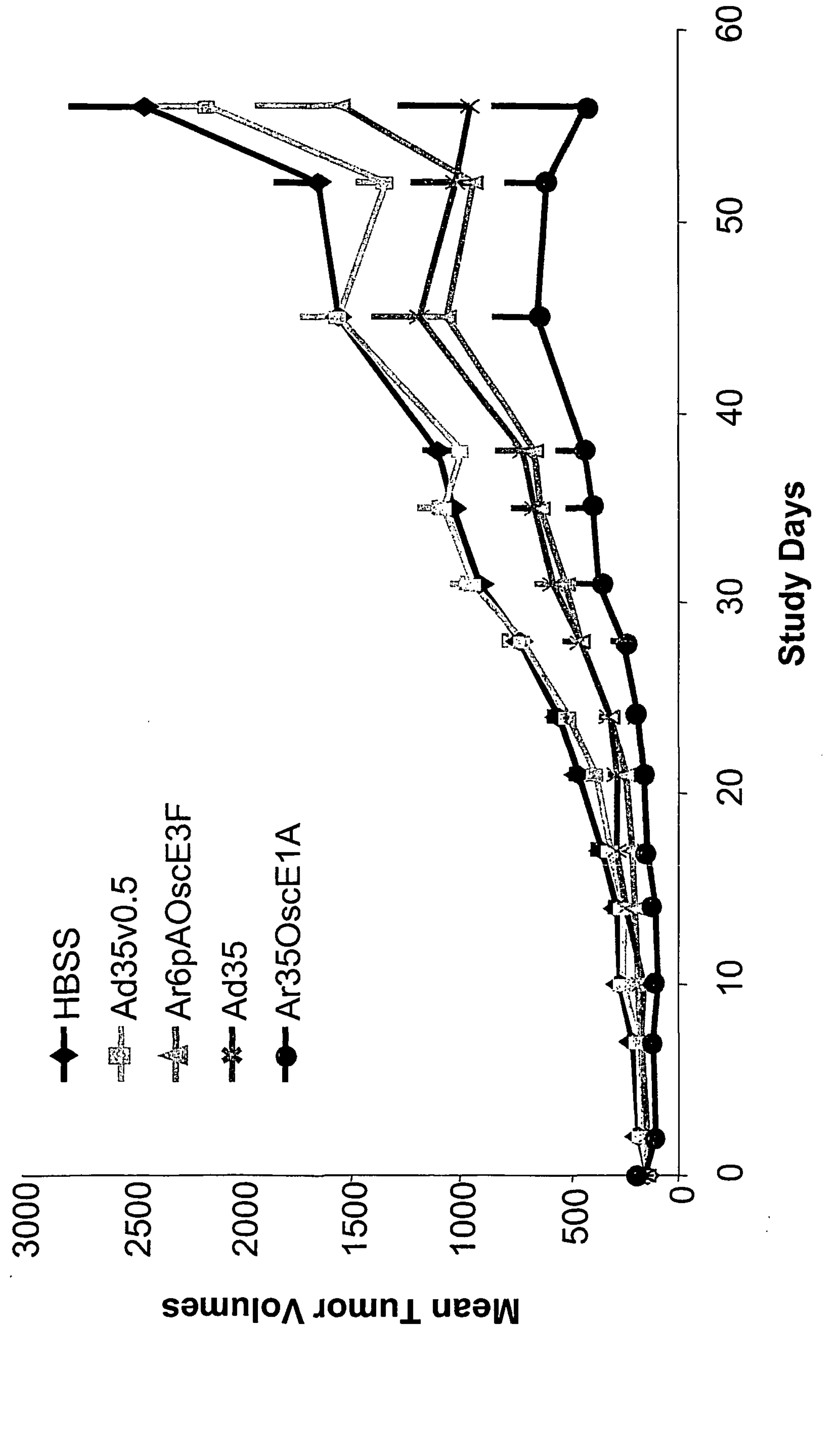
HBSS
Ad35v0.5
Ar6pAOscE3F
Ad35
Ar35OscE1A
Mean Tumor Volumes
3000
2500
2000
1500
1000
500
0
0
10
20
30
40
50
60
Study Days

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 60270922 B
- US 60295037 B
- US 60348670 B
- US 5998205 A, Hallenbeck
- US 1999 A
- WO 9813508 A
- US 5994128 A, Fallaux
- US 6033908 A, Bout
- WO 9633746 A
- US 5672510 A
- WO 9943320 A


### Non-patent literature cited in the description


- **HORIKAWA I ; CABLE PL ; AFSHARI C ; BARRETT JC.** Cloning and characterization of the promoter region of human telomerase reverse transcriptase gene. *Cancer Res.,* 15 February 1999, vol. 59 (4), 826-30
- **TAKAKURA M ; KYO S ; KANAYA T ; HIRANO H ; TAKEDA J ; YUTSUDO M ; INOUE M.** Cloning of human telomerase catalytic subunit (hTERT) gene promoter and identification of proximal core promoter sequences essential for transcriptional activation in immortalized and cancer cells. *Cancer Res.,* 01 February 1999, vol. 59 (3), 551-7
- **ZWICKER J ; MULLER R.** Cell cycle-regulated transcription in mammalian cells. *Prog. Cell Cycle Res,* 1995, vol. 1, 91-99
- **DYSON, N.** The regulation of E2F by pRB-family proteins. *Genes and Development,* 1998, vol. 12, 2245-2262
- **STRAUSS M. ; LUKASS J ; BARTEK J.** Unrestricted cell cycling and cancer. *Nat Med,* 1995, vol. 12, 1245-1246
- **WEINBERG, RA.** The retinoblastoma protein and cell cycle control. *Cell,* 1995, vol. 81, 323-330
- **PARR MJ et al.** Tumor-selective transgene expression in vivo mediated by an E2F-responsive adenoviral vector. *Nature Med,* October 1997, vol. 3 (10), 1145-1149
- **CHAO et al.** *Molecular and Cellular Biology,* August 1999, 5588-5600
- **KILIAN A et al.** *Hum Mol Genet.,* November 1997, vol. 6 (12), 2011-9
- **KIM NW et al.** *Science,* 23 December 1994, vol. 266 (5193), 2011-5
- **SHAY JW et al.** *European Journal of Cancer,* 1997, vol. 5, 787-791
- **STEWART SA et al.** *Semin Cancer Biol.,* December 2000, vol. 10 (6), 399-406
- **HIYAMA K et al.** *J Natl Cancer Inst.,* 21 June 1995, vol. 87 (12), 895-902
- **POOLE JC et al.** *Gene,* 16 May 2001, vol. 269 (1-2), 1-12
- **SHAY JW et al.** *Hum Mol Genet.,* April 2001, vol. 10 (7), 677-85
- **KIYONO T et al.** *Nature,* 1998, vol. 396, 84
- **BRYAN TM et al.** *Nat Med.,* November 1997, vol. 3 (11), 1271-4
- **ARMITAGE JO et al.** *Blood,* 15 December 1998, vol. 92 (12), 4491-508
- **GASSON et al.** *Blood,* 15 March 1991, vol. 77 (6), 1131-45
- **MACH et al.** *Cancer Res.,* 15 June 2000, vol. 60 (12), 3239-46
- **MACH ; DRANOFF.** *Curr Opin Immunol.,* October 2000, vol. 12 (5), 571-5
- **BOON ; OLD.** *Curr Opin Immunol.,* 01 October 1997, vol. 9 (5), 681-3
- **DRANOFF et al.** *Proc National Acad Sciences,* 1993, vol. 90, 3539-3543
- **JAFFEE et al.** *J Clin Oncol,* 2001, vol. 19, 145-156
- **PARDOLL.** *Annu Rev Immunol,* 1995, vol. 13, 399-415
- **SIMONS JW et al.** *Cancer Res.,* 1999, vol. 59, 5160-5168
- **SIMONS JW et al.** *Cancer Res,* 1997, vol. 57, 1537-1546
- **SOLFFER R et al.** *Proc. Natl. Acad. Sci USA,* 1998, vol. 95, 13141-13146
- **; JAFFEE et al.** . *J Clin Oncol,* 2001, vol. 19, 145-156
- **DONG Z et al.** *Cell,* 21 March 1997, vol. 88 (6), 801-10
- **DONA Z et al.** *J Exp Med,* 1998, vol. 188, 755-763
- **STRAUSS M ; LUKASS J ; BARTEK J.** Unrestricted cell cycling and cancer. *Nat Med,* 1995, vol. 12, 1245-1246

- **WEINBERG, RA.** The retinoblastoma protein and cell cycle control. *Cell,* 1995, vol. 81, 323-330
- **TAKAHASHI Y ; RAYMAN JB ; DYNLACHT BD.** Analysis of promoter binding by the E2F and pRB families in vivo: distinct E2F proteins mediate activation and repression. *Genes Dev.,* 01 April 2000, vol. 14 (7), 804-16
- **HE et al.** A simplified system for generating recombinant adenoviruses. *PNAS,* 1998, vol. 95, 2509-2514
- **GORZIGLIA et al.** Elimination of both E1 and E2a from adenovirus vectors further improves prospects for in vivo human gene therapy. *J. Virol.,* 1996, vol. 6, 4173-4178
- **JAKUBCZAK et al.** Adenovirus type 5 viral particles pseudotyped with mutagenized fiber proteins show diminished infectivity of coxsackie B-Adenovirus receptor-bearing cells. *J. Virol.,* 2001, vol. 75, 2972-2981
- **MITTEREDER et al.** Evaluation of the concentration and bioactivity of adenovirus vectors for gene therapy. *J Virol.,* 1996, vol. 70, 7498-7509
- **FALLAUX et al.** New helper cells and matched early region 1-deleted adenovirus vectors prevent generation of replication-competent adenoviruses. *Human. Gene Ther,* 1998, vol. 9, 1909-1917
- Biology of the cultured cell in Culture of Animal Cells. Wiley-Liss, 1994, 9-19
- **PARR MJ et al.** *Nat Med,* October 1997, vol. 3 (10), 1145-9
- **FARSHID et al.** *J Viral Hepat,* 1994, vol. 1 (1), 45-53
- **KAINO M.** *J Gastroenterol,* February 1997, vol. 32 (1), 40-6
- **KATAOKA M et al.** *Oncogene,* 16 March 2000, vol. 19 (12), 1589-95
- **SPILLARE EA et al.** *Mol Carcinog,* May 1996, vol. 16 (1), 53-60
- Adenoviridae: The viruses and their replication. **SHENK T.** Fields Virology. Lippincott-Raven, 1996, 2111-2148
- **RUSSELL WC.** Update on adenovirus and its vectors. *J. General Virol,* 2000, vol. 81, 2573-2604
- Adenoviridae: The viruses and their replication. **SHENK T.** Fields Virology. Lippincott-Raven, 1996, 2111-2148
- **KASAMATSU H ; NAKANISHI A.** *Annu Rev Microbiol,* 1998, vol. 52, 627-86
- **HEISE CC et al.** *Cancer Res,* 01 June 1999, vol. 59 (11), 2623-8
- **GANLY I et al.** *Clinical Cancer Res,* March 2000, vol. 6, 798-806
- **TOLLEFSON AE et al.** *J Virol,* April 1996, vol. 70 (4), 2296-306
- The genetic system. **YOUNG CSH et al.** The adenoviruses comprehensive virology. Plenum Press, 1984, vol. 4, 125-172
- **MITTEREDER N ; MARCK KL ; TRAPNELL BC.** *J. Virology,* 1996, vol. 70, 7498-7509
- **GORZIGLIA et al.** Elimination of both E1 and E2a from adenovirus vectors further improves prospects for in vivo human gene therapy. *J. Virol,* 1996, vol. 6, 4173-4178
- **O'REILLY DR et al.** Appendix 6 In: Baculovirus Expression Vectors: A Laboratory Manual. Oxford: Oxford University press, 1994, 132-134
- **GORZIGLIA et al.** Generation of an adenovirus vector lacking E1, E2a, E3, and all of E4 except open reading frame 3. *J Virol,* 1999, vol. 73 (7), 6048-6055
- **SAMBROOK et al.** Molecular cloning: A laboratory manual. 1989, 9.31-9.52
- **MITTEREDER et al.** Evaluation of the Concentration and Bioactivity of Adenovirus Vectors for Gene Therapy. *J. Virol.,* 1996, vol. 70, 7498-7509
- The Molecular Repertoire of Adenoviruses I. **WOLD et al.** E3 transcription unit of adenovirus. Springer-Verlag, 1995, 237-274
- **KATAOKA et al.** Downregulation of bcl-2 is associated with p16INK4 mediated apoptosis in non-small cell lung cancer cells. *Oncogene,* 2000, vol. 19 (12), 1589-1595
- **SPILLARE et al.** Suppression of growth in vitro and tumorigenesis in vivo of human carcinoma cell lines by transfected p16INK4. *Mol Carcinogenesis,* 1996, vol. 16 (1), 53-60
- **FARSHID et al.** Alterations of the RB tumor suppressor gene in hepatocellular carcinoma and hepatoblastoma cell lines in association with abnormal p53 expression. *J Viral Hepat,* 2000, vol. 1 (1), 45-53
- **GHATTAS et al.** The encephalomyocarditis virus ribosomal entry site allows efficient coexpression of two genes from a recombinant provirus in cultured cells and embryos. *Mol Cell Biol,* 1991, vol. 11, 5848-5859
- **MITTEREDER et al.** Evaluation of the concentration and bioactivity of adenovirus vectors for gene therapy. *J Virol,* 1996, vol. 70, 7498-7509
- **ALBERT ML et al.** Dendritic Cells Acquire Antigen from Apoptotic Cells and Induce Class I-Restricted CTLs. *Nature,* 1998, vol. 392, 86-89
- **WOLD et al.** E3 transcription unit of adenovirus. *Curr Top Microbiol Immunol,* 1995, vol. 199, 237-274
- **SAMBROOK et al.** Molecular cloning: A laboratory manual. 1989, 9.31-9.52
- **SLANSKY et al.** A protein synthesis-dependent increase in E2F1 mRNA correlates with growth regulation of the dihydrofolate reductase promoter. *Mol Cell Biol.,* March 1993, vol. 13, 1610-8
- **NEUMAN et al.** Structure and partial genomic sequence of the human E2F1 gene. *Gene,* 16 September 1996, vol. 173 (2), 163-9

- Adenoviridae: The viruses and their replication. **SHENK T.** Fields Virology. 1996, 2111-2148
- **RUSSELL et al.** Update on adenovirus and its vectors. *Gen.Virol.,* 2000, vol. 81, 2573-2604
- **EMENENS et al.** Chemotherapy: friend or foe to cancer vaccines?. *Curr Opin Mol Ther,* 2001, vol. 3 (1), 77-84
- **HORWITZ, MS.** Adenovirus immunoregulatory genes and their cellular targets. *Virology,* 2001, vol. 279, 1-8
- **FANG B ; KOCH P ; ROTH JA.** Diminishing adenovirus gene expression and viral replication by promoter replacement. *J. Virol,* 1997, vol. 71, 4798-4803
- **TAKAKURA M ; KYO S ; KANAYA T ; HIRANO H ; TAKEDA J ; YUTSUDO M ; INOUE M.** *Cancer Research,* 1999, vol. 59, 551-557
- **RUSSELL WC.** Update on adenovirus and its vectors. *J. of Gen. Virol.,* 2000, vol. 81, 2573-2604
- **OH S ; SONG YH ; KIM UJ ; YIM J ; KIM TK.** In vivo and in vitro analyses of Myc for differential promoter activities of the human telomerase (hTERT) gene in normal and tumor cells. *Biochem Biophys Res Commun.,* 24 September 1999, vol. 263 (2), 361-5
- **GOPE R ; GOPE ML.** Abundance and state of phosphorylation of the retinoblastoma susceptibility gene product in human colon cancer. *Mol Cell Biochem.,* 25 March 1992, vol. 110 (2), 123-33
- **O'REILLY DR ; MULLER LK ; LUCKOW VA.** Appendix 6. In: Baculovirus Expression Vectors: A Laboratory Manual. Oxford University Press, 1994, 132-134
- Primer Extension Assay in Current Protocols in Molecular Biology. John Wiley & Sons, Inc, 1995, 4.8.1-4.9.1
- The genetic system. **YOUNG CSH ; SHENK T ; GINSBERG HS.** The adenoviruses comprehensive virology. Plenum Press, 1984, vol. 4, 125-172
- **MITTEREDER et al.** *J. Virology,* 1996, vol. 70, 7498-7509
- **GORZIGLIA MI ; LAPCEVICH C ; ROY S, ; KANG Q ; KADAN M ; WU V ; PECHAN P ; KALEKO M.** Generation of an adenovirus vector lacking E1, e2a, E3, and all of E4 except open reading frame 3. *J Virol.,* July 1999, vol. 73 (7), 6048-55
- **GU J ; KAGAWA S ; TAKAKURA M ; KYO S ; INOUE M ; ROTH JA ; FANG B.** Tumor-specific Transgene Expression from the Human Telomerase Reverse Transcriptase Promoter Enables Targeting of the Therapeutic Effects of the Bax Gene to Cancers. *Cancer Res.,* 2000, vol. 60, 5359-5364
- **ALTMANN K ; WARTMANN M ; O'REILLY T.** *Biochimica et Biophysica Acta,* 1998, 1470
- **TALLARIDA RJ.** *Pain,* 1992, vol. 49, 93-97
- **STEEL GG ; PECKHAM MJ.** *Int. J. Radiation Oncology Biol. Phys.,* 1979, vol. 5, 85-91
- **HOLDEN, STALDER H. et al.** *J. Clin. Microbiol.,* 1977, vol. 6, 257-265